# EUROPEAN PATENT APPLICATION

(11) **EP 4 148 051 A1**
(43) Date of publication of application: **15.03.2023**
(21) Application number: 21196175.0
(22) Date of filing: 10.09.2021
(51) Int. Cl.: C07D 401/14, A61K 49/08, A61K 49/12, C07D 471/22, C07F 9/38, C07K 7/06

(54) **COMPOUNDS FOR COMPLEXATION OF RARE EARTH ELEMENTS AND/OR S-, P-, D- BLOCK METALS, THEIR COORDINATION COMPOUNDS, PEPTIDE CONJUGATES, METHOD OF THEIR PREPARATION AND USE THEREOF**

(71) Applicant: Ustav organicke chemie a biochemie AV CR, v.v.i., 166 10 Praha 6 (CZ)
(72) Inventor: Polasek, Miloslav, 16000 Praha 6 (CZ); David, Tomas, 25064 Hovorcovice (CZ); Straka, Michal, 26601 Beroun (CZ); Jaros, Adam, 14000 Praha 4 (CZ)
(74) Representative: Hartvichova, Katerina

(57) **Abstract**

The present invention relates to compounds of general formula (I) .

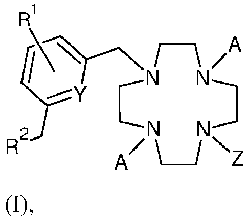

The invention further relates to the coordination compounds thereof, peptide conjugates, method of their preparation and use thereof.

## Description

### State of Art

The present invention relates to new macrocyclic compounds suitable as cross-bridged chelators for complexation of rare earth elements and/or s-, p-, d- block metals, forming extremely stable coordination compounds. These coordination compounds are suitable for use as labels for quantitative detection of peptide conjugates, and/or as contrast agents for magnetic resonance imaging. The invention further relates to a method of preparation of the chelators and to a method of tracing peptide/protein containing medicaments.

### Background Art

Metal elements find biomedical applications such as imaging contrast agents, radiotherapeutic agents or bioanalytical labels. For most of these applications, it is necessary to bind the metal in a stable chelate that can be covalently linked to other molecules, such as targeting vectors based on peptides or antibodies. The most universal example of a chelator applicable to majority of metal elements is DOTA (1,4,7,10-tetraazacyclododecane-1,4,7,10-tetraacetic acid) and its derivatives. A broad range of other chelators have been developed specifically for particular metal elements. [Price E. W., Orvig C. (2014), Chem. Soc. Rev. 43(1), 260 - 290].

Rare earth elements (scandium - Sc, yttrium - Y, lanthanum - La, cerium - Ce, praseodymium - Pr, neodymium - Nd, promethium - Pm, samarium - Sm, europium - Eu, gadolinium - Gd, terbium - Tb, dysprosium - Dy, holmium - Ho, erbium - Er, thulium - Tm, ytterbium - Yb and lutetium - Lu) are a group of metals that offer a broad range of medical applications. Radiopharmaceuticals based on ⁹⁰Y, ¹⁵³Sm and ¹⁷⁷Lu are approved by FDA, clinical trials are ongoing with ¹⁶⁶Ho, and others show advantageous properties for Positron Emission Tomography (PET), Single-Photon Emission Computed Tomography (SPECT) or therapy (⁴⁴Sc, ⁴⁷Sc, ⁸⁶Y, ¹⁴⁹Pm, ¹⁵⁹Gd, ¹⁴⁹Tb, ¹⁶¹Tb, ¹⁶⁵Dy, ¹⁶¹Ho, ¹⁶⁹Er and ¹⁷⁵Yb). Stable, non-radioactive Gd chelates are in clinical use as contrast agents for Magnetic Resonance Imaging (MRI). Stable isotopes of rare earth elements also serve as labels for analytical purposes. Compounds of interest labeled in this way can be visualized, traced and quantified based on unique luminescence or isotope mass of the elements that have zero background in biological systems. The unique isotope mass is especially useful, as it allows many compounds to be quantified simultaneously in a single analysis (multiplexing), typically with the use of inductively-coupled plasma mass spectrometry (ICP-MS) [Bodenmiller B. et al. (2012), Nat. Biotechnol. 30(9), 858-867]. Other metal elements from s-, p- and d-block of the periodic system, such as ⁸⁹Sr, ²²³Ra, ^{117m}Sn, ²¹²Pb, ²¹³Bi, ⁶⁴Cu, ²²⁵Ac, find use in radiopharmaceutical compounds for imaging or therapy.

For practical use, metal chelates must be very stable, so that the metal ion cannot easily escape from the chelator and detach from the carrier molecule. In this respect, thermodynamic stability constant provides insufficient information, because most applications proceed under conditions far from thermodynamic equilibrium (e.g. *in-vivo*). Instead, kinetic inertness that characterizes the rate at which the metal ion escapes from the chelate under given conditions must be considered. Kinetic inertness strongly correlates with rigidity of the chelator. Acyclic chelators of the type DTPA (diethylenetriaminepentaacetic acid) have more flexible structure and provide lower kinetic inertness than macrocyclic chelators of the type DOTA, which are more rigid. Chelates with high kinetic inertness are desirable particularly for *in-vivo* applications, where the metal chelate is challenged with excess of competing biogenic chelators and metal ions. For this reason, the rigid and kinetically inert macrocyclic chelators are preferred to the flexible acyclic ones. For example, practically all radiopharmaceuticals based on the radionuclide ¹⁷⁷Lu (half-life 6.7 days) that are approved or in development make use of the macrocyclic chelator DOTA, as the metal must remain bound to the targeting molecule *in-vivo* for weeks in order to have the desired curative effect. Similarly, in MRI contrast agents, macrocyclic chelates of gadolinium(III) are preferred to the acyclic DTPA type. It has been recently found that significant amount of free gadolinium is released *in-vivo* from the acyclic agents and deposited in human brain for long time. [Fur M. L., Caravan P. (2019), Metallomics 11(2), 240-254] In response to this finding and to prevent potential harm to patients, the use of the acyclic MRI contrast agents has been severely restricted around the globe by drug-regulating agencies. It is therefore likely that the importance of kinetic inertness of metal chelates will grow in medical and other applications, and chelators providing higher inertness will be needed.

Methods to further increase kinetic inertness rely on reinforcement and rigidification of the macrocyclic chelators with additional rings. For example, a cross-bridged macrocyclic chelator cb-TE2A (4,11-bis(carboxymethyl)-1,4,8,11-tetraazabicyclo[6.6.2]hexadecane) provides much more inert chelates with copper(II) ion than the non-cross-bridged analogues [Boswell C. A. et al. (2004), J. Med. Chem. 47(6), 1465-1474]. Another similar chelator cb-TEDPA (6,6'-((1,4,8,11-Tetraazabicyclo[6.6.2]hexadecane-4,11-diyl)bis(methylene))dipicolinic acid) was shown to provide exceptionally inert lanthanide(III) chelates [Rodriguez-Rodriguez A. et al. (2016), Inorg. Chem. 55(5), 2227-2239]. Another cyclen-based cross-bridged chelator provided extremely inert copper(II) chelates [Esteves, C. V. et al. (2013), Inorg. Chem. 52(9), 5138-5153]. A common feature of these chelators is that the bridge is independent from the coordinating pendant arms and is oriented on the opposite side from the pendant arms in the chelate. Chelators where the bridge connected the pendant arms themselves have also been made, but the effect on stability of the chelates was strongly negative [Vipond, J. et al. (2007), Inorg. Chem. 46(7), 2584-2595].

There are also practical limitations when too high kinetic inertness becomes counterproductive. This is because the energy barrier that the metal ion must overcome on the way out of the chelate is similar to the barrier that it has to overcome on the way in during chelate formation. Generally, the more inert a chelate is, the more difficult it is to make. The formation kinetics can be accelerated with high temperature and long reaction times. However, such reaction conditions are often incompatible with sensitive targeting vectors (e.g. antibodies) and/or half-life of metal radionuclides in preparation of radiopharmaceuticals.

There remains a strong need for new types of chelators, which would form highly kinetically inert metal complexes quickly and under mild synthetic conditions.

### Disclosure of Invention

We have circumvented problems of the background art with a new type of chelators, which form a bridge after complexation of the metal ion that rigidifies the structure and increases kinetic inertness. The bridge is formed between two coordinating pendant arms based on cycloaddition reaction between alkyne and azide substituents on the opposite pendant arms. This reaction does not require catalyzation by Cu(I) ions as is usually the case, and occurs spontaneously after formation of a non-bridged chelate. The chelator therefore acts as a one-way trap for the metal ion. Initial formation of the non-bridged chelate is relatively fast. Then, a bridge is formed that traps the metal inside the chelator. For rare earth elements, the resulting bridged chelates show extremely high kinetic inertness that is up to 6 orders of magnitude higher than for the analogous chelates with DOTA. Such unusually high inertness allows new uses of the chelates as analytical labels that can withstand harsh hydrolytic conditions in concentrated acids and can be quantified in the lysate as intact chelates with liquid chromatography - mass spectrometry (LC-MS). The use of LC-MS is advantageous, as it is much more common instrument than ICP-MS. The extremely high inertness is also advantageous for potential *in-vivo* use of the chelates, such as MRI contrast agents or radiopharmaceuticals, as no free metal is released *in-vivo* from the coordination compounds, therefore no metal deposit occurs in human or animal body.

The compounds according to the present invention are capable of acting as extremely efficient molecular traps, which can coordinate metal ions in an extremely stable, rigid and well defined manner. The ligands can be prepared in relatively a few number of steps of organic synthesis. After coordination of the metal ion, the click reaction is performed between the azide group and a triple bond present in opposite pendant arms of the macrocycle, forming a triazole bridge, and enclosing the metal ion within the cage (forming the so-called click-zipped complex). The formation of triazole is irreversible and is depicted in Scheme 1 below.

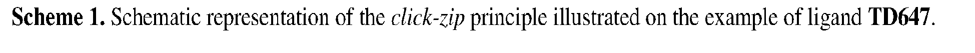

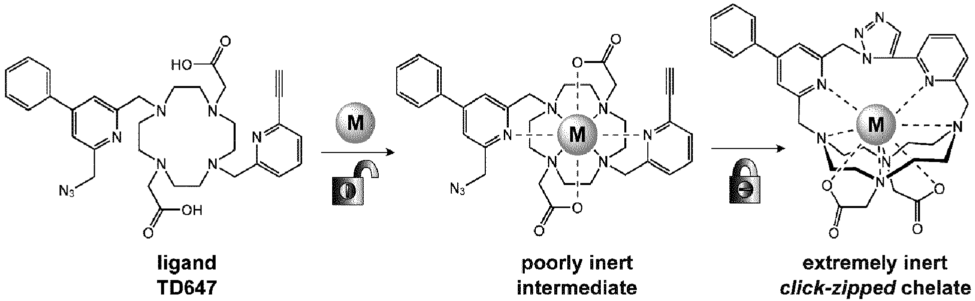

In first aspect, the subject of the present invention relates to compounds of general formula (I) 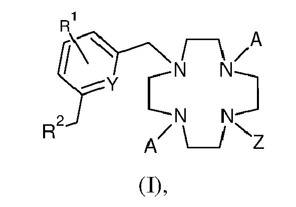 wherein
Y is selected from a group consisting of nitrogen (N); N-oxide (N⁺-O⁻);
R¹ is selected from the group consisting of H; halogen; -OH; -N₃; -CH₂N₃; -NR₂, wherein R is independently selected from H or C₁ to C₆ alkyl, which may be branched or linear; C₆ to C₁₀ aryl, which can optionally be substituted with -NH₂, -NO₂, -COOH and/or -CH₂COOH; C₇ to C₁₀ arylalkyl, which can optionally be substituted with -NH₂, -NO₂, -COOH, -CH₂Cl and/or -CH₂COOH; -CF₃; -COOR, wherein R is as defined above; 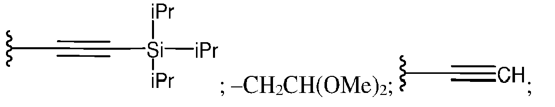
R² is 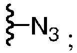
A are independently selected from the group consisting of H; -(CH₂)ₙCOOH, wherein n is an integer from 1 to 3; -CH(CH₃)COOH; -CH((CH₂)ₙCH₃)COOH, wherein n is as defined above; -CH₂P(=O)(OR)₂, wherein R is as defined above;
-CH₂C(=O)(NH₂); -CH₂C(=O)(NH)-CH₂COOH; -CH₂P(=O)(OH)(Ar), wherein Ar is phenyl, which can optionally be substituted with C₁ to C₆ alkyl;
Z is selected from a group consisting of 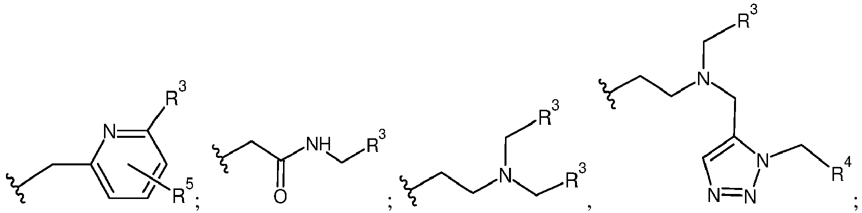 wherein
   R³ is 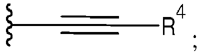
   R⁴ is selected from the group consisting of H; halogen; piperidinyl; trimethylsilyl; triisopropylsilyl; phenyl; C₁ to C₆ alkyl, which may be branched or linear; C₃ to C₆ cycloalkyl; -CF₃; -CH₂NHR⁶, wherein R⁶ is selected from H, fluorenylmethyloxykarbonyl and benzyloxycarbonyl; -C(CH₃)₂(NHR⁶), wherein R⁶ is as defined above; adamantyl; 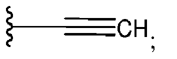
   R⁵ is H; -CF₃; halogen; -OH; C₇ to C₁₀ arylalkyl, which can optionally be substituted with -NH₂, -NO₂, -COOH, -CH₂Cl and/or -CH₂COOH; or 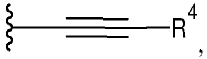 wherein R⁴ is as defined above;
   and/or R² and R³ together form a 1,2,3-triazole group of formula 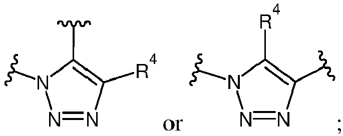 wherein
      R⁴ is defined above;
      with the proviso that at most one A is H.

When the compounds of this invention contain a chiral centre, then all enantiomers, mixtures of enantiomers and racemates fall within the framework of the present invention. The present invention further includes the compounds of general formula (I) in the form of salts with alkali metals, ammonium or amines, as well as in the form of addition salts with acids.

In one preferred embodiment, Y is nitrogen, thus forming a pendant arm comprising a pyridyl moiety, substituted with R¹ and R².

In one embodiment, R¹ is in meta-position from Y, and para-position from R²-CH₂-.

In one embodiment, R¹ is in meta-position from Y, and orto-position from R²⁻CH₂-.

In another embodiment, R¹ is in para-position from Y.

In one preferred embodiment, R² is

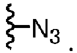

Preferably, R² is 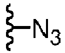 and R¹ is selected from the group comprising H, phenyl, carboxyphenyl, halogen (preferably Cl), trifluoromethyl, carboxyl, carboxylic ester or amine, more preferably R¹ is H or carboxyphenyl.

In one embodiment, Z is

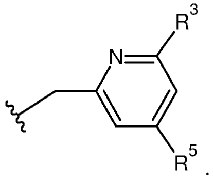

In one preferred embodiment, Z is 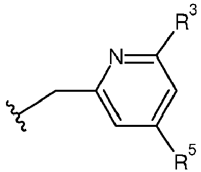 and R³ and R⁵ are as defined above. Preferably, Z is 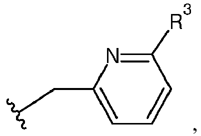 even more preferably, Z is

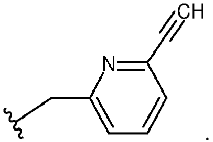

In the compound of general formula (I), A can be the same or different. Preferably, A are the same.

In one embodiment, Z is

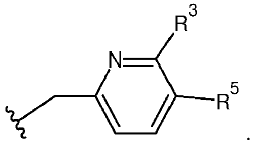

In one embodiment, Z is

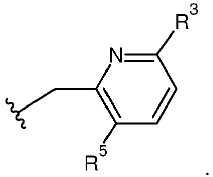

In one embodiment, A is selected from the group comprising -(CH₂)COOH; -(CH₂)₂COOH; -CH₂P(=O)(OEt)₂; -CH₂P(=O)(OH)₂; -CH₂P(=O)(OH)(OEt); -CH₂P(=O)(Ph)(OH); -(CH₂)COONH₂; -(CH₂)C(=O)NH-CH₂COOH; -(CH₂)COO^{t}Bu.

Preferably, A is selected from the group comprising -(CH₂)COOH; -CH₂P(=O)(OEt)₂; -CH₂P(=O)(OH)₂; -CH₂P(=O)(OH)(OEt); and -CH₂P(=O)(Ph)(OH). Most preferably, A is -(CH₂)COOH.

In one preferred embodiment, R⁴ is selected from the group comprising H; trifluoromethyl; 4-piperidinyl; -CH₂-NH₂; phenyl; cyclopropyl; adamantyl; terc-butyl; trimethylsilyl (TMS); triisopropylsilyl (TIPS); halogen (F, Cl, Br, I); -C(CH₃)₂NH₂; -C(CH₃)₂NHBoc. More preferably, R⁴ is hydrogen.

In one embodiment, Y is nitrogen; R¹ is selected from the group consisting of H; Cl; -N(CH₃)₂; phenyl, which can optionally be substituted with -COOH or -CH₂COOH; benzyl, which can optionally be substituted with -COOH or -CH₂COOH; -CF₃; -COOCH₃; -COOCH(CH₃)₂;
-COOtBu;

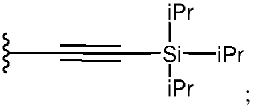

A are independently selected from the group consisting of H; -(CH₂)ₙCOOH, wherein n is 1 or 2; -CH₂P(=O)(OH)₂; -CH₂P(=O)(OH)(OEt); -CH₂P(=O)(OEt)₂; -CH₂C(=O)(NH₂); -CH₂C(=O)(NH)-CH₂COOH; -CH₂P(=O)(OH)(Ph);

Z is selected from a group consisting of 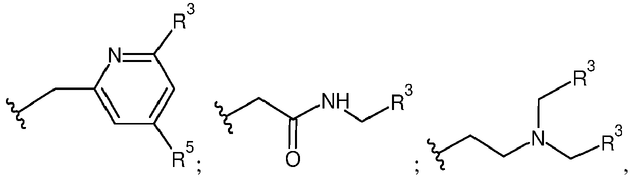 wherein
R³ is 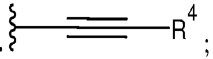
R⁴ is selected from the group consisting of H; halogen; piperidinyl; trimethylsilyl; triisopropylsilyl; phenyl; cyclopropyl, terc-butyl; -CF₃; -CH₂NH₂; -CH₂N(H)(Fmoc); -C(CH₃)₂(NH₂); -C(CH₃)₂(NHBoc); adamantyl;
R⁵ is H or 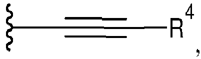 wherein R⁴ is as defined above;
with the proviso that at most one A is H.

In one preferred embodiment, Z is 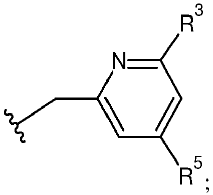 R³ is 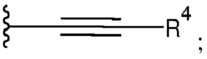 R⁴ and R⁵ are as defined above, preferably R⁴ and R⁵ are H.

In another embodiment, R² and R³ together form a 1,2,3-triazole group of formula 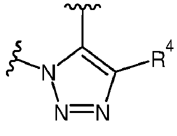 or 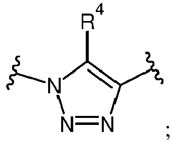 wherein R⁴ is as defined above, preferably R⁴ is hydrogen. In such embodiment, a bridge is formed between two opposite nitrogen atoms of the cyclen moiety, and the compound of general formula (I) thus forms a cage-like structure. The bridge is an entity of general formula (IIa) or (IIb) 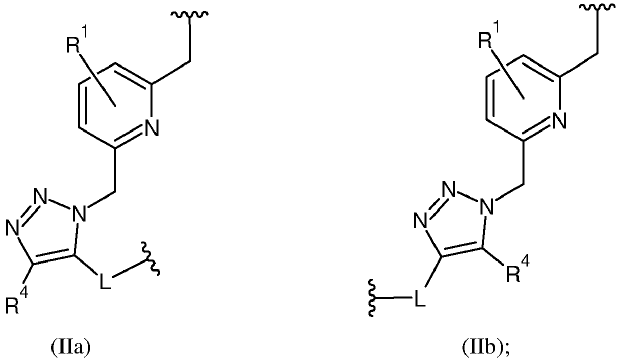 wherein
L is a linker selected from the group consisting of 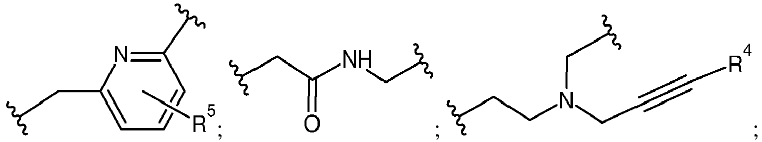 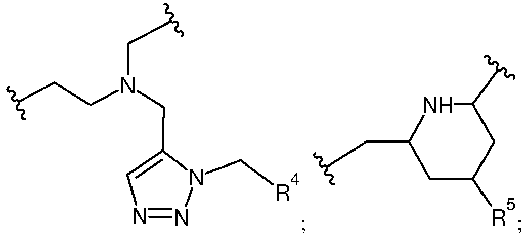
and R¹, R⁴ and R⁵ are as defined above. The linker L is derived from the group Z defined above.

Preferably, L is 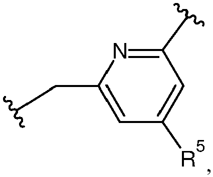 more preferably L is

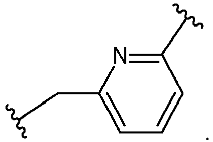

In one preferred embodiment, the compound of general formula (I) is selected from the group comprising compounds with the following combinations of substituents:
Y is N⁺-O⁻; R¹, R⁴ and R⁵ is H:

| Compound | A | Z | R² | R³ |
|---|---|---|---|---|
| **TD604** | -(CH₂)COOH | 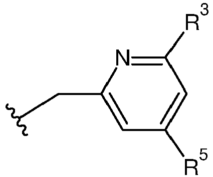 | 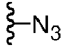 | 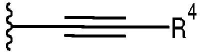 |

Y is N; R¹, R⁴ and R⁵ is H:

| Compound | A | Z | R² | R³ |
|---|---|---|---|---|
| **TD556** | -(CH₂)COOH | 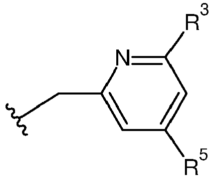 | 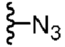 | 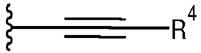 |
| **TD1204** | -(CH₂)COOH | 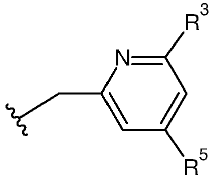 | 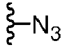 | 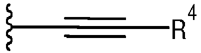 |
| **TD703** | -(CH₂)COOH and H | 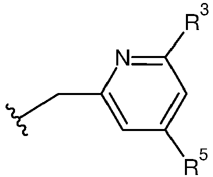 | 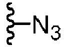 | 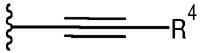 |
| **TD705** | -CH₂P(=O)(OEt)₂ and -(CH₂)COO^{t}Bu | 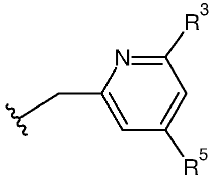 | 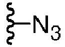 | 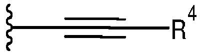 |
| **TD714** | -CH₂P(=O)(OH)₂ and -(CH₂)COOH | 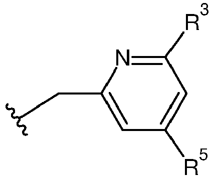 | 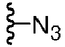 | 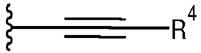 |
| **TD921** | -CH₂P(=O)(Ph)(OH) | 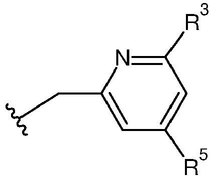 | 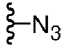 | 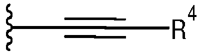 |
| **TD580** | -CH₂P(=O)(OEt)₂ | 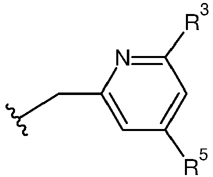 | 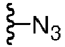 | 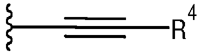 |
| **TD582** | -CH₂P(=O)(OH)(OEt) | 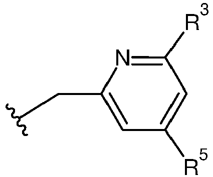 | 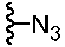 | 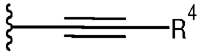 |
| **TD581** | -CH₂P(=O)(OH)₂ | 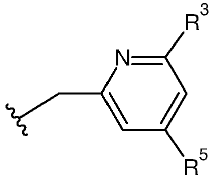 | 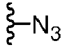 | 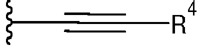 |
| **TD801** | -(CH₂)₂COOH | 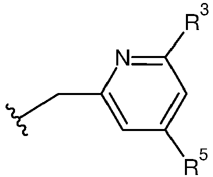 | 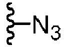 | 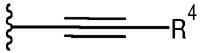 |
| **cz-TD556** | -(CH₂)COOH | 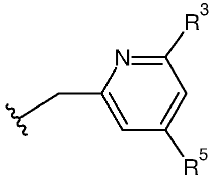 | 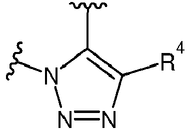 | |

Y is N; Z is 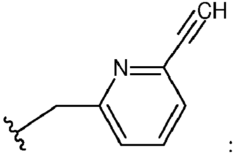 and R² is

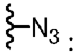

| Compound | R¹ | A |
|---|---|---|
| **TD827** | 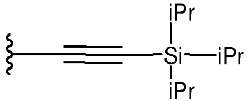 | -(CH₂)COOH |
| **TD575** | Ph | -CH₂P(=O)(OEt)₂ |
| **TD576** | Ph | -CH₂P(=O)(OH)₂ |
| **TD764** | Cl | -(CH₂)COOH |
| **TD1063** | -CF₃ | -(CH₂)COOH |
| **TD1092** | -COOCH₃ | -(CH₂)COOH |
| **TD1148** | -COOⁱPr | -(CH₂)COOH |
| **TD1160** | -COO^{t}Bu | -(CH₂)COOH |
| **TD1176** | -N(CH₃)₂ | -(CH₂)COOH |
| **TD647** | Ph | -(CH₂)COOH |
| **TD742** | 4-carboxyphenyl | -(CH₂)COOH |
| **TD779** | Ph | -(CH₂)COONH₂ |
| **TD778** | Ph | -(CH₂)CONH-CH₂COOH |

Y is N; and R² and R³ together form a 1,2,3-triazole group of formula

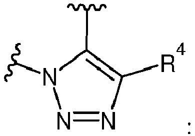

| Compound | R¹ | A | Z | R⁴ |
|---|---|---|---|---|
| **cz-TD425** | Ph | -(CH₂)COOH | 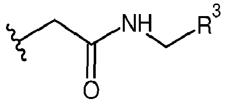 | H |
| **cz-TD764** | Cl | -(CH₂)COOH | 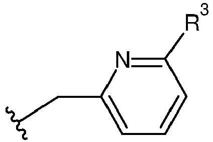 | H |
| **cz-TD1063** | -CF₃ | -(CH₂)COOH | 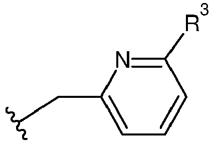 | H |
| **TD1188** | H | -(CH₂)COOH | 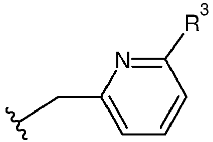 | -CF₃ |
| **TD650** | Ph | -(CH₂)COOH | 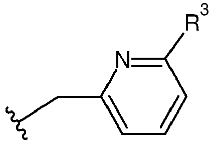 | H |

Y is N; and R² and R³ together form a 1,2,3-triazole group of formula

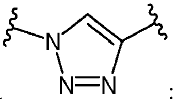

| Compound | R¹ | A | Z |
|---|---|---|---|
| **TD871** | Ph | -(CH₂)COOH | 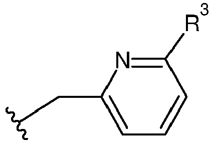 |

Y is N; R¹ is phenyl; and R² is 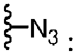 and R³ is

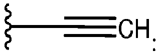

| Compound | A | Z |
|---|---|---|
| **TD425** | -(CH₂)COOH | 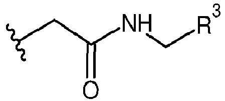 |
| **TD669** | -(CH₂)COOH | 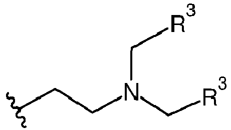 |

Y is N; A is -(CH₂)COOH; R¹ is H; and R² is

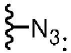

| Compound | Z | R³ | R⁴ | R⁵ |
|---|---|---|---|---|
| **TD810** | 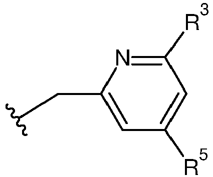 | 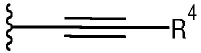 | H | 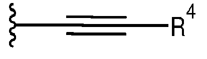 |
| **TD718** | 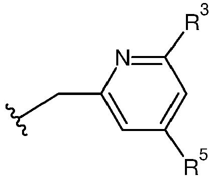 | 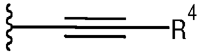 | TMS | H |
| **TD728** | 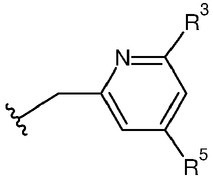 | 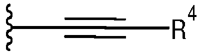 | TIPS | H |
| **TD1204** | 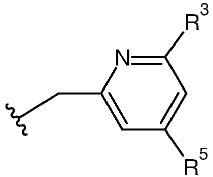 | 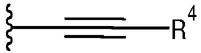 | I | H |
| **TD944** | 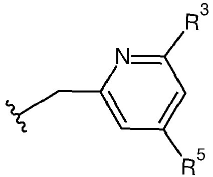 | 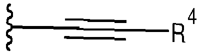 | Ph | H |
| **TD943** | 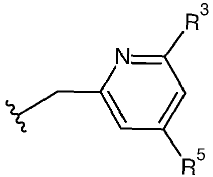 | 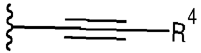 | cyclopropyl | H |
| **TD959** | 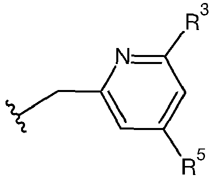 | 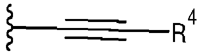 | tBu | H |
| **TD992** | 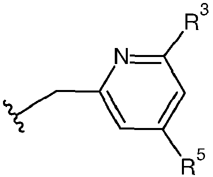 | 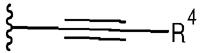 | adamantyl | H |
| **TD1105** | 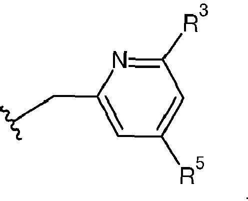 | 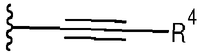 | - C(CH₃)₂N HBoc | H |
| **TD1127** | 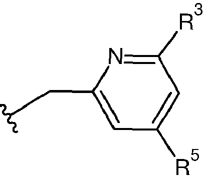 | 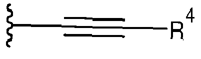 | 4-piperidinyl | H |

Y is N; A is -(CH₂)COOH; R² is 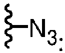 and Z is

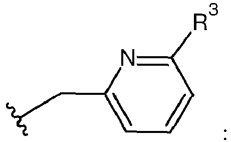

| Compound | R¹ | R³ | R⁴ |
|---|---|---|---|
| **TD722** | Ph | 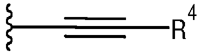 | -CH₂NH₂ |
| **TD1054** | Ph | 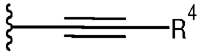 | -C(CH₃)₂NH₂ |
| **TD744** | 4-carboxyphenyl | 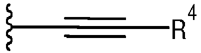 | -CH₂-NH₂ |

In the most preferred embodiment, the compound of general formula (I) is selected from the group comprising compounds, wherein Y is nitrogen, Z is 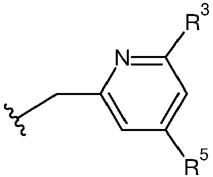 and the remaining substituents are present in the following combinations:

| Compou nd | R¹ | A | R² | R³ | R⁴ | R⁵ |
|---|---|---|---|---|---|---|
| TD556 | H | -(CH₂)COOH | 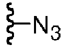 | 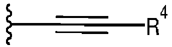 | H | H |
| TD810 | H | -(CH₂)COOH | 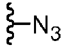 | 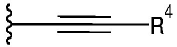 | H | 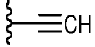 |
| TD827 | 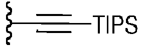 | -(CH₂)COOH | 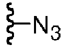 | 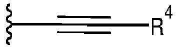 | H | H |
| TD718 | H | -(CH₂)COOH | 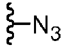 | 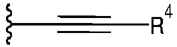 | TMS | H |
| TD728 | H | -(CH₂)COOH | 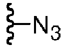 | 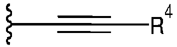 | TIPS | H |
| TD1204 | H | -(CH₂)COOH | 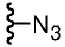 | 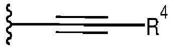 | I | H |
| TD944 | H | -(CH₂)COOH | 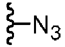 | 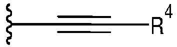 | Ph | H |
| TD943 | H | -(CH₂)COOH | 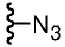 | 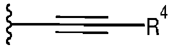 | cyclopropyl | H |
| TD959 | H | -(CH₂)COOH | 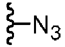 | 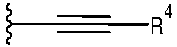 | tBu | H |
| TD992 | H | -(CH₂)COOH | 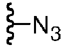 | 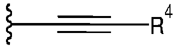 | adamantyl | H |
| TD705 | H | -CH₂P(=O)(OEt)₂ and -(CH₂)COO^{t}Bu | 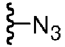 | 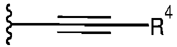 | H | H |
| TD714 | H | -CH₂P(=O)(OH)₂ and -(CH₂)COOH | 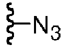 | 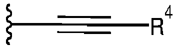 | H | H |
| TD921 | H | -CH₂P(=O)(Ph)(OH) | 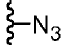 | 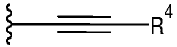 | H | H |
| TD580 | H | -CH₂P(=O)(OEt)₂ | 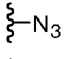 | 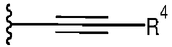 | H | H |
| TD582 | H | -CH₂P(=O)(OH)(OEt) | 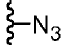 | 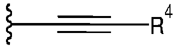 | H | H |
| TD581 | H | -CH₂P(=O)(OH)₂ | 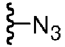 | 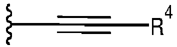 | H | H |
| TD575 | Ph | -CH₂P(=O)(OEt)₂ | 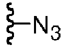 | 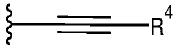 | H | H |
| TD576 | Ph | -CH₂P(=O)(OH)₂ | 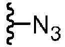 | 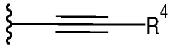 | H | H |
| TD764 | Cl | -(CH₂)COOH | 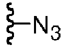 | 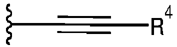 | H | H |
| TD1063 | -CF₃ | -(CH₂)COOH | 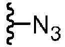 | 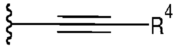 | H | H |
| TD1092 | -COOCH₃ | -(CH₂)COOH | 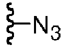 | 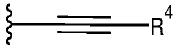 | H | H |
| TD1148 | -COOⁱPr | -(CH₂)COOH | 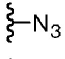 | 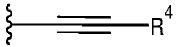 | H | H |
| TD1160 | -COO^{t}Bu | -(CH₂)COOH | 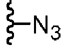 | 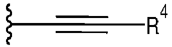 | H | H |
| TD1176 | -N(CH₃)₂ | -(CH₂)COOH | 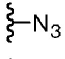 | 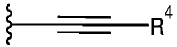 | H | H |
| TD647 | Ph | -(CH₂)COOH | 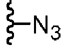 | 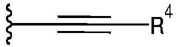 | H | H |
| TD722 | Ph | -(CH₂)COOH | 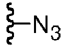 | 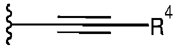 | -CH₂NH₂ | H |
| TD1054 | Ph | -(CH₂)COOH | 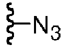 | 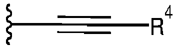 | -C(CH₃)₂NH₂ | H |
| TD1105 | H | -(CH₂)COOH | 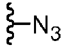 | 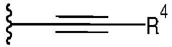 | -C(CH₃)₂NHBoc | H |
| TD1127 | H | -(CH₂)COOH | 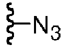 | 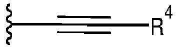 | 4-piperidinyl | H |
| TD742 | 4-carboxyphen yl | -(CH₂)COOH | 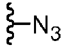 | 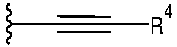 | H | H |
| TD744 | 4-carboxyphen yl | -(CH₂)COOH | 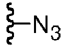 | 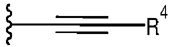 | -CH₂-NH₂ | H |
| TD779 | Ph | -(CH₂)COONH₂ | 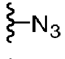 | 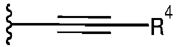 | H | H |
| TD778 | Ph | -(CH₂)CONH-CH₂COOH | 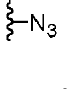 | 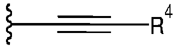 | H | H |
| cz-TD556 | H | -(CH₂)COOH | 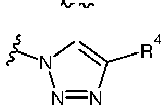 | | H | H |
| cz-TD764 | Cl | -(CH₂)COOH | 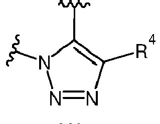 | H | H | |
| cz-TD1063 | -CF₃ | -(CH₂)COOH | 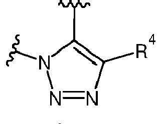 | H | H | |
| TD1188 | H | -(CH₂)COOH | 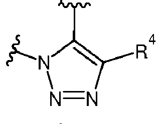 | -CF₃ | H | |
| TD650 | Ph | -(CH₂)COOH | 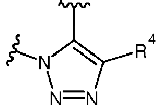 | H | H | |
| TD871 | Ph | -(CH₂)COOH | 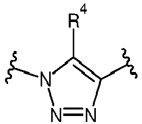 | H | H | |

In another aspect, the subject of the present invention is a method of synthesis of the compounds of general formula (I), comprising the following steps:
i) providing an alkyne intermediate of general formula Z-Cl, wherein Z is as defined above;
ii) providing an azide intermediate of general formula (III) 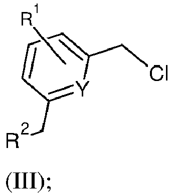 wherein Y, R¹ and R² are as defined above;
iii) providing a cyclen derivative of general formula (IV) 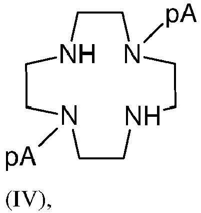 wherein pA is selected from the group comprising -(CH₂)ₙCOO^{t}Bu, wherein n is an integer from 1 to 3; benzyloxycarbonyl; -CH(CH₃)COO^{t}Bu; -CH₂P(=O)(OR)₂, wherein R is C₁ to C₆ alkyl, which may be branched or linear; -CH₂C(=O)(NH₂); -CH₂C(=O)(NH)-CH₂COOH; -CH₂P(=O)(OH)(Ar), wherein Ar is phenyl, which can optionally be substituted with C₁ to C₆ alkyl; preferably pA is selected from the group comprising -CH₂COOtBu; -CH₂P(O)(OEt)₂; -(CH₂)₂COOtBu;
iv-A) reacting the cyclen derivative of general formula (IV) with alkyne intermediate Z-Cl to obtain an intermediate of general formula (V) 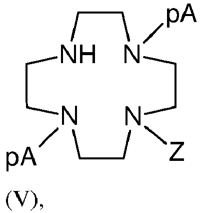 wherein Z and pA are as defined above;
   or
iv-B) reacting the cyclen derivative of general formula (IV) with the azide intermediate of general formula (III) to obtain an intermediate of general formula (VI) 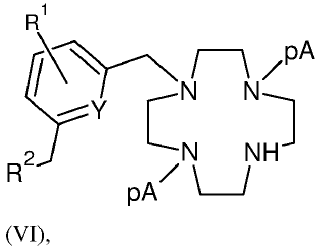 wherein Y, 3R¹, R² and pA are as defined above;
v-A) reacting the intermediate of general formula (V) with the azide intermediate of general formula (III) to obtain an intermediate of general formula (VII) 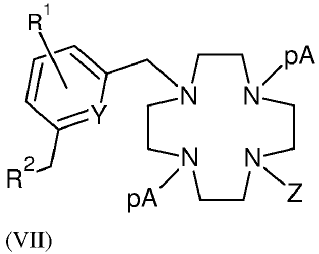 wherein Y, R¹, R², pA and Z are as defined above;
   or
v-B) reacting the intermediate of general formula (VI) with the alkyne intermediate Z-Cl to obtain the intermediate of general formula (VII);
vi) optionally, hydrolysis of protecting groups, resulting in the compound of general formula (I).

In general, compounds of general formula Z-Cl can be obtained commercially or can be synthesised using Pd(0) catalyzed Sonogashira cross-coupling reaction from corresponding 2-halopyridines (preferably Br, I) and terminal alkynes or silyl-protected acetylenes.

Compounds of general formula (III) can be obtained from 2,6-bis(halomethyl)pyridines (preferably Cl) by desymetrization using sodium azide as a source of azide moiety.

Cyclene derivatives of general formula (IV) are commercially available or may be prepared by reaction of cyclen or *trans*-*N*-bis-protected cyclen (preferably carbamate protected) with alkyl haloacetates (alkylation), alkyl acrylates (Michael addition) or trialkylphosphites in the presence of (para)formaldehyde (Kabachnik-Fields reaction).

Steps iv-A), iv-B), v-A) and v-B) take place in a solvent selected from the group comprising MeCN (preferably), DMSO or DMF. The reaction takes place at room temperature for usually several hours up to several days.

Step vi) of deprotecting pA groups is optional, for example if pA is -CH₂P(O)(OEt)₂, then if no deprotection takes place, the resulting A in general formula (I) equals pA. Specific conditions for deprotection of the protecting group depend on the chemical nature of the protecting group, and are known to the person skilled in the art. Typically, terc-butyl ester protecting groups and Boc protecting group are hydrolyzed under acidic conditions (TFA) or thermally, methyl or isopropyl protecting groups are hydrolyzed under alkaline conditions, ethyl phosphonate ester are converted to corresponding phosphonic acids by transesterification using trimethylsilylbromide in presence of pyridine base.

Another subject of the present invention is a coordination compound of the compound of the general formula (I) according to any one of the claims 1 to 5 with a metal cation, selected from the group consisting of lanthanide(III) cations, Na⁺, Ba²⁺, Pb²⁺, Sr²⁺, Ca²⁺, Cd²⁺, Zn²⁺, Mn²⁺, Pt²⁺, Cu²⁺, Ni²⁺, Sc³⁺, Y³⁺, Bi³⁺, In³⁺, Ru³⁺, Ir³⁺, Ga³⁺, Tl³⁺, Pd²⁺; preferably the metal cation is selected from the group consisting of La³⁺, Ce³⁺, Pr³⁺, Nd³⁺, Sm³⁺, Eu³⁺, Gd³⁺, Tb³⁺, Dy³⁺, Ho³⁺, Er³⁺, Tm³⁺, Yb³⁺, Lu³⁺, Y³⁺, Sc³⁺, Bi³⁺, In³⁺, Tl³⁺, Pb²⁺, Ca²⁺, Cd²⁺, Zn²⁺, Cu²⁺, Ni²⁺, Mn²⁺, Pd²⁺, Na⁺.

The metal ions shall be undestood as comprising any izotope of the particular metal, including stable isotopes such as ⁴⁰Ca, ⁴²Ca, ⁴³Ca, ⁴⁴Ca, ⁴⁶Ca, ⁴⁸Ca, ⁴⁵Sc, ⁸⁹Y, ¹³⁸La, ¹³⁹La, ¹³⁶Ce, ¹³⁸Ce, ¹⁴⁰Ce, ¹⁴²Ce, ¹⁴¹Pr, ¹⁴²Nd, ¹⁴³Nd, ¹⁴⁴Nd, ¹⁴⁵Nd, ¹⁴⁶Nd, ¹⁴⁸Nd, ¹⁵⁰Nd, ¹⁴⁴Sm, ¹⁴⁷Sm, ¹⁴⁸Sm, ¹⁴⁹Sm, ¹⁵⁰Sm, ¹⁵²Sm, ¹⁵⁴Sm, ¹⁵¹Eu, ¹⁵³Eu, ¹⁵²Gd, ¹⁵⁴Gd, ¹⁵⁵Gd, ¹⁵⁶Gd, ¹⁵⁷Gd, ¹⁵⁸Gd, ¹⁶⁰Gd, ¹⁵⁹Tb, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Ho, ¹⁶²Er, ¹⁶⁴Er, ¹⁶⁶Er, ¹⁶⁷Er, ¹⁶⁸Er, ¹⁷⁰Er, ¹⁶⁹Tm, ¹⁶⁸Yb, ¹⁷⁰Yb, ¹⁷¹Yb, ¹⁷²Yb, ¹⁷³Yb, ¹⁷⁴Yb, ¹⁷⁶Yb, ¹⁷⁵Lu, ¹⁷⁶Lu, and radioisotopes such as ⁴⁴Sc, ⁴⁷Sc, ⁶⁴Cu, ⁶⁷Cu, ⁸⁶Y,⁹⁰Y, ¹⁴⁰Nd, ¹⁴⁹Pm, ¹⁵¹Pm, ¹⁵³Sm, ¹⁵⁹Gd, ¹⁴⁹Tb, ¹⁶¹Tb, ¹⁶⁵Dy, ¹⁶¹Ho, ¹⁶⁶Ho, ¹⁶⁹Er, ¹⁶⁷Tm, ¹⁷⁵Yb, ¹⁷⁷Lu.

Lanthanides (Ln) are defined as chemical elements comprising 15 metallic chemical elements with atomic numbers in the range of from 57 to 71 (from lanthanum through lutetium).

Preferably, the coordination compound has a general formula (VIII) 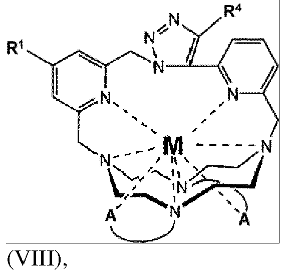 wherein M is the metal cation, and R¹, R⁴ and A are as defined above.

The coordination compounds according to the present invention comprise also solvates and salts of pharmaceutically acceptable acids. The positive charge of the metal cation is counterbalanced by two negative charges arising from A pendant arms (e.g. acetates). Therefore, the overall charge of the coordination compound is -1 (for monovalent metal ions), 0 (for divalent metal ions) or +1 (trivalent metal ions). The counterion, compensating for the overall charge of the coordination compound, is selected from the group, comprising anions derived from the following pharmaceutically acceptable acids:
1-hydroxy-2-naphthoic acid; 2,2-dichloroacetic acid; 2-hydroxyethanesulfonic acid; 2-oxoglutaric acid; 4-acetamidobenzoic acid; 4-aminosalicylic acid; acetic acid; adipic acid; ascorbic acid (L); aspartic acid (L); benzenesulfonic acid; benzoic acid; camphoric acid (+); camphor- 10-sulfonic acid (+); capric acid (decanoic acid); caproic acid (hexanoic acid); caprylic acid (octanoic acid); carbonic acid; cinnamic acid; citric acid; cyclamic acid; dodecylsulfuric acid; ethane-1,2-disulfonic acid; ethanesulfonic acid; formic acid; fumaric acid; galactaric acid; gentisic acid; glucoheptonic acid (D); gluconic acid (D); glucuronic acid (D); glutamic acid; glutaric acid; glycerophosphoric acid; glycolic acid; hippuric acid; hydrobromic acid; hydrochloric acid; isobutyric acid; lactic acid (DL); lactobionic acid; lauric acid; maleic acid; malic acid (- L); malonic acid; mandelic acid (DL); methanesulfonic acid; naphthalene-1,5-disulfonic acid; naphthalene-2-sulfonic acid; nicotinic acid; nitric acid; oleic acid; oxalic acid; palmitic acid; pamoic acid; phosphoric acid; proprionic acid; pyroglutamic acid (- L); salicylic acid; sebacic acid; stearic acid; succinic acid; sulfuric acid; tartaric acid (+ L); thiocyanic acid; toluenesulfonic acid (*p*); undecylenic acid; trifluoroacetic acid.

The coordination compounds can be prepared according to Scheme 1 above from the free ligand of general formula (I) (preferably the non-caged, therefore preferably without the trazole bridge) and a metal salt (typically water soluble metal salt, preferably selected from a group comprising chloride, nitrate, acetate, formate, trifluoroacetate, trifluoromethylsulfonate, more preferably chloride or nitrate salt). By stirring of these reactants in an aqueous environment (pH 5-7) at temperature in the range of from 25 °C to 100 °C, the metal cation coordinates into the compound of general formula (I) and the R² and R³ substituents then "click-zip" the metal cation inside the cage by forming the triazole bridge (reaction temperatures and times are strongly dependent on the choice of ligand and metal cation).

However, the suitable ligands are not limited to the non-caged compounds of general formula (I), eventhough they are preferred because wider range of metal cations (such as lanthanides) may form the caged coordination compounds. The bridged (caged) compounds of general formula (I) with 1,5-triazole bridge are capable of forming coordination compounds with metal cations, typically selected from the group comprising Tl³⁺, Pb²⁺, Bi³⁺, In³⁺, Cd²⁺, Ca²⁺, Cu²⁺, Ni²⁺, Mn²⁺, Pd²⁺, Na⁺. The bridged (caged) compounds of general formula (I) with 1,4-triazole bridge are capable of forming coordination compounds with metal cations, typically selected from the group comprising La³⁺, Ce³⁺, Pr³⁺, Nd³⁺, Sm³⁺, Eu³⁺, Gd³⁺, Tb³⁺, Dy³⁺, Ho³⁺, Er³⁺, Tm³⁺, Yb³⁺, Lu³⁺, Y³⁺, Tl³⁺, Pb²⁺, Bi³⁺, In³⁺, Cd²⁺, Ca²⁺, Cu²⁺, Ni²⁺, Mn²⁺, Pd²⁺, Na⁺.

The resulting coordination compounds are very inert and stable, therefore suitable for various applications, such as MRI contract agents, radiodiagnostics or radiotherapy, peptide or protein containing drug tracing.

The coordination of the compound of general formula (I) to a metal cation takes place via the macrocyclic nitrogen atoms of the cyclene moiety, oxygen atoms present in groups A (pendant arms) and Y group (when Y is N-oxide), and via nitrogen atoms of Y group (when Y is nitrogen) and nitrogen atom present in Z group.

In one embodiment, the coordination compounds according to the present invention may undergo a post-clickzip transformation. It means that after the metal cation is coordinated within the cage of the compound of general formula (I) by either following the Scheme 1 or coordinating with the caged compound of general formula (I), some functional groups, if present, may undergo a further reaction. Typically, such functional group is a halogen, preferably Cl, present within substituent R¹ or R⁴ of the coordination compound.

For example, when R¹ is halogen, preferably Cl; or R¹ is C₆ to C₁₀ aryl, substituted with -CH₂Cl, then the coordination compound of general formula (VIII) may undergo post-click transformation using Pd(0) catalyzed Suzuki cross-coupling reaction with phenylboronic acids (3-borono-5-nitrobenzoic acid or 2-(4-(4,4,5,5-tetramethyl-1,3,2-dioxaborolan-2-yl)phenyl)acetic acid or 2-(4-boronophenyl)acetic acid), during which the halogen is replaced, thus the resulting coordination compound of general formula (VIII) would have R¹ = COOH or C₆ to C₁₀ aryl, substituted with -COOH or -CH₂COOH. The resulting carboxylate moiety can be then utilized in amide coupling reaction for synthesis of peptide conjugates. Conjugates with oligoarginines are capable of crossing cell membrane, allowing metal cages to be incorporated to the cells.

Another possible post-click transformation is the replacement of halogen present within R¹ or R⁴ substituent by -N₃ group by reaction with NaN₃. The resulting azide can be used for coupling of the metal cage to another alkyne-bearing moiety using copper-catalysed azide-alkyne cycloaddition (CuAAC) reaction.

Another possible post-click transformation is the hydrolysis of TIPS protecting group present in R¹ or R⁴ substituent and protecting the triple bond (R¹ or R⁴ is 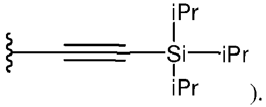 The hydrolysis is performed using aqueous K₂CO₃, and results in R¹ or R⁴ being CuAAC reaction. 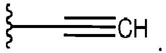 The resulting alkyne can be utilized in

In another embodiment, the post-click transformation may be addition reaction of methanol to R¹, wherein R¹ is 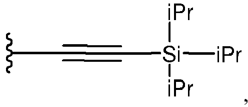 thus transforming the triple bond to dimethyl acetal (protected version of an aldehyde). This demonstrates conversion of one functional group (alkyne) to another (aldehyde). Aldehydes are usable for conjugation reactions based on formation of Schiff bases with amines, hydrazines and acid hydrazides.

In yet another embodiment, the post-click transformation may be - hydrolysis of triisopropylsilyl group from R¹, wherein R¹ is 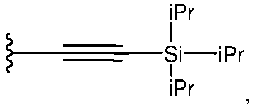 thus transforming R¹ into 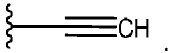 The resulting deprotected triple bond is suitable for further conjugations via reaction with the alkyne, for example click reactions with azides and forming triazole bridges.

In yet another embodiment, the post-click transformation may be deuteration reaction of CH₂ groups of the pendant arms in D₂O in presence of DBU, transforming them into CD₂ groups. This conversion doesn't alter the physico-chemical properties of the molecule (in terms of stability or reactivity), but changes the overall mass of the molecule, to be entirely distinguishable from the parent non-deuterated molecule using mass spectrometry. This means, that the metal ion cage with one particular metal can be used as two different mass tags.

In yet another embodiment, the post-click transformation may be a selective reduction of pyridyl cycle of Z substituent, wherein Z is 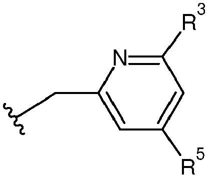 and R³ and R⁵ are as defined above, using NaBH₄ or NaBD₄ as the reducing agent, thus transforming the Z group into 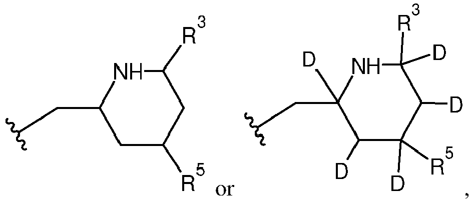 respectively. Similiarly to the deuteration of CH₂ groups, this conversion changes the mass of the molecule for metal tags purposes.

In yet another embodiment, the post-click transformation may be reaction of NH₂ group of R¹ or R⁴, wherein R¹ or R⁴ is -CH₂NH₂, with FmocCl, resulting in transformation of -CH₂NH₂ group into -CH₂NHFmoc group. Fmoc group is the most commonly encountered amine protecting group used in solid-phase peptide synthesis (SPPS). Introducting of Fmoc group therefore facilitates use of the metal cages for SPPS.

In yet another embodiment, the post-click transformation may be reaction of -COOH group present in R¹ or R⁴, with an amino group of an aminoacid or peptide, thus forming a peptide bond for conjugation purposes.

In yet another embodiment, the post-click transformation may be S-alkylation reaction of a halogen, preferably Cl, of R¹ or R⁴ substituent with a thiol, resulting in trasforming the halogen into sulfide. The thiol may be e.g. N-Boc-cysteine or a cysteine-containing peptide and the reaction may take place in presence of DIPEA. Incorporating cysteine (aminoacid) into the structure of the coordination compound according to the present invention opens futher possibilities for amide bonds (e.g. peptide attachment).

In yet another embodiment, the post-click transformation may be reaction of 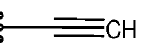 group of R¹ or R³,wherein R¹ or R³ contains 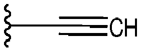 , with an azide, preferably with alkyl- or arylazide, thereby forming a triazole bridge connecting the alkyl- or aryl- to R¹ or R³ group. The arylazide is preferably C6 to C10 arylazide, e.g. benzyl azide, optionally substituted with -COOH or -CH₂COOH. The alkyl azide is preferably C1 to C7 alkyl azide, wherein the alkyl may be linear or branched.

In yet another embodiment, the post-click transformation may be reaction of-N₃ group of R¹, wherein R¹ contains -N₃ group, with a substituent comprising a triple bond, thereby forming a triazole bridge connecting the substituent with the coordination compound. The substituent comprising a triple bond may be for example C7 to C10 arylalkynyl, optionally substituted with -COOH or -CH₂COOH, e.g. phenylacetylene, wherein the phenyl moiety may optionally be substituted with -COOH or -CH₂COOH.

In one embodiment, the post-click transformation may include reaction with another coordination compound of the present invention, thereby forming a chain or a dimer. The coordination compound chain or dimer may contain the same metal cation in both click-zipped cages or it may contain different metal cations in each of the chelates. The advantage of the chains and dimers lies in the possibility to combine properties of the two caged chelates, such as their unique weights to produce mass tags with higher variability of weights.

Typically, the coordination compounds are bound into the chain by forming a triazole bridge between R¹ or R⁵ group of the first coordination compound and R¹ or R⁵ group of the following coordination compound (the use of R⁵ group is only possible for Z being

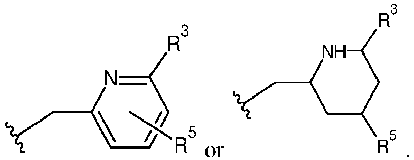

The general basic structure of the coordination compound chain is thus (IXa) or (IXb)

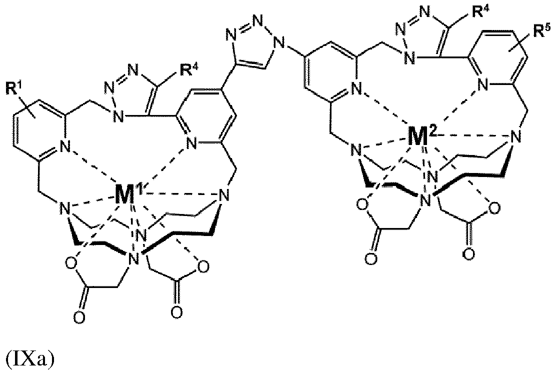

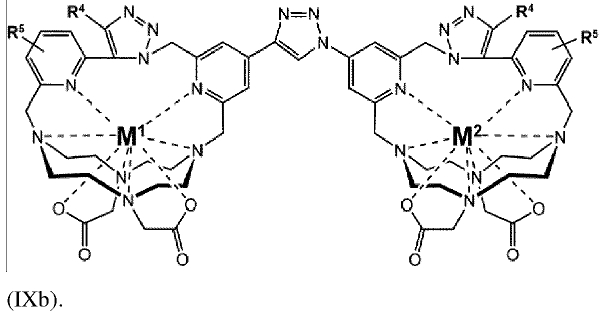

The chain usually contains two coordination compounds according to the present invention, however it may contain three, four or more coordination compounds. Each two neighbouring coordination compounds are linked by one triazole bridge, leaving R⁵ or R¹ substituent available for linking another coordination compound according to the present invention.

The coordination compound dimer contains two coordination compounds according to the present invention, bound together by forming a triazole bridge between R¹ group of the first coordination compound and R⁵ group of the second coordination compound, and, at the same time, by forming a triazole bridge between R⁵ group of the first coordination compound and R¹ group of the second coordination compound.

The general structure of the coordination compound dimer is thus (X)

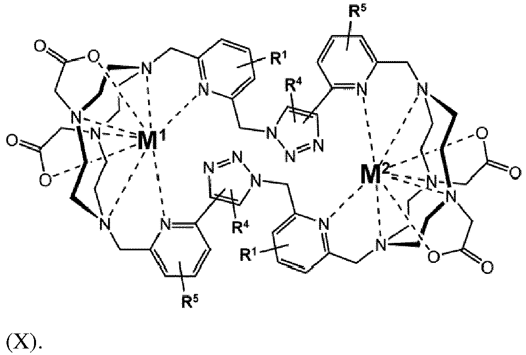

The synthesis of the coordination compound chain or dimer is based on a click reaction between an azido group of the first coordination compound and a triple bond of the second coordination compound, thereby forming a triazole bridge, linking the two coordination compound into a dimer. The triazole bridge is selected from the group comprising:
1,2,3-triazole group of formula 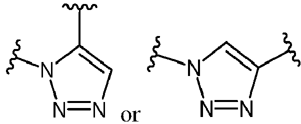 formed between -N₃ group of R¹ or R⁵ of first coordination compound and the triple bond of R¹ group or R⁵ group of substituent Z of the second coordination compound;

Preferably, the metal cations are different, more preferably the metal cations are selected from Tb³⁺ and ¹⁷⁶Yb³⁺.

Yet another object of the present invention is a conjugate suitable for use as markers for drug tracing. Many drugs are based on peptides or proteins, and their biodistribution can be traced by attachment of a fluorescent or radioactive marker to the peptide or protein structure. The coordination compounds and dimers of the present invention are suitable for their attachment to the peptide or protein of a drug to be traced. The isolated cell culture or a tissue can then be analysed for the presence of the metal complex using conventional methods, such as LC-MS, which is commonly used and relatively unexpensive instrument. The cells or tissues can be entirely hydrolyzed in a strong acid without any decomposition of the coordination compound according to the present invention. The coordination compound can then be detected in a hydrolyzed sample. Such method is unexpensive and reliable thanks to the extremely high stability of the coordination compounds. LC-MS method also allows for using a plurality of different coordination compounds with different metal isotops (multiplexing), which may then be all analyzed during one LC-MS analysis.

The conjugate according to the present invention contains the coordination compound according to the present invention as defined above or the coordination compound dimer or chain as defined above, conjugated to a peptide or to a protein. The conjugation takes place via an amide bond, formed between the amino group of the peptide or the protein and a carboxyl group of the coordination compound or of the chain or dimer or vice versa. Thus, the coordination compound or the chain or dimer need to contain either -NH₂ or -COOH group prior to peptide or protein conjugation. Preferably, the -NH₂ or -COOH group is part of the R¹ group, therefore the conjugates are formed from coordination compounds or coordination compound dimers, which have R¹ group selected from the group comprising -NH₂; -COOH; and

C₆ to C₁₀ aryl, substituted with -NH₂, -COOH, or -CH₂COOH.

The conjugation reaction conditions are known to the person skilled in the art, typically the reaction takes place at 25 °C in DMSO, using commonly used peptide coupling agents (preferebaly HATU, PyAOP) in the presence of organic base (triethylamine, ethyldiisopropylamine). Reaction are usually completed within minutes.

The peptide is preferably selected from the group comprising oligopeptides of 3 to 20 aminoacids;

The protein is preferably selected from the group comprising antibodies, preferably monoclonal antibodies.

Another object of the present invention is a method of drug tracing, preferably of tracing of peptide-based or protein-based drugs, which comprises the following steps:
i) providing a cell culture or a tissue to be analyzed, containing at least one conjugate defined above, wherein the peptide or the protein is of the peptide-based or protein-based drug to be traced;
ii) hydrolyzing the cell culture or tissue from step i) using a strong acid, preferably non-oxidizing aq. HCl, obtaining a hydrolyzate;
iii) qualitative and/or quantitative analysis of the hydrolyzate of step ii) for the presence of the coordination compound or of the coordination compound dimer according to the present invention, preferably using LC-MS.

Further object of the present invention is the use of the coordination compound or of the coordination compound dimer or of the conjugate according to the present invention in pharmacy, preferably for development and testing of new drugs, more preferably for drug marking and tracing.

Another object of the present invention is the use of the coordination compounds or of the coordination compound dimers according to the present invention in medical diagnostics, preferably as MRI contrast agents. Preferably, Gd³⁺ coordination compounds or dimers shall be used as MRI contrast agents, as Gd has a very high relaxivity. The extremely high kinetic inertness of the coordination compounds (approximately 100x higher than for GdDOTA, a commonly used MRI contrast agent) allows for using even higher dosages and no free gadolinium, which itself is toxic for humans or animals, is released from the coordination compounds according to the present invention. The claimed coordination compounds are thus safe for use in human or animal beings.

Another object of the present invention is the use of the coordination compound or the coordination compound chain or the coordination compound dimer according to the present invention, wherein M is a radionuclide, preferably selected from the group comprising ⁴⁴Sc, ⁴⁷Sc, ⁶⁴Cu, ⁶⁷Cu, ⁸⁶Y,⁹⁰Y, ¹⁴⁰Nd, ¹⁴⁹Pm, ¹⁵¹Pm, ¹⁵³Sm, ¹⁵⁹Gd, ¹⁴⁹Tb, ¹⁶¹Tb, ¹⁶⁵Dy, ¹⁶¹Ho, ¹⁶⁶Ho, ¹⁶⁹Er, ¹⁶⁷Tm, ¹⁷⁵Yb, ¹⁷⁷Lu, in medicine as radiodiagnostic and/or radiopharmaceutic agents.

The present invention is further demostrated by the following examples.

### Examples

### Example 1: Synthesis of intermediates of alkyne pendant arms

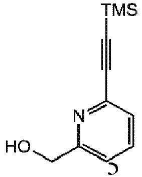

**Synthesis of TD701:** Pear-shape glass flask (50 mL) was charged with **(6-Bromopyridin-2-yl)methanol** (2.47 g; 13.1 mmol; 1.0 equiv.) and magnetic stirrer and three times secured with argon. Solid **CuI** (149 mg; 782 µmol; 6.0 mol%) and **[Pd(PPh₃)₂Cl₂]** (225 mg; 321 µmol; 2.4 mol%) were subsequently added followed by another securing with argon (three times). Under constant flow of argon was then added dry THF (25 mL) through septum followed by addition of **Ethynyltrimethylsilane** (2.0 mL; 14.5 mmol; 1.1 equiv.) and **TEA** (5.5 mL; 39.5 mmol; 3.0 equiv.) after which the mixture changed color to dark brown. The flask was left stirring under septum (but without external argon) for 3 h at RT. The mixture was then transferred to a separatory funnel with EtOAc (150 mL) and H₂O (100 mL). After shaking, the dark colored (and not entirely homogenous) organic layer was separated and the aqueous layer was further extracted with EtOAc (5 × 50 mL). Combined organic layers were filtered through cotton plug and the filtrate was further dried with anhydrous Na₂SO₄, filtered through glass frit (S3) and evaporated to dryness. Resulting dark brown oily residue was purified on flash chromatography (330 g SiO₂, 100% DCM to 10% EtOAc in DCM). Combined fractions with product were evaporated to dryness and further co-evaporated once with DCM. The residue was further dried on high vacuum overnight to give product in the form of free base as dark yellow oil. **Yield:** 2.16 g (80%; 1 step; based on **(6-Bromopyridin-2-yl)methanol). NMR (DMSO-d*₆*): ¹H** δ_{H} 0.24 (C*H*₃, s, 9H); 4.52 (C*H*₂, d, 2H, ³*J*_{HH} = 6); 5.48 (OH, t, 1H, ³*J*_{HH} = 6); 7.39 *(arom.,* dd, 1H, ³*J*_{HH} = 8; ⁴*J*_{HH} = 1); 7.46 *(arom.,* dd, 1H, ³*J*_{HH} = 8; ⁴*J*_{HH} = 1); 7.80 *(arom.,* t, 1H, ³*J*_{HH} = 8). **¹³Ce{¹H}** δ_{C} -0.3 (*CH*₃, s); 63.9 (*CH*₂, s); 93.4 and 104.5 (*C*≡*C*; 2 × s); 120.2 (*arom.,* s); 125.4 (*arom.,* s); 137.2 *(arom.,* s); 140.8 *(arom.,* s); 162.7 *(arom.,* s). **ESI-HRMS:** 206.0995 [M+H]⁺ (theor. [C₁₁H₁₆N₁O₁Si₁]⁺ = 206.0996). 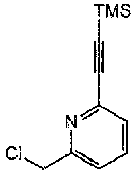 **Synthesis of TD557:** In a round-bottom glass flask (100 mL), **TD701** (2.16 g; 10.5 mmol; 1.0 equiv.) was dissolved in DCM (40 mL). Freshly prepared solution of **SOCl₂** (1.50 mL; 20.7 mmol; 2.0 equiv.) in DCM (10 mL) was then added and the open flask was stirred 1 h at RT. Dil. aq. solution of NaHCO₃ (50 mL) was then added and resulting biphasic mixture was vigorously stirred for additional 1 h at RT, during which bubbles of gas slowly evolved. Mixture was then transferred to a separatory funnel. After shaking, the bottom phase was separated. Aqueous phase was further extracted with DCM (4 × 50 mL). Combined organic layers were dried with anhydrous Na₂SO₄, filtered through glass frit (S3) and evaporated to dryness. The brown residue (already pure according to ¹H NMR) was purified on flash chromatography (330 g SiO₂,60% P.E. in DCM to 20% P.E. in DCM) to remove the intensively colored impurities. Combined fractions with product were evaporated to dryness and further co-evaporated once with DCM. The residual nearly colorless oil was left in a freezer to solidify. Resulting solid was crushed and further dried on high vacuum overnight to give product in the form of free base as fine white powder. **Yield:** 2.14 g (87%; 1 step; based on **TD701). NMR (DMSO-d*₆*): ¹H** δ_{H} 0.25 (C*H*₃, s, 9H); 4.76 (C*H*₂, s, 2H); 7.50 *(arom.,* dd, 1H, ³*J*_{HH} = 8; ⁴*J*_{HH} = 1); 7.55 *(arom.,* dd, 1H, ³*J*_{HH} = 8; ⁴*J*_{HH} = 1); 7.86 (*arom.,* t, 1H, ³*J*_{HH} = 8). **¹³C{¹H}** δ_{C} -0.4 (*C*H₃, s); 46.3 (*C*H₂, s); 94.3 and 103.8 (*C*≡*C;* 2 × s); 123.3 (*arom.,* s); 126.7 (*arom.,* s); 138.1 (*arom.,* s); 141.5 (*arom.,* s); 157.0 (*arom.,* s). **ESI-HRMS:** 224.0657 [M+H]⁺ (theor. [C₁₁H₁₅N₁Cl₂Si₁]⁺ = 224.0657). 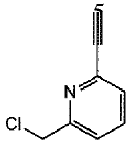 **Synthesis of TD558:** In a pear-shaped glass flask (100 mL), **TD557** (1.05 g; 4.69 mmol; 1.0 equiv.) was dissolved in MeCN (40 mL). Freshly prepared solution of **KF** (406 mg; 7.0 mmol; 1.5 equiv.) in H₂O (7 mL) was then added and the flask was vigorously stirred 2 h at RT. The mixture was concentrated to ~10 mL and then diluted with DCM (50 mL) and dil. aq. NaHCO₃ (50 mL). The mixture was transferred to a separatory funnel. After shaking, the bottom phase was separated. Aqueous phase was further extracted with DCM (4 × 25 mL). Combined organic layers were dried with anhydrous Na₂SO₄, filtered through glass frit (S3) and evaporated to dryness. The residue was further briefly dried on high vacuum to give product in the form of free base as pale yellow oil. **Yield:** 702 mg (99%; 1 step; based on **TD557). NMR (DMSO-d*₆*): ¹H** δ_{H} 4.37 (C≡C*H*, s, 1H); 4.76 (C*H*₂, s, 2H); 7.53 (*arom.,* dd, 1H, ³*J*_{HH} = 8; ⁴*J*_{HH} = 1); 7.57 (*arom.,* dd, 1H, ³*J*_{HH} = 8; ⁴*J*_{HH} = 1); 7.87 (*arom.,* t, 1H, ³*J*_{HH} = 8). **¹³Ce{¹H}** 46.3 (*C*H₂, s); 80.6 and 82.6 (*C*≡*C*H; 2 × s); 123.4 (*arom.,* s); 126.8 (*arom.,* s); 138.1 (*arom.,* s); 141.3 (*arom.,* s); 157.0 (*arom.,* s). **ESI-HRMS:** 152.0262 [M+H]⁺ (theor. [C₈H₇N₁Cl₁]⁺ = 152.0262). 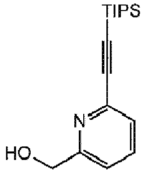 **Synthesis of TD712:** Pear-shape glass flask (10 mL) was charged with **(6-Bromopyridin-2-yl)methanol** (284 mg; 1.51 mmol; 1.0 equiv.) and magnetic stirrer and three times secured with argon. Solid **CuI** (14.5 mg; 76 µmol; 5.0 mol%) and **[Pd(PPh₃)₂Cl₂]** (21 mg; 30 µmol; 2.0 mol%) were subsequently added followed by another securing with argon (three times). Under constant flow of argon was then added dry THF (4 mL) through septum followed by addition of **Ethynyltriisopropylsilane** (370 µL; 1.65 mmol; 1.1 equiv.) and **TEA** (630 µL; 4.52 mmol; 3.0 equiv.) after which the mixture changed color to dark brown. The flask was left stirring under septum (but without external argon) for 16 h at RT. The mixture was then transferred to a separatory funnel with EtOAc (30 mL) and H₂O (30 mL). After shaking, the dark colored (and not entirely homogenous) organic layer was separated and the aqueous layer was further extracted with EtOAc (5 × 25 mL). Combined organic layers were filtered through cotton plug and the filtrate was further dried with anhydrous Na₂SO₄, filtered through glass frit (S3) and evaporated to dryness. Resulting dark brown oily residue was purified on flash chromatography (120 g SiO₂,100% DCM to 10% EtOAc in DCM). Combined fractions with product were evaporated to dryness and further co-evaporated once with DCM. The residue was further dried on high vacuum overnight to give product in the form of free base as yellow oil. **Yield:** 387 mg (89%; 1 step; based on **(6-Bromopyridin-2-yl)methanol). NMR (DMSO-d*₆*): ¹H** δ_{H} 0.92-1.26 (*i-Pr,* m, 21H); 4.53 (C*H*₂, d, 2H, ³*J*_{HH} = 6); 5.48 (O*H*, t, 1H, ³*J*_{HH} = 6); 7.40 (*arom.,* dd, 1H, ³*J*_{HH} = 8; ⁴*J*_{HH} = 1); 7.47 (*arom.,* dd, 1H, ³*J*_{HH} = 8; ⁴*J*_{HH} = 1); 7.80 (*arom.,* t, 1H, ³*J*_{HH} = 8). **¹³Ce{¹H}** δ_{C} 10.7 (*i-Pr,* s); 18.5 (*i-Pr,* s); 64.0 (*C*H₂, s); 89.4 and 106.6 (*C*≡*C*; 2 × s); 120.2 (*arom.,* s); 125.8 (*arom.,* s); 137.2 (*arom.,* s); 140.9 (*arom.,* s); 162.7 (*arom.,* s). **ESI-HRMS:** 290.1932 [M+H]⁺ (theor. [C₁₇H₂₈N₁O₁Si₁]⁺ = 290.1935). 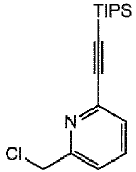 **Synthesis of TD723:** In a round-bottom glass flask (25 mL), **TD712** (384 g; 1.33 mmol; 1.0 equiv.) was dissolved in DCM (5 mL). Freshly prepared solution of **SOCh** (200 µL; 2.75 mmol; 2.1 equiv.) in DCM (1 mL) was then added and the open flask was stirred 1 h at RT. Dil. aq. solution of NaHCO₃ (10 mL) was then added and resulting biphasic mixture was vigorously stirred for additional 1 h at RT, during which bubbles of gas slowly evolved. Mixture was then transferred to a separatory funnel. After shaking, the bottom phase was separated. Aqueous phase was further extracted with DCM (4 × 10 mL). Combined organic layers were dried with anhydrous Na₂SO₄, filtered through glass frit (S3) and evaporated to dryness. The brown residue (already pure according to ¹H NMR) was purified on flash chromatography (120 g SiO₂,20% DCM in P.E. to 30% DCM in P.E.) to remove the intensively colored impurities. Combined fractions with product were evaporated to dryness and further co-evaporated once with DCM. The residual colorless oil was further dried on high vacuum overnight to give product in the form of free base as colourless solid. **Yield:** 356 mg (87%; 1 step; based on **TD712). NMR (DMSO-d*₆*): ¹H** δ_{H} 0.91-1.28 (*i-Pr,* m, 21H); 4.78 (C*H*₂, s); 7.52 (*arom.,* dd, 1H, ³*J*_{HH} = 8; ⁴*J*_{HH} = 1); 7.57 (*arom.,* dd, 1H, ³*J*_{HH} = 8; ⁴*J*_{HH} = 1); 7.86 (*arom.,* t, 1H, ³*J*_{HH} = 8). **ESI-HRMS:** 308.1593 [M+H]⁺ (theor. [C₁₇H₂₇N₁Cl₁Si₁]⁺ = 308.1596). 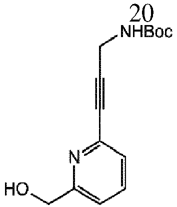 **Synthesis of TD530:** Pear-shape glass flask (50 mL) was charged with **(6-Bromopyridin-2-yl)methanol** (1.00 g; 5.35 mmol; 1.0 equiv.), ***N*-Boc-propargylamine** (1.00 g; 6.44 mmol; 1.2 equiv.) and magnetic stirrer and three times secured with argon. Solid **CuI** (102 mg; 536 µmol; 10.0 mol%) and **[Pd(PPh₃)₂Cl₂]** (187 mg; 266 µmol; 5.0 mol%) were subsequently added followed by another securing with argon (three times). Under constant flow of argon was then added dry THF (27 mL) through septum followed by addition of **TEA** (2.24 mL; 16.1 mmol; 3.0 equiv.) after which the mixture changed color to dark brown. The flask was left stirring under septum (but without external argon) for 18 h at RT. The mixture was then transferred to a separatory funnel with EtOAc (30 mL) and H₂O (50 mL). After shaking, the dark colored (and not entirely homogenous) organic layer was separated and the aqueous layer was further extracted with EtOAc (3 × 30 mL). Combined organic layers were filtered through cotton plug and the filtrate was further dried with anhydrous Na₂SO₄, filtered through glass frit (S3) and evaporated to dryness. Resulting dark brown oily residue was purified on flash chromatography (220 g SiO₂,100% DCM to 100% EtOAc). Combined fractions with product were evaporated to dryness and further co-evaporated once with DCM. The residue was further dried on high vacuum overnight to give product in the form of free base as yellow oil. **Yield:** 610 mg (43%; 1 step; based on **(6-Bromopyridin-2-yl)methanol). NMR (DMSO-d*₆*): ¹H** δ_{H} 1.40 (C*H*₃, s, 9H); 3.99 (C*H*₂, d, 2H, ³*J*_{HH} = 6); 4.51 (C*H*₂, d, 2H, ³*J*_{HH} = 6); 5.47 (O*H,* t, 1H, ³*J*_{HH} = 6); 7.32 (*arom.,* dd, 1H, ³*J*_{HH} = 8; ⁴*J*_{HH} = 1); 7.40 (N*H*, t, 1H, ³*J*_{HH} = 6); 7.44 (*arom.,* dd, 1H, ³*J*_{HH} = 8; ⁴*J*_{HH} = 1); 7.79 (*arom.,* t, 1H, ³*J*_{HH} = 8). **¹³C{¹H}** δ_{C} 28.2 (*C*H₃, s); 29.9 (*C*H₂, s); 64.0 (*C*H₂, s); 78.4 (*C*-CH₃, s); 81.3 and 86.9 (*C*≡*C*; 2 × s); 119.8 (*arom.,* s); 125.0 (*arom.,* s); 137.2 (*arom.,* s); 141.1 (*arom.,* s); 155.3 (CO, s); 162.5 (*arom.,* s). ESI-HRMS: 263.1390 [M+H]⁺ (theor. [C₁₄H₁₉N₂O₃₁]⁺ = 263.1390). 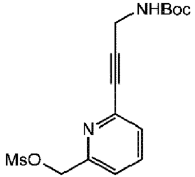 **Synthesis of TD538:** In a round-bottom glass flask (250 mL), **TD530** (568 mg; 2.17 mmol; 1.0 equiv.) was dissolved in DCM (20 mL) followed by addition of **TEA** (900 µL; 6.46 mmol; 3.0 equiv.). Freshly prepared solution of **MsCl** (355 µL; 4.33 mmol; 2.0 equiv.) in DCM (5 mL) was then added. Resulting solution was stirred 20 min at RT. Mixture was then diluted with DCM (50 mL) followed by addition of dil. aq. solution of NaHCO₃ (50 mL). The resulting biphasic mixture was then transferred to a separatory funnel. After shaking, the bottom phase was separated. Aqueous phase was further extracted with DCM (3 × 50 mL). Combined organic layers were dried with anhydrous Na₂SO₄, filtered through glass frit (S3) and evaporated to dryness. **ESI-MS** (LC-MS): 341.1 [M+H]⁺ (theor. [C₁₅H₂₁N₂O₅S₁]⁺ = 341.1). All of **TD538** obtained in this way was directly used for **TD539** without further purification or characterization. 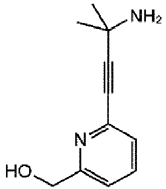 **Synthesis of TD1045:** Pear-shaped glass flask (25 mL) was charged with **(6-Bromopyridin-2-yl)methanol** (500 mg; 2.67 mmol; 1.0 equiv.), ***1,1*-Dimethylpropargylamine** (420 µL; 3.99 mmol; 1.5 equiv.) and magnetic stirrer and three times secured with argon. Solid **CuI** (21 mg; 110 µmol; 4.0 mol%) and **[Pd(PPh₃)₂Cl₂]** (19 mg; 27 µmol; 1.0 mol%) were subsequently added followed by another securing with argon (three times). Under constant flow of argon was then added dry THF (10 mL) through septum followed by addition of **TEA** (1.10 mL; 7.89 mmol; 3.0 equiv.) after which the mixture changed color to dark brown. The flask was left stirring under septum (but without external argon) for 18 h at RT. The mixture was filtered with syringe microfilter (PTFE) and the solid phase was further washed with MeCN. Filtrate was evaporated to dryness and the resulting dark brown solid residue was purified on flash chromatography (120 g SiO₂,100% EtOAc to 40% EtOAc in MeOH). Combined fractions with product were evaporated to dryness and further co-evaporated once with DCM. The residue was further dried on high vacuum overnight to give product in the form of free base as yellow solidified oil. **Yield:** 283 mg (56%; 1 step; based on **(6-Bromopyridin-2-yl)methanol). NMR (DMSO-d*₆*): ¹H** δ_{H} 1.38 (C*H*₃, s, 6H); 3.17 (N*H*₂, s, 2H); 4.51 (C*H*₂, s, 2H); 5.44 (O*H*, bs, 1H); 7.26 (*arom.,* dd, 1H, ³*J*_{HH} = 8; ⁴*J*_{HH} = 1); 7.40 (*arom.,* dd, 1H, ³*J*_{HH} = 8; ⁴*J*_{HH} = 1); 7.76 (*arom.,* t, 1H, ³*J*_{HH} = 8). **ESI-HRMS:** 191.1180 [M+H]⁺ (theor. [C₁₁H₁₅N₂O₁]⁺ = 191.1180). 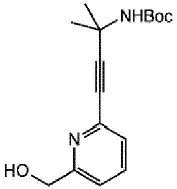 **Synthesis of TD1048:** Pear-shaped glass flask (25 mL) was charged with **TD1045** (280 mg; 1.47 mmol; 1.0 equiv.) followed by addition of MeCN (10 mL) and solution of **Boc₂O** (2.0 M in dry THF; 1.0 mL; 2.00 mmol; 1.4 equiv.). The resulting mixture was stirred 16 h at RT. Mixture was then evaporated to dryness and the resulting yellow residue was purified on flash chromatography (120 g SiO₂,100% DCM to 100% EtOAc). Combined fractions with product were evaporated to dryness and further co-evaporated once with DCM. The residue was further dried on high vacuum overnight to give product in the form of free base as colourless solid. **Yield:** 242 mg (57%; 1 step; based on **TD1045**). **NMR (DMSO-d*₆*): ¹H** δ_{H} 1.40 (C*H*₃, s, 9H), 1.53 (C*H*₃, s, 6H); 4.51 (C*H*₂, d, 2H, ³*J*_{HH} = 4); 5.44 (OH, t, 1H, ³*J*_{HH} = 4); 7.12 (NH, bs, 1H); 7.25 (*arom.,* dd, 1H, ³*J*_{HH} = 8; ⁴*J*_{HH} = 1); 7.41 (*arom.,* dd, 1H, ³*J*_{HH} = 8; ⁴*J*_{HH} = 1); 7.77 (*arom.,* t, 1H, ³*J*_{HH} = 8). **ESI-HRMS:** 291.1704 [M+H]⁺ (theor. [C₁₆H₂₃N₂O₃]⁺ = 291.1703). 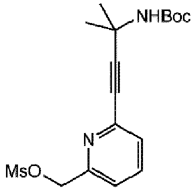 **Synthesis of TD1050:** In a glass vial (20 mL), **TD1048** (19 mg; 65 µmol; 1.0 equiv.) was dissolved in DCM (3 mL) followed by addition of **TEA** (28 µL; 201 µmol; 3.1 equiv.). Freshly prepared solution of **MsCl** (10 µL; 129 µmol; 2.0 equiv.) in DCM (1 mL) was then added. Resulting solution was stirred 20 min at RT. Mixture was then diluted with DCM (15 mL) followed by addition of dil. aq. solution of NaHCO₃ (15 mL). The resulting biphasic mixture was then transferred to a separatory funnel. After shaking, the bottom phase was separated. Aqueous phase was further extracted with DCM (3 × 20 mL). Combined organic layers were dried with anhydrous Na₂SO₄, filtered through glass frit (S3) and evaporated to dryness. **ESI-MS** (LC-MS): 369.1 [M+H]⁺ (theor. [C₁₇H₂₅N₂O₅S₁]⁺ = 369.1). All of **TD1050** obtained in this way was directly used for **TD1054** (and analogically for **TD1105**) without further purification or characterization.

**Synthesis of TD966:** Pear-shape glass flask (100 mL) was charged with **(6-Bromoridin-2-yl)methanol** 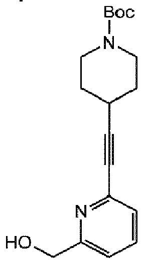 (1.00 g; 5.35 mmol; 1.0 equiv.), ***N*-Boc-4-ethynylpiperidine** (1.23 g; 5.88 mmol; 1.1 equiv.) and magnetic stirrer and three times secured with argon. Solid **CuI** (41 mg; 215 µmol; 4.0 mol%) and **[Pd(PPh₃)₂Cl₂]** (75 mg; 107 µmol; 2.0 mol%) were subsequently added followed by another securing with argon (three times). Under constant flow of argon was then added dry THF (40 mL) through septum followed by addition of **TEA** (2.20 mL; 15.7 mmol; 3.0 equiv.) after which the mixture changed color to dark brown. The flask was left stirring under septum (but without external argon) for 2 d at RT. The mixture was then transferred to a separatory funnel with EtOAc (30 mL) and H₂O (50 mL). After shaking, the dark colored (and not entirely homogenous) organic layer was separated and the aqueous layer was further extracted with EtOAc (3 × 30 mL). Combined organic layers were filtered through cotton plug and the filtrate was further dried with anhydrous Na₂SO₄, filtered through glass frit (S3) and evaporated to dryness. Resulting dark brown oily residue was purified on flash chromatography (220 g SiO₂, 100% DCM to 100% EtOAc). Combined fractions with product were evaporated to dryness and further co-evaporated once with DCM. The residue was further dried on high vacuum overnight to give product in the form of free base as yellow oil. **Yield:** 1.47 g (87%; 1 step; based on **(6-Bromopyridin-2-yl)methanol). NMR (DMSO-d*₆*): ¹H** δ_{H} 1.40 (C*H*₃, s, 9H); 1.44-1.57 (C*H*₂, m, 2H); 1.75-1.89 (C*H*₂, m, 2H); 2.87 (C*H*-C≡C tt, 1H, ³*J*_{HH} = 8, ³*J*_{HH} = 4); 3.01-3.17 (C*H*₂, m, 2H); 3.59-3.73 (C*H*₂, m, 2H); 4.51 (C*H*₂-O, d, 2H, ³*J*_{HH} = 6); 5.44 (O*H,* t, 1H, ³*J*_{HH} = 6); 7.32 (*arom.,* dd, 1H, ³*J*_{HH} = 8; ⁴*J*_{HH} = 1); 7.41 (*arom.,* dd, 1H, ³*J*_{HH} = 8; ⁴*J*_{HH} = 1); 7.76 (*arom.,* t, 1H, ³*J*_{HH} = 8). **ESI-HRMS:** 317.1860 [M+H]⁺ (theor. [C₁₈H₂₅N₂O₃]⁺ = 317.1860). 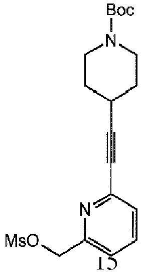 **Synthesis of TD1117:** In a glass vial (20 mL), **TD952** (50.0 mg; 158 µmol; 1.0 equiv.) was dissolved in DCM (6 mL) followed by addition of **TEA** (66 µL; 473 µmol; 3.0 equiv.) and neat **MsCl** (25 µL; 323 µmol; 2.0 equiv.). Resulting solution was stirred 20 min at RT followed by addition of DCM (10 mL) dil. aq. solution of NaHCO₃ (10 mL). The resulting biphasic mixture was then transferred to a separatory funnel. After shaking, the bottom phase was separated. Aqueous phase was further extracted with DCM (4 × 10 mL). Combined organic layers were dried with anhydrous Na₂SO₄, filtered through glass frit (S3) and evaporated to dryness. **ESI-MS** (LC-MS): 395.2 [M+H]⁺ (theor. [C₁₉H2₇N₂O₅S₁]⁺= 395.2). All of TD1117 obtained in this way was directly used for **TD1118** without further purification or characterization. 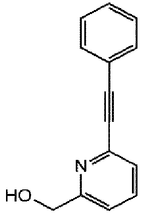 **Synthesis of TD936:** Glass vial (4 mL) was charged with **(6-Bromopyridin-2-yl)methanol** (135 mg; 722 µmol; 1.0 equiv.), **CuI** (5.5 mg; 29 µmol; 4.0 mol%), **[Pd(PPh₃)₂Cl₂**] (10.0 mg; 14 µmol; 2.0 mol%) and magnetic stirrer and three times secured with argon. Under constant flow of argon was then added dry THF (3 mL) through septum followed by addition of **phenylacetylene** (95 µL; 865 µmol; 1.2 equiv.) and **TEA** (300 µL; 2.15 mmol; 3.0 equiv.) after which the mixture changed color to dark brown. The vial was left stirring under septum (but without external argon) for 16 h at RT. The mixture was then filtered through syringe microfilter (PTFE) followed by washing with MeCN. Filtrate was evaporated to dryness, re-dissolved in MeCN (3 mL) and directly purified by preparative HPLC (C18; H₂O-MeCN gradient with TFA additive). Fractions containing product were neutralized with dil. aq. NaHCO₃ and evaporated to dryness. Residue was dissolved in a mixture of DCM (50 mL) and H₂O(50 mL) and transferred to a separatory funnel. After brief shaking, the bottom phase was separated and the aq. layer was further extracted with DCM (4 × 25 mL). Combined organic layers were dried with anhydrous Na₂SO₄, filtered through glass frit (S3) and evaporated to dryness. The residual solid was briefly dried on high vacuum to give product in the form of free base as a yellow oil. **Yield:** 141 mg (93%; 1 step; based on **(6-Bromopyridin-2-yl)methanol). NMR (DMSO-d*₆*): ¹H** δ_{H} 4.57 (C*H*₂, d, 2H, ³*J*_{HH} = 6); 5.50 (O*H,* t, 1H, ³*J*_{HH} = 6); 7.42-7.54 (*arom., Ph,* m, 2+3H); 7.57-7.64 (*Ph,* m, 2H); 7.85 (*arom.,* t, 1H, ³*J*_{HH} = 8). **ESI-HRMS:** 210.0913 [M+H]⁺ (theor. [C₁₄H₁₂N₁O₁]⁺= 210.0913). 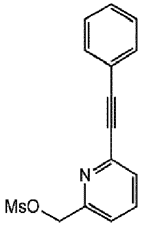 **Synthesis of TD939:** In a glass vial (20 mL), **TD936** (22 mg; 105 µmol; 1.0 equiv.) was dissolved in DCM (6 mL) followed by addition of **TEA** (44 µL; 316 µmol; 3.0 equiv.). and neat **MsCl** (16 µL; 207 µmol; 2.0 equiv.). Resulting solution was stirred 20 min at RT followed by addition of dil. aq. solution of NaHCO₃ (5 mL). The resulting biphasic mixture was then transferred to a separatory funnel. After shaking, the bottom phase was separated. Aqueous phase was further extracted with DCM (4 × 25 mL). Combined organic layers were dried with anhydrous Na₂SO₄, filtered through glass frit (S3) and evaporated to dryness. **ESI-MS** (LC-MS): 288.1 [M+H]⁺ (theor. [C₁₅H₁₄N₁O₃S₁]⁺ = 288.1). All of **TD939** obtained in this way was directly used for **TD944** without further purification or characterization. 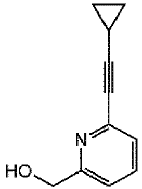 **Synthesis of TD937:** Glass vial (4 mL) was charged with **(6-Bromopyridin-2-yl)methanol** (135 mg; 722 µmol; 1.0 equiv.), **CuI** (5.5 mg; 29 µmol; 4.0 mol%), [**Pd**(**PPh₃**)**₂Cl₂**] (10.0 mg; 14 µmol; 2.0 mol%) and magnetic stirrer and three times secured with argon. Under constant flow of argon was then added dry THF (3 mL) through septum followed by addition of **Cyclopropylacetylene** (75 µL; 886 µmol; 1.2 equiv.) and **TEA** (300 µL; 2.15 mmol; 3.0 equiv.) after which the mixture changed color to dark brown. The vial was left stirring under septum (but without external argon) for 16 h at RT. The mixture was then filtered through syringe microfilter (PTFE) followed by washing with MeCN. Filtrate was evaporated to dryness, re-dissolved in MeCN (3 mL) and directly purified by preparative HPLC (C18; H₂O-MeCN gradient with TFA additive). Fractions containing product were neutralized with dil. aq. NaHCO₃ and evaporated to dryness. Residue was dissolved in a mixture of DCM (50 mL) and H₂O (50 mL) and transferred to a separatory funnel. After brief shaking, the bottom phase was separated and the aq. layer was further extracted with DCM (4 × 25 mL). Combined organic layers were dried with anhydrous Na₂SO₄, filtered through glass frit (S3) and evaporated to dryness. The residual solid was briefly dried on high vacuum to give product in the form of free base as a yellow oil. **Yield:** 98 mg (78%; 1 step; based on (**6-Bromopyridin-2-yl**)**methanol**). **NMR** (**DMSO-d*₆***)**: ¹H** δ_{H} 0.69-0.83 (C*H*₂-CH, m, 2H); 0.83-0.99 (C*H*₂-CH, m, 2H); 1.56 (C*H*, tt, ³*J*_{HH} = 8, ³*J*_{HH} = 5); 4.49 (C*H*₂, d, 2H, ³*J*_{HH} = 6); 5.42 (O*H,* t, 1H, ³*J*_{HH} = 6); 7.27 (*arom.,* dd, 1H, ³*J*_{HH} = 8, ⁴*J*_{HH} = 1); 7.38 (*arom.,* dd, 1H, ³*J*_{HH} = 8, ⁴*J*_{HH} = 1); 7.74 (*arom.,* t, 1H, ³*J*_{HH} = 8). **ESI-HRMS:** 174.0913 [M+H]⁺ (theor. [C₁₁H₁₂N₁O₁]⁺ = 174.0913). 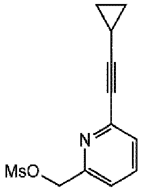 **Synthesis of TD940:** In a glass vial (20 mL), **TD937** (26 mg; 150 µmol; 1.0 equiv.) was dissolved in DCM (9 mL) followed by addition of **TEA** (63 µL; 452 µmol; 3.0 equiv.) and neat **MsCl** (23 µL; 297 µmol; 2.0 equiv.). Resulting solution was stirred 20 min at RT followed by addition of dil. aq. solution of NaHCO₃ (5 mL). The resulting biphasic mixture was then transferred to a separatory funnel. After shaking, the bottom phase was separated. Aqueous phase was further extracted with DCM (4 × 25 mL). Combined organic layers were dried with anhydrous Na₂SO₄, filtered through glass frit (S3) and evaporated to dryness. **ESI-MS** (LC-MS): 252.0 [M+H]⁺ (theor. [C₁₂H₁₄N₁O₃S₁]⁺ = 252.1). All of **TD940** obtained in this way was directly used for **TD943** without further purification or characterization. 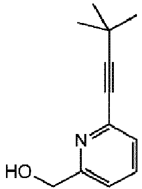 **Synthesis of TD952:** Glass vial (4 mL) was charged with (**6-Bromopyridin-2-yl**)**methanol** (150 mg; 802 µmol; 1.0 equiv.), **CuI** (6.1 mg; 32 µmol; 4.0 mol%), [**Pd**(**PPh₃**)**₂Cl₂**] (11.5 mg; 16 µmol; 2.0 mol%) and magnetic stirrer and three times secured with argon. Under constant flow of argon was then added dry THF (3 mL) through septum followed by addition of ***tert-*butylacetylene** (200 µL; 1.62 mmol; 2.0 equiv.) and **TEA** (340 µL; 2.44 mmol; 3.0 equiv.) after which the mixture changed color to dark brown. The vial was left stirring under septum (but without external argon) for 24 h at RT. The mixture was then filtered through syringe microfilter (PTFE) followed by washing with MeCN. Filtrate was evaporated to dryness, re-dissolved in MeCN (3 mL) and directly purified by preparative HPLC (C18; H₂O-MeCN gradient with TFA additive). Fractions containing product were neutralized with dil. aq. NaHCO₃ and evaporated to dryness. Residue was dissolved in a mixture of DCM (50 mL) and H₂O (50 mL) and transferred to a separatory funnel. After brief shaking, the bottom phase was separated and the aq. layer was further extracted with DCM (4 × 25 mL). Combined organic layers were dried with anhydrous Na₂SO₄, filtered through glass frit (S3) and evaporated to dryness. The residual solid was briefly dried on high vacuum to give product in the form of free base as a yellow oil. **Yield:** 146 mg (96%; 1 step; based on (**6-Bromopyridin-2-yl**)**methanol**)**. NMR** (**DMSO-d*₆***): **¹H** δ_{H} 1.30 (C*H*₃, s, 9H); 4.51 (C*H*₂, d, 2H, ³*J*_{HH} = 6); 5.43 (*OH,* t, 1H, ³*J*_{HH} = 6); 7.26 (*arom.,* dd, 1H, ³*J*_{HH} = 8, ⁴*J*_{HH} = 1); 7.39 (*arom.,* dd, 1H, ³*J*_{HH} = 8, ⁴*J*_{HH} = 1); 7.74 (*arom.,* t, 1H, ³*J*_{HH} = 8). **ESI-HRMS:** 190.1227 [M+H]⁺ (theor. [C₁₂H₁₆N₁O₁]⁺ = 190.1226). 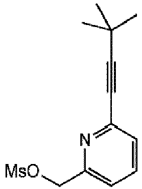 **Synthesis of TD956:** In a glass vial (20 mL), **TD952** (8 mg; 42 µmol; 1.0 equiv.) was dissolved in DCM (3 mL) followed by addition of **TEA** (18 µL; 129 µmol; 3.0 equiv.) and neat **MsCl** (6.5 µL; 84 µmol; 2.0 equiv.). Resulting solution was stirred 20 min at RT followed by addition of DCM (5 mL) dil. aq. solution of NaHCO₃ (5 mL). The resulting biphasic mixture was then transferred to a separatory funnel. After shaking, the bottom phase was separated. Aqueous phase was further extracted with DCM (4 × 10 mL). Combined organic layers were dried with anhydrous Na₂SO₄, filtered through glass frit (S3) and evaporated to dryness. **ESI-MS** (LC-MS): 268.1 [M+H]⁺ (theor. [C₁₃H₁₈N₁O₃S₁]⁺ = 268.1). All of **TD956** obtained in this way was directly used for **TD959** without further purification or characterization. 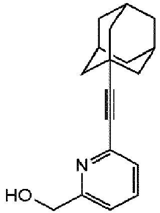 **Synthesis of TD965:** Glass vial (4 mL) was charged with (**6-Bromopyridin-2-yl**)**methanol** (53 mg; 283 µmol; 1.0 equiv.), ***1*-Ethynyladamantane** (50 mg; 312 µmol; 1.1 equiv.), **CuI** (2.7 mg; 14 µmol; 5.0 mol%), [**Pd**(**PPh₃**)**₂Cl₂**] (5.0 mg; 7 µmol; 2.5 mol%) and magnetic stirrer and three times secured with argon. Under constant flow of argon was then added dry THF (1.5 mL) through septum followed by addition of **TEA** (120 µL; 861 µmol; 3.0 equiv.) after which the mixture changed color to dark brown. The vial was left stirring under septum (but without external argon) for 2 d at RT. The mixture was then filtered through syringe microfilter (PTFE) followed by washing with MeCN. Filtrate was evaporated to dryness, re-dissolved in MeCN (3 mL) and directly purified by preparative HPLC (C18; H₂O-MeCN gradient with TFA additive). Fractions containing product were neutralized with dil. aq. NaHCO₃ and evaporated to dryness. Residue was dissolved in a mixture of DCM (25 mL) and H₂O (25 mL) and transferred to a separatory funnel. After brief shaking, the bottom phase was separated and the aq. layer was further extracted with DCM (4 × 10 mL). Combined organic layers were dried with anhydrous Na₂SO₄, filtered through glass frit (S3) and evaporated to dryness. The residual solid was briefly dried on high vacuum to give product in the form of free base as a colourless oil. **Yield:** 67 mg (89%; 1 step; based on (**6-Bromopyridin-2-yl**)**methanol**). **NMR** (**DMSO-d*₆***)**: ¹H** δ_{H} 1.61-1.76 (*Adm.,* m, 6H); 1.82-1.93 (*Adm.,* m, 6H); 1.94-2.00 (*Adm.,* m, 3H); 4.50 (C*H*₂, d, 2H, ³*J*_{HH} = 6); 5.43 (O*H,* t, 1H, ³*J*_{HH} = 6); 7.25 (*arom.,* dd, 1H, ³*J*_{HH} = 8, ⁴*J*_{HH} = 1); 7.38 (*arom.,* dd, 1H, ³*J*_{HH} = 8, ⁴*J*_{HH} = 1); 7.74 (*arom.,* t, 1H, ³*J*_{HH} = 8). **ESI-HRMS:** 268.1694 [M+H]⁺ (theor. [C₁₈H₂₂N₁O₁]⁺ = 268.1696). 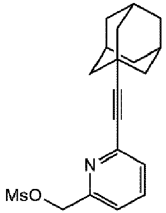 **Synthesis of TD990:** In a glass vial (20 mL), **TD965** (21 mg; 79 µmol; 1.0 equiv.) was dissolved in DCM (3 mL) followed by addition of **TEA** (33 µL; 237 µmol; 3.0 equiv.) and neat **MsCl** (12 µL; 155 µmol; 2.0 equiv.). Resulting solution was stirred 20 min at RT followed by addition of DCM (10 mL) dil. aq. solution of NaHCO₃ (10 mL). The resulting biphasic mixture was then transferred to a separatory funnel. After shaking, the bottom phase was separated. Aqueous phase was further extracted with DCM (3 × 15 mL). Combined organic layers were dried with anhydrous Na₂SO₄, filtered through glass frit (S3) and evaporated to dryness. **ESI-MS** (LC-MS): 346.1 [M+H]⁺ (theor. [C₁₉H₂₄N₁O₃S₁]⁺ = 346.1). All of **TD990** obtained in this way was directly used for **TD992** without further purification or characterization. 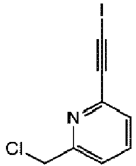 **Synthesis** of **TD1194:** In a glass vial (4 mL), **TD558** (110 mg; 726 µmol; 1.0 equiv.) was dissolved in MeCN (3.3 mL) followed by addition of **NIS** (180 mg; 800 µmol; 1.1 equiv.) and **AcOH** (50 µL; 875 µmol; 1.2 equiv.) and the resulting mixture was stirred 3 h at 80 °C. Mixture was then directly purified by preparative HPLC (C18; H₂O-MeCN gradient with FA additive). Fractions containing product were joined and diluted with DCM (50 mL). The resulting biphasic mixture was transferred to a separatory funnel followed by addition of dil. aq. NaHCO₃ and aq. Na₂S₃O₃. After brief shaking, the bottom phase was separated and the aq. layer was further extracted with DCM (3 × 25 mL). Combined organic layers were dried with anhydrous Na₂SO₄, filtered through glass frit (S3) and evaporated to dryness. The residue was briefly dried on high vacuum to give product in the form of free base as a pale red solid. **Yield:** 61.5 mg (31%; 1 step; based on **TD558**)**. NMR** (**DMSO-d*₆***)**: ¹H** δ_{H} 4.74 (C*H*₂, s, 2H); 7.48 (*arom.,* dd, 1H, ³*J*_{HH} = 8, ⁴*J*_{HH} = 1); 7.54 (*arom.,* dd, 1H, ³*J*_{HH} = 8, ⁴*J*_{HH} = 1); 7.85 (*arom.,* t, 1H, ³*J*_{HH} = 8). **ESI-HRMS:** 277.9230 [M+H]⁺ (theor. [C₈H₆N₁I₁Cl₁]⁺ = 277.9228). 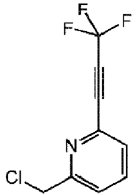 **Synthesis of TD1178:** Glass vial (20 mL) was charged with **CuI** (60.0 mg; 315 µmol; 40 mol%), **Phen** (114 mg; 633 µmol; 80.0 mol%), **KHCO₃** (159 mg; 1.59 mmol; 2.0 equiv.) and magnetic stirrer and three times secured with argon. Under constant flow of argon was then added solution of **TD558** (120 mg; 792 µmol; 1.0 equiv.) and **Togni I** (288 mg; 872 µmol; 1.1 equiv.) in dry DCM (8 mL) through septum and the resulting mixture was stirred 2 d at RT. Mixture was then filtered through syringe microfilter (PTFE) and the solid phase was further washed with DCM. Filtrate was evaporated to dryness and the residue was resuspended in MeCN (3 mL). Mixture was again filtered through syringe microfilter (PTFE) and the filtrate was purified by preparative HPLC (C18; H₂O-MeCN gradient with FA additive). Fractions containing product were joined and diluted with DCM (50 mL). The resulting biphasic mixture was transferred to a separatory funnel followed by addition of dil. aq. NaHCO₃. After brief shaking, the bottom phase was separated and the aq. layer was further extracted with DCM (3 × 25 mL). Combined organic layers were dried with anhydrous Na₂SO₄, filtered through glass frit (S3) and evaporated to dryness. The resulting yellow oil was briefly dried on high vacuum (until the oil crystallized) to give product in the form of free base as pale yellow solid (portion of the product sublimed as colourless crystals on the upper part of the flask). **Yield:** 48.5 mg (28%; 1 step; based on **TD558). NMR** (**DMSO-d*₆***)**: ¹H** δ_{H} 4.83 (C*H*₂, s, 2H); 7.78 (*arom.,* dd, 1H, ³*J*_{HH} = 8, ⁴*J*_{HH} = 1); 7.88 (*arom.,* dd, 1H, ³*J*_{HH} = 8, ⁴*J*_{HH} = 1); 8.04 (*arom.,* t, 1H, ³*J*_{HH} = 8). **¹⁹F{¹H}** δ_{F} -49.2 (s). **ESI-HRMS:** 220.0140 [M+H]⁺ (theor. [C₉H₆N₁F₃Cl₁]^{÷} = 220.0135). 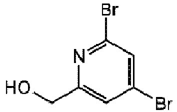 **Synthesis of TD797:** In a round-bottom glass flask (50 mL), **Methyl 4,6-dibromopicolinate** (200 mg; 678 µmol; 1.0 equiv.) was dissolved in a mixture of THF (4 mL) and MeOH (2 mL). To the resulting slightly yellow solution was added portion wise (over course of 10 min, with no stopper on the flask) solid **NaBH₄** (205 mg; 5.42 mmol; 8.0 equiv.) during which hydrogen gas evolved intensively and the color of the mixture changed to nearly colorless. Mixture was then transferred to a separatory funnel and diluted with DCM (100 mL) and H₂O (100 mL). After shaking, the bottom phase was separated. Aqueous phase was further extracted with DCM (3 × 50 mL). Combined organic layers were dried with anhydrous Na₂SO₄, filtered through glass frit (S3) and evaporated to dryness. The residue was further dried on high vacuum overnight to give product in the form of free base as white solid. **Yield:** 179 mg (99%; 1 step; based on **Methyl 4,6-dibromopicolinate**)**. NMR** (**DMSO-d*₆***)**: ¹H** δ_{H} 4.53 (C*H*₂, s, 2H); 5.68 (O*H*, bs, 1H); 7.67 (*arom.,* m, 1H); 7.89 (*arom.,* m, 1H). **ESI-HRMS:** 265.8809 [M+H]⁺ (theor. [C₆H₆N₁O₁Br₂]⁺ = 265.8811). 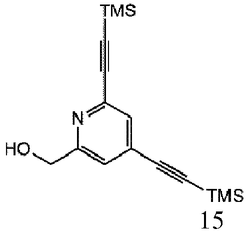 **Synthesis of TD804:** Glass vial (4 mL) was charged with **TD797** (100 mg; 375 µmol; 1.0 equiv.) and magnetic stirrer and three times secured with argon. Solid **CuI** (4.3 mg; 23 µmol; 6.0mol%) and [**Pd**(**PPh₃**)**₂Cl₂**] (8.0 mg; 11 µmol; 3.0 mol%) were subsequently added followed by another securing with argon (three times). Under constant flow of argon was then added dry THF (1.5 mL) through septum followed by addition of **Ethynyltrimethylsilane** (160 µL; 1.16 mmol; 3.1 equiv.) and **TEA** (160 µL; 1.15 mmol; 3.1 equiv.) after which the mixture changed color to dark brown. The flask was left stirring under septum (but without external argon) for 16 h at RT. The mixture was then transferred to a separatory funnel with EtOAc (50 mL) and H₂O (50 mL). After shaking, the dark colored organic layer was separated and the aqueous layer was further extracted with EtOAc (3 × 25 mL). Combined organic layers were dried with anhydrous Na₂SO₄, filtered through glass frit (S3) and evaporated to dryness. Resulting dark brown oily residue was purified on flash chromatography (120 g SiO₂, 100% DCM to 20% EtOAc in DCM). Combined fractions with product were evaporated to dryness and further co-evaporated once with DCM. The residue was further dried on high vacuum overnight to give product in the form of free base as dark brown oil. **Yield:** 99 mg (88%; 1 step; based on **TD797). NMR** (**DMSO-d*₆***)**: ¹H** δ_{H} 0.24 (C*H*₃, s, 9H); 0.25 (C*H*₃, s, 9H); 4.51 (C*H*₂, d, 2H, ³*J*_{HH} = 6); 5.53 (O*H,* t, 1H, ³*J*_{HH} = 6); 7.40 (*arom.,* d, 1H, ⁴*J*_{HH} = 2); 7.42 (*arom.,* d, 1H, ⁴*J*_{HH} = 2). **ESI-HRMS:** 302.1389 [M+H]⁺ (theor. [C₁₆H₂₄N₁O₁Si₂]⁺ = 302.1391). 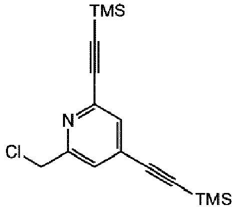 **Synthesis of TD806:** In a pear-shaped glass flask (50 mL), **TD804** (98 mg; 325 µmol; 1.0 equiv.) was dissolved in DCM (3 mL). Freshly prepared solution of **SOCl₂** (47 µL; 647 µmol; 2.0 equiv.) in DCM (1 mL) was then added and the open flask was stirred 1 h at RT. Reaction mixture was diluted with DCM (16 mL) and dil. aq. solution of NaHCO₃ (20 mL) was then added and resulting biphasic mixture was vigorously stirred for additional 1 h at RT, during which bubbles of gas slowly evolved. Mixture was then transferred to a separatory funnel. After shaking, the bottom phase was separated. Aqueous phase was further extracted with DCM (3 × 20 mL). Combined organic layers were dried with anhydrous Na₂SO₄, filtered through glass frit (S3) and evaporated to dryness. The residue was further dried on high vacuum overnight to give product in the form of free base as brown oil. Yield: 102 mg (98%; 1 step; based on **TD804**)**. NMR** (**DMSO-d*₆***)**: ¹H** δ_{H} 0.25 (C*H*₃, s, 18H); 4.74 (C*H*₂, s, 2H); 7.53 (*arom.,* d, 1H, ⁴*J*_{HH} = 2); 7.60 (*arom.,* d, 1H, ⁴*J*_{HH} = 2). **ESI-HRMS:** 320.1051 [M+H]⁺ (theor. [C₁₆H₂₃N₁O₁Cl₁Si₂]⁺ = 320.1052). 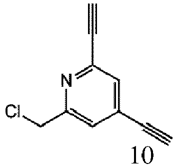 **Synthesis of TD807:** In a round-bottom glass flask (50 mL), **TD806** (101 mg; 316 µmol; 1.0 equiv.) was dissolved in MeCN (5 mL). Freshly prepared solution of **KF** (46.4 mg; 800 µmol; 2.5 equiv.) in H₂O (800 µL) was then added and the flask was stirred 5 h at RT. The mixture was concentrated to ~1 ml and diluted with DCM (50 mL) and dil. aq. NaHCO₃ (50 mL). The mixture was transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as brown solid. **Yield:** 48 mg (87%; 1 step; based on **TD806**)**. NMR** (**DMSO-d*₆***)**: ¹H** δ_{H} 4.48 (C=CH, s, 1H); 4.75 (C=CH, s, 1H); 4.76 (C*H*₂, s, 2H); 7.61 (*arom.,* d, 1H, ⁴*J*_{HH} = 1); 7.65 (*arom.,* d, 1H, ⁴*J*_{HH} = 1). **ESI-HRMS:** 176.0267 [M+H]⁺ (theor. [C₁₀H₇N₁Cl₁]⁺ = 176.0267). 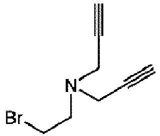 **Synthesis of TD662:** In a glass vial (4 mL), **Dipropargylamine** (116 µL; 1.12 mmol; 1.0 equiv.) was dissolved in MeCN (2.5 mL) followed by addition of neat **1,2-Dibromoethane** (965 µL; 11.2 mmol; 10 equiv.) and **NaHCO₃** (113 mg; 1.35 mmol; 1.2 equiv.) and the resulting mixture was stirred 2 days at 80 °C. Mixture was then diluted with DCM (50 mL) followed by addition of dil. aq. solution of NaHCO₃ (50 mL). The resulting biphasic mixture was then transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. Residue was further purified by column chromatography (SiO2; 25 g; DCM). Combined fractions with product were evaporated to dryness. The residue was further dried on high vacuum overnight to give product in the form of free base as nearly colorless oil. **Yield:** 69 mg (31%; 1 step; based on **Dipropargylamine**)**. NMR** (**DMSO-d*₆***)**: ¹H** δ_{H} 2.86 (C*H*₂, t, 2H, ³*J*_{HH} = 7); 3.21 (C≡C*H*, t, 2H, ⁴*J*_{HH} = 2); 3.21 (CH₂, t, 4H, ⁴*J*_{HH} = 2); 3.55 (C*H*₂, t, 2H, ³*J*_{HH} = 7). **¹³C{¹H}** δ_{C} 30.3 (*C*H₂, s); 41.6 (*C*H₂, s); 53.9 (*C*H₂, s); 76.4 and 78.5 (*C*≡*C*H, 2 × s). **ESI-HRMS:** 200.0070 [M+H]⁺ (theor. [C₈H₁₁N₁Br₁]⁺ = 200.0070).

### Example 2: Synthesis of intermediates of azide pendant arms

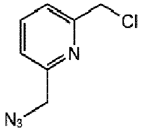

**Synthesis of TD406:** In a round-bottom glass flask (250 mL), **2,6-Bis**(**chloromethyl**)**pyridine** (6.17 g; 35.0 mmol; 1.2 equiv.) was dissolved in MeCN (80 mL) followed by addition of solid NaN₃ (1.90 g; 29.2 mmol; 1.0 equiv.) and anhydrous K₂CO₃ (4.04 g; 29.2 mmol; 1.0 equiv.) and the resulting suspension was stirred 5 days at 50 °C. The mixture was filtered on glass frit S3 and the solid residue was washed with MeCN. Filtrate was evaporated to dryness and the orange oily residue was purified by column chromatography (SiO₂, 30% P.E. in DCM to 100% DCM). Fractions with product were combined and evaporated to dryness. The residue was further dried on high vacuum overnight to give product in the form of free base as faint yellow oil. **Yield:** 2.62 g (49%; 1 step; based on **NaN₃**)**. Recovery:** 2.21 g of **2,6-Bis**(**chloromethyl**)**pyridine** (36% of the initial amount used). **NMR** (**DMSO-d*₆***)**: ¹H** δ_{H} 4.53 (C*H*₂, s, 2H); 4.78 (C*H*₂, s, 2H); 7.40 (*arom.,* d, 1H, ³*J*_{HH} = 8; ⁴*J*_{HH} = 1); 7.52 (*arom.,* d, 1H, ³*J*_{HH} = 8; ⁴*J*_{HH} = 1); 7.89 (*arom.,* t, 1H, ³*J*_{HH} = 8). **¹³C{¹H}** δ_{C} 46.6 (*C*H₂, s); 54.2 (*C*H₂, s); 121.9 (*arom.,* s); 122.6 (*arom.,* s); 138.5 (*arom.,* s); 155.7 (*arom.,* s); 156.3 (*arom.,* s). **ESI-HRMS:** 183.0433 [M+H]⁺ (theor. [C₇H₈N₄Cl₁]⁺ = 183.0432). 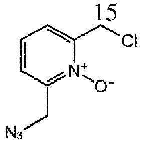 **Synthesis of TD595:** In a glass vial (20 mL), **TD406** (30 mg; 162 µmol; 1.0 equiv.) was dissolved in DCM (5 mL) followed by addition of freshly prepared solution of **MCPBA** (77%; 72 mg; 320 µmol; 2.0 equiv.) in DCM (1 mL). The resulting solution was stirred 3 h at RT. Mixture was then diluted with DCM (20 mL) and dil. aq. NaHCO₃ (25 mL) and transferred to a separatory funnel. After shaking, the bottom layer was separated and aqueous layer was further extracted with DCM (3 × 20 mL). Combined organic layers were dried with anhydrous Na₂SO₄, filtered through glass frit (S3) and evaporated to dryness. **ESI-MS** (LC-MS): 199.0 [M+H]⁺ (theor. [C₇H₈N₄O₁Cl₁]⁺ = 199.0). All of **TD595** obtained in this way was directly used for **TD596** without further purification or characterization. 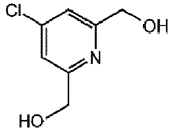 **Synthesis of TD726:** In a round-bottom glass flask (1000 mL), **Dimethyl 4-chloropyridine-2,6-dicarboxylate** (15.0 g; 65.3 mmol; 1.0 equiv.) was gradually dissolved in a mixture of THF (540 mL) and MeOH (120 mL). The resulting slightly yellow solution was cooled with an ice bath (~5 °C) followed by portion wise additions (over course of 1 h, with no stopper on the flask) of solid **NaBH₄** (12.4 g; 328 mmol; 5.0 equiv.) during which hydrogen gas evolved intensively and the color of the mixture changed to red, orange and eventually yellow. After the addition, the flask was allowed to warm up to RT and was further stirred 16 h at RT. Resulting faint yellow opalescent solution was filtered on glass frit (S3), the filtrate was evaporated to dryness and further co-evaporated with DCM (as a suspension). Residue was dissolved in boiling H₂O (~600 mL) and the resulting highly alkaline solution was continuously extracted by DCM overnight. The organic layer (containing partly crystallized product) was evaporated to dryness. The residue was mechanically crushed and further dried on high vacuum (till constant mass) to give product in the form of free base as nearly colorless microcrystalline powder. **Yield:** 10.84 g (96%; 1 step; based on **Dimethyl 4-chloropyridine-2,6-dicarboxylate**)**. NMR** (**DMSO-d*₆***)**: ¹H** δ_{H} 4.53 (C*H*₂, d, 4H, ³*J*_{HH} = 6); 5.54 (O*H,* t, 2H, ³*J*_{HH} = 6); 7.36 (*arom.,* s, 2H). **¹³C{¹H}** δ_{C} 63.7 (*C*H₂, s); 118.0 (*arom.,* s); 144.0 (*arom.,* s); 163.5 (*arom.,* s). **ESI-HRMS:** 174.0317 [M+H]⁺ (theor. [C₇H₉N₁O₂Cl₁]⁺ = 174.0316). **EA** (C₇H₈N₁O₂Cl₁, *M*_{R} = 173.6): C 48.4 (48.5); H 4.7 (4.5); N 7.7 (8.1); Cl 20.4 (21.2). 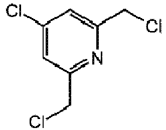 **Synthesis of TD759:** In a round-bottom glass flask (100 mL), **TD726** (626 mg; 3.61 mmol; 1.0 equiv.) was suspended in DCM (20 mL). Freshly prepared solution of **SOCl₂** (780 µL; 10.7 mmol; 3.0 equiv.) in DCM (10 mL) was then added and the open flask was stirred 90 min at RT, producing clear solution. Dil. aq. solution of NaHCO₃ (40 mL) was then added and resulting biphasic mixture was vigorously stirred for additional 1 h at RT, during which bubbles of gas slowly evolved. Mixture was then transferred to a separatory funnel. After shaking, the bottom phase was separated. Aqueous phase was further extracted with DCM (3 × 50 mL). Combined organic layers were dried with anhydrous Na₂SO₄, filtered through glass frit (S3) and evaporated to dryness. The resulting crystalized residue was crushed and further dried on high vacuum overnight to give product in the form of free base as nearly colorless microcrystalline powder. **Yield:** 711 mg (94%; 1 step; based on **TD726**)**. NMR** (**DMSO-d*₆***)**: ¹H** δ_{H} 4.79 (C*H*₂, s, 4H); 7.70 (*arom.,* s, 2H). **¹³C{¹H}** δ_{C} 45.7 (*C*H₂, s); 122.8 (*arom.,* s); 144.4 (*arom.,* s); 158.2 (*arom.,* s). **ESI-HRMS:** 209.9638 [M+H]⁺ (theor. [C₇H₇N₁Cl₃]⁺ = 209.9639). 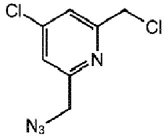 **Synthesis of TD760:** In a glass vial (20 mL), **TD759** (708 mg; 3.36 mmol; 1.3 equiv.) was dissolved in MeCN (20 mL) followed by addition of solid **NaN₃** (168 mg; 2.58 mmol; 1.0 equiv.) and dried **K₂CO₃** (360 mg; 2.61 mmol; 1.0 equiv.) and the resulting suspension was stirred 24 h at 80 °C. The mixture was filtered on through syringe microfilter (PTFE) and the solid residue was washed with MeCN. Filtrate was evaporated to dryness and yellow oily residue was purified by column chromatography (SiO₂, 40% P.E. in DCM to 10% P.E. in DCM). Fractions with product were combined and evaporated to dryness. The residue was further dried on high vacuum 1 h to give product in the form of free base as faint yellow oil. **Yield:** 323 mg (58%; 1 step; based on **NaN₃**)**. Recovery:** 218 mg of **TD759** (31% of the initial amount used). **NMR** (**DMSO-d*₆***)**: ¹H** δ_{H} 4.56 (C*H*₂, s, 2H); 4.78 (C*H*₂, s, 2H); 7.58 (*arom.,* d, 1H, ⁴*J*_{HH} = 2); 7.69 (*arom.,* d, 1H, ⁴*J*_{HH} = 8). **¹³C{¹H}** δ_{C} 45.8 (*C*H₂, s); 53.6 (*C*H₂, s); 121.9 (*arom.,* s); 122.5 (*arom.,* s); 144.4 (*arom.,* s); 157.9 (*arom.,* s); 158.2 (*arom.,* s). **ESI-HRMS:** 217.0040 [M+H]⁺ (theor. [C₇H₇N₄Cl₂]⁺ = 217.0042). 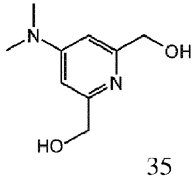 **Synthesis of TD1146:** In a glass vial (20 mL), **TD726** (495 mg; 2.85 mmol; 1.0 equiv.) was dissolved in **DMF** (11.0 mL; 143 mmol; 50 equiv.) followed by addition of freshly ground **KOH** (1.6 g; 28.5 mmol; 10 equiv.). The resulting suspension was stirred 3 days at 100 °C in the presence of air (vial septum was punctured by two thick needles). The mixture was then filtered through syringe microfilter (PTFE; the solids were washed with DMF). Filtrate was evaporated to dryness and purified by preparative HPLC (C18; H₂O-MeCN gradient with TFA additive). Fractions with product were combined and directly lyophilized to give product in the form of trifluoroacetate salt as white foam. **Yield:** 187 mg (22%; 1 step; based on **TD726**)**. NMR** (**DMSO-d*₆***)**: ¹H** δ_{H} 3.18 (C*H*₃, s, 6H); 4.59 (C*H*₂, d, 4H, ³*J*_{HH} = 6); 5.91 (O*H,* t, 2H, ³*J*_{HH} = 6); 6.84 (*arom.,* s, 2H). **ESI-HRMS:** 183.1128 [M+H]⁺ (theor. [C₉H₁₅N₂O₂]⁺ = 183.1128). **EA** (C₉H₁₄N₂O₂·1.0TFA, *M*_{R} = 296.2): C 44.6 (44.1); H 5.1 (4.9); N 9.5 (9.1); F 19.2 (18.2). 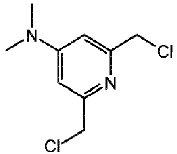 **Synthesis of TD1154:** In a round-bottom glass flask (100 mL), **TD1146·**1.0TFA (173.3 mg; 585 µmol; 1.0 equiv.) was suspended in DCM (17 mL) followed by addition of neat **SOCl₂** (780 µL; 2.34 mmol; 4.0 equiv.). The resulting mixture was stirred 90 min at RT, producing clear solution. Dil. aq. solution of NaHCO₃ (20 mL) was then added and resulting biphasic mixture was vigorously stirred for additional 1 h RT, during which bubbles of gas slowly evolved. Mixture was then transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as white solid. **Yield:** 105.6 mg (82%; 1 step; based on **TD726**)**. NMR** (**DMSO-d*₆***)**: ¹H** δ_{H} 2.98 (C*H*₃, s, 6H); 4.60 (C*H*₂, s, 4H); 6.73 (*arom.,* s, 2H). **ESI-HRMS:** 219.0450 [M+H]⁺ (theor. [C₉H₁₃N₂Cl₂]⁺ = 219.0450). 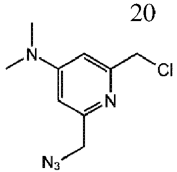 **Synthesis of TD1163:** In a glass vial (20 mL), **TD1154** (103.9 mg; 474 µmol; 1.3 equiv.) was dissolved in MeCN (7 mL) followed by addition of solid **NaN₃** (23.7 mg; 365 µmol; 1.0 equiv.) and dried **K₂CO₃** (50.3 mg; 364 µmol; 1.0 equiv.) and the resulting suspension was stirred 24 h at 70 °C. The mixture was filtered on through syringe microfilter (PTFE) and the solid residue was washed with MeCN. Filtrate was evaporated to dryness and the residue was purified by column chromatography (SiO₂, 5% EtOAc in DCM to 10% EtOAc in DCM). Fractions with product were combined and evaporated to dryness. The residue was further dried on high vacuum 1 h to give product in the form of free base as colourless oil. **Yield:** 33.8 mg (41%; 1 step; based on **NaN₃**). **Recovery:** 11.0 mg of **TD1154** (11% of the initial amount used). **NMR** (**DMSO-d*₆***)**: ¹H** δ_{H} 2.99 (C*H*₃, s, 6H); 4.32 (C*H*₂, s, 2H); 4.60 (C*H*₂, s, 2H); 6.60 (*arom.,* d, 1H, ⁴*J*_{HH} = 2); 6.73 (*arom.,* d, 1H, ⁴*J*_{HH} = 2). **ESI-HRMS:** 226.0853 [M+H]⁺ (theor. [C₉H₁₃N₅Cl₁]⁺ = 226.0854). 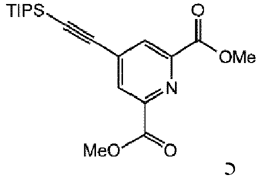 **Synthesis of TD1024:** Glass vial (100 mL) was charged with **CuI** (70 mg; 0.37 mmol; 4.0 mol%) and [**Pd**(**PPh₃**)**₂Cl₂**] (125 mg; 0.18 mmol; 2.0 mol%) and magnetic stirrer and three times secured with argon. Solution of **Dimethyl 4-iodopyridine-2,6-dicarboxylate** (2.93 g; 9.13 mmol; 1.0 equiv.) in mixture of dry THF (40 ml), dry Toluene (40 ml) and dry DMF (2 mL) was subsequently added through septum followed by addition of **Ethynyltriisopropylsilane** (2.25 mL; 10.0 mmol; 1.1 equiv.) and **TEA** (3.80 mL; 27.3 mmol; 3.0 equiv.) after which the mixture changed color to pale red. The flask was left stirring under septum (but without external argon) for 20 h at RT. The resulting dark mixture was filtered through syringe microfilter (PTFE) and the filtrate was evaporated to dryness. Residue was dissolved in DCM (250 mL) and the resulting red solution was washed once with aq. solution of Na₂S₂O₃. The organic layer was separated, dried with Na₂SO₄, filtered through glass frit (S3) and evaporated to dryness. Residue was purified on flash chromatography (220 g SiO₂, 100% DCM to 20% EtOAc in DCM). Combined fractions with product were evaporated to dryness and further co-evaporated once with DCM. The residue was further dried on high vacuum overnight to give pre-purified product in the form of free base as orange oil. **Yield:** 3.16 g (92%; 1 step; based on **Dimethyl 4-iodopyridine-2,6-dicarboxylate). NMR** (**DMSO-d*₆***)**: ¹H** δ_{H} 0.92-1.27 *(i-Pr,* m, 21H); 3.92 (C*H*₃, s, 6H); 8.16 (*arom.,* s, 2H). **¹³C{¹H}** δ_{C} 10.6 (*i-Pr,* s); 18.4 (*i-Pr,* s); 52.9 (*C*H₃, s); 99.0 (C≡*C-*Si, s); 102.5 (C≡*C*-arom.; s); 129.3 (*arom.,* s); 132.7 (*arom.,* s); 148.4 (*arom.,* s); 164.0 (*C*O, s). **ESI-HRMS:** 376.1939 [M+H]⁺ (theor. [C₂₀H₃₀N₁O₄Si₁]⁺ = 376.1939). 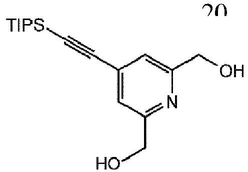 **Synthesis of TD808:** In a round-bottom glass flask (250 mL), **TD1024** (3.15 g; 8.39 mmol; 1.0 equiv.) was dissolved in a mixture of THF (60 mL) and MeOH (30 mL). Then, solid **NaBH₄** (2.55 g; 67.4 mmol; 8 equiv.) was added portion wise over the course of 1 h (during which hydrogen gas evolved intensively) and the color of the mixture changed to dark red. After the addition, the flask was further stirred 1 h at RT. The mixture was evaporated to dryness. Residue was resuspended in DCM (200 mL) and H₂O (200 mL) and transferred to a separatory funnel. After shaking, the bottom phase (as a suspension) was separated. Aqueous phase was further extracted with DCM (3 × 100 mL). Combined organic layers were diluted with EtOAc (500 mL). The resulting brown solution was then dried with anhydrous Na₂SO₄, filtered through glass frit (S3) and evaporated to dryness. The residue was further dried on high vacuum overnight to give product in the form of free base as pale brown solid. **Yield:** 2.61 g (97%; 1 step; based on **TD1024**)**. NMR** (**DMSO-d*₆***)**: ¹H** δ_{H} 1.04-1.17 (*i-Pr,* m, 21H); 4.51 (C*H*₂, d, 4H, ³*J*_{HH} = 6); 5.48 (O*H*, t, 2H, ³*J*_{HH} = 6); 7.31 (*arom.,* s, 2H). **¹³C{¹H}** δ_{C} 10.6 (*i-Pr,* s); 18.5 (*i-Pr,* s); 63.9 (*C*H2, s); 94.6 (C≡*C*-Si, s); 105.1 (C≡*C*-arom.; s); 119.8 (*arom.,* s); 130.8 (*arom.,* s); 161.9 (*arom.,* s). **ESI-HRMS:** 320.2036 [M+H]⁺ (theor. [C₁₈H₃₀N₁O₂]⁺ = 320.2040). 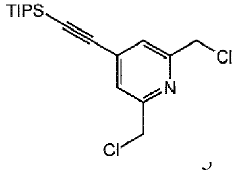 **Synthesis of TD815:** In a round-bottom glass flask (250 mL), pre-purified **TD808** (2.61 g; 8.17 mmol; 1.0 equiv.) was suspended in DCM (100 mL). Freshly prepared solution of **SOCl₂** (1.80 mL; 24.8 mmol; 3.0 equiv.) in DCM (10 mL) was then added and the open flask was stirred 1 h at RT. Dil. aq. solution of NaHCO₃ (100 mL) was then added and resulting biphasic mixture was vigorously stirred for additional 1 h at RT, during which bubbles of gas slowly evolved. Mixture was then transferred to a separatory funnel. After shaking, the bottom phase was separated. Aqueous phase was further extracted with DCM (3 × 75 mL). Combined organic layers were dried with anhydrous Na₂SO₄, filtered through glass frit (S3) and evaporated to dryness. The residue was further dried on high vacuum overnight to give product in the form of free base as yellow oil. **Yield:** 2.87 g (99%; 1 step; based on **TD808**). **NMR** (**DMSO-d*₆***)**: ¹H** δ_{H} 0.97-1.20 (*i-Pr,* m, 21H); 4.78 (C*H*₂, s, 4H); 7.59 (*arom.,* s, 2H). **¹³C{¹H}** δ_{C} 10.6 (*i-Pr,* s); 18.4 (*i-Pr,* s); 45.9 (*C*H₂, s); 96.6 (C≡*C*-Si, s); 103.5 (C≡*C*-arom.; s); 124.6 (*arom.,* s); 132.0 (*arom.,* s); 157.1 (*arom.,* s). **ESI-HRMS:** 356.1368 [M+H]⁺ (theor. [C₁₈H₂₈N₁Cl₂Si₁]⁺ = 356.1368). 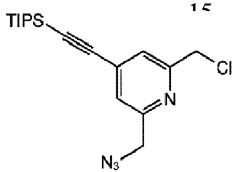 **Synthesis of TD817:** In a round-bottom glass flask (250 mL), **TD815** (2.86 g; 8.02 mmol; 1.2 equiv.) was dissolved in MeCN (130 mL) followed by addition of solid **NaN₃** (435 mg; 6.69 mmol; 1.0 equiv.) and dried **K₂CO₃** (920 mg; 6.67 mmol; 1.0 equiv.) and the resulting suspension was stirred 16 h at 80 °C. The mixture was filtered through syringe microfilter (PTFE) and the solid residue was washed with MeCN. Filtrate was evaporated to dryness and orange oily residue was purified by column chromatography (220 g SiO₂, 30% P.E. in DCM to 10% P.E. in DCM). Fractions with product were combined and evaporated to dryness. The residue was further dried on high vacuum 1 h to give product in the form of free base as faint yellow oil. **Yield:** 1.23 g (51%; 1 step; based on **NaN₃**)**. Recovery:** 709 mg of **TD815** (25% of the initial amount used). **NMR** (**DMSO-d*₆***)**: ¹H** δ_{H} 1.01-1.21 (*i-Pr,* m, 21H); 4.55 (C*H*₂, s, 2H); 4.78 (C*H*₂, s, 2H); 7.47 (*arom*., d, 1H, ⁴*J*_{HH} = 1); 7.58 (*arom*., d, 1H, ⁴*J*_{HH} = 1). **¹³C{¹H}** δ_{C} 10.6 (*i*-*Pr*, s); 18.4 (*i*-*Pr*, s); 46.0 (*C*H₂, s); 53.7 (*C*H₂, s); 96.5 (C≡*C*-Si, s); 103.7 (C≡*C*-arom.; s); 123.7 (*arom.,* s); 124.3 (*arom.,* s); 131.8 (*arom.,* s); 156.6 (*arom.,* s); 157.1 (*arom.,* s). **ESI-HRMS:** 363.1763 [M+H]⁺ (theor. [C₁₈H₂₈N₄Cl₁Si₁]⁺ = 363.1766). 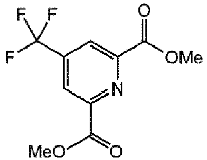 **Synthesis of TD1052:** In a glass vial (20 mL), **Dimethyl 4-iodopyridine-2,6-dicarboxylate** (500 mg; 1.56 mmol; 1.0 equiv.), **CuI** (600 mg; 3.15 mmol, 2.0 equiv.) and [(**dppf**)**PdCl₂**] (7.0 mg, 10 µmol; 0.6 mol%) were dissolved in dry DMF (8 mL) followed by addition of **Methyl 2,2-difluoro-2-(fluorosulfonyl)acetate** (600 mg; 3.12 mmol; 2.0 equiv.) in dry DMF (2 mL). The resulting dark mixture was stirred 16 h at 100 °C. After cooling down, the mixture was diluted with DCM (10 mL) and filtered through syringe microfilter (PTFE). The solids were further washed with DCM. Filtrate was further diluted with DCM (50 mL) and washed with dil. aq. NaHCO₃ (5 × 25 mL). Organic layer was dried with anhydrous Na₂SO₄, filtered through glass frit (S3) and evaporated to dryness. Residue was purified by column chromatography (120 g SiO₂, 100 % DCM to 15% EtOAc in DCM). Fractions with product were combined and evaporated to dryness. The residue was further dried on high vacuum overnight to give product in the form of free base as colorless solid. **Yield:** 366 mg (89%; 1 step; based on **Dimethyl 4-iodopyridine-2,6-dicarboxylate**). **NMR** (**DMSO-d*₆***)**: ¹H** δ_{H} 3.97 (C*H*₃, s, 6H); 8.49 (*arom.,* q, 2H, ⁴*J*_{HF} = 1). **¹⁹F{¹H}** δ_{F} -63.4 (s). **ESI-HRMS:** 264.0480 [M+H]⁺ (theor. [C₁₀H₉N₁O₄F₃]⁺ = 264.0478). 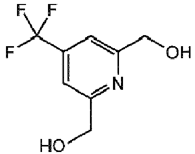 **Synthesis of TD1053:** In a round-bottom glass flask (100 mL), **TD1052** (365 mg; 1.39 mmol; 1.0 equiv.) was dissolved in a mixture of MeOH (5 mL) and THF (10 mL). The resulting colourless solution was cooled with an ice bath (~5 °C) followed by portion wise additions (over course of 30 min, with no stopper on the flask) of solid **NaBH₄** (420 mg; 11.1 mmol; 8.0 equiv.) during which hydrogen gas evolved intensively and the color of the mixture changed to red and orange. After the addition, the flask was allowed to warm up to RT and was further stirred 30 min at RT. Resulting yellow solution was evaporated to dryness. Residue was purified by column chromatography (120 g SiO₂, solid load technique, 100 % EtOAc to 15% MeOH in EtOAc). Fractions with product were combined and evaporated to dryness. The residue was further dried on high vacuum overnight to give product in the form of free base as yellow oil. **Yield:** 171 mg (60%; 1 step; based on **TD1052**)**. NMR** (**DMSO-d*₆***)**: ¹H** δ_{H} 4.62 (C*H*₂, s, 4H); 5.65 (O*H*, s, 2H); 7.60 (*arom.,* q, 2H, ⁴*J*_{HF} = 1). **¹⁹F{¹H}** δ_{F} -63.6 (s). **ESI-HRMS:** 208.0580 [M+H]⁺ (theor. [C₈H₉N₁O₂F₃]⁺ = 208.0580). 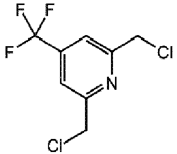 **Synthesis of TD1055:** In a round-bottom glass flask (100 mL), **TD1053** (169 mg; 816 µmol; 1.0 equiv.) was dissolved in DCM (20 mL). Freshly prepared solution of **SOCl₂** (237 µL; 3.26 mmol; 4.0 equiv.) in DCM (5 mL) was then added and the resulting mixture was stirred 16 h at RT. Dil. aq. solution of NaHCO₃ (25 mL) was then added and resulting biphasic mixture was vigorously stirred for additional 10 min at RT, during which bubbles of gas slowly evolved. Mixture was then transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as yellow oil. **Yield:** 168 mg (84%; 1 step; based on **TD1053**). **NMR** (**DMSO-d*₆***)**: ¹H** δ_{H} 4.90 (C*H*₂, s, 4H); 7.94 (*arom.,* s, 2H). **¹⁹F{¹H}** δ_{F} -63.4 (s). **APCI-HRMS:** 243.9902 [M+H]⁺ (theor. [C₈H₇N₁F₃Cl₂]⁺ = 243.9902). 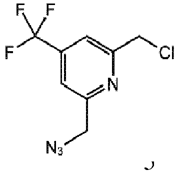 **Synthesis of TD1057:** In a glass vial (4 mL), **TD1055** (143 mg; 586 µmol; 1.8 equiv.) was dissolved in MeCN (3.5 mL) followed by addition of solid **NaN₃** (21.5 mg; 331 µmol; 1.0 equiv.) and dried **K₂CO₃** (46 mg; 333 µmol; 1.0 equiv.) and the resulting suspension was stirred 3 d at 70 °C. The mixture was filtered through syringe microfilter (PTFE) and the solid residue was washed with MeCN. Filtrate was evaporated to dryness and orange oily residue was purified by column chromatography (80 g SiO₂, 30% P.E. in DCM to 40% DCM in P.E.). Fractions with product were combined and evaporated to dryness. The residue was further dried on high vacuum 1 h to give product in the form of free base as colourless oil. **Yield:** 43.9 g (53%; 1 step; based on **NaN₃**). **Recovery:** 32.1 mg of **TD1055** (22% of the initial amount used). **NMR** (**DMSO-d*₆***): **¹H** δ_{H} 4.69 (C*H*₂, s, 2H); 4.90 (C*H*₂, s, 2H); 7.81 (*arom.,* d, 1H, ⁴*J*_{HH} = 1); 7.93 (*arom.,* d, 1H, ⁴*J*_{HH} = 1). **¹⁹F{¹H}** δ_{F} -63.4 (s). **APCI-HRMS:** 251.0306 [M+H]⁺ (theor. [C₈H₇N₄F₃Cl₁]⁺ = 251.0306). 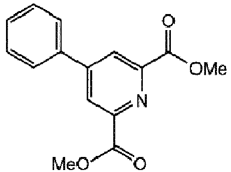 **Synthesis of TD549:** Pear-shape glass flask (250 mL) was charged with **Dimethyl 4-chlororidine-2,6-dicarboxylate** (5.00 g; 21.8 mmol; 1.0 equiv.), **Phenylboronic acid** (3.20 g; 26.2 mmol; 1.2 equiv.) and **XPhos Pd G2** (510 mg; 648 µmol; 3.0 mol%) and was then three times secured with argon. Under constant flow of argon was then added dry DMF (110 mL) through septum followed by addition of freshly dried (using heat gun under high vacuum) **Cs₂CO₃** (15.6 g; 47.9 mmol; 2.2 equiv.; the flask was briefly opened for addition). The mixture was then stirred 20 h under septum (but without external argon) at 80 °C. Resulting dark mixture was filtered through glass frit (S3) and the filtrate was poured to a stirred beaker with H₂O (500 mL). Precipitate was collected on glass frit (S2), washed with H₂O and further dried on high vacuum overnight to give product in the form of free base as off white solid. **Yield:** 3.01 g (51%; 1 step; based on **Dimethyl 4-chloropyridine-2,6-dicarboxylate**). **NMR** (**DMSO-d*₆***): **¹H** δ_{H} 3.95 (C*H*₃, s, 6H); 7.45-7.57 (*Ph,* m, 3H); 7.82-8.00 (*Ph,* m, 2H); 8.48 (*arom.,* s, 2H); **¹³C{¹H}** δ_{C} 52.7 (*C*H₃, s); 125.0 (*arom.,* s); 127.2 (*Ph.,* s); 129.5 (*Ph.,* s); 130.2 (*Ph.,* s); 135.5 (*Ph.,* s); 148.6 (*arom.,* s); 150.0 (*arom.,* s); 164.7 (CO., s). **ESI-HRMS:** 272.0916 [M+H]⁺ (theor. [C₁₅H₁₄N₁O₄]⁺ = 272.0917). 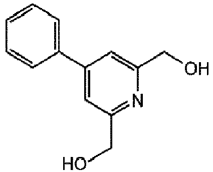 **Synthesis of TD563:** In a round-bottom glass flask (500 mL), **TD549** (2.98 g; 11.0 mmol; 1.0 equiv.) was dissolved in a mixture of MeOH (100 mL) and THF (100 mL). The resulting slightly yellow solution was cooled with an ice bath (~5 °C) followed by portion wise additions (over course of 30 min, with no stopper on the flask) of solid **NaBH₄** (2.90 g; 76.7 mmol; 9.0 equiv.) during which hydrogen gas evolved intensively and the color of the mixture changed to red and orange. After the addition, the flask was allowed to warm up to RT and was further stirred 30 min at RT. Resulting faint yellow opalescent solution was filtered through syringe microfilter (PTFE). The filtrate was evaporated to dryness and further co-evaporated with DCM (as a suspension). Residue was dissolved in a mixture of H₂O (300 mL) and DCM (300 mL) and transferred to a separatory funnel. After shaking, the bottom phase was separated. Aqueous phase was further extracted with DCM (8 × 75 mL). Combined organic layers were dried with anhydrous Na₂SO₄, filtered through glass frit (S3) and evaporated to dryness. The resulting crystalized residue was crushed and further dried on high vacuum overnight to give product in the form of free base as nearly colorless solid. **Yield:** 2.29 g (97%; 1 step; based on **TD549). NMR** (**DMSO-d*₆***): **¹H** δ_{H} 4.59 (C*H*₂, s, 4H); 5.45 (O*H*, s, 2H); 7.45-7.57 (*Ph,* m, 3H); 7.60 (*arom.,* s, 2H); 7.68-7.82 (*Ph,* m, 2H). **¹³C{¹H}** δ_{C} 64.2 (*C*H₂, s); 115.7 (*arom.,* s); 126.7 (*Ph,* s); 129.1 (*Ph,* s); 129.3 (*Ph,* s); 138.1 (*Ph,* s); 148.2 (*arom.,* s); 161.7 (*arom.,* s). **ESI-HRMS:** 216.1022 [M+H]⁺ (theor. [C₁₃H₁₄N₁O₂]⁺ = 216.1019). 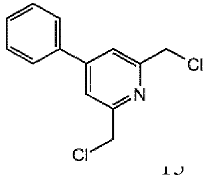 **Synthesis of TD564:** In a round-bottom glass flask (500 mL), **TD563** (2.25 g; 10.5 mmol; 1.0 equiv.) was dissolved (with gentle heating) in DCM (250 mL). Freshly prepared solution of **SOCl₂** (2.27 mL; 31.3 mmol; 3.0 equiv.) in DCM (10 mL) was then added, after which precipitate started to form. After 1 h of stirring at RT, all of the previously formed precipitate was dissolved again. Dil. aq. solution of NaHCO₃ (150 mL) was then added to the clear yellow solution and the resulting biphasic mixture was vigorously stirred for additional 1 h at RT, during which bubbles of gas slowly evolved. Mixture was then transferred to a separatory funnel. After shaking, the bottom phase was separated. Aqueous phase was further extracted with DCM (3 × 100 mL). Combined organic layers were dried with anhydrous Na₂SO₄, filtered through glass frit (S3) and evaporated to dryness. The residue was further dried on high vacuum overnight to give product in the form of free base as faint yellow solid. **Yield:** 2.59 g (98%; 1 step; based on **TD563). NMR** (**DMSO-d*₆***)**: ¹H** δ_{H} 4.88 (C*H*₂, s, 4H); 7.47-7.58 (*Ph,* m, 3H); 7.77-7.85 (*Ph,* m, 2H); 7.86 (*arom.,* s, 2H). **¹³C{¹H}** δ_{C} 46.6 (*C*H₂, s); 120.3 (*arom.,* s); 126.9 (*Ph,* s); 129.3 (*Ph,* s); 129.7 (*Ph,* s); 136.5 (*Ph,* s); 149.5 (*arom.,* s); 157.1 (*arom.,* s); 157.1 (*arom.,* s); **ESI-HRMS:** 252.0342 [M+H]⁺ (theor. [C₁₃H₁₂N₁Cl₂]⁺ = 252.0341).

**Synthesis of TD566:** In a pear-shaped glass flask (100 mL), **TD564** (1.60 g; 6.35 mmol; 1.3 equiv.) was 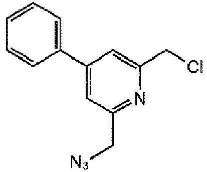 dissolved (with gentle heating) in MeCN (60 mL) followed by addition of solid **NaN₃** (320 mg; 4.92 mmol; 1.0 equiv.) and dried **K₂CO₃** (680 mg; 4.93 mmol; 1.0 equiv.) and the resulting suspension was stirred 24 h at 80 °C. The mixture was filtered on glass frit S3 and the solid residue was washed with MeCN. Filtrate was evaporated to dryness and the orange oily residue was purified by column chromatography (SiO₂, 15% P.E. in DCM to 100% DCM). Fractions with product were combined and evaporated to dryness. The residue was further dried 1 h on high vacuum to give product in the form of free base as white solid. **Yield:** 691 mg (54%; 1 step; based on **NaN₃**). **Recovery:** 613 mg of **TD564** (38% of the initial amount used). **NMR** (**DMSO-d*₆***)**: ¹H** δ_{H} 4.59 (C*H*₂, s, 2H); 4.84 (C*H*₂, s, 2H); 7.46-7.60 (*Ph,* m, 3H); 7.74 (*arom.,* d, 1H, ⁴*J*_{HH} = 2); 7.77-7.85 (*Ph,* m, 2H); 7.86 (*arom.,* d, 1H, ⁴*J*_{HH} = 2). **¹³C{¹H}** δ_{C} 46.7 (*C*H₂, s); 54.2 (*C*H₂, s); 119.4 (*arom.,* s); 120.0 (*arom.,* s); 126.9 (*Ph,* s); 129.3 (*Ph,* s); 129.7 (*Ph,* s); 136.6 (*Ph,* s); 149.3 (*arom.,* s); 156.6 (*arom.,* s); 157.1 (*arom.,* s). **ESI-HRMS:** 259.0747 [M+H]⁺ (theor. [C₁₃H₁₂N₄Cl₁]⁺ = 259.0745). 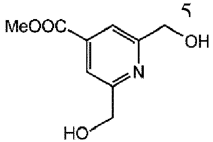 **Synthesis of TD1083:** In a round-bottom glass flask (100 mL), **methyl isonicotinate** (250 mg; 1.83 mmol; 1.0 equiv.) was dissolved in MeOH (10 mL) followed by addition of conc. **H₂SO₄** (25 µL). The resulting mixture was stirred 30 min at 55 °C. After cooling down, solution of (**NH₄**)**₂S₂O₈** (4.16 g; 18.2 mmol; 10 equiv.) in H₂O (10 mL) was added dropwise and the resulting mixture was then further stirred 16 h at 55 °C. Reaction was then carefully quenched by aq. NaHCO₃. Mixture was then transferred to a separatory funnel and extracted with EtOAc (5 × 50 mL). Combined organic layers were dried with anhydrous Na₂SO₄, filtered through glass frit (S3) and evaporated to dryness. Residue was purified by preparative HPLC (C18; H₂O-MeCN gradient with FA additive). Fractions with product were joined and directly lyophilized to give product in the form of free base as white fluffy solid. **Yield:** 99.5 mg (28%; 1 step; based on **methyl isonicotinate). NMR** (**DMSO-d*₆***)**: ¹H** δ_{H} 3.92 (C*H*₃, s, 3H); 4.60 (C*H*₂, d, 4H, ³*J*_{HH} = 6); 5.58 (O*H,* t, 2H, ³*J*_{HH} = 6); 7.80 (*arom.,* s, 2H). **ESI-HRMS:** 198.0759 [M+H]⁺ (theor. [C₉H₁₂N₁O₄]⁺ = 198.0761). **EA** (C₉H₁₁N₁O₄, *M*_{R} = 197.2): C 54.8 (54.8); H 5.6 (5.5); N 7.1 (7.0). 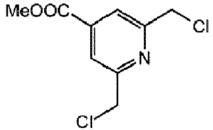 **Synthesis of TD1087:** In a round-bottom glass flask (50 mL), **TD1083** (96.2 mg; 488 µmol; 1.0 equiv.) was dissolved in DCM (20 mL) followed by addition of **SOCl₂** (106 µL; 1.46 mmol; 3.0 equiv.). The mixture was stirred 1 h at RT. Mixture was then quenched by addition of dil. aq. solution of NaHCO₃ (20 mL). The resulting biphasic mixture was vigorously stirred for additional 1 h at RT, during which bubbles of gas slowly evolved. Mixture was then transferred to a separatory funnel. After shaking, the bottom phase was separated. Aqueous phase was further extracted with DCM (3 × 30 mL). Combined organic layers were dried with anhydrous Na₂SO₄, filtered through glass frit (S3) and evaporated to dryness. The residue was further dried on high vacuum overnight (where product slowly crystallized) to give product in the form of free base as bright yellow solid. **Yield:** 112.8 mg (99%; 1 step; based on **TD1083). NMR** (**DMSO-d*₆***)**: ¹H** δ_{H} 3.92 (C*H*₃, s, 3H); 4.90 (C*H*₂, s, 4H); 7.98 (*arom.,* s, 2H). **ESI-HRMS:** 234.0085 [M+H]⁺ (theor. [C₉H₁₀N₁O₂Cl₂]⁺ = 234.0083). 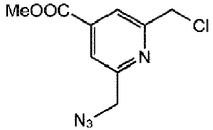 **Synthesis of TD1089:** In a glass vial (4 mL), **TD1087** (112.0 mg; 478 µmol; 1.2 equiv.) was dissolved in MeCN (2 mL) followed by addition of solid **NaN₃** (26.0 mg; 400 µmol; 1.0 equiv.) and dried **K₂CO₃** (55.0 mg; 400 µmol; 1.0 equiv.) and the resulting suspension was stirred 2 h at 70 °C. The mixture was filtered on through syringe microfilter (PTFE) and the solid residue was washed with MeCN. Filtrate was evaporated to dryness and oily residue was purified by column chromatography (SiO₂, DCM to 10% EtOAc in DCM). Fractions with product were combined and evaporated to dryness. The residue was further dried on high vacuum overnight to give pre-purified product (containing ~20% of bis azide byproduct) in the form of free base as yellow oil. **Yield:** 49.6 mg. **ESI-HRMS:** 241.0490 [M+H]⁺ (theor. [C₉H₁₀N₄O₂Cl₁]⁺ = 241.0487). Part of **TD1089** obtained in this way was directly used for **TD1092** without further purification or characterization. 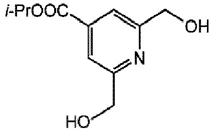 **Synthesis of TD1101:** In a round-bottom glass flask (100 mL), **isopropyl isonicotinate** (600 mg; 3.63 mmol; 1.0 equiv.) was dissolved in MeOH (20 mL) followed by addition of conc. **H₂SO₄** (50 µL). Then, solution of (**NH₄**)**₂S₂O₈** (8.32 g; 36.5 mmol; 10 equiv.) in H₂O (20 mL) was added dropwise and the resulting mixture was further stirred 16 h at 70 °C. Reaction was then carefully neutralized by aq. NaHCO₃. Mixture was then transferred to a separatory funnel and extracted with EtOAc (5 × 50 mL). Combined organic layers were dried with anhydrous Na₂SO₄, filtered through glass frit (S3) and evaporated to dryness. Residue was purified by preparative HPLC (C18; H₂O-MeCN gradient with FA additive). Fractions with product were joined and directly lyophilized to give product in the form of free base as white fluffy solid. **Yield:** 284 mg (35%; 1 step; based on **isopropyl isonicotinate**)**. NMR** (**DMSO-d*₆***)**: ¹H** δ_{H} 1.34 (C*H*₃, d, 6H, ³*J*_{HH} = 6); 4.59 (C*H*₂, d, 4H, ³*J*_{HH} = 6); 5.19 (C*H*, hept, 1H, ³*J*_{HH}= 6); 5.57 (O*H,* t, 2H, ³*J*_{HH} = 6); 7.78 (*arom.,* s, 2H). **ESI-HRMS:** 226.1076 [M+H]⁺ (theor. [C₁₁H₁₆N₁O₄]⁺ = 226.1074). 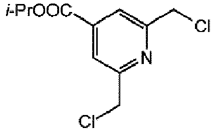 **Synthesis of TD1109:** In a round-bottom glass flask (100 mL), **TD1101** (283 mg; 1.26 mmol; 1.0 equiv.) was dissolved in DCM (40 mL) followed by addition of **SOCl₂** (275 µL; 3.79 mmol; 3.0 equiv.). The mixture was stirred 1 h at RT. Mixture was then quenched by addition of dil. aq. solution of NaHCO₃ (40 mL). The resulting biphasic mixture was vigorously stirred for additional 1 h at RT, during which bubbles of gas slowly evolved. Mixture was then transferred to a separatory funnel. After shaking, the bottom phase was separated. Aqueous phase was further extracted with DCM (3 × 50 mL). Combined organic layers were dried with anhydrous Na₂SO₄, filtered through glass frit (S3) and evaporated to dryness. The residue was further dried on high vacuum overnight (where product slowly crystallized) to give product in the form of free base as pale yellow solid. **Yield:** 322 mg (98%; 1 step; based on **TD1101**). **NMR** (**DMSO-d*₆***)**: ¹H** δ_{H} 1.35 (C*H*₃, d, 6H, ³*J*_{HH} = 6); 4.90 (C*H*₂, s, 4H); 5.20 (C*H*, hept, 1H, ³*J*_{HH} = 6); 7.96 (*arom.,* s, 2H). **ESI-HRMS:** 262.0399 [M+H]⁺ (theor. [C₁₁H₁₄N₁O₂Cl₂]⁺ = 262.0396). 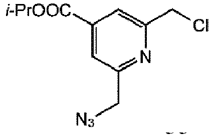 **Synthesis of TD1112:** In a glass vial (20 mL), **TD1109** (321 mg; 1.23 mmol; 1.2 equiv.) was dissolved in MeCN (6 mL) followed by addition of solid **NaN₃** (66.5 mg; 1.02 mmol; 1.0 equiv.) and dried **K₂CO₃** (141 mg; 1.02 mmol; 1.0 equiv.) and the resulting suspension was stirred 16 h at 70 °C. The mixture was filtered on through syringe microfilter (PTFE) and the solid residue was washed with MeCN. Filtrate was evaporated to dryness and oily residue was purified by column chromatography (SiO₂, DCM to 3% EtOAc in DCM). Fractions with product were combined and evaporated to dryness. The residue was further dried on high vacuum overnight to give product in the form of free base as colourless oil. **Yield:** 116 mg (42%; 1 step; based on **NaN₃**). **Recovery:** 124 mg of **TD1109** (39% of the initial amount used). **NMR** (**DMSO-d*₆***): **¹H** δ_{H} 1.35 (C*H*₃, d, 6H, ³*J*_{HH} = 6); 4.67 (C*H*₂, s, 2H); 4.89 (C*H*₂, s, 4H); 5.19 (C*H*, hept, 1H, ³*J*_{HH} = 6); 7.83 (*arom.,* d, 1H, ⁴*J*_{HH} = 1).7.95 (*arom.,* d, 1H, ⁴*J*_{HH} = 1). **ESI-HRMS:** 269.0800 [M+H]⁺ (theor. [C₁₁H₁₄N₄O₂Cl₁]⁺ = 269.0800). 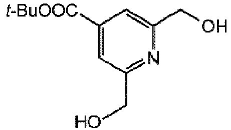 **Synthesis of TD1119:** In a glass vial (40 mL), ***tert*-butyl isonicotinate** (486 mg; 2.71 mmol; 1.0 equiv.) was dissolved in MeOH (15 mL) followed by addition of conc. **H₂SO₄** (40 µL). Then, solution of (**NH₄**)**₂S₂O₈** (6.20 g; 27.2 mmol; 10 equiv.) in H₂O (15 mL) was added dropwise and the resulting mixture was further stirred 30 min at 80 °C. Reaction was then carefully neutralized by aq. NaHCO₃. Mixture was then transferred to a separatory funnel and extracted with EtOAc (5 × 30 mL). Combined organic layers were dried with anhydrous Na₂SO₄, filtered through glass frit (S3) and evaporated to dryness. Residue was purified by preparative HPLC (C18; H₂O-MeCN gradient with FA additive). Fractions with product were joined and directly lyophilized to give product in the form of free base as white fluffy solid. **Yield:** 161 mg (25%; 1 step; based on **tert-butyl isonicotinate**). **NMR** (**DMSO-d*₆***)**: ¹H** δ_{H} 1.57 (C*H*₃, s, 9H); 4.58 (C*H*₂, d, 4H, ³*J*_{HH} = 4); 5.54 (O*H,* t, 2H, ³*J*_{HH} = 4); 7.73 (*arom.,* s, 2H). **ESI-HRMS:** 240.1230 [M+H]⁺ (theor. [C₁₂H₁₈N₁O₄]⁺ = 240.1230). 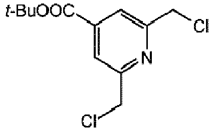 **Synthesis of TD1129:** In a round-bottom glass flask (100 mL), **TD1119** (159 mg; 664 µmol; 1.0 equiv.) was dissolved in DCM (20 mL) followed by addition of **SOCl₂** (145 µL; 2.00 mmol; 3.0 equiv.). The mixture was stirred 1 h at RT. Mixture was then quenched by addition of dil. aq. solution of NaHCO₃ (20 mL). The resulting biphasic mixture was vigorously stirred for additional 1 h at RT, during which bubbles of gas slowly evolved. Mixture was then transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight (where product slowly crystallized) to give product in the form of free base as faint yellow solid. **Yield:** 175 mg (95%; 1 step; based on **TD1119**). **NMR** (**DMSO-d*₆***)**: ¹H** δ_{H} 1.57 (C*H*₃, s, 9H); 4.89 (C*H*₂, s, 4H); 7.91 (*arom.,* s, 2H). **ESI-HRMS:** 276.0552 [M+H]⁺ (theor. [C₁₂H₁₆N₁O₂Cl₂]⁺ = 276.0553). 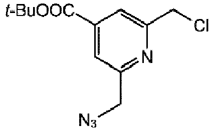 **Synthesis of TD1130:** In a glass vial (4 mL), **TD1129** (174.0 mg; 630 µmol; 1.2 equiv.) was dissolved in MeCN (3 mL) followed by addition of solid **NaN₃** (34.0 mg; 523 µmol; 1.0 equiv.) and dried **K₂CO₃** (72.0 mg; 522 µmol; 1.0 equiv.) and the resulting suspension was stirred 6 h at 70 °C. The mixture was filtered on through syringe microfilter (PTFE) and the solid residue was washed with MeCN. Filtrate was evaporated to dryness and oily residue was purified by column chromatography (SiO₂, DCM to 3% EtOAc in DCM). Fractions with product were combined and evaporated to dryness. The residue was further dried on high vacuum overnight to give product in the form of free base as colourless oil. **Yield:** 58.2 mg (39%; 1 step; based on **NaN₃**). **Recovery:** 90.5 mg of **TD1129** (52% of the initial amount used). **NMR** (**DMSO-d*₆***)**: ¹H** δ_{H} 1.57 (C*H*₃, s, 9H); 4.66 (C*H*₂, s, 2H); 4.89 (C*H*₂, s, 2H); 7.79 (*arom.,* d, 1H, ⁴*J*_{HH} = 2).7.91 (*arom.,* d, 1H, ⁴*J*_{HH} = 2). **ESI-HRMS:** 305.0772 [M+Na]⁺ (theor. [C₁₂H₁₅N₄O₂Cl₁Na₁]⁺ = 305.0776). 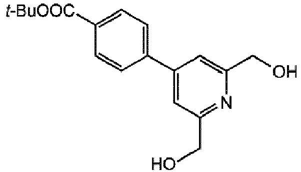 **Synthesis of TD725:** Glass vial (40 mL) was charged with **TD726** (628 mg; 3.62 mmol; 1.0 equiv.), (**4-**(**tert-Butoxycarbonyl**)**phenyl**)**boronic acid** (880 mg; 3.96 mmol; 1.1 equiv.) and **XPhos Pd G2** (57 mg; 7.2 µmol; 2.0 mol%) and was then three times secured with argon. Under constant flow of argon was then added dry *1,4*-Dioxane (16 mL) through septum followed by addition of freshly prepared (and briefly washed with argon prior addition) solution of **K₃PO₄·**H₂O (920 mg; 4.00 mmol; 1.1 equiv.) in H₂O (8 mL). The mixture was then stirred 16 h under septum (but without external argon) at 80 °C. Resulting dark mixture was transferred to a separatory funnel and diluted with EtOAc (40 mL) and H₂O (50 ml). After shaking, upper layer was separated and aqueous layer was further extracted with EtOAc (5 × 30 mL). Combined organic layers were dried with anhydrous Na₂SO₄, filtered through glass frit (S3) and evaporated to dryness. Residue was purified on flash chromatography (120 g SiO₂, 100% EtOAc to 30% MeOH in EtOAc). Combined fractions with product were evaporated to dryness and further co-evaporated once with DCM. The residue was further dried on high vacuum overnight to give pre-purified product in the form of free base as off-white powder. **Yield:** 1.04 g. **NMR** (**DMSO-d*₆***)**: ¹H** δ_{H} 1.57 (C*H*₃, s, 9H); 4.61 (C*H*₂, d, 4H, ³*J*_{HH} = 6); 5.48 (O*H*, t, 2H, ³*J*_{HH} = 6); 7.64 (*arom.,* s, 2H); 7.88 (*arom.,* dm, 2H, ³*J*_{HH} = 9); 8.04 (*arom.,* dm, 2H, ³*J*_{HH} = 9). **¹³C{¹H}** δ_{C} 27.8 (*C*H₃, s); 64.2 (*C*H₂, s); 81.0 (*C*-CH₃, s); 115.8 (*arom.,* s); 127.0 (*arom.,* s); 129.9 (*arom.,* s); 131.6 (*arom.,* s); 142.2 (*arom.,* s); 147.0 (*arom.,* s); 162.0 (*arom.,* s); 164.6 (*C*O, s). **ESI-HRMS:** 316.1539 [M+H]⁺ (theor. [C₁₈H₂₂N₁O₄]⁺ = 316.1543). 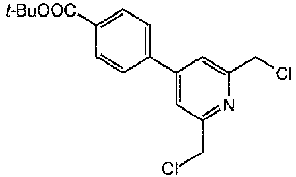 **Synthesis of TD730:** In a round-bottom glass flask (250 mL), pre-purified **TD725** (1.04 g; ≤3.30 mmol; 1.0 equiv.) was suspended in DCM (35 mL). Freshly prepared solution of **SOCl₂** (721 µL; 9.93 mmol; ≥3.0 equiv.) in DCM (5 mL) was then added. Clear solution was quickly formed and the open flask was stirred 1 h at RT. Mixture was then quenched by addition of dil. aq. solution of NaHCO₃ (60 mL). The resulting biphasic mixture was vigorously stirred for additional 1 h at RT, during which bubbles of gas slowly evolved. Mixture was then transferred to a separatory funnel. After shaking, the bottom phase was separated. Aqueous phase was further extracted with DCM (5 × 30 mL). Combined organic layers were dried with anhydrous Na₂SO₄, filtered through glass frit (S3) and evaporated to dryness. The residue was purified on flash chromatography (120 g SiO₂, 100% DCM to 20% EtOAc in DCM). Combined fractions with product were evaporated to dryness and further co-evaporated once with DCM. The residual faint yellow oil slowly crystallized. The solid was crushed and further dried on high vacuum overnight to give product in the form of free base as off-white powder. **Yield:** 839 mg (66%; 2 steps; based on **TD726**). **NMR** (**DMSO-d*₆***)**: ¹H** δ_{H} 1.58 (C*H*₃, s, 9H); 4.86 (C*H*₂, s, 4H); 7.92 (*arom.,* s, 2H); 7.94 (*arom.,* dm, 2H, ³*J*_{HH} = 9); 8.05 (*arom.,* dm, 2H, ³*J*_{HH} = 9). **¹³C{¹H}** δ_{C} 27.8 (*C*H₃, s); 46.5 (*C*H₂, s); 81.2 (*C*-CH₃, s); 120.6 (*arom.,* s); 127.2 (*arom.,* s); 129.9 (*arom.,* s); 132.1 (*arom.,* s); 140.6 (*arom.,* s); 148.3 (*arom.,* s); 157.3 (*arom.,* s); 164.5 (*C*O, s). **ESI-HRMS:** 352.0866 [M+H]⁺ (theor. [C₁₈H₂₀N₁O₂Cl₂]⁺ = 352.0866). 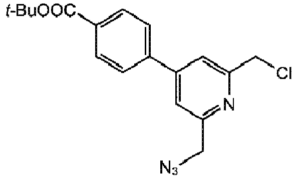 **Synthesis of TD733:** In a glass vial (20 mL), **TD730** (837 mg; 2.38 mmol; 1.3 equiv.) was dissolved in MeCN (18 mL) followed by addition of solid **NaN₃** (119 mg; 1.83 mmol; 1.0 equiv.) and dried **K₂CO₃** (253 mg; 1.83 mmol; 1.0 equiv.) and the resulting suspension was stirred 24 h at 80 °C. The mixture was filtered on through syringe microfilter (PTFE) and the solid residue was washed with MeCN. Filtrate was evaporated to dryness and oily residue was purified by column chromatography (SiO₂, 20% Pentane in DCM to 1% EtOAc in DCM). Fractions with product were combined and evaporated to dryness. The resulting nearly colorless oil was further dried on high vacuum overnight (where product slowly crystallized) to give product in the form of free base as colorless solid. **Yield:** 230 mg (35%; 1 step; based on **NaN₃**). **Recovery:** 442 mg of **TD730** (53% of the initial amount used). **NMR** (**DMSO-d*₆***)**: ¹H** δ_{H} 1.57 (C*H*₃, s, 9H); 4.62 (C*H*₂, s, 2H); 4.86 (C*H*₂, s, 2H); 7.80 (*arom.,* d, 1H, ⁴*J*_{HH} = 2); 7.91 (*arom.,* d, 1H, ⁴*J*_{HH} = 2); 7.94 (*arom.,* dm, 2H, ³*J*_{HH} = 9); 8.05 (*arom.,* dm, 2H, ³*J*_{HH} = 9). **¹³C{¹H}** δ_{C} 27.8 (*C*H₃, s); 46.6 (*C*H₂, s); 54.2 (*C*H₂, s); 81.2 (*C*-CH₃, s); 119.6 (*arom.,* s); 120.3 (*arom.,* s); 127.2 (*arom.,* s); 129.9 (*arom.,* s); 132.1 (*arom.,* s); 140.7 (*arom.,* s); 148.1 (*arom.,* s); 156.8 (*arom.,* s); 157.3 (*arom.,* s); 164.5 (CO, s). **ESI-HRMS:** 359.1270 [M+H]⁺ (theor. [C₁₈H₂₀N₄O₂Cl₁]⁺ = 359.1269).

### Example 3: Synthesis of protected macrocyclic intermediates

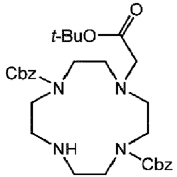

**Synthesis of TD680:** In a pear-shape glass flask (250 mL), **Cbz2cyclen** (free base; 1.54 g; 3.50 mmol; 1.6 equiv.) was dissolved in MeCN (100 mL) followed by addition of dried **K₂CO₃** (300 mg; 2.17 mmol; 1.0 equiv.). To the vigorously stirred reaction mixture, solution of ***tert*-Butyl bromoacetate** (427 mg; 2.19 mmol; 1.0 equiv.) in dry MeCN (25 mL) was added dropwise (over the course of 2 h). Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (150 mL) and H₂O (150 mL) and transferred to a separatory funnel. After shaking, the bottom phase was separated. Aqueous phase was further extracted with DCM (3 × 75 mL). Combined organic layers were dried with anhydrous Na₂SO₄, filtered through glass frit (S3) and evaporated to dryness. The residue was further dried on high vacuum overnight to give product in the form of free base as nearly colorless oil. **Yield:** 624 mg (51%; 1 step; based on ***tert*-Butyl bromoacetate**). **NMR** (**CD₃CN**): **¹H** δ_{H} 1.32-1.49 (C*H*₃, m, 9H); 2.57-2.76 (*mc,* m, 4H); 2.81-2.98 (*mc,* m, 4H); 3.23-3.64 (*mc,* C*H*₂-CO, m, 8+2H); 5.00-5.15 (C*H*₂-Ph, m, 4H); 7.19-7.48 (*Ph,* m, 10H). **ESI-HRMS:** 555.3171 [M+H]⁺ (theor. [C₃₀H₄₃N₄O₆]⁺ = 555.3177). 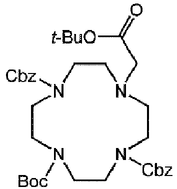 **Synthesis of TD686:** In a glass vial (4 mL), **TD680** (150 mg; 270 µmol; 1.0 equiv.) was dissolved in MeCN (3 mL) followed by addition of **Boc₂O** (2.0 M solution in THF; 200 µL; 400 µmol; 1.5 equiv.). Resulting mixture was stirred 16 h at RT. Reaction mixture was directly purified by preparative HPLC (C18; H₂O-MeCN gradient with FA additive). Fractions with product were combined, immediately neutralized with dil. aq. NaHCO₃ and evaporated to dryness. Residue was dissolved in DCM (50 mL) and H₂O (50 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as colorless oil. **Yield:** 170 mg (96%; 1 step; based on **TD680**). **NMR** (**CD₃CN**): **¹H** δ_{H} 1.17-1.56 (C*H*₃, m, 18H); 2.62-2.99 (*mc,* m, 4H); 3.10-3.53 (*mc,* C*H*₂-CO, m, 12+2H); 4.96-5.16 (C*H*₂-Ph, m, 4H); 7.20-7.47 (*Ph,* m, 10H). **ESI-HRMS:** 655.3691 [M+H]⁺ (theor. [C₃₅H₅₁N₄O₈]⁺ = 655.3701). 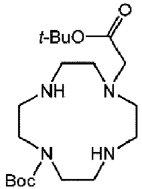 **Synthesis of TD691:** Pear-shape glass flask (25 mL) was charged with **TD686** (170 mg; 260 µmol) and magnetic stirrer and three times secured with argon. Solid **Pd@C** (17 mg) was then added followed by another securing with argon (three times). Under constant flow of argon was then added MeOH (10 mL) through septum. Argon input was then removed and **H₂ gas** (from balloon) was allowed to bubble through the mixture for 30 min at RT. Catalyst was then filtered off using syringe microfilter (PTFE; filter was further washed with MeOH). Filtrate was evaporated to dryness and once co-evaporated with DCM. The residue was further dried on high vacuum overnight to give product in the form of free base as colorless oil. **Yield:** 100 mg (≥99%; 1 step; based on **TD686**). **ESI-MS** (LC-MS): 387.3 [M+H]⁺ (theor. [C₁₉H₃₉N₄O₄]⁺ = 387.3). All of **TD691** was directly used for **TD692** without further characterization. 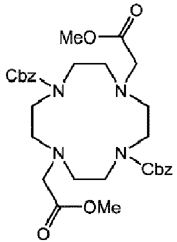 **Synthesis of TD1106:** In a pear-shape glass flask (50 mL), **Cbz2cyclen** (free base; 500 mg; 1.14 mmol; 1.0 equiv.) was dissolved in MeCN (10 mL) followed by addition of dried **K₂CO₃** (870 mg; 5.69 mmol; 5.0 equiv.) and **Methyl bromoacetate** (235 µL; 2.51 mmol; 2.2 equiv.) in MeCN (5 mL). The resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (50 mL) and H₂O (50 mL) and transferred to a separatory funnel. After shaking, the bottom phase was separated. Aqueous phase was further extracted with DCM (3 × 50 mL). Combined organic layers were dried with anhydrous Na₂SO₄, filtered through glass frit (S3) and evaporated to dryness. The residue was further dried on high vacuum overnight to give product in the form of free base as colorless oil. **Yield:** 422 mg (64%; 1 step; based on **Cbz2cyclen). NMR** (**CD₃CN**)**: ¹H** δ_{H} 2.63-2.88 (*mc,* m, 8H); 3.19-3.49 (*mc,* C*H*₂-CO, m, 8+4H); 3.59 (C*H*₃, s, 6H); 5.07 (C*H*₂-Ph, s, 4H); 7.21-7.41 (*Ph,* m, 10H). **ESI-LCMS:** 585.3 [M+H]⁺ (theor. [C₃₀H₄₁N₄O₈]⁺ = 585.3). 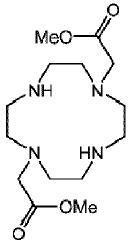 **Synthesis of TD1116:** Pear-shape glass flask (50 mL) was charged with **Pd@C** (42 mg) and magnetic stirrer and three times secured with argon. Under constant flow of argon was then added solution of **TD1106** (418 mg; 715 µmol) in MeOH (25 mL) through septum. Argon input was then removed and **H₂ gas** (from balloon) was allowed to bubble through the mixture for 1 h at RT. Catalyst was then filtered off using syringe microfilter (PTFE; filter was further washed with MeOH). Filtrate was evaporated to dryness and once co-evaporated with DCM. The residue was further dried on high vacuum overnight to give product in the form of free base as colorless oil that crystallized on standing. **Yield:** 223 mg (99%; 1 step; based on **TD1106**). **NMR** (**CD₃CN**)**: ¹H** δ_{H} 2.48-2.57 (*mc,* m, 8H); 2.63-2.74 (*mc,* m, 8H); 3.36 (C*H*₂-CO, s, 4H); 3.64 (C*H*₃, s, 6H). **ESI-MS** (LC-MS): 317.2 [M+H]⁺ (theor. [C₁₄H₂₉N₄O₄]⁺ = 317.2). 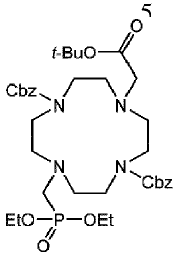 **Synthesis of TD687:** In a glass vial (2 mL), **TD680** (150 mg; 270 µmol; 1.0 equiv.) and **P**(**OEt**)**₃** (232 µL; 1.35 mmol; 5.0 equiv.) were mixed together followed by addition of solid (**CH₂O**)***ₙ*** (12 mg; 400 µmol; 1.5 equiv.). The resulting suspension was stirred 3 d at RT. Reaction mixture was then diluted with MeCN (700 µL) and filtered through syringe microfilter (PTFE). Filter was further washed with MeCN. Filtrate was then directly purified by preparative HPLC (C18; H₂O-MeCN gradient with FA additive). Fractions with product were combined, immediately neutralized with dil. aq. NaHCO₃ and evaporated to dryness. Residue was dissolved in DCM (25 mL) and H₂O (25 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as slightly yellow oil. **Yield:** 167 mg (87%; 1 step; based on **TD680**). **ESI-MS** (LC-MS): 705.4 [M+H]⁺ (theor. [C₃₅H₅₄N₄O₉P₁]⁺ = 705.4). All of **TD687** was directly used for **TD695** without further characterization. 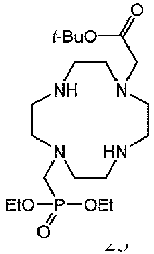 **Synthesis of TD695:** Pear-shape glass flask (25 mL) was charged with **TD687** (167 mg; 237 µmol) and magnetic stirrer and three times secured with argon. Solid **Pd@C** (17 mg) was then added followed by another securing with argon (three times). Under constant flow of argon was then added MeOH (10 mL) through septum. Argon input was then removed and **H₂ gas** (from balloon) was allowed to bubble through the mixture for 30 min at RT. The reaction mixture was further stirred under hydrogen atmosphere (from balloon) 16 h at RT. Catalyst was then filtered off using syringe microfilter (PTFE; filter was further washed with MeOH). Filtrate was evaporated to dryness and once co-evaporated with DCM. The residue was further dried on high vacuum overnight to give product in the form of free base as slightly yellow oil. **Yield:** 100 mg (97%; 1 step; based on **TD686**). **ESI-MS** (LC-MS): 437.2 [M+H]⁺ (theor. [C₁₉H₄₁N₄O₅P₁]⁺ = 437.3). All of **TD695** was directly used for **TD700** without further characterization. 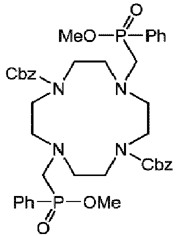 **Synthesis of TD913:** In a glass vial (20 mL), **Cbz2cyclen** (free base; 242 mg; 549 µmol; 1.0 equiv.) was dissolved in MeCN (12 mL) followed by addition of solid **(CH₂O)*ₙ*** (50 mg; 1.67 mmol; 3.0 equiv.) and **PhP(OMe)₂** (350 µL; 2.2 mmol; 4.0 equiv.). The resulting suspension was stirred 24 h at 80°C. Reaction mixture was then filtered through syringe microfilter (PTFE) and the filtrate was evaporated to dryness. Residue was purified by preparative HPLC (C18; H₂O-MeCN gradient with TFA additive). Fractions with product were combined, neutralized with dil. aq. NaHCO₃ and evaporated to dryness. Residue was dissolved in DCM (75 mL) and H₂O (75 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as nearly colourless oil. **Yield:** 259 mg (61%; 1 step; based on **Cbz2cyclen). NMR (CD₃CN): ¹H** δ_{H} 2.30-3.38 (*mc,* C*H*₂-P, m, 16+4H); 3.47 (C*H*₃, d, 6H, ³*J*_{HP} = 11); 5.00-5.12 (C*H*₂-Ph, m, 4H); 7.27-7.40 *(Ph,* m, 10H); 7.40-7.51 *(Ph,* m, 4H); 7.51-7.60 *(Ph,* m, 2H); 7.60-7.82 *(Ph,* m, 4H). **³¹P** δ_{P} 42.8 (m). **ESI-HRMS:** 777.3178 [M+H]⁺ (theor. [C₄₀H₅₁N₄O₈P_{2]}+ = 777.3177). 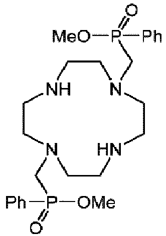 **Synthesis of TD910:** Pear-shape glass flask (50 mL) was charged with **TD913** (250 mg; 322 µmol) and magnetic stirrer and three times secured with argon. Solid **Pd@C** (50 mg) was then added followed by another securing with argon (three times). Under constant flow of argon was then added MeOH (25 mL) through septum. Argon input was then removed and **H₂ gas** (from balloon) was allowed to bubble through the mixture for 2 h at RT. Catalyst was then filtered off using syringe microfilter (PTFE; filter was further washed with MeOH). Filtrate was evaporated to dryness and once co-evaporated with DCM. The residue was further dried on high vacuum overnight to give product in the form of free base as nearly colourless oil. **Yield:** 161 mg (98%; 1 step; based on **TD913). NMR (CD₃CN): ¹H** δ_{H} 2.28-3.14 (*mc*,C*H*₂-P, m, 16+4H); 3.53 (C*H*₃, d, 3H, ³*J*_{HP} = 11); 3.54 (C*H*₃, d, 3H, ³*J*_{HP} = 11); 7.46-7.63 *(Ph,* m, 6H); 7.71-7.83 *(Ph,* m, 4H). **³¹P** δ_{P} 43.3 (m). **ESI-HRMS:** 509.2438 [M+H]⁺ (theor. [C₂₄H₃₉N₄O₄P₂]⁺ = 509.2441). 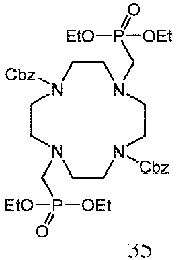 **Synthesis of TD571:** In a glass vial (4 mL), **Cbz2cyclen** (free base; 1.00 g; 2.27 mmol; 1.0 equiv.) and **P(OEt)₃** (2.00 mL; 11.7 mmol; 5.1 equiv.) were mixed together followed by addition of solid **(CH₂O)*ₙ*** (164 mg; 5.47 mmol; 2.4 equiv.). The resulting suspension was stirred 24 h at RT. Reaction mixture was then evaporated to dryness and the residue was purified by column chromatography (SiO₂; 80 g; DCM-MeOH-aq. NH₃ 150:10:1). Fractions with product were combined and evaporated to dryness. The residue was further dried on high vacuum overnight to give product in the form of free base as slightly yellow oil. **Yield:** 1.29 g (77%; 1 step; based on **Cbz2cyclen). ESI-MS** (LC-MS): 741.3 [M+H]⁺ (theor. [C₃₄H₅₅N₄O₁₀P₂]⁺ = 741.3). All of **TD571** was directly used for **TD573** without further characterization. 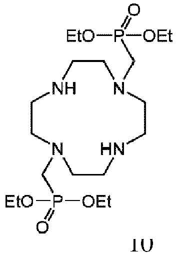 **Synthesis of TD573:** Pear-shape glass flask (100 mL) was charged with **TD571** (1.29 g; 1.74 mmol) and magnetic stirrer and three times secured with argon. Solid **Pd@C** (129 mg) was then added followed by another securing with argon (three times). Under constant flow of argon was then added EtOH (96%, 70 mL) through septum. Argon input was then removed and **H₂ gas** (from balloon) was allowed to bubble through the mixture for 30 min at RT. The reaction mixture was further stirred under hydrogen atmosphere (from balloon) 16 h at RT. Catalyst was then filtered off using syringe microfilter (PTFE; filter was further washed with EtOH). Filtrate was evaporated to dryness and once co-evaporated with DCM. The residue was further dried on high vacuum overnight to give product in the form of free base as slightly yellow oil. **Yield:** 776 mg (95%; 1 step; based on **TD571). ESI-MS** (LC-MS): 473.2 [M+H]⁺ (theor. [C₁₈H₄₃N₄O₄P₂]⁺ = 473.3). Major portion of **TD573** was directly used for **TD579** without further characterization. 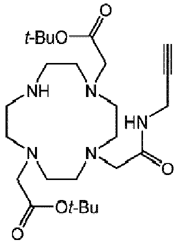 **Synthesis of TD423:** In a pear-shape glass flask (25 mL), **tBuDO2A** (free base; 410 mg; 1.03 mmol; 1.4 equiv.) was dissolved in dry MeCN (8 mL) followed by addition of **Cs₂CO₃** (840 mg; 2.58 mmol; 3.4 equiv.) and of **KI** (172 mg; 1.04 mmol; 1.4 equiv.). Solution of **2-Chloro-*N*-(prop-2-yn-1-yl)acetamide** (100 mg; 760 µmol; 1.0 equiv.) in dry MeCN (2 mL) was added and the resulting mixture was stirred 3 d at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (100 mL) and H₂O (100 mL) and transferred to a separatory funnel. After shaking, the bottom phase was separated. Aqueous phase was further extracted with DCM (3 × 50 mL). Combined organic layers were dried with anhydrous Na₂SO₄, filtered through glass frit (S3) and evaporated to dryness to give prepurified product in the form of free base as yellow oil. **Yield:** 180 mg (~75% of **TD423** in a mixture with ~25% of bis(substituted) by-product). **ESI-MS** (LC-MS): 496.4 [M+H]⁺ (theor. [C₂₅H₄₆N₅O₅]⁺ = 496.3). All of **TD423** was directly used for **TD425** without further purification and characterization. 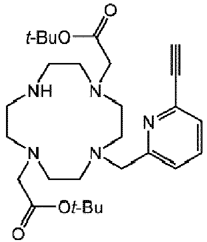 **Synthesis of TD635:** In a pear-shape glass flask (250 mL), **tBuDO2A** (free base; 1.21 g; 3.02 mmol; 2.3 equiv.) was dissolved in MeCN (150 mL). To the vigorously stirred reaction mixture, solution of **TD558** (1202 mg; 1.33 mmol; 1.0 equiv.) in MeCN (100 mL) was added dropwise (over the course of 2 h). Resulting mixture was further stirred 2 d at RT after which was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (100 mL) and H₂O (100 mL) and transferred to a separatory funnel. After shaking, the bottom phase was separated. Aqueous phase was further extracted with DCM (4 × 50 mL). Combined organic layers were dried with anhydrous Na₂SO₄, filtered through glass frit (S3) and evaporated to dryness. The residue was further dried on high vacuum overnight to give product in the form of free base as faint yellow oil that crystallized on standing. **Yield:** 453 mg (66%; 1 step; based on **TD558). NMR (CD₃CN): ¹H** δ_{H} 1.39 (C*H*₃, s, 18H); 2.53 *(mc,* m, 4H); 2.61 *(mc,* m, 4H); 2.73 *(mc,* m, 4H); 2.77 *(mc,* m, 4H); 3.01 (C*H*₂-CO, s, 4H); 3.44 (C≡C*H*, s, 1H); 3.66 (C*H*₂-arom., s, 2H); 7.38 *(arom.,* dd, 1H, ³*J*_{HH} = 8, ⁴*J*_{HH} = 1); 7.70 *(arom.,* t, 1H, ³*J*_{HH} = 8); 7.76 *(arom.,* dd, 1H, ³*J*_{HH} = 8, ⁴*J*_{HH} = 1). **¹³C{¹H}** δ_{C} 28.4 (C*H*₃, s); 48.2 *(mc,* s); 51.8 *(mc,* s); 52.6 *(mc,* s); 54.9 *(mc,* s); 57.6 (*C*H₂-CO, s); 59.3 (*C*H₂-arom., s); 78.0 (C=CH, s); 81.3 (*C*-CH₃, s); 84.0 (C=CH, s); 124.9 (*arom.,* s); 126.5 (*arom.,* s); 137.8 (*arom.,* s); 141.6 (*arom.,* s); 162.8 (*arom.,* s); 172.0 (CO, s). **ESI-HRMS:** 516.3542 [M+H]⁺ (theor. [C₂₈H₄₆N₅O₄]⁺ = 516.3544). 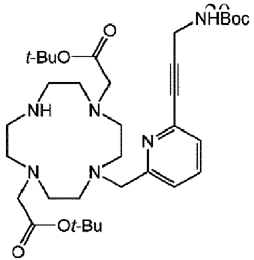 **Synthesis of TD539:** In a pear-shape glass flask (100 mL), **tBuDO2A** (free base; 1.73 g; 4.32 mmol; ≥2.0 equiv.) was dissolved in dry MeCN (15 mL) followed by addition of dried **K₂CO₃** (900 mg; 6.52 mmol; ≥3.0 equiv.). To the vigorously stirred reaction mixture, solution of freshly prepared and isolated **TD538** (≤2.17 mmol; 1.0 equiv.) in dry MeCN (10 mL) was added dropwise (over the course of 15 min). Resulting mixture was further stirred 20 h at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (150 mL) and H₂O (150 mL) and transferred to a separatory funnel. After shaking, the bottom phase was separated. Aqueous phase was further extracted with DCM (3 × 100 mL). Combined organic layers were dried with anhydrous Na₂SO₄, filtered through glass frit (S3) and evaporated to dryness. The residue was further dried on high vacuum overnight to give product in the form of free base as yellow solidified oil. **Yield:** 653 mg (47%; 2 steps; based on **TD530). NMR (CD₃CN): ¹H** δ_{H} 1.39 (C*H*₃, s, 18H); 1.43 (C*H*₃, s, 9H); 2.51-2.84 (*mc,* m, 16H); 3.01 (C*H*₂-CO, s, 4H); 3.64 (C*H*₂-arom., s, 2H); 4.05 (C*H*₂-C≡C, bd, 2H, ³*J*_{HH} = 5); 5.97 (NH-CO, bt, 1H, ³*J*_{HH} = 5); 7.27 *(arom.,* dd, 1H, ³*J*_{HH} = 7, ⁴*J*_{HH} = 2); 7.66-7.75 *(arom.,* m, 2H). **¹³C{¹H}** δ_{C} 28.4 (C*H*₃, s); 28.6 (C*H*₃, s); 31.2 (*C*H₂-C≡C, bs); 48.1 *(mc,* s); 51.8 *(mc,* s); 52.8 (*mc,* s); 55.1 *(mc,* s); 57.6 (C*H*₂-CO, s); 59.6 (*C*H₂-arom., s); 81.3 (*C*-CH₃, 2 × s); 82.6 and 87.0 (C=C, 2 × s); 124.3 *(arom.,* s); 126.0 *(arom.,* s); 137.7 (*arom.,* s); 142.3 (*arom.,* s); 162.7 (*arom.,* s); 172.0 (CO, s). **ESI-HRMS:** 645.4331 [M+H]⁺ (theor. [C₃₄H₅₇N₆O₆]⁺ = 645.4334). 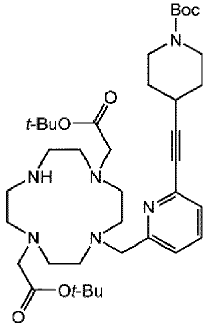 **Synthesis of TD1118:** In a pear-shape glass flask (50 mL), **tBuDO2A** (free base; 160 mg; 399 µmol; ≥2.5 equiv.) was dissolved in MeCN (10 mL) followed by addition of dried **K₂CO₃** (65 mg; 470 µmol; ≥3 equiv.). To the vigorously stirred reaction mixture, solution of freshly prepared and isolated **TD1117** (≤470 mmol; 1.0 equiv.) in MeCN (10 mL) was added dropwise (over the course of 5 min). Resulting mixture was further stirred 16 h at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (50 mL) and H₂O (50 mL) and transferred to a separatory funnel. After shaking, the bottom phase was separated. Aqueous phase was further extracted with DCM (3 × 30 mL). Combined organic layers were dried with anhydrous Na₂SO₄, filtered through glass frit (S3) and evaporated to dryness. The residue was further dried on high vacuum overnight to give product in the form of free base as colourless solidified oil. **Yield:** 62.2 mg (57%; 2 steps; based on **TD966). NMR (CD₃CN): ¹H** δ_{H} 1.40 (C*H*₃, s, 18H); 1.43 (C*H*₃, s, 9H); 1.53-1.64 (C*H*₂, m, 2H); 1.82-1.91 (C*H*₂, m, 2H); 2.47-2.79 *(mc,* m, 16H); 2.79-2.88 (C*H*-C≡C, m, 1H); 3.01 (C*H*₂-CO, s, 4H); 3.06-3.18 (C*H*₂, m, 2H); 3.63 (C*H*₂-arom., s, 2H); 3.71-3.82 (C*H*₂, m, 2H); 7.27 *(arom.,* dd, 1H, ³*J*_{HH} = 7, ⁴*J*_{HH} = 2); 7.62-7.72 *(arom.,* m, 2H). **ESI-HRMS:** 699.4801 [M+H]⁺ (theor. [C₃₈H₆₃N₆O₆]⁺ = 699.4804). 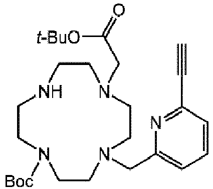 **Synthesis** of TD692: In a pear-shape glass flask (250 mL), TD681 (100 mg; 259 µmol; 1.5 equiv.) was dissolved in MeCN (25 mL). To the vigorously stirred reaction mixture, solution of TD558 (26 mg; 172 µmol; 1.0 equiv.) in MeCN (25 mL) was added dropwise (over the course of 1 h). Resulting mixture was further stirred 2 d at RT. Solids were filtered off using syringe microfilter (PTFE) and filtrate was evaporated to dryness. Residue was purified by preparative HPLC (C18; H₂O-MeCN gradient with FA additive). Fractions with product were combined, immediately neutralized with dil. aq. NaHCO₃ and evaporated to dryness. Residue was dissolved in DCM (25 mL) and H₂O (25 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as yellow oil. **Yield:** 38 mg (44%; 1 step; based on **TD558). ESI-MS** (LC-MS): 502.4 [M+H]⁺ (theor. [C₂₇H₄₄N₅O₄]⁺ = 502.3). All of **TD692** was directly used for **TD703** without further characterization. 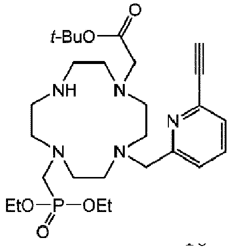 **Synthesis of TD700:** In a pear-shape glass flask (100 mL), **TD695** (100 mg; 229 µmol; 1.5 equiv.) was dissolved in MeCN (25 mL) followed by addition of dried **K₂CO₃** (21 mg; 152 µmol; 1.0 equiv.). To the vigorously stirred reaction mixture, solution of **TD558** (23 mg; 152 µmol; 1.0 equiv.) in MeCN (25 mL) was added dropwise (over the course of 1 h). Resulting mixture was further stirred 16 h at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (75 mL) and H₂O (75 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as yellow oil. **Yield:** 20 mg (24%; 1 step; based on **TD558). ESI-MS** (LC-MS): 552.3 [M+H]⁺ (theor. [C₂₇H₄₇N₅O₅P₁]⁺ = 552.3). All of **TD700** was directly used for **TD705** without further characterization. 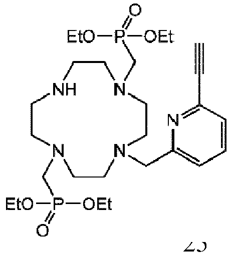 **Synthesis of TD579:** In a pear-shape glass flask (25 mL), **TD573** (600 mg; 1.27 mmol; 2.0 equiv.) was dissolved in MeCN (10 mL). To the vigorously stirred reaction mixture, solution of **TD558** (30 mg; 488 µmol; 1.0 equiv.) in MeCN (10 mL) was added dropwise (over the course of 30 min). Resulting mixture was further stirred 16 h at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (125 mL), H₂O (125 mL) and aq. NaOH (1%; 20 mL) and transferred to a separatory funnel. After shaking, the bottom phase was separated. Aqueous phase was further extracted with DCM (3 × 50 mL). Combined organic layers were dried with anhydrous Na₂SO₄, filtered through glass frit (S3) and evaporated to dryness. The residue was further dried on high vacuum overnight to give product in the form of free base as yellow oil. **Yield:** 137 mg (48%; 1 step; based on **TD558). ESI-MS** (LC-MS): 588.3 [M+H]⁺ (theor. [C₂₆H₄₈N₅O₆P₂]⁺ = 588.3). **TD579** was directly used for **TD575** and **TD580** and without further characterization. 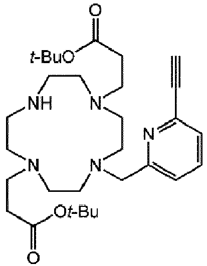 **Synthesis of TD799:** In a pear-shape glass flask (100 mL), **tBuDO2Prop** (free base; 165 mg; 385 µmol; 2.0 equiv.) was dissolved in MeCN (30 mL). To the vigorously stirred reaction mixture, solution of **TD558** (29 mg; 191 µmol; 1.0 equiv.) in MeCN (30 mL) was added dropwise (over the course of 6 h). Resulting mixture was further stirred 10 h at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (50 mL) and H₂O (50 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as slightly yellow oil. **Yield:** 47 mg (45%; 1 step; based on **TD558). NMR (CD₃CN): ¹H** δ_{H} 1.38 (C*H*₃, s, 18H); 2.19-2.25 (C*H*₂-CO, m, 4H); 2.44-2.64 *(mc,* C*H*₂-CH₂-CO, m, 16+4H); 3.41 (C≡C*H*, s, 1H); 3.66 (C*H*₂-arom., s, 2H); 7.36 *(arom.,* dd, 1H, ³*J*_{HH} = 8, ⁴*J*_{HH} = 1); 7.70 (*arom.,* t, 1H, ³*J*_{HH} = 8); 7.77 (*arom.,* bd, 1H, ³*J*_{HH} = 8). **ESI-HRMS:** 544.3854 [M+H]⁺ (theor. [C₃₀H₅₀N₅O₄]⁺ = 544.3857). 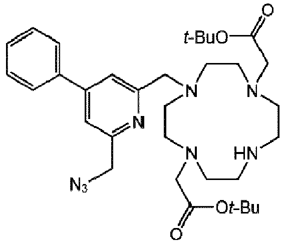 **Synthesis of TD663:** In a pear-shape glass flask (50 mL), **tBuDO2A** (free base; 210 mg; 524 µmol; 2.7 equiv.) was dissolved in MeCN (15 mL) followed by addition of dried **K₂CO₃** (27 mg; 195 µmol; 1.0 equiv.). To the vigorously stirred reaction mixture, solution of **TD566** (50 mg; 193 µmol; 1.0 equiv.) in MeCN (15 mL) was added dropwise (over the course of 15 min). Resulting mixture was further stirred 2 d at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (50 mL) and H₂O (50 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as nearly colorless oil. **Yield:** 83 mg (69%; 1 step; based on **TD566). NMR (CD₃CN): ¹H** δ_{H} 1.33 (C*H*₃, s, 18H); 2.62 *(mc,* m, 4H); 2.66 *(mc,* m, 4H); 2.74 *(mc,* m, 4H); 2.80 *(mc,* m, 4H); 2.82 (C*H*₂-CO, s, 4H); 3.74 (C*H*₂-arom., s, 2H); 4.46 (C*H*₂-N₃, s, 2H); 2.46-7.58 *(Ph, arom.,* m, 3+1H); 7.78-7.87 *(Ph,* m, 2H); 8.18 *(arom.,* bs, 1H). **ESI-HRMS:** 623.4022 [M+H]⁺ (theor. [C₃₃H₅₁N₈O₄]⁺ = 623.4028). 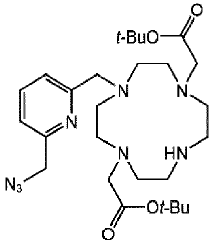 **Synthesis of TD711:** In a pear-shape glass flask (100 mL), **tBuDO2A** (free base; 564 mg; 1.41 mmol; 2.5 equiv.) was dissolved in MeCN (40 mL). To the vigorously stirred reaction mixture, solution of **TD406** (103 mg; 564 µmol; 1.0 equiv.) in MeCN (40 mL) was added dropwise (over the course of 2 h). Resulting mixture was further stirred 3 d at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (100 mL) and H₂O (100 mL) and transferred to a separatory funnel. After shaking, the bottom phase was separated. Aqueous phase was further extracted with DCM (3 × 50 mL). Combined organic layers were dried with anhydrous Na₂SO₄, filtered through glass frit (S3) and evaporated to dryness. The residue was further dried on high vacuum overnight to give product in the form of free base as nearly colourless oil. **Yield:** 217 mg (70%; 1 step; based on **TD406). NMR (CD₃CN): ¹H** δ_{H} 1.39 (C*H*₃, s, 18H); 2.55 *(mc,* m, 4H); 2.60 *(mc,* m, 4H); 2.76 *(mc,* m, 8H); 3.03 (C*H*₂-CO, s, 4H); 3.70 (C*H*₂-arom., s, 2H); 4.39 (C*H*₂-N₃, s, 2H); 7.21 (*arom.,* dd, 1H, ³*J*_{HH} = 8, ⁴*J*_{HH} = 1); 7.67 *(arom.,* dd, 1H, ³*J*_{HH} = 8, ⁴*J*_{HH} = 1); 7.73 *(arom.,* t, 1H, ³*J*_{HH} = 8). **¹³C{¹H}** δ_{C} 28.4 (C*H*₃, s); 48.3 *(mc,* s); 51.8 *(mc,* s); 52.7 *(mc,* s); 54.7 (*mc,* s); 56.1 (*C*H₂-N₃, s); 57.7 (C*H*₂-CO, s); 59.0 (C*H*₂-arom., s); 81.2 (*C*-CH₃, s); 121.2 *(arom.,* s); 124.1 (*arom.,* s); 138.2 (*arom.,* s); 155.6 (*arom.,* s); 161.9 (*arom.,* s); 172.0 (CO, s). **ESI-HRMS:** 547.3716 [M+H]⁺ (theor. [C₂₇H₄₇N₈O₄]⁺ = 547.3715). 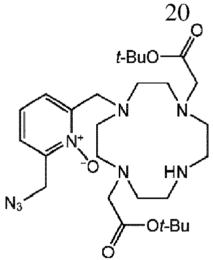 **Synthesis of TD596:** In a pear-shape glass flask (25 mL), **tBuDO2A** (free base; 162 mg; 405 µmol; ≥2.5 equiv.) was dissolved in MeCN (10 mL). To the vigorously stirred reaction mixture, solution of crude **TD633** (≤162 µmol; 1.0 equiv.) in MeCN (10 mL) was added dropwise (over the course of 15 min). Resulting mixture was further stirred 2 d at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (50 mL) and H₂O (50 mL) and transferred to a separatory funnel. After shaking, the bottom phase was separated and the aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as nearly colorless oil. **Yield:** 44 mg (48%; 2 steps; based on **TD406). ESI-MS** (LC-MS): 563.4 [M+H]⁺ (theor. [C₂₇H₄₇N₈O₅]⁺ = 563.4). All of **TD596** was directly used for **TD604** without further characterization.

### Example 4: Synthesis of non-caged macrocyclic ligands

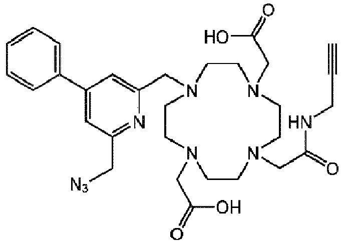

**Synthesis of TD425:** In a pear-shape glass flask (50 mL), crude **TD423** (~75% in a mixture with ~25% of bis(substituted) by-product; 180 mg; ≥1.0 equiv.) was dissolved in dry MeCN (10 mL) followed by addition of **Cs₂CO₃** (360 mg; 1.11 mmol; 3.8 equiv.). Solution of **TD566** (75 mg; 290 µmol; 1.0 equiv.) in MeCN (5 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was purified by preparative HPLC (C18; H₂O-MeCN gradient with FA additive). Fractions with product were joined and directly lyophilized to give product in nearly zwitterionic form as white fluffy solid. **Yield:** 129 mg (27%; 3 steps; based on **2-Chloro-*N*-(prop-2-yn-1-yl)acetamide). NMR (D₂O, pD ~5): ¹H** δ_{H} 2.62 (C=CH, t, 1H, ⁴*J*_{HH} = 3); 2.89-3.74 *(mc,* C*H*₂-CO, m, 16+6H); 3.79 (C*H*₂-C≡CH, t, 2H, ⁴*J*_{HH} = 3); 4.03 (C*H*₂-arom., bs, 2H); 4.68 (C*H*₂-N₃, s, 2H); 7.55-7.67 *(Ph, arom.,* m, 3+1H); 7.78 (*arom.,* d, 1H, ⁴*J*_{HH} = 2); 7.85-7.92 *(Ph,* m, 2H); 8.17 (*arom.,* d, 1H, ⁴*J*_{HH} = 2). **ESI-HRMS:** 606.3151 [M+H]⁺ (theor. [C₃₀H₄₀N₉O₅]⁺ = 606.3147). **EA** (C₃₀H₃₉N₉O_{5•}0.1FA•1.3H₂O, *M*_{R} = 633.7): C 57.0 (56.8); H 6.6 (6.3); N 19.9 (19.5). 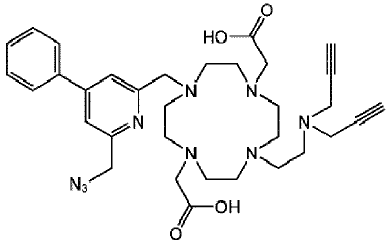 **Synthesis of TD669:** In a glass vial (4 mL), **TD663** (30 mg; 48 µmol, 1.0 equiv.) was dissolved in dry MeCN (500 µL) followed by addition of **K₂CO₃** (20 mg; 145 µmol; 3 equiv.). Solution **of TD662** (10 mg; 50 µmol; 1.0 equiv.) in MeCN (500 µL) was then added and the resulting suspension was stirred 2 d at RT. Another portion of **TD662** (6 mg; 30 µmol; 0.6 equiv.) in MeCN (200 µL) was then added and the mixture was further stirred 2 d at RT. Solids were filtered off using syringe microfilter (PTFE) and filtrate was directly purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was purified by preparative HPLC (C18; H₂O-MeCN gradient with FA additive). Fractions with product were joined and directly lyophilized to give product in nearly zwitterionic form as white fluffy solid. **Yield:** 27 mg (≤88% assuming M•0.1FA•xH₂O; *M*_{R} = 634.4; 2 steps; based on **TD663). NMR (D₂O, pD ~5): ¹H** δ_{H} 2.59-3.82 *(mc,* C*H*₂-CO, C*H*₂-C*H*₂-N(C*H*₂C=C*H*)₂, m, 16+4+10H); 4.00 (C*H*₂-arom., bs, 2H); 4.64 (C*H*₂-N₃, s, 2H); 7.55-7.65 *(Ph,* m, 3H); 7.74 (*arom.,* d, 1H, ⁴*J*_{HH} = 2); 7.83-7.91 *(Ph,* m, 2H); 8.17 *(arom.,* d, 1H, ⁴*J*_{HH} = 2). **ESI-HRMS:** 630.3510 [M+H]⁺ (theor. [C₃₃H₄₄N₉O₄]⁺ = 630.3511). 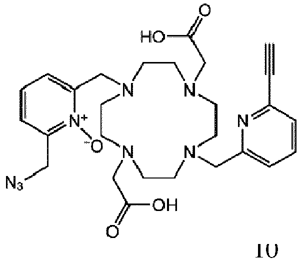 **Synthesis of TD604:** In a glass vial (4 mL), **TD596** (44 mg; 78 µmol; 1.0 equiv.) was dissolved in MeCN (3 mL) followed by addition of dried **K₂CO₃** (33 mg; 239 µmol; 3.0 equiv.). Solution of **TD558** (13 mg; 86 µmol; 1.1 equiv.) in MeCN (600 µL) was then added and the resulting suspension was stirred 16 d at RT. Then additional portion of **TD558** (2 mg; 13 µmol; 0.2 equiv.) in MeCN (400 µL). Reaction mixture was further stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was purified by preparative HPLC (C18; H₂O-MeCN gradient with FA additive). Fractions with product were joined and directly lyophilized to give product in nearly zwitterionic form as white fluffy solid. **Yield:** 38 mg (81%; 2 steps; based on **TD596). NMR (D₂O, pD ~4): ¹H** δ_{H} 2.80-3.68 *(mc,* C*H*₂-CO, m, 16+4H); 3.72 (C=CH, s, 1H); 3.93 (C*H*₂-arom., bs, 2H); 4.17 (C*H*₂-arom., s, 2H); 4.74 (C*H*₂-N₃, s, 2H); 7.48-7.67 *(arom.,* m, 3H); 7.74-7.95 *(arom.,* m, 3H). **¹³C{¹H}** δ_{C} 48.3 *(mc,* s); 48.9 *(mc,* s); 50.6 (*C*H₂-N₃, s); 51.4 (*C*H₂-arom., s); 51.5 *(mc,* s); 51.7 *(mc,* s); 56.7 (C*H*₂-CO, s); 58.8 (*C*H₂-arom., s); 80.7 and 82.2 (C=CH; 2 × s); 126.0 (*arom.,* s); 126.5 (*arom.,* s); 127.9 (*arom.,* s); 128.5 (*arom.,* s); 131.5 (*arom.,* s); 140.3 (*arom.,* s); 141.2 (*arom.,* s); 148.4 (*arom.,* s); 148.7 (*arom.,* s); 157.3 (*arom.,* s); 169.5 (CO, s). **ESI-HRMS:** 566.2836 [M+H]⁺ (theor. [C₂₇H₃₆N₉O₅]⁺ = 566.2834). **EA** (C₂₇H₃₅N₉O_{5•}0.1FA•1.6H₂O, *M*_{R} = 599.1): C 54.3 (54.4); H 6.5 (6.1); N 21.0 (20.7). 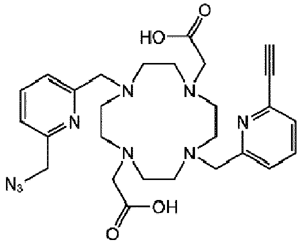 **Synthesis of TD556:** In a pear-shape glass flask (25 mL), **TD635** (216 mg; 419 µmol; 1.0 equiv.) was dissolved in MeCN (12 mL) followed by addition of dried **K₂CO₃** (174 mg; 1.26 mmol; 3.0 equiv.). Solution of **TD406** (92 mg; 504 µmol; 1.2 equiv.) in MeCN (3 mL) was then added and the resulting suspension was stirred 3 d at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in nearly zwitterionic form as white fluffy solid. **Yield:** 161 mg (66%; 2 steps; based on **TD635). NMR (D₂O, pD ~4): ¹H** δ_{H} 2.80-3.60 *(mc, CH₂*-CO, m, 16+4H); 3.74 (C≡C*H*, s, 1H); 3.86 (C*H*₂-arom., bs, 2H); 3.94 (C*H*₂-arom., bs, 2H); 4.72 (C*H*₂-N₃, s, 2H); 7.65 *(arom.,* dd, 1H, ³*J*_{HH} = 8, ⁴*J*_{HH} = 1); 7.67 *(arom.,* dd, 1H, ³*J*_{HH} = 8, ⁴*J*_{HH} = 1); 7.95-8.03 *(arom.,* m, 3H); 8.12 *(arom.,* t, 1H, ³*J*_{HH} = 8); **¹³C[¹H}** δ_{C} 48.3 *(mc,* s); 48.4 *(mc,* s); 51.4 *(mc,* s); 51.5 *(mc,* s); 54.3 (*C*H₂-N₃, s); 56.6 (C*H*₂-CO, s); 58.0 (*C*H₂-arom., s); 58.9 (*C*H₂-arom., s); 80.9 and 82.0 (C=CH; 2 × s); 124.5 (*arom.,* s); 126.2 (*arom.,* s); 126.3 (*arom.,* s); 128.5 (*arom.,* s); 140.5 (*arom.,* s); 141.2 (*arom.,* s); 142.7 (*arom.,* s); 155.0 (*arom.,* s); 155.5 (*arom.,* s); 157.2 (*arom.,* s); 169.2 (CO, s). **ESI-HRMS:** 550.2880 [M+H]⁺ (theor. [C₂₇H₃₆N₉O₄]⁺ = 550.2885). **EA** (C₂₇H₃₅N₉O_{4•}0.1FA• 1.6H₂O, *M*_{R} = 583.1): C 55.8 (55.5); H 6.6 (6.1); N 21.6 (21.2). 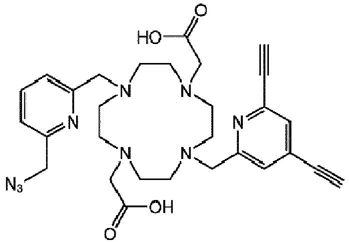 **Synthesis of TD810:** In a glass vial (20 mL), **TD711** (103 mg; 188 µmol; 1.0 equiv.) was dissolved in MeCN (5 mL) followed by addition of dried **K₂CO₃** (130 mg; 942 µmol; 5.0 equiv.). Solution of **TD807** (33 mg; 188 µmol; 1.0 equiv.) in MeCN (2 mL) was then added and the resulting suspension was stirred 3 d at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in the form of mixed trifluoroacetate/formate salt as off white fluffy solid. **Yield:** 55 mg (43%; 2 steps; based on **TD711). NMR (D₂O, pD ~4): ¹H** δ_{H} 2.79-3.63 *(mc,* C*H*₂-CO, m, 16+4H); 3.74 (C=CH, s, 1H); 3.82 (C*H*₂-arom., bs, 2H); 3.87 (C*H*₂-arom., bs, 2H); 3.92 (C≡C*H*, s, 1H); 4.63 (C*H*₂-N₃, s, 2H); 7.55 *(arom.,* dd, 1H, ³*J*_{HH} = 8, ⁴*J*_{HH} = 1); 7.72 *(arom.,* d, 1H, ⁴*J*_{HH} = 1); 8.10 *(arom.,* dd, 1H, ³*J*_{HH} = 8, ⁴*J*_{HH} = 1); 8.16 *(arom.,* t, 1H, ³*J*_{HH} = 8); 8.26 *(arom.,* d, 1H, ⁴*J*_{HH} = 1). **ESI-HRMS:** 574.2882 [M+H]⁺ (theor. [C₂₉H₃₆N₉O₄]⁺ = 574.2885). **EA** (C₂₉H₃₅N₉O_{4•}0.5TFA•0.1FA•2.1H₂O, *M*_{R} = 673.1): C 53.7 (53.8); H 6.0 (6.2); N 18.7 (18.7). 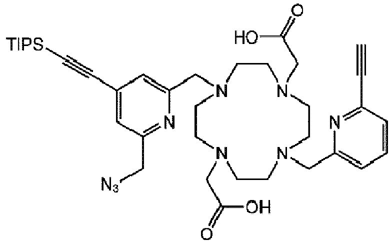 **Synthesis of TD827:** In a glass vial (20 mL), **TD635** (157 mg; 304 µmol; 1.0 equiv.) was dissolved in dry MeCN (7 mL) followed by addition of dried **K₂CO₃** (210 mg; 1.52 mmol; 5.0 equiv.). Solution of **TD817** (110 mg; 303 µmol; 1.0 equiv.) in dry MeCN (3 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, concentrated to approx. half volume (to remove most of MeCN) and then diluted with DCM (125 mL). The resulting biphasic mixture was transferred to a separatory funnel. Aqueous phase was only then neutralized with dil. aq. NaHCO₃. After shaking, the bottom phase was separated and the aqueous phase was further extracted with DCM (3 × 50 mL). Combined organic layers were with anhydrous Na₂SO₄, filtered through glass frit (S3) and evaporated to dryness. Residue was dissolved in TFA (3 mL) and the resulting clear mixture was stirred 16 h at RT. Mixture was evaporated to dryness and once co-evaporated with MeOH to remove most of TFA. Residue was directly purified by preparative HPLC (C18; H₂O-MeCN gradient with FA additive). Fractions with product were joined and directly lyophilized to give product in the form of formate salt as off white fluffy solid. **Yield:** 149 mg (62%; 2 steps; based on **TD711). NMR (D₂O+FA, pD ~3): ¹H** δ_{H} 0.86-1.33 (*i-Pr,* m, 21H); 2.58-3.78 *(mc,* C*H*₂-CO, C≡C*H*, bm, 16+4+1H); 3.93 (C*H*₂-arom., bs, 2H); 4.06 (C*H*₂-arom., bs, 2H); 4.56 (C*H*₂-N₃, s, 2H); 7.37 *(arom.,* s, 1H); 7.53 *(arom.,* d, 1H, ³*J*_{HH} = 8); 7.74 *(arom.,* s, 1H); 7.80 (*arom.,* d, 1H, ³*J*_{HH} = 8); 7.96 (*arom.,* t, 1H, ³*J*_{HH} = 8). **ESI-HRMS:** 730.4216 [M+H]⁺ (theor. [C₃₈H₅₆N₉O₄Si₁]⁺ = 730.4219). **EA** (C₃₈H₅₅N₉O₄Si_{1•}0.8FA•1.6H₂O, *M*_{R} = 795.6): C 58.6 (58.7); H 7.6 (7.7); N 15.8 (15.7); Si 3.5 (3.2). 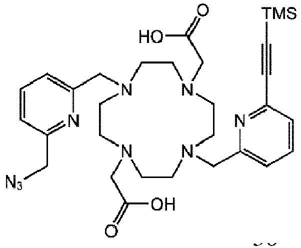 **Synthesis of TD718:** In a pear-shape glass flask (10 mL), **TD711** (31 mg; 57 µmol; 1.0 equiv.) was dissolved in dry MeCN (2 mL) followed by addition of dried **K₂CO₃** (31 mg; 225 µmol; 4.0 equiv.). Solution of **TD706** (13 mg; 58 µmol; 1.0 equiv.) in dry MeCN (2 mL) was then added and the resulting suspension was stirred 24 h at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, diluted with DCM (100 mL) and transferred to a separatory funnel. Aqueous phase was only then neutralized with dil. aq. NaHCO₃. After shaking, bottom phase was separated, dried with anhydrous Na₂SO₄, filtered through glass frit (S3) and evaporated to dryness. Residue was dissolved in TFA (3 mL) and the resulting clear mixture was stirred 16 h at RT. Mixture was evaporated to dryness and once co-evaporated with MeOH to remove most of TFA. Residue was directly purified by preparative HPLC (C18; H₂O-MeCN gradient with FA additive). Fractions with product were joined and directly lyophilized to give product in zwitterionic form as white fluffy solid. **Yield:** 23 mg (61%; 2 steps; based on **TD706). NMR (D₂O, pD ~5): ¹H** δ_{H} 0.28 (C*H*₃, s, 9H); 2.28-3.67 *(mc,* C*H*₂-CO, m, 16+4H); 3.89 (C*H*₂-arom., bs, 4H); 4.57 (C*H*₂-N₃, s, 2H); 7.49 (*arom.,* dd, 1H, ³*J*_{HH}= 7, ⁴*J*_{HH}= 2); 7.58-7.64 *(arom.,* m, 1H); 7.78-7.98 (*arom.,* m, 4H). **ESI-HRMS:** 622.6277 [M+H]⁺ (theor. [C₃₀H₄₄N₉O₄Si₁]⁺ = 622.3280). **EA** (C₃₀H₄₃N₉O₄Si_{1•}2.6H₂O, *M*_{R} = 668.7): C 53.9 (53.9); H 7.3 (7.0); N 18.9 (18.7). 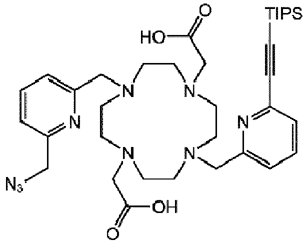 **Synthesis of TD728:** In a pear-shape glass flask (10 mL), **TD711** (66 mg; 121 µmol; 1.0 equiv.) was dissolved in MeCN (2 mL) followed by addition of dried **K₂CO₃** (67 mg; 486 µmol; 4.0 equiv.). Solution of **TD723** (37 mg; 120 µmol; 1.0 equiv.) in MeCN (2 mL) was then added and the resulting suspension was stirred 24 h at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, diluted with DCM (150 mL) and the resulting biphasic mixture was transferred to a separatory funnel. Aqueous phase was only then neutralized with dil. aq. NaHCO₃. After shaking, the bottom phase was separated and the aqueous phase was further extracted with DCM (2 × 50 mL). Combined organic layers were with anhydrous Na₂SO₄, filtered through glass frit (S3) and evaporated to dryness. Residue was dissolved in TFA (3 mL) and the resulting clear mixture was stirred 16 h at RT. Mixture was evaporated to dryness and once co-evaporated with MeOH to remove most of TFA. Residue was directly purified by preparative HPLC (C18; H₂O-MeCN gradient with FA additive). Fractions with product were joined and directly lyophilized to give product in zwitterionic form as white fluffy solid. **Yield:** 64 mg (71%; 2 steps; based on **TD723). NMR (D₂O, pD ~5): ¹H** δ_{H} 0.84-1.34 (*i-Pr,* m, 21H); 2.41-3.78 *(mc,* C*H*₂-CO, m, 16+4H); 3.89 (C*H*₂-arom., bs, 2H); 3.95 (C*H*₂-arom., bs, 2H); 4.52 (C*H*₂-N₃, s, 2H); 7.42 (*arom.,* d, 1H, ³*J*_{HH} = 8); 7.46 *(arom.,* d, 1H, ³*J*_{HH} = 8); 7.74 *(arom.,* d, 1H, ³*J*_{HH} = 8); 7.78 *(arom.,* d, 1H, ³*J*_{HH} = 8); 7.85 (*arom.,* t, 1H, ³*J*_{HH} = 8); 7.87 (*arom.,* t, 1H, ³*J*_{HH} = 8). **ESI-HRMS:** 706.4221 [M+H]⁺ (theor. [C₃₆H₅₆N₉O₄Si₁]⁺ = 706.4219). EA (C₃₆H₅₅N₉O₄Si₁ • 2.6H₂O, *M*_{R} = 752.8): C 57.4 (57.7); H 8.1 (8.0); N 16.7 (16.5); Si 3.7 (3.8). 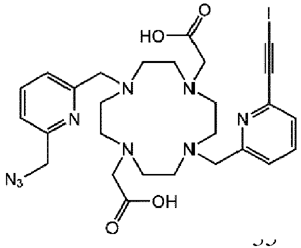 **Synthesis of TD1204:** In a glass vial (20 mL), **TD711** (35.0 mg; 64.0 µmol; 1.0 equiv.) was dissolved in MeCN (1 mL) followed by addition of dried **K₂CO₃** (44.2 mg; 320 µmol; 5.0 equiv.). Solution of **TD1194** (21.4 mg; 77.1 µmol; 1.2 equiv.) in MeCN (2 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in the form of formate salt as white fluffy solid. **Yield:** 23.3 mg (49%; 2 steps; based on **TD711). NMR (D₂O, pD ~7): ¹H** δ_{H} 2.83-3.67 *(mc,* C*H*₂-CO, m, 16+4H); 3.88 (C*H*₂-arom., bs, 2H); 3.92 (C*H*₂-arom., bs, 2H); 4.56 (C*H*₂-N₃, s, 2H); 7.49 (*arom.,* d, 1H, ³*J*_{HH} = 8); 7.54 *(arom.,* d, 1H, ³*J*_{HH} = 8); 7.87-8.00 *(arom.,* m, 4H). **ESI-HRMS:** 676.1855 [M+H]⁺ (theor. [C₂₇H₃₅N₉O₄I₁]⁺ = 676.1851). **EA** (C₂₇H₃₄N₉O₄I_{1•}0.3FA•2.7H₂O, *M*_{R} = 738.0): C 44.4 (44.6); H 5.5 (5.1); N 17.1 (16.7). 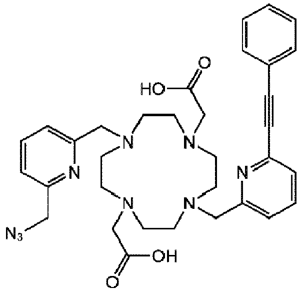 **Synthesis of TD944:** In a pear-shape glass flask (10 mL), **TD711** (58 mg; 106 µmol; 1.0 equiv.) was dissolved in MeCN (2 mL) followed by addition of dried **K₂CO₃** (87 mg; 629 µmol; 6.0 equiv.). Solution of freshly prepared and isolated **TD939** (≤105 µmol; ≤1.0 equiv.) in MeCN (2 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in zwitterionic form as faint yellow solid. **Yield:** 38 mg (55%; 3 steps; based on **TD936). NMR (D₂O, pD ~7): ¹H** δ_{H} 2.82-3.67 (*mc,* C*H*₂-CO, m, 16+4H); 3.91 (C*H*₂-arom., bs, 2H); 3.95 (C*H*₂-arom., bs, 2H); 4.58 (C*H*₂-N₃, s, 2H); 7.40-8.07 *(Ph, arom.,* m, 5+6H). **ESI-HRMS:** 626.3197 [M+H]⁺ (theor. [C₃₃H₄ₒN₉O₄]⁺ = 626.3198). **EA** (C₃₀H₃₉N₉O₄ • 1.5H₂O, *M*_{R} = 652.8): C 60.7 (61.0); H 6.5 (6.3); N 19.3 (18.8). 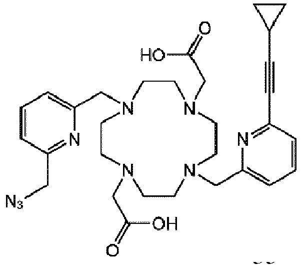 **Synthesis of TD943:** In a pear-shape glass flask (10 mL), **TD711** (82 mg; 150 µmol; 1.0 equiv.) was dissolved in MeCN (2 mL) followed by addition of dried **K₂CO₃** (125 mg; 904 µmol; 6.0 equiv.). Solution of freshly prepared and isolated **TD940** (≤150 µmol; ≤1.0 equiv.) in MeCN (2 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in zwitterionic form as faint yellow solid. **Yield:** 66 mg (71%; 3 steps; based on **TD937). NMR (D₂O, pD ~7): ¹H** δ_{H} 0.74-1.06 (C*H*₂-CH, m, 4H); 1.35-1.66 (CH₂-C*H*, m, 1H); 2.69-3.65 *(mc,* C*H*₂-CO, m, 16+4H); 3.86 (C*H*₂-arom., bs, 2H); 3.92 (C*H*₂-arom., bs, 2H); 4.57 (C*H*₂-N₃, s, 2H); 7.42-7.53 *(arom.,* m, 2H); 7.83-8.01 *(arom.,* m, 4H). **ESI-HRMS:** 590.3196 [M+H]⁺ (theor. [C₃₀H₄ₒN₉O₄]⁺ = 590.3198). **EA** (C₃₀H₃₉N_{9P}O₄ • 1.5H₂O, *M*_{R} = 616.7): C 58.4 (58.6); H 6.9 (6.9); N 20.4 (20.3). 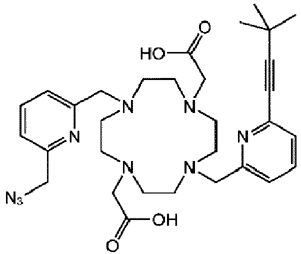 **Synthesis of TD959:** In a pear-shape glass flask (10 mL), **TD711** (23 mg; 42 µmol; 1.0 equiv.) was dissolved in MeCN (1 mL) followed by addition of dried **K₂CO₃** (35 mg; 253 µmol; 6.0 equiv.). Solution of freshly prepared and isolated TD956 (≤42 µmol; ≤1.0 equiv.) in MeCN (1 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in zwitterionic form as nearly colourless solid. **Yield:** 18 mg (67%; 3 steps; based on **TD952). NMR (D₂O, pD ~6): ¹H** δ_{H} 1.34 (C*H*₃, s, 9H); 2.72-3.69 *(mc,* C*H*₂-CO, m, 16+4H); 3.88 (C*H*₂-arom., bs, 2H); 3.92 (C*H*₂-arom., bs, 2H); 4.57 (C*H*₂-N₃, s, 2H); 7.46-7.55 (*arom.,* m, 2H); 7.84-8.03 (*arom.,* m, 4H). **ESI-HRMS:** 606.3513 [M+H]⁺ (theor. [C₃₁H₄₄N₉O₄]⁺ = 606.3511). **EA** (C₃₁H₄₃N₉O₄ • 1.8H₂O, *M*_{R} = 638.2): C 58.3 (58.7); H 7.4 (7.0); N 19.8 (19.4 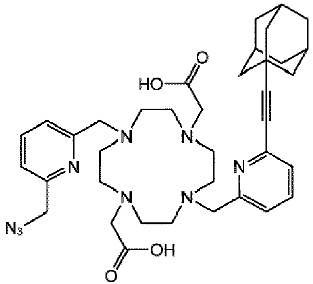 **Synthesis of TD992:** In a glass vial (4 mL), **TD711** (43 mg; 79 µmol; 1.0 equiv.) was dissolved in MeCN (1 mL) followed by addition of dried **K₂CO₃** (44 mg; 318 µmol; 4.1 equiv.). Solution of freshly prepared and isolated **TD990** (≤79 µmol; ≤1.0 equiv.) in MeCN (1 mL) was then added and the resulting suspension was stirred 16 h at 40 °C. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in the form of formate salt as off white fluffy solid. **Yield:** 42.0 mg (73%; 3 steps; based on **TD965). NMR (D₂O, pD ~5): ¹H** δ_{H} 1.62-1.83 (*Adm.,* m, 6H); 1.86-2.04 *(Adm.,* m, 9H); 2.77-3.70 *(mc,* C*H*₂-CO, m, 16+4H); 3.90 (C*H*₂-arom., bs, 2H); 3.94 (C*H*₂-arom., bs, 2H); 4.57 (C*H*₂-N₃, s, 2H); 7.44-7.56 (*arom.,* m, 2H); 7.82-8.00 *(arom.,* m, 4H). **ESI-HRMS:** 684.3982 [M+H]⁺ (theor. [C₃₇H₅₀N₉O₄]⁺ = 684.3980). **EA** (C₃₇H₄₉N₉O_{4•}0.3FA 1.9H₂O, *M*_{R} = 731.9): C 61.2 (61.2); H 7.1 (7.1); N 17.2 (17.0). 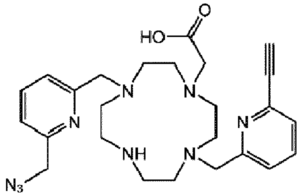 **Synthesis of TD703:** In a glass vial (20 mL), **TD692** (38 mg; 76 µmol; 1.0 equiv.) was dissolved in MeCN (3 mL) followed by addition of dried **K₂CO₃** (31 mg; 217 µmol; 3.0 equiv.). Solution of **TD406** (14 mg; 77 µmol; 1.0 equiv.) in MeCN (2 mL) was then added and the resulting suspension was stirred 16 h at RT. Then, another portion of dried **K₂CO₃** (31 mg; 217 µmol; 3.0 equiv.) and of **TD406** (10 mg; 55 µmol; 0.7 equiv.) in MeCN (1 mL) was added and the resulting suspension was further stirred 3 d at RT. Solids were filtered off using syringe microfilter (PTFE) and filtrate was evaporated to dryness. Residue was purified by preparative HPLC (C18; H₂O-MeCN gradient with FA additive). Fractions with protected product were combined, evaporated to dryness and further two-times co-evaporated with MeOH. Residue was dissolved in TFA (3 mL) and the resulting clear mixture was stirred 16 h at RT. Mixture was evaporated to dryness and three-times co-evaporated with MeOH to remove most of TFA. Residue was directly purified by preparative HPLC (C18; H₂O-MeCN gradient with FA additive). Fractions with product were joined and directly lyophilized to give product in the form of mixed trifluoroacetate/formate salt as slightly yellow viscous oil. **Yield:** 16 mg (34%; 2 steps; based on **TD692). NMR (D₂O, pD ~5): ¹H** δ_{H} 2.59-3.73 *(mc,* C*H*₂-CO, m, 16+2H); 3.75 (C≡C*H*, s, 1H); 3.84 (C*H*₂-arom., bs, 2H); 4.29-4.62 (C*H*₂-arom., C*H*₂-N₃, m, 2+2H); 7.37 (*arom.,* dd, 1H, ³*J*_{HH} = 8, ⁴*J*_{HH} = 1); 7.45 *(arom.,* dd, 1H, ³*J*_{HH} = 8, ⁴*J*_{HH} = 1); 7.52 *(arom.,* t, 1H, ³*J*_{HH} = 8); 7.56 *(arom.,* dd, 1H, ³*J*_{HH} = 8, ⁴*J*_{HH} = 1); 7.59 (*arom.,* dd, 1H, ³*J*_{HH} = 8, ⁴*J*_{HH} = 1); 7.89 (*arom.,* t, 1H, ³*J*_{HH} = 8). **ESI-HRMS:** 492.2829 [M+H]⁺ (theor. [C₂₅H₃₄N₉O₂]⁺ = 492.2830). EA (C₂₅H₃₃N₉O_{2•}0.3TFA•0.7FA•3.5H₂O, *M*_{R} = 621.1): C 50.9 (51.1); H 6.8 (6.8); N 20.3 (20.0). 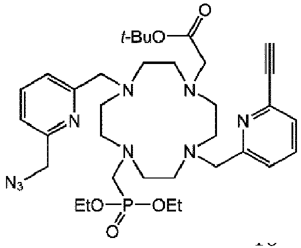 **Synthesis of TD705:** In a glass vial (20 mL), **TD700** (20 mg; 36 µmol; 1.0 equiv.) was dissolved in MeCN (3 mL) followed by addition of dried **K₂CO₃** (20 mg; 145 µmol; 4.0 equiv.). Solution of **TD406** (9 mg; 49 µmol; 1.4 equiv.) in MeCN (1 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (50 mL) and H₂O (50 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as nearly colorless oil. **Yield:** 22 mg (87%; 1 step; based on **TD406). ESI-MS** (LC-MS): 698.4 [M+H]⁺ (theor. [C₃₄H₅₃N₉O₅P₁]⁺ = 698.4). All of **TD705** was directly used for **TD714** without further characterization. 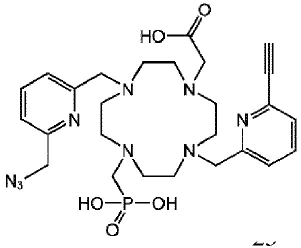 **Synthesis of TD714:** In a glass vial (4 mL), **TD705** (22 mg; 32 µmol; 1.0 equiv.) was dissolved in dry Pyridine (1 mL) followed by addition of neat **TMSBr** (50 µL; 379 µmol; 12.0 equiv.) and the resulting mixture was stirred 16 h at RT. Reaction was then quenched with 50% aq. MeOH (1 mL) and the mixture was further stirred 1 h at RT. The mixture was then directly purified by preparative HPLC (C18; H₂O-MeCN gradient with FA additive). Fractions with *tert-butyl* protected product were combined, evaporated to dryness and further two-times co-evaporated with MeOH. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in nearly zwitterionic form as white fluffy solid. **Yield:** 12 mg (≤64% assuming M•0.1FA•*x*H₂O; *M*_{R} = 590.2; 2 steps; based on **TD705). NMR (D₂O, pD ~5): ¹H** δ_{H} 2.73-3.71 *(mc,* C*H*₂-CO, C*H*₂-P, m, 16+2+1H); 3.74 (C=CH, s, 1H); 3.84 (C*H*₂-arom, bs, 1H); 3.99 (C*H*₂-arom., bs, 1H); 4.17 (C*H*₂-arom., bs, 2H); 4.47 (C*H₂*-P; bd, ²*J*_{H} = 13); 4.67 (C*H*₂-N₃, s, 1H); 4.68 (C*H*₂-N₃, s, 1H); 7.54 *(arom.,* d, 1H, ³*J*_{HH} = 8); 7.61 *(arom.,* d, 1H, ³*J*_{HH} = 8); 7.62 *(arom.,* d, 1H, ³*J*_{HH} = 8); 7.64 *(arom.,* d, 1H, ³*J*_{HH} = 8); 7.90 *(arom.,* t, 1H, ³*J*_{HH} = 8); 7.93 *(arom.,* t, 1H, ³*J*_{HH} = 8). **ESI-HRMS:** 586.2649 [M+H]⁺ (theor. [C₂₆H₃₇N₉O₅P₁]⁺ = 586.2650). 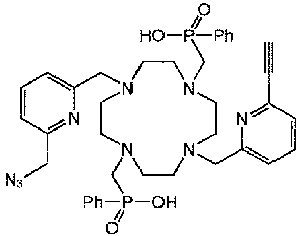 **Synthesis of TD921:** In a pear-shape glass flask (25 mL), **TD910** (160 mg; 315 µmol; 2.0 equiv.) was dissolved in MeCN (10 mL) followed by addition of solution of dried **K₂CO₃** (22 mg; 160 µmol; 1.0 equiv.). Solution **of TD558** (24 mg; 158 µmol; 1.0 equiv.) in MeCN (5 mL) was then added dropwise over the course of 15 min and the resulting suspension was stirred for 24 h at RT. Solids were filtered off using syringe microfilter (PTFE) and filtrate was evaporated to dryness. Residue was purified by preparative HPLC (C18; H₂O--MeCN gradient with TFA additive). Fractions with product were combined, neutralized with dil. aq. NaHCO₃ and evaporated to dryness. Residue was s dissolved in DCM (50 mL) and H₂O (50 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The resulting alkyne bearing intermediate (together with partially N-methylated/demethylated by-products as a consequence of ongoing self-methylation; 49 mg; ≤78 µmol; ≤1.0 equiv.) was dissolved in MeCN (7 mL) followed by addition of **TD406** (18 mg; 119 µmol; ≥1.5 equiv.) and K₂CO₃ (54 mg; 391 µmol; ≥5.0 equiv.) and the resulting suspension was stirred 16 h at RT. Solids were filtered off using syringe microfilter (PTFE) and filtrate was evaporated to dryness. Residue was purified by preparative HPLC (C18; H₂O-MeCN gradient with TFA additive). Fractions with product were combined, neutralized with dil. aq. NaHCO₃ and evaporated to dryness. Residue was s dissolved in DCM (50 mL) and H₂O (50 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The resulting methyl ester of product (together with partially N-methylated/demethylated by-products as a consequence of ongoing self-methylation; 37 mg; ≤48 µmol; ≤1.0 equiv.) was dissolved in dry Pyridine (2 mL) followed by addition of neat **TMSBr** (65 µL; 492 µmol; ≥ 12.0 equiv.) and the resulting mixture was stirred 16 h at RT. Reaction was then quenched with 50% aq. MeOH (1 mL) and the mixture was further stirred 1 h at RT. The mixture was then directly purified by preparative HPLC (C18; H₂O-MeCN gradient with FA additive). Fractions with product were joined and directly lyophilized to give product in zwitterionic form as white fluffy solid. **Yield:** 25.6 mg (20%; 3 steps; based on **TD558). NMR (D₂O, pD ~4): ¹H** δ_{H} 2.56-3.75 (*mc,* C*H*₂-P, C≡CH, m, 16+4+1H); 4.20 (C*H*₂-arom., bs, 2H); 4.29 (C*H*₂-arom., bs, 2H); 4.45 (C*H*₂-N₃, s, 2H); 7.23-7.77 *(Ph, arom.,* m, 10+4H); 7.96 (*arom.,* t, 1H, ³*J*_{HH} = 8); 7.97 *(arom.,* t, 1H, ³*J*_{HH} = 8). **³¹P{¹H}** δ_{P} 25.1 (bs). **ESI-HRMS:** 796.2334 [M+H]⁺ (theor. [C₄₄H₃₄N₁₀O₂P₂]⁺ = 796.2336). **EA** (C₃₇H₄₅N₉O₄P_{2•}4.6H₂O, *M*_{R} = 824.6): C 53.9 (54.5); H 6.6 (6.3); N 15.3 (14.7). 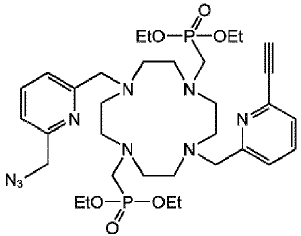 **Synthesis of TD580:** In a glass vial (20 mL), **TD579** (137 mg; 233 µmol; 1.0 equiv.) was dissolved in MeCN (7 mL) followed by addition of dried **K₂CO₃** (141 mg; 1.02 mmol; 4.4 equiv.). Solution of **TD406** (50 mg; 274 µmol; 1.2 equiv.) in MeCN (3 mL) was then added and the resulting suspension was stirred 3 d at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (50 mL) and H₂O (50 mL) and transferred to a separatory funnel. After shaking, the bottom phase was separated. Aqueous phase was further extracted with DCM (3 × 50 mL). Combined organic layers were dried with anhydrous Na₂SO₄, filtered through glass frit (S3) and evaporated to dryness. The residue was further dried on high vacuum overnight to give product in the form of free base as colorless oil. **Yield:** 125 mg (73%; 1 step; based on **TD579). NMR (CD₃CN): ¹H** δ_{H} 1.21 (C*H*₃, t, 12H, ³*J*_{HH} = 7); 2.90 *(mc,* m, 8H); 2.97 (C*H*₂-P, d, 4H, ²*J*_{HP} = 10); 2.99 (*mc,* m, 4H); 3.24 (*mc,* m, 8H); 3.55 (C≡C*H*, s, 1H); 4.01 (C*H*₂-O-P, m, 8H); 4.21 (C*H*₂-arom., s, 2H); 4.48 (C*H*₂-N₃, s, 2H); 4.55 (C*H*₂-arom., s, 2H); 7.37 (*arom.,* dd, 1H, ³*J*_{HH} = 8, ⁴*J*_{HH} = 1); 7.49 *(arom.,* dd, 1H, ³*J*_{HH} = 8, ⁴*J*_{HH} = 1); 7.51 *(arom.,* dd, 1H, ³*J*_{HH} = 8, ⁴*J*_{HH} = 1); 7.54 *(arom.,* dd, 1H, ³*J*_{HH} = 8, ⁴*J*_{HH} = 1); 7.80 *(arom.,* t, 1H, ³*J*_{HH} = 8); 7.84 *(arom.,* t, 1H, ³*J*_{HH} = 8). **³¹P{¹H}** δ_{P} 24.8 (s). **ESI-MS** (LC-MS): 734.5 [M+H]⁺ (theor. [C₃₃H₅₄N₉O₆P₂]⁺ = 734.4). 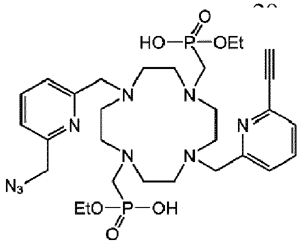 **Synthesis of TD582:** In a glass vial (4 mL), **TD580** (25 mg; 34 µmol; 1.0 equiv.) was dissolved in a mixture of aq. NaOH (10%; 1 mL) and EtOH (300 µL) and the resulting solution was stirred 2 d at RT. Reaction was then quenched with AcOH (200 µL) and the mixture was directly purified by preparative HPLC (C18; H₂O-MeCN gradient with FA additive). Fractions with product were joined and directly lyophilized to give product in nearly zwitterionic form as white fluffy solid. **Yield:** 9 mg (≤39% assuming M•0.1FA·*x*H₂O; *M*_{R} = 682.3; 1 step; based on **TD580). ESI-MS** (LC-MS): 678.4 [M+H]⁺ (theor. [C₂₉H₄₆N₉O₆P₂]⁺ = 678.3). 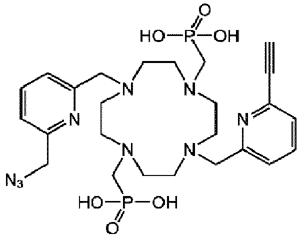 **Synthesis of TD581:** In a glass vial (4 mL), **TD580** (25 mg; 34 µmol; 1.0 equiv.) was dissolved in dry Pyridine (1 mL) followed by addition of neat **TMSBr** (110 µL; 833 µmol; ~25 equiv.) and the resulting mixture was stirred 16 h at RT. Reaction was then quenched with 50% aq. MeOH (1 mL) and the mixture was further stirred 1 h at RT. The mixture was then directly purified by preparative HPLC (C18; H₂O-MeCN gradient with FA additive). Fractions with product were joined and directly lyophilized to give product in nearly zwitterionic form as slightly yellow fluffy solid. **Yield:** 6 mg (≤28% assuming **M**·0.1FA·*x*H₂O; *M*_{R} = 626.2; 1 step; based on **TD580**). **ESI-MS** (LC-MS): 622.3 [M+H]⁺ (theor. [C₂₅H₃₈N₉O₆P₂]⁺ = 622.2). 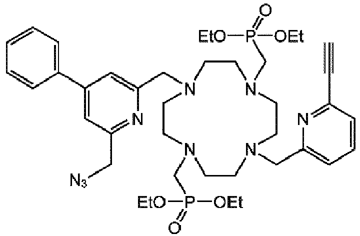 **Synthesis of TD575:** In a glass vial (20 mL), **TD579** (51 mg; 87 µmol; 1.2 equiv.) was dissolved in MeCN (3 mL) followed by addition of dried **K₂CO₃** (30 mg; 217 µmol; 3.0 equiv.). Solution of **TD566** (20 mg; 73 µmol; 1.0 equiv.) in MeCN (2 mL) was then added and the resulting suspension was stirred 16 h at RT. Solids were filtered off using syringe microfilter (PTFE) and filtrate was evaporated to dryness. Residue was purified by preparative HPLC (C18; H₂O-MeCN gradient with FA additive). Fractions with product were combined, neutralized with dil. aq. NaHCO₃ and evaporated to dryness. Residue was dissolved in DCM (50 mL) and H₂O (50 mL) and transferred to a separatory funnel. After shaking, the bottom phase was separated. Aqueous phase was further extracted with DCM (3 × 25 mL). Combined organic layers were dried with anhydrous Na₂SO₄, filtered through glass frit (S3) and evaporated to dryness. The residue was further dried on high vacuum overnight to give product in the form of free base as nearly colorless oil. **Yield:** 46 mg (90%; 1 step; based on **TD566**). **ESI-MS** (LC-MS): 810.4 [M+H]⁺ (theor. [C₃₉H₅₈N₉O₆P₂]⁺ = 810.4). **TD575** was directly used for **TD576** and without further characterization. 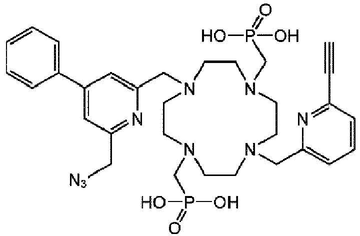 **Synthesis of TD576:** In a glass vial (4 mL), **TD575** (23 mg; 28 µmol; 1.0 equiv.) was dissolved in dry Pyridine (1 mL) followed by addition of neat **TMSBr** (220 µL; 1.67 mmol; ~60 equiv.) and the resulting mixture was stirred 16 h at RT. Reaction was then quenched with 50% aq. MeOH (1 mL) and the mixture was further stirred 1 h at RT. The mixture was then directly purified by preparative HPLC (C18; H₂O-MeCN gradient with FA additive). Fractions with product were joined and directly lyophilized to give product in nearly zwitterionic form as white fluffy solid. **Yield:** 15 mg (≤75% assuming **M**·0.1FA·*x*H₂O; *M*_{R} = 702.3; 1 step; based on **TD575**). **NMR** (**D₂O+NaOD, pD ~8**): **¹H** δ_{H} 2.54-3.76 (*mc,* C*H*₂-P, m, 16+4H); 3.93 (C*H*₂-arom., bs, 2H); 4.10 (C*H*₂-arom., bs, 2H); 4.60 (C*H*₂-N₃, s, 2H); 7.44 (*arom.,* dd, 1H, ³*J*_{HH} = 8, ⁴*J*_{HH} = 1); 7.46-7.51 *(Ph,* m, 3H); 7.53 *(arom.,* dd, 1H, ³*J*_{HH} = 8, ⁴*J*_{HH} = 1); 7.66-7.70 *(Ph,* m, 2H); 7.71 *(arom.,* d, 1H, ⁴*J*_{HH} = 2); 7.77 *(arom.,* d, 1H, ⁴*J*_{HH} = 2); 7.81 *(arom.,* t, 1H, ³*J*_{HH} = 8). **³¹P{¹H}** δ_{P} 8.2 (bs). **ESI-HRMS:** 696.2575 [M-H]⁻ (theor. [C₃₁H₄₀N₉O₆P₂]⁻ = 696.2582). 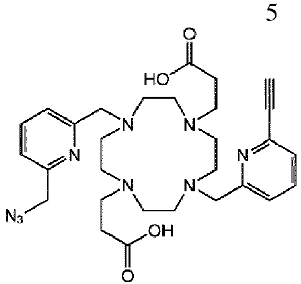 **Synthesis of TD801:** In a glass vial (20 mL), **TD799** (46 mg; 85 µmol; 1.0 equiv.) was dissolved in MeCN (5 mL) followed by addition of dried **K₂CO₃** (48 mg; 348 µmol; 4.1 equiv.). Solution of **TD406** (20 mg; 110 µmol; 1.3 equiv.) in MeCN (1 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert*-butyl protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in nearly zwitterionic form as white fluffy solid. **Yield:** 33 mg (48%; 2 steps; based on **TD799**). **NMR (D₂O, pD ~3): ¹H** δ_{H} 2.41-3.78 (*mc,* C*H*₂-CO, C*H*₂-P, m, 16+2+2H); 3.89 (C*H*₂-arom., bs, 2H); 3.95 (C*H*₂-arom., bs, 2H); 4.52 (C*H*₂-N₃, s, 2H); 7.42 *(arom.,* d, 1H, ³*J*_{HH} = 8); 7.46 *(arom.,* d, 1H, ³*J*_{HH} = 8); 7.74 *(arom.,* d, 1H, ³*J*_{HH} = 8); 7.78 (*arom.,* d, 1H, ³*J*_{HH} = 8); 7.85 (*arom.,* t, 1H, ³*J*_{HH} = 8); 7.87 (*arom.,* t, 1H, ³*J*_{HH} = 8). **ESI-HRMS:** 578.3208 [M+H]⁺ (theor. [C₂₉H₄₀N₉O₄]⁺ = 578.3203). **EA** (C₂₉H₃₉N₉O₄·2.0TFA, *M*_{R} = 805.7): C 49.2 (49.3); H 5.1 (5.4); N 15.6 (15.7). 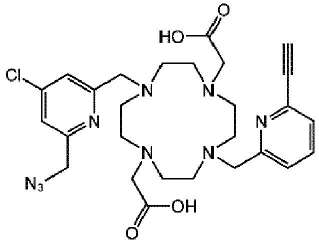 **Synthesis of TD764:** In a glass vial (20 mL), **TD635** (304 mg; 589 µmol; 1.1 equiv.) was dissolved in MeCN (7 mL) followed by addition of dried **K₂CO₃** (305 mg; 2.21 mmol; 4.0 equiv.). Solution of **TD760** (120 mg; 553 µmol; 1.0 equiv.) in MeCN (3 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in the form of trifluoroacetate salt as white fluffy solid. **Yield:** 196 mg (51%; 2 steps; based on **TD635**). **NMR (D₂O, pD ~3): ¹H** δ_{H} 2.86-3.60 *(mc,* C*H*₂-CO, m, 16+4H); 3.77 (C≡C*H*, s, 1H); 3.79-4.06 (C*H*₂-arom., bm, 4H); 4.62 (C*H*₂-N₃, s, 2H); 7.62 *(arom.,* d, 1H, ⁴*J*_{HH} = 2); 7.67-7.73 *(arom.,* m, 1H); 8.07-8.14 *(arom.,* m, 2H); 8.17 *(arom.,* d, 1H, ⁴*J*_{HH} = 2). **ESI-HRMS:** 582.2344 [M-H]⁻ (theor. [C₂₇H₃₃N₉O₄Cl₁]⁻ = 582.2350). **EA** (C₂₇H₃₄N₉O₄Cl₁·0.7TFA·1.7H₂O, *M*_{R} = 694.5): C 49.1 (49.0); H 5.5 (5.4); N 18.2 (18.4); Cl 5.1 (5.3). 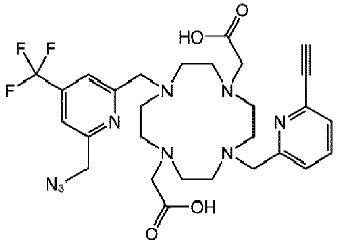 **Synthesis of TD1063:** In a glass vial (4 mL), **TD635** (50 mg; 97 µmol; 1.0 equiv.) was dissolved in MeCN (1 mL) followed by addition of dried **K₂CO₃** (67 mg; 485 µmol; 5.0 equiv.). Solution of **TD1057** (24 mg; 96 µmol; 1.0 equiv.) in MeCN (1 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert*-butyl protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in nearly zwitterionic form as white fluffy solid. **Yield:** 33.4 mg (55%; 2 steps; based on **TD635**). **NMR (D₂O, pD ~7): ¹H** δ_{H} 2.81-3.68 *(mc,* C*H*₂-CO, m, 16+4H); 3.72 (C≡C*H*, s, 1H); 3.82 (C*H*₂-arom., bs, 2H); 4.05 (C*H*₂-arom., bs, 2H); 4.70 (C*H*₂-N₃, s, 2H); 7.62-7.69 (*arom.,* m, 1H); 7.82 (*arom.,* s, 1H); 7.92-7.99 (*arom.,* m, 2H); 8.38 (*arom.,* s, 1H). **¹⁹F{¹H}** δ_{F} -64.2 (s). **ESI-HRMS:** 618.2759 [M+H]⁺ (theor. [C₂₈H₃₅N₉O₄F₃]⁺ = 618.2759). **EA** (C₂₈H₃₄N₉O₄F₃·0.1FA·1.2H₂O, *M*_{R} = 643.8): C 52.4 (52.8); H 5.7 (5.4); N 19.6 (19.4); F 8.8 (7.8). 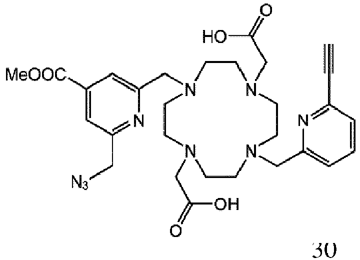 **Synthesis of TD1092:** In a glass vial (4 mL), **TD635** (35 mg; 68 µmol; 1.0 equiv.) was dissolved in MeCN (2 mL) followed by addition of dried **K₂CO₃** (44 mg; 318 µmol; 4.7 equiv.). Solution of pre-purified **TD1089** (containing ~20% of the bis azide byproduct; 20 mg; <83 µmol; <1.2 equiv.) in MeCN (1 mL) was then added and the resulting suspension was stirred 24 h at RT. Solids were filtered off using syringe microfilter (PTFE) and filtrate was evaporated to dryness. Residue was purified by preparative HPLC (C18; H₂O-MeCN gradient with FA additive). Fractions with *tert*-butyl protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in nearly zwitterionic form as white fluffy solid. **Yield:** 31.9 mg (74%; 2 steps; based on **TD635**). **NMR (D₂O, pD ~6)**: **¹H** δ_{H} 2.76-3.62 *(mc,* C*H*₂-CO, m, 16+4H); 3.73 (C≡C*H*, s, 1H); 3.83 (C*H*₂-arom., bs, 2H); 3.99 (C*H*₃, s, 3H); 4.05 (C*H*₂-arom., bs, 2H); 4.69 (C*H*₂-N₃, s, 2H); 7.70 *(arom.,* dd, 1H, ³*J*_{HH} = 8, ⁴*J*_{HH} = 1); 8.02 *(arom.,* d, 1H, ⁴*J*_{HH} = 1); 8.05 *(arom.,* dd, 1H, ³*J*_{HH} = 8, ⁴*J*_{HH} = 1); 8.15 *(arom.,* t, 1H, ³*J*_{HH} = 8); 8.58 *(arom.,* d, 1H, ⁴*J*_{HH} = 1). **ESI-HRMS:** 608.2939 [M+H]⁺ (theor. [C₂₉H₃₈N₉O₆]⁺ = 608.2940). **EA** (C₂₉H₃₇N₉O₆·0.1FA·1.1H₂O, *M*_{R} = 632.1): C 55.3 (55.1); H 6.3 (6.0); N 19.9 (19.6). 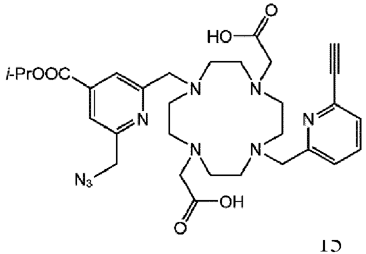 **Synthesis of TD1148:** In a glass vial (4 mL), **TD635** (44 mg; 85 µmol; 1.0 equiv.) was dissolved in MeCN (2.5 mL) followed by addition of dried **K₂CO₃** (47 mg; 340 µmol; 4.0 equiv.). Solution of **TD1112** (23 mg; 86 µmol; 1.0 equiv.) in MeCN (1 mL) was then added and the resulting suspension was stirred 3 d at RT. Solids were filtered off using syringe microfilter (PTFE) and filtrate was evaporated to dryness. Residue was purified by preparative HPLC (C18; H₂O-MeCN gradient with FA additive). Fractions with *tert*-butyl protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in nearly zwitterionic form as white fluffy solid. **Yield:** 30.7 mg (55%; 2 steps; based on **TD635**). **NMR (D₂O, pD ~6): ¹H** δ_{H} 1.40 (C*H*₃, d, 6H, ³*J*_{HH} = 6); 2.77-3.63 (*mc,* C*H*₂-CO, m, 16+4H); 3.70 (C≡C*H*, s, 1H); 3.80 (C*H*₂-arom., bs, 2H); 4.06 (C*H*₂-arom., bs, 2H); 4.65 (C*H*₂-N₃, s, 2H); 5.26 (C*H*, hept, 1H, ³*J*_{HH} = 6); 7.65 *(arom.,* dd, 1H, ³*J*_{HH} = 8, ⁴*J*_{HH} = 1); 7.93-8.00 *(arom.,* m, 2H); 8.12 *(arom.,* t, 1H, ³*J*_{HH} = 8); 8.45 *(arom.,* d, 1H, ⁴*J*_{HH} = 2). **ESI-HRMS:** 636.3247 [M+H]⁺ (theor. [C₃₁H₄₂N₉O₆]⁺ = 636.3253). **EA** (C₃₁H₄₁N₉O₆·0.1FA·1.0H₂O, *M*_{R} = 658.3): C 56.7 (56.4); H 6.6 (6.4); N 19.1 (18.8). 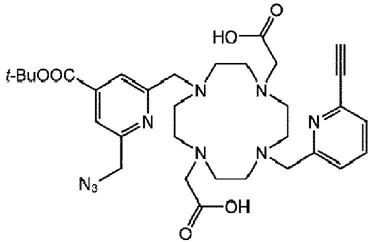 **Synthesis of TD1160:** In a pear-shape glass flask (100 mL), **TD1116** (50 mg; 158 µmol; 2.0 equiv.) was dissolved in MeCN (20 mL) followed by addition of dried **K₂CO₃** (11 mg; 80 µmol; 1.0 equiv.). Solution of **TD558** (12 mg; 79 µmol; 1.0 equiv.) in MeCN (20 mL) was then added dropwise over the course of 2 h and the resulting suspension was stirred 7 d at RT. Solids were filtered off using syringe microfilter (PTFE) and filtrate was evaporated to dryness. Residue was purified by preparative HPLC (C18; H₂O-MeCN gradient with FA additive). Fractions with mono alkylated intermediate were combined and directly lyophilized. Resulting solid (15.4 mg; ≤36 µmol; 1.0 equiv.) was dissolved in MeCN (2 mL) followed by addition of dried **K₂CO₃** (25 mg; 181 µmol; ≥5.0 equiv.). Solution of **TD1130** (15 mg; 53 µmol; ≥1.5 equiv.) in MeCN (1 mL) was then added and the resulting suspension was stirred 7 d at RT. Solids were filtered off using syringe microfilter (PTFE) and filtrate was evaporated to dryness. Residue was purified by preparative HPLC (C18; H₂O-MeCN gradient with FA additive). Fractions with methyl protected product were joined and directly lyophilized. Residue was re-dissolved in a mixture of MeOH (2 mL) and H₂O (1 mL) followed by addition of solid **LiOH·**H₂O (15 mg; 360 µmol; >10 equiv.). The resulting mixture was stirred 15 min at RT, quenched with FA (15 µL) and then directly purified by preparative HPLC (C18; H₂O-MeCN gradient with FA additive). Fractions with product were joined and directly lyophilized to give product as white solid. **Yield:** 1.7 mg (<5%; 3 steps; based on **TD558**). **ESI-HRMS:** 650.3407 [M+H]⁺ (theor. [C₃₂H₄₄N₉O₆]⁺ = 650.3409). 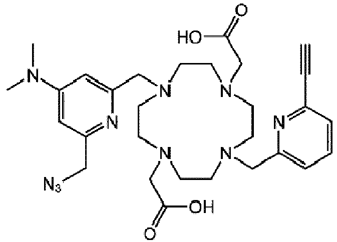 **Synthesis of TD1176:** In a glass vial (4 mL), **TD635** (43.5 mg; 84.4 µmol; 1.1 equiv.) was dissolved in MeCN (2 mL) followed by addition of dried **K₂CO₃** (42.5 mg; 308 µmol; 4.0 equiv.). Solution of **TD1163** (17.3 mg; 76.7 µmol; 1.0 equiv.) in MeCN (1 mL) was then added and the resulting suspension was stirred 2 d at RT. Solids were filtered off using syringe microfilter (PTFE) and filtrate was evaporated to dryness. Residue was purified by preparative HPLC (C18; H₂O-MeCN gradient with FA additive). Fractions with *tert*-butyl protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in the form of formate salt as white fluffy solid. **Yield:** 43.6 mg (80%; 2 steps; based on **TD1163**). **NMR (D₂O, pD ~7): ¹H** δ_{H} 2.74-3.62 *(mc,* C*H*₂-CO, C*H*₃, m, 16+4+6H); 3.70 (C≡C*H*, s, 1H); 3.76 (C*H*₂-arom., bs, 2H); 3.84 (C*H*₂-arom., bs, 2H); ~4.7-4.8 (C*H*₂-N₃, obscured by HOD signal); 6.85 *(arom.,* bs, 1H); 7.02 *(arom.,* bs, 1H); 7.64 *(arom.,* dd, 1H, ³*J*_{HH} = 8, ⁴*J*_{HH} = 1); 7.88 (*arom.,* bt, 1H, ³*J*_{HH} = 8); 7.94 (*arom.,* bd, 1H, ³*J*_{HH} = 8). **ESI-HRMS:** 593.3310 [M+H]⁺ (theor. [C₂₉H₄₁N₁₀O4]⁺ = 593.3307). **EA** (C₂₉H₄₀N₁₀O_{4·}0.8FA·4.4H₂O, *M*_{R} = 708.8): C 50.5 (50.8); H 7.2 (7.1); N 19.8 (19.5). 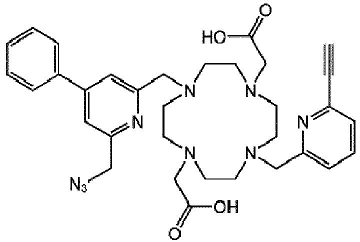 **Synthesis of TD647:** In a pear-shape glass flask (50 mL), **TD635** (375 mg; 727 µmol; 1.0 equiv.) was dissolved in dry MeCN (20 mL) followed by addition of dried **K₂CO₃** (300 mg; 2.17 mmol; 3.0 equiv.). Solution of **TD566** (189 mg; 731 µmol; 1.0 equiv.) in dry MeCN (5 mL) was then added and the resulting suspension was stirred 24 h at RT. Solids were filtered off using syringe microfilter (PTFE) and filtrate was evaporated to dryness. Residue was purified by preparative HPLC (C18; H₂O-MeCN gradient with FA additive). Fractions with *tert*-butyl protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in nearly zwitterionic form as white fluffy solid. **Yield:** 342 mg (72%; 2 steps; based on **TD635**). **NMR (D₂O, pD ~4): ¹H** δ_{H} 2.70-3.59 *(mc,* C*H*₂-CO, m, 16+4H); 3.66 (C*H*₂-arom., bs, 2H); 3.69 (C≡C*H*, s, 1H); 3.90 (C*H*₂-arom., bs, 2H); 4.64 (C*H*₂-N₃, s, 2H); 7.46-7.60 (*Ph, arom.,* m, 3+1H); 7.64 *(arom.,* dd, 1H, ³*J*_{HH} = 8, ⁴*J*_{HH} = 2); 7.68 *(arom.,* t, 1H, ³*J*_{HH} = 8); 7.69 *(arom.,* d, 1H, ⁴*J*_{HH} = 2); 7.79-7.88 (*Ph,* m, 2H); 8.28 (*arom.,* d, 1H, ⁴*J*_{HH} = 2). **¹³C{¹H}** δ_{C} 48.2 (*mc,* s); 48.3 (*mc,* s); 51.2 (*mc,* s); 51.3 (*mc,* s); 54.7 (*C*H₂-N₃, s); 56.4 (*C*H₂-CO, s); 57.8 (*C*H₂-arom., s); 59.1 (*C*H₂-arom., s); 80.3 and 82.4 (*C*≡*C*H; 2 × s); 121.9 *(arom.,* s); 124.4 *(arom.,* s); 125.6 *(arom.,* s); 128.2 *(Ph,* s); 128.5 (*Ph, s*); 130.0 (*Ph,* s); 130.8 *(arom.,* s); 137.7 *(Ph,* s); 140.3 (*arom.,* s); 141.2 (*arom.,* s); 153.6 (*arom.,* s); 155.4 (*arom.,* bs); 156.2 (*arom.,* s); 157.6 (*arom.,* s); 168.7 (*C*O, s). **ESI-HRMS:** 626.3195 [M+H]⁺ (theor. [C₃₃H₄₀N₉O₄]⁺ = 626.3198). **EA** (C₃₃H₃₉N₉O₄·0.2FA·0.9H₂O, *M*_{R} = 651.2): C 61.2 (61.6); H 6.4 (6.1); N 19.4 (19.1). 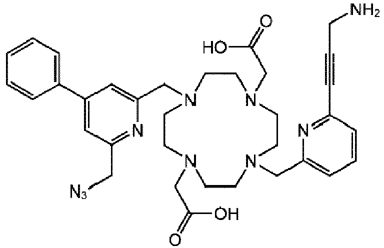 **Synthesis of TD722:** In a glass vial (20 mL), **TD539** (350 mg; 543 µmol; 1.0 equiv.) was dissolved in MeCN (15 mL) followed by addition of dried **K₂CO₃** (375 mg; 2.72 mmol; 5.0 equiv.). Solution of **TD566** (140 mg; 541 µmol; 1.0 equiv.) in MeCN (3 mL) was then added and the resulting suspension was stirred 24 h at RT. Solids were filtered off using syringe microfilter (PTFE) and filtrate was evaporated to dryness. Residue was purified by preparative HPLC (C18; H₂O-MeCN gradient with FA additive). Fractions with *tert*-butyl/Boc protected product were combined, evaporated to dryness and twice co-evaporated with MeOH to remove most of MeCN. Residue was dissolved in TFA (5 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in the form of trifluoroacetate salt as slightly yellow solid. **Yield:** 295 mg (69%; 2 steps; based on **TD566**). **NMR (D₂O, pD ~7): ¹H** δ_{H} 2.74-3.64 *(mc,* C*H*₂-CO, m, 16+4H); 3.72 (C*H*₂-arom., bs, 2H); 3.98 (C*H*₂-arom., bs, 2H); 4.10 (C*H*₂-C≡C, s, 2H); 4.65 (C*H*₂-N₃, s, 2H); 7.47-7.69 *(Ph, arom.,* m, 3+3H); 7.72 (*arom.,* d, 1H, ⁴*J*_{HH} = 2); 7.85-7.96 *(Ph,* m, 2H); 8.41 *(arom.,* d, 1H, ⁴*J*_{HH} = 2). **ESI-HRMS:** 655.3460 [M+H]⁺ (theor. [C₃₄H₄₃N₁₀O₄]⁺ = 655.3463). **EA** (C₃₄H₄₂N₁₀O₄·0.7TFA·2.0H₂O, *M*_{R} = 770.6): C 55.2 (54.9); H 6.1 (5.8); N 18.2 (18.3); F 5.2 (5.3). 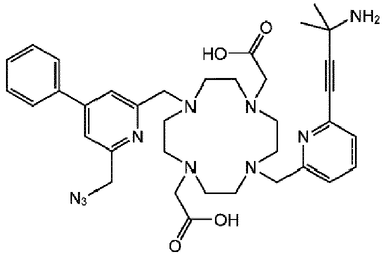 **Synthesis of TD1054:** In a glass vial (4 mL), **TD663** (30.0 mg; 45.3 µmol; 1.0 equiv.) was dissolved in MeCN (1 mL) followed by addition of dried **K₂CO₃** (37.5 mg; 271 µmol; 5.0 equiv.). Solution of freshly prepared and isolated **TD1050** (≤65.3 µmol; ≤1.5 equiv.) in MeCN (1 mL) was then added and the resulting suspension was stirred 3 d at RT. Solids were filtered off using syringe microfilter (PTFE) and filtrate was evaporated to dryness. Residue was purified by preparative HPLC (C18; H₂O-MeCN gradient with FA additive). Fractions with *tert*-butyl/Boc protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in the form of mixed trifluoroacetate/formate salt as colourless solid. **Yield:** 23.0 mg (65%; 2 steps; based on **TD663**). **NMR (D₂O, pD ~8): ¹H** δ_{H} 1.72 (C*H*₃, s, 6H); 2.67-3.61 *(mc,* C*H*₂-CO, m, 16+4H); 3.70 (C*H*₂-arom., bs, 2H); 3.97 (C*H*₂-arom., bs, 2H); 4.64 (C*H*₂-N₃, s, 2H); 7.45-7.67 *(Ph, arom.,* m, 3+3H); 7.72 *(arom.,* d, 1H, ⁴*J*_{HH} = 2); 7.86-7.93 *(Ph,* m, 2H); 8.40 *(arom.,* d, 1H, ⁴*J*_{HH} = 2). **ESI-HRMS:** 683.3775 [M+H]⁺ (theor. [C₃₆H₄₇N₁₀O₄]⁺ = 683.3776). **EA** (C₃₆H₄₆N₁₀O₄·0.3TFA·0.8FA·2.3H₂O, *M*_{R} = 777.3): C 57.8 (58.0); H 6.5 (6.5); N 18.0 (17.7). 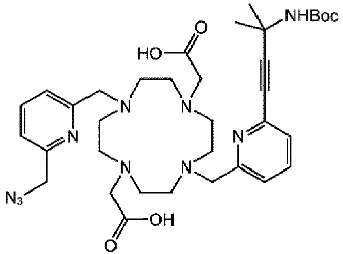 **Synthesis of TD1105:** In a glass vial (4 mL), **TD711** (101 mg; 185 µmol; 1.0 equiv.) was dissolved in MeCN (2 mL) followed by addition of dried **K₂CO₃** (102 mg; 738 µmol; 4.0 equiv.). Solution of freshly prepared and isolated **TD1050** (≤222 µmol; ≤1.2 equiv.) in MeCN (1 mL) was then added and the resulting suspension was stirred 24 h at 40 °C. Solids were filtered off using syringe microfilter (PTFE) and filtrate was evaporated to dryness. Residue was purified by preparative HPLC (C18; H₂O-MeCN gradient with FA additive). Fractions with *tert*-butyl/Boc protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with fully deprotected intermediate were joined and directly lyophilized. The resulting solid was dissolved in a mixture of H₂O (6 mL), MeCN (6 mL) and THF (2 mL) followed by addition of **Boc₂O** (2.0 M in dry THF; 315 µL; 630 µmol; ≥3.4 equiv.) and **NaHCO₃** (210 mg; 2.50 mmol; ≥13.5 equiv.) The resulting mixture was stirred 2 d at RT after which another portion of **Boc₂O** (2.0 M in dry THF; 315 µL; 630 µmol; ≥3.4 equiv.) was added. The mixture was further stirred 16 h at RT. Mixture was evaporated to dryness and the residue was directly purified by preparative HPLC (C18; H₂O-MeCN gradient with FA additive). Fractions with product were joined to give product in nearly zwitterionic form as white fluffy solid. **Yield:** 36.8 mg (27%; 3 steps; based on **TD711**). **NMR (D₂O, pD ~7): ¹H** δ_{H} 1.47 (C*H*₃, s, 9H); 1.65 (C*H*₃, s, 6H); 2.85-3.65 *(mc,* C*H*₂-CO, m, 16+4H); 3.91 (C*H*₂-arom., bs, 2H); 3.95 (C*H*₂-arom., bs, 2H); 4.59 (C*H*₂-N₃, s, 2H); 7.51 *(arom.,* dd, 1H, ³*J*_{HH} = 7, ⁴*J*_{HH} = 2); 7.59 *(arom.,* dd, 1H, ³*J*_{HH} = 7, ⁴*J*_{HH} = 2); 7.89-8.05 (*arom.,* m, 4H). **ESI-HRMS:** 707.3988 [M+H]⁺ (theor. [C₃₅H₅₁N₁₀O₆]⁺ = 707.3988). **EA** (C₃₅H₅₀N₁₀O₆·0.1FA·2.0H₂O, *M*_{R} = 747.5): C 56.4 (56.7); H 7.3 (7.0); N 18.7 (18.5). 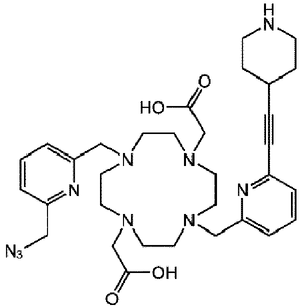 **Synthesis of TD1127:** In a glass vial (20 mL), **TD1118** (56.9 mg; 81.4 µmol; 1.0 equiv.) was dissolved in MeCN (2 mL) followed by addition of dried **K₂CO₃** (45.0 mg; 326 µmol; 4.0 equiv.) and **TD406** (16.0 mg; 87.6 µmol; 1.1 equiv.) in MeCN (1 mL). The resulting suspension was stirred 16 h at RT. Solids were filtered off using syringe microfilter (PTFE) and filtrate was directly purified by preparative HPLC (C18; H₂O-MeCN gradient with FA additive). Fractions with *tert*-butyl/Boc protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in the form of mixed trifluoroacetate/formate salt as colourless solid. **Yield:** 49.0 mg (71%; 2 steps; based on **TD1118**). **NMR (D₂O, pD ~7): ¹H** δ_{H} 1.96-2.11 (C*H*₂, m, 2H); 2.14-2.29 (C*H*₂, m, 2H); 2.86-3.63 *(mc,* C*H*₂-CO, C*H*₂*, CH,* m, 16+4+4+1H); 3.90 (C*H*₂-arom., bs, 2H); 3.94 (C*H*₂-arom., bs, 2H); 4.59 (C*H*₂-N₃, s, 2H); 7.49-7.55 (*arom.,* m, 1H); 7.60 (*arom.,* dd, 1H, ³*J*_{HH} = 8, ⁴*J*_{HH} = 2); 7.92-8.04 *(arom.,* m, 4H). **ESI-HRMS:** 633.3612 [M+H]⁺ (theor. [C₃₂H₄₅N₁₀O₄]⁺ = 633.3620). **EA** (C₃₂H₄₄N₁₀O₄·1.8TFA·0.1FA, *M*_{R} = 878.6): C 50.9 (50.7); H 5.5 (5.9); N 16.6 (16.9). 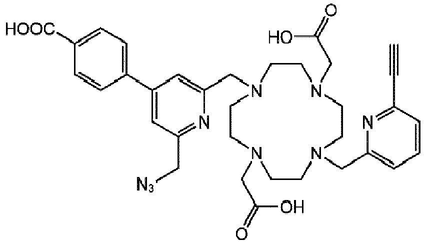 **Synthesis of TD742:** In a glass vial (20 mL), **TD635** (90 mg; 163 µmol; 1.1 equiv.) was dissolved in MeCN (6 mL) followed by addition of dried **K₂CO₃** (85 mg; 616 µmol; 4.0 equiv.). Solution of **TD733** (55 mg; 153 µmol; 1.0 equiv.) in MeCN (2 mL) was then added and the resulting suspension was stirred 18 h at RT. Solids were filtered off using syringe microfilter (PTFE) and filtrate was evaporated to dryness. Residue was purified by preparative HPLC (C18; H₂O-MeCN gradient with FA additive). Fractions with *tert-butyl* protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in nearly zwitterionic form as white fluffy solid. **Yield:** 87 mg (80%; 2 steps; based on **TD733**). **NMR (D₂O, pD ~4): ¹H** δ_{H} 2.76-3.64 *(mc,* C*H*₂-CO, m, 16+4H); 3.69 (C≡C*H*, s, 1H); 3.72 (C*H*₂-arom., bs, 2H); 3.99 (C*H*₂-arom., bs, 2H); 4.77 (C*H*₂-N₃, s, 2H); 7.54-7.70 (*arom.,* m, 3H); 7.85 (*arom.,* d, 1H, ⁴*J*_{HH} = 2); 7.96 (*arom.,* d, 2H, ³*J*_{HH} = 8); 8.11 (*arom.,* d, 2H, ³*J*_{HH} = 8); 8.38 (*arom.,* d, 1H, ⁴*J*_{HH} = 2). **ESI-HRMS:** 670.3094 [M+H]⁺ (theor. [C₃₄H₄₀N₉O₆]⁺ = 670.3096). **EA** (C₃₄H₄₀N₉O₆·0.2FA·1.6H₂O, *M*_{R} = 707.8): C 58.0 (58.4); H 6.1 (5.9); N 17.8 (17.5). 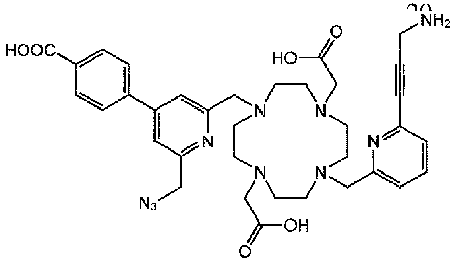 **Synthesis of TD744:** In a glass vial (20 mL), **TD539** (80 mg; 124 µmol; 1.1 equiv.) was dissolved in MeCN (3 mL) followed by addition of dried **K₂CO₃** (65 mg; 471 µmol; 4.0 equiv.). Solution of **TD733** (42 mg; 117 µmol; 1.0 equiv.) in MeCN (3 mL) was then added and the resulting suspension was stirred 24 h at RT. Solids were filtered off using syringe microfilter (PTFE) and filtrate was evaporated to dryness. Residue was purified by preparative HPLC (C18; H₂O-MeCN gradient with FA additive). Fractions with protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in the form of mixed trifluoroacetate/formate salt as off white fluffy solid. **Yield:** 44 mg (45%; 2 steps; based on **TD733**). **NMR (D₂O, pD ~4): ¹H** δ_{H} 2.71-3.64 (*mc,* C*H*₂-CO, m, 16+4H); 3.70 (C*H*₂-arom., bs, 2H); 3.98 (C*H*₂-arom., bs, 2H); 4.12 (C*H*₂-C≡C, s, 2H); 4.70 (C*H*₂-N₃, s, 2H); 7.50-7.59 *(arom.,* m, 2H); 7.63 *(arom.,* dd, 1H, ³*J*_{HH} = 7, ⁴*J*_{HH} = 2); 7.78 *(arom.,* d, 1H, ⁴*J*_{HH} = 2); 7.95 (*arom.,* d, 2H, ³*J*_{HH} = 9); 8.07 *(arom.,* d, 2H, ³*J*_{HH} = 9); 8.42 *(arom.,* d, 1H, ⁴*J*_{HH} = 2). **ESI-HRMS:** 699.3358 [M+H]⁺ (theor. [C₃₅H₄₃N₁₀O₆]⁺ = 699.3362). **EA** (C₃₅H₄₂N₁₀O₆·1.0TFA·1.3H₂O, *M*_{R} = 836.2): C 53.1 (52.8); H 5.5 (5.8); N 16.8 (16.9). **Synthesis of TD750:** In a glass vial (2 mL), **TD744·**1.0TFA·1.3H₂O (1.5 mg; ~1.8 µmol; 1.0 equiv.) was dissolved in aq. **Borate/NaOH buffer** (200 mM; pH 9.0; 900 µL; 180 mmol; ~100 equiv.). followed by addition of freshly prepared solution of **FmocCl** (0.6 mg; ~2.3 µmol; 1.3 equiv.) in MeCN (900 µL). The resulting clear solution was stirred 2 h at RT. Mixture was then directly purified by preparative HPLC (C18; H₂O-MeCN gradient with FA additive). Fractions with product were joined and directly lyophilized to give product in nearly zwitterionic form as white fluffy solid. **Yield:** 0.8 mg (≤48% assuming **M**·0.2FA·*x*H₂O; *M*_{R} = 930.2; 1 step; based on **TD744**·1.0TFA·1.3H₂O). **ESI-HRMS:** 921.4044 [M+H]⁺ (theor. [C₅₀H₅₃N₁₀O₈]⁺ = 921.4042). 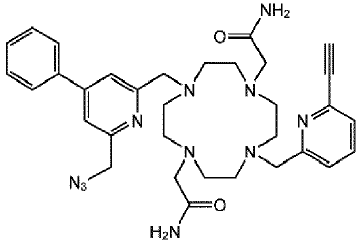 **Synthesis of TD779:** In a glass vial (4 mL), **TD647**·0.2FA·0.9H₂O (10 mg; 15 µmol; 1.0 equiv.) and **Ammonium chloride** (2 mg; 38 µmol; 2.5 equiv.) were dissolved in DMSO (2 mL) followed by addition of **DIPEA** (22 µL; 126 µmol; 8.2 equiv.) and solid **HATU** (18 mg; 47 µmol; 3.1 equiv.). The resulting yellow solution was stirred 15 min at RT. Mixture was then quenched with **FA** (6 µL; 169 µmol; 10 equiv.) and directly purified by preparative HPLC (C18; H₂O-MeCN gradient with FA additive). Fractions with product were joined and directly lyophilized to give product in nearly zwitterionic form as white fluffy solid. **Yield:** 7 mg (≤73% assuming **M·**0.1FA·*x*H₂O; *M*_{R} = 628.4; 1 step; based on **TD647**·0.2FA·0.9H₂O). **NMR (D₂O+FA, pD ~3): ¹H** δ_{H} 2.83-3.74 *(mc,* C*H*₂-CO, bm, 16+4H); 3.65 (C≡C*H*, s, 1H); 4.13-4.68 (C*H*₂-arom., bm, 4H); 4.69 (C*H*₂-N₃, s, 2H); 7.52-7.66 *(Ph, arom.,* m, 3+1H); 7.71 *(arom.,* dd, 1H, ³*J*_{HH} = 8, ⁴*J*_{HH} = 1); 7.80-7.88 *(Ph,* m, 2H); 7.90 *(arom.,* d, 1H, ⁴*J*_{HH} = 2); 7.91 *(arom.,* t, 1H, ³*J*_{HH} = 8); 8.00 (*arom.,* bs, 1H). **ESI-HRMS:** 624.3503 [M+H]⁺ (theor. [C₃₃H₄₂N₁₁O₂]⁺ = 624.3504). 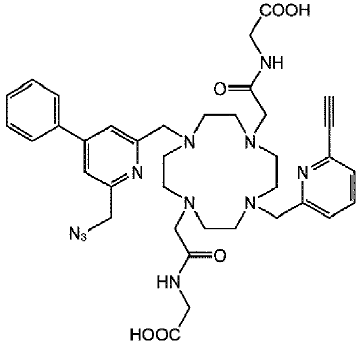 **Synthesis** of TD778: In a glass vial (4 mL), **TD647·**0.2FA·0.9H₂O (12 mg; 18 µmol; 1.0 equiv.) and **Glycine *tert-butyl* ester hydrochloride** (8 mg; 48 µmol; 2.6 equiv.) were dissolved in DMSO (2 mL) followed by addition of DIPEA (26 µL; 149 µmol; 8.1 equiv.) and solid HATU (21 mg; 55 µmol; 3.0 equiv.). The resulting yellow solution was stirred 20 min at RT. Mixture was then quenched with **FA** (7 µL; 186 µmol; 10 equiv.) and directly purified by preparative HPLC (C18; H₂O-MeCN gradient with FA additive). Fractions with *tert*-butyl protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in nearly zwitterionic form as white fluffy solid. **Yield:** 9 mg (62%; 2 steps; based on **TD647·**0.2FA·0.9H₂O). **NMR (D₂O, pD ~5): ¹H** δ_{H} 2.83-3.93 *(mc,* C*H*₂-CO, C≡C*H*, bm, 16+8+1H); 4.35-4.69 (C*H*₂-arom., C*H*₂-N₃., bm, 4+2H); 7.49 (*arom.,* d, 1H, ³*J*_{HH} = 8); 7.56-7.73 *(Ph, arom.,* m, 3+1H); 7.75-7.92 *(Ph, arom.,* m, 2+2H); 8.06 (*arom.,* s, 1H). **ESI-HRMS:** 738.3471 [M-H]⁻ (theor. [C₃₇H₄₄N₁₁O₆]⁻ = 738.3482). **EA** (C₃₇H₄₅N₁₁O₆·0.1FA·2.4H₂O, *M*_{R} = 787.7): C 56.6 (55.9); H 6.4 (5.8); N 19.6 (20.3).

### Example 5: Synthesis of caged macrocyclic ligands (bridged with a triazole group)

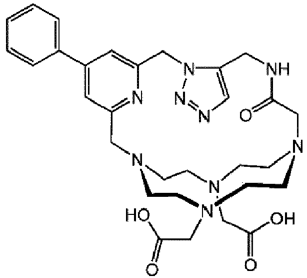

**Synthesis of cz-TD425:** Obtained as side product during synthesis of **TD425** in nearly zwitterionic form as white fluffy solid. **Yield:** 3 mg (≤1% assuming **M·**0.1FA·*x*H₂O; *M*_{R} = 609.9; 3 steps; based on **2-Chloro-*N*-(prop-2-yn-1-yl)acetamide). NMR (D₂O, pD ~3): ¹H** δ_{H} 2.52-5.20 (*mc,* C*H*₂-CO, C*H*₂-NH-CO-C*H*₂, C*H*₂-arom., bm, 16+4+4+2H); 6.24 (C*H*₂-N₃., bs, 2H); 7.56-7.72 *(Ph,* m, 3H); 7.88 (C*H*-N₃, s, 1H); 7.88-7.94 *(Ph,* m, 2H); 8.33 (*arom.,* d, 1H, ⁴*J*_{HH} = 2); 8.73 *(arom.,* d, 1H, ⁴*J*_{HH} = 2). **ESI-HRMS:** 606.3143 [M+H]⁺ (theor. [C₃₀H₄₀N₉O₅]⁺ = 606.3147). 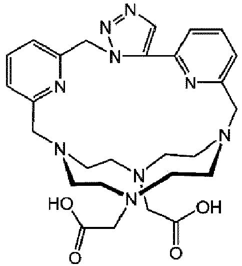 **Synthesis of cz-TD556:** Obtained as side product during synthesis of **TD556** in the form of trifluoroacetate salt as white fluffy solid. **Yield:** 9 mg (~2%; 2 steps; based on **TD635**). **NMR (D₂O, pD ~3): ¹H** δ_{H} 2.54-4.06 *(mc,* C*H*₂-CO, bm, 16+4H); 4.59 (C*H*₂-arom., bs, 2H); 5.02 (C*H*₂-arom., bs, 2H); 5.86 (C*H*₂-N₃, bs, 2H); 7.67 (*arom.,* bd, 1H, ³*J*_{HH} = 8); 7.70 *(arom.,* bd, 1H, ³*J*_{HH} = 8); 7.83 *(arom.,* bd, 1H, ³*J*_{HH} = 8); 7.87 *(arom.,* bd, 1H, ³*J*_{HH} = 8); 8.14 *(arom.,* bt, 1H, ³*J*_{HH} = 8); 8.16 (C*H*-N₃, bs, 1H); 8.17 *(arom.,* bt, 1H, ³*J*_{HH} = 8). **ESI-HRMS:** 550.2889 [M+H]⁺ (theor. [C₂₇H₃₆N₉O₄]⁺ = 550.2885). **EA** (C₂₇H₃₅N₉O₄·2.7TFA·0.5H₂O, *M*_{R} = 866.4): C 44.9 (44.8); H 4.5 (4.8); N 14.5 (14.8). 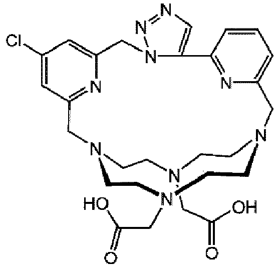 **Synthesis of cz-TD764:** Obtained as side product during synthesis of **TD764** in the form of trifluoroacetate salt as white fluffy solid. **Yield:** 9 mg (~2%; 2 steps; based on **TD635**). **NMR (D₂O, pD ∼3): ¹H** δ_{H} 2.48-3.94 *(mc,* C*H*₂-CO, bm, 16+4H); 4.57 (C*H*₂-arom., bs, 2H); 5.00 (C*H*₂-arom., bs, 2H); 5.94 (C*H*₂-N₃, bs, 2H); 7.64 (*arom.,* d, 1H, ³*J*_{HH} = 8); 7.79 (*arom.,* s, 1H); 7.84 (*arom.,* d, 1H, ³*J*_{HH} = 8); 7.92 (*arom.,* s, 1H); 8.14 *(arom.,* t, 1H, ³*J*_{HH} = 8); 8.15 (C*H*-N₃, s, 1H). **ESI-HRMS:** 582.2344 [M-H]⁻ (theor. [C₂₇H₃₃N₉O₄Cl₁]⁻ = 582.2350). **EA** (C₂₇H₃₄N₉O₄Cl₁·1.8TFA·3.3H₂O, *M*_{R} = 848.7): C 43.3 (43.3); H 5.0 (4.5); N 14.9 (14.5); Cl 4.2 (4.2); F 12.1 (12.0). 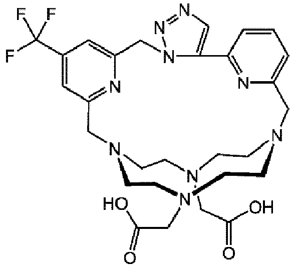 **Synthesis of cz-TD1063:** Obtained as side product during synthesis of **TD1113** in the form of trifluoroacetate salt as white fluffy solid. **Yield:** 1.7 mg. **NMR (D₂O, pD ~3): ¹H** δ_{H} 2.57-3.84 (*mc,* C*H*₂-CO, m, 16+4H); 4.70 (C*H*₂-arom., s, 2H); 4.99 (C*H*₂-arom., bs, 2H); 6.03 (C*H*₂-N₃, bs, 2H); 7.65 (*arom.,* d, 1H, ³*J*_{HH} = 8); 7.86 (*arom.,* d, 1H, ³*J*_{HH} = 8); 8.05 (*arom.,* s, 1H); 8.12-8.18 (*arom.,* C*H*-N₃, m, 1+1H); 8.19 (*arom.,* s, 1H). **¹⁹F** δ_{F} -65.82 (s). **NMR (∼0.5 mM cz-TD1063 in ∼50 mM MOPS/NaOH buffer in 10% H₂O, pH 7.0): ¹⁹F** δ_{F} -65.66 (s; ΔH_{½} = 4.6 Hz). **ESI-HRMS:** 618.2754 [M+H]⁺ (theor. [C₂₉H₃₅N₉O₄F₃]⁺ = 618.2759). 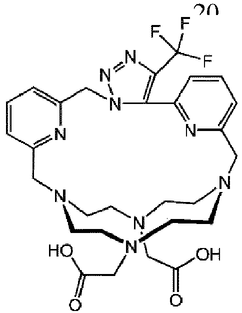 **Synthesis of TD1188:** In a glass vial (4 mL), **TD711** (70.0 mg; 128 µmol; 1.0 equiv.) was dissolved in MeCN (1.5 mL) followed by addition of dried **K₂CO₃** (71 mg; 514 µmol; 4.0 equiv.). Solution of **TD1178** (31 mg; 141 µmol; 1.1 equiv.) in MeCN (1.5 mL) was then added and the resulting suspension was stirred 7 d at RT. Solids were filtered off using syringe microfilter (PTFE) and filtrate was evaporated to dryness. Residue was purified by preparative HPLC (C18; H₂O-MeCN gradient with FA additive). Fractions with *tert*-butyl protected product were combined, evaporated to dryness and twice co-evaporated with MeOH to remove most of MeCN. Residue was dissolved in TFA (3 mL) and the resulting clear mixture was stirred 16 h at RT. Mixture was evaporated to dryness and twice co-evaporated with MeOH to remove most of TFA. Residue was directly purified by preparative HPLC (C18; H₂O-MeCN gradient with FA additive). Fractions with product were joined and directly lyophilized to give product in zwitterionic form as white fluffy solid. **Yield:** 9.5 mg (10%; 2 steps; based on **TD711**). **NMR (D₂O, pD ~5): ¹H** δ_{H} 2.11-4.00 (*mc,* C*H*₂-CO, bm, 16+4H); 4.52 (C*H*₂-arom., bs, 2H); 4.88-6.58 (C*H*₂-arom., C*H*₂-N₃, bm, 4H); 7.66 (*arom.,* bd, 1H, ³*J*_{HH} = 8); 7.68 (*arom.,* d, 1H, ³*J*_{HH} = 8); 7.75 (*arom.,* bd, 1H, ³*J*_{HH} = 8); 7.85 (*arom.,* d, 1H, ³*J*_{HH} = 8); 8.08 (*arom.,* bt, 1H, ³*J*_{HH} = 8); 8.16 (*arom.,* t, 1H, ³*J*_{HH} = 8). **¹⁹F** δ_{F} -59.35 (s). **ESI-HRMS:** 618.2756 [M+H]⁺ (theor. [C₂₈H₃₅N₉O₄F₃]⁺ = 618.2759). **EA** (C₂₈H₃₄N₉O₄F₃·0.2TFA·0.4FA·4.3H₂O, *M*_{R} = 736.3): C 47.0 (46.6); H 6.0 (5.6); N 17.1 (16.8); F 9.3 (10.1). 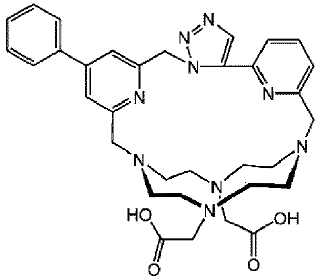 **Synthesis of TD650:** In a glass vial (40 mL), **TD647·**0.2FA·0.9H₂O (130 mg; 200 µmol; 1.0 equiv.) was dissolved in H₂O (20 mL) followed by addition aq. **Citric acid** (100 mM; 20 mL; 2.0 mmol; 10 equiv.) to reach pH 2.4. Resulting mixture was then stirred 2 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 109 mg (77%; 1 step; based on **TD647·**0.2FA·0.9H₂O). **NMR (D₂O, pD ~5,95 °C): ¹H** δ_{H} 2.69-3.63 *(mc,* C*H*₂-CO, m, 16+4H); 4.22 (C*H*₂-arom., s, 2H); 4.58 (C*H*₂-arom., s, 2H); 6.22 (C*H*₂-N₃, s, 2H); 7.61 (*arom.,* dd, 1H, ³*J*_{HH} = 8, ⁴*J*_{HH} = 1); 7.60-7.67 *(Ph,* m, 3H); 7.83 (*arom.,* s, 1H); 7.85 (*arom.,* dd, 1H, ³*J*_{HH} = 8, ⁴*J*_{HH} = 1); 7.86-7.92 *(Ph,* m, 2H); 8.00 *(arom.,* s, 1H); 8.11 *(arom.,* t, 1H, ³J_{HH} = 8); 8.21 (C*H*-N₃, s, 1H). **¹³C{¹H}** δ_{C} 48.5 *(mc,* s); 49.9 *(mc,* s); 50.2 *(mc,* s); 50.4 *(mc,* s); 53.4 (CH₂-N₃, s); 55.6 (*C*H₂-CO, s); 57.8 (C*H*₂-arom., s); 59.2 (*C*H₂-arom., s); 122.6 (*arom.,* s); 124.2 (*arom.,* s); 125.4 (*arom.,* s); 125.9 (*arom.,* s); 127.8 *(Ph,* s); 129.9 *(Ph,* s); 130.7 *(Ph,* s); 134.5 (*C*H-N₃, s); 137.0 *(Ph,* s); 138.0 (*arom.,* s); 139.9 (*arom.,* s); 147.1 (*arom.,* s); 152.3 (*arom.,* bs); 153.6 (*arom.,* bs); 153.8 (*arom.,* bs); 156.8 (*arom.,* s); 172.6 (CO, s). **ESI-HRMS:** 626.3200 [M+H]⁺ (theor. [C₃₃H₄₀N₉O₄]⁺ = 626.3198). **EA** (C₃₃H₃₉N₉O₄·0.9FA·2.0H₂O, *M*_{R} = 703.2): C 57.9 (58.3); H 6.4 (5.9); N 17.9 (17.5). 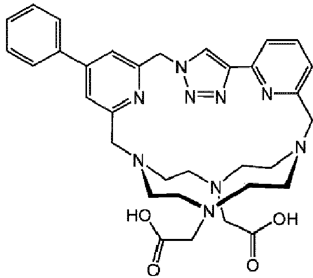 **Synthesis of TD871:** In a glass vial (40 mL), **TD647**·1.3TFA (78.0 mg; 103 µmol; 1.0 equiv.) was dissolved in H₂O (34 mL) followed by addition aq. **Borate/CsOH buffer** (200 mM; pH 9.0; 6.0 mL; 1.2 mmol; ~12 equiv.). Resulting mixture was then stirred 6 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 49.3 mg (70%; 1 step; based on **TD647·**1.3TFA). **NMR (D₂O, pD ~5,95 °C): ¹H** δ_{H} 2.93-3.56 *(mc,* C*H*₂-CO, m, 16+4H); 4.41 (C*H*₂-arom., s, 2H); 4.49 (C*H*₂-arom., s, 2H); 6.00 (C*H*₂-N₃, s, 2H); 7.54 *(arom.,* dd, 1H, ³*J*_{HH} = 7, ⁴*J*_{HH} = 2); 7.56-7.66 *(Ph,* m, 3H); 7.77 (*arom.,* d, 1H, ⁴*J*_{HH} = 1); 7.79-7.87 *(Ph,* m, 2H); 7.94 (*arom.,* d, 1H, ⁴*J*_{HH} = 1); 8.05 *(arom.,* dd, 1H, ³*J*_{HH} = 8, ⁴*J*_{HH} = 2); 8.07 *(arom.,* dd, 1H, ³*J*_{HH} = 8, ³*J*_{HH} = 7); 8.93 (C*H*-N₃, s, 1H). **¹³C{¹H}** δ_{C} 50.0 (*mc,* s); 50.7 *(mc,* bs); 52.0 (*mc,* s); 52.3 (*mc,* s); 54.8 (*C*H₂-N₃, s); 56.6 (*C*H₂-CO, s); 59.9 (C*H*₂-arom., s); 60.2 (*C*H₂-arom., bs); 121.2 (*arom.,* s); 121.7 (*arom.,* s); 123.3 (*arom.,* s); 124.5 (*arom.,* s); 127.0 (*C*H-N₃, s); 127.7 (*Ph,* s); 129.9 (*Ph,* s); 130.5 (*Ph,* s); 137.1 (*Ph,* s); 140.2 (*arom.,* s); 148.0 (*arom.,* s); 150.3 (*arom.,* s); 151.7 (*arom.,* s); 152.5 (*arom.,* bs); 154.9 (*arom.,* s); 155.0 (*arom.,* s); 174.4 (CO, s). ESI-HRMS: 626.3199 [M+H]⁺ (theor. [C₃₃H₄₀N₉O₄]⁺ = 626.3198). EA (C₃₃H₃₉N₉O₄·0.6FA·2.3H₂O, *M*_{R} = 696.8): C 58.1 (57.9); H 6.5 (6.2); N 18.1 (18.2).

### Example 6: Synthesis of coordination compounds

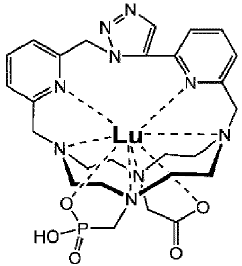

**Synthesis of TD734:** In a glass vial (20 mL), **TD714·0.1FA·*x*H₂O** (6 mg; ~10 µmol; 1.0 equiv.) was dissolved in H₂O (4 mL) followed by addition aq. **MOPS/NaOH buffer** (500 mM; pH 7.0; 1.00 mL; 500 µmol; ~50 equiv.) and aq. **LuCl₃** (100 mM; 110 µL; 11 µmol; ~1.1 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product as a mixture of two diastereoisomer (~11:1) in the form of formate salt as white fluffy solid. **Yield:** 5 mg (≤61% assuming [**M**]⁺[FA]⁻·*x*H₂O; *M*_{R} = 803.6; 1 step; based on **TD714·**0.1FA·*x*H₂O). **NMR (D₂O, pD ~4, signals of major isomer): ¹H** δ_{H} 2.50-3.82 (*mc,* C*H*₂-CO, C*H*₂-P, m, 16+2+1H); 3.85-4.00 (*C*H₂-arom., C*H*₂-P, m, 1+1H); 4.10 (*C*H₂-arom., d, 1H, ²*J*_{HH} = 16); 4.23 (C*H*₂-arom., d, 1H, ²*J*_{HH} = 15); 4.99 (*C*H₂-arom., d, 1H, ²*J*_{HH} = 15); 5.53 (C*H*₂-N₃, d, 1H, ²*J*_{HH} = 15); 7.56 (C*H*₂-N₃, d, 1H, ²*J*_{HH} = 15); 7.67 (*arom.,* dd, 1H, ³*J*_{HH} = 8, ⁴*J*_{HH} = 1); 7.73 (*arom.,* dd, 1H, ³*J*_{HH} = 8, ⁴*J*_{HH} = 1); 7.81 (*arom.,* dd, 1H, ³*J*_{HH} = 8, ⁴*J*_{HH} = 2); 7.83 (*C*H-N₃, s, 1H); 7.98 (*arom.,* dd, 1H, ³*J*_{HH} = 8, ⁴*J*_{HH} = 2); 7.18 (*arom.,* t, 1H, ³*J*_{HH} = 8); 8.20 (*arom.,* t, 1H, ³*J*_{HH} = 8); 8.35 (FA, s, 1H). **¹³C{1H}** δ_{C} 48.9 (*mc,* s); 51.3 (*mc,* s); 51.6 (*C*H₂-P, d, ¹*J*_{CP} = 73); 52.0 (*mc,* s); 52.3 (*mc,* s); 53.4 (*mc,* s); 53.5 (C*H*₂-N₃, s); 53.6 (*mc,* s); 55.0 (*mc,* s); 56.3 (*mc,* s); 59.7 (*C*H₂-arom., s); 60.0 (*C*H₂-arom., s); 61.0 (*C*H₂-CO, s); 125.9 (*arom.,* s); 127.6 (*arom.,* s); 128.9 (*arom.,* s); 130.4 (*arom.,* s); 136.4 (*C*H-N₃, s); 138.7 (*arom.,* s); 142.0 (*arom.,* s); 142.9 (*arom.,* s); 146.2 (*arom.,* s); 156.8 (*arom.,* s); 157.6 (*arom.,* s); 158.6 (*arom.,* s); 169.5 (FA, s); 178.2 (CO, s). **³¹P{¹H}** δ_{P} 17.8 (s). **ESI-HRMS:** 758.1822 [M]⁺ (theor. [C₂₆H₃₄N₉O₅P₁Lu₁]⁺ = 758.1823). 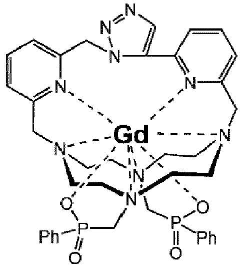 **Synthesis of TD925:** In a glass vial (20 mL), **TD921**·4.6H₂O (6.1 mg; 7.4 µmol; 1.0 equiv.) was dissolved in H₂O (18 mL) and *i*-PrOH (1 mL) followed by addition aq. **MOPS/NaOH buffer** (500 mM; pH 7.0; 400 µL; 200 µmol; 27 equiv.) and aq. **GdCl₃** (100 mM; 95 µL; 9.5 µmol; 1.3 equiv.) and the mixture was stirred 7 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. Yield: 2.5 mg (≤33% assuming [**M**]⁺[TFA]⁻·*x*H₂O; *M*_{R} = 1010.0; 1 step; based on **TD921·**4.6H₂O). **ESI-HRMS:** 897.2158 [M]⁺ (theor. [C₃₇H₄₃N₉O₄P₂Gd₁]⁺ = 897.2149). 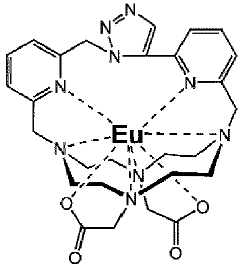 **Synthesis of TD560:** In a glass vial (20 mL), **TD556·**0.1FA·1.6H₂O (39 mg; 69 µmol; 1.0 equiv.) was dissolved in H₂O (15 mL) followed by addition aq. MOPS/NaOH buffer (500 mM; pH 7.0; 3.40 mL; 1.70 mmol; 25 equiv.) and aq. **EuCl₃** (100 mM; 745 µL; 75 µmol; 1.1 equiv.) and the mixture was stirred 4 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 24 mg (43%; 1 step; based on **TD556·**0.1FA1.6H₂O). **ESI-HRMS:** 700.1867 [M]⁺ (theor. [C₂₇H₃₃N₉O₄Eu₁]⁺ = 700.1862). **EA** ([C₂₇H₃₃N₉O₄Eu₁]⁺[FA]⁻·4.5H₂O, *M*_{R} = 825.7): C 40.7 (40.7); H 5.2 (4.8); N 15.3 (15.0); Eu 18.4 (17.7). 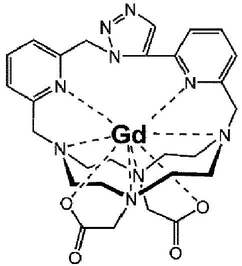 **Synthesis of TD561:** In a glass vial (20 mL), **TD556** · 0.1FA · 1.6H₂O (40 mg; 69 µmol; 1.0 equiv.) was dissolved in H₂O (15 mL) followed by addition aq. **MOPS/NaOH buffer** (500 mM; pH 7.0; 3.40 mL; 1.70 mmol; 25 equiv.) and aq. **GdCl₃** (100 mM; 745 µL; 75 µmol; 1.1 equiv.) and the mixture was stirred 4 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 24 mg (42%; 1 step; based on **TD556·**0.1FA1.6H₂O). **ESI-HRMS:** 705.1893 [M]⁺ (theor. [C₂₇H₃₃N₉O₄Gd₁]⁺ = 705.1891). **EA** ([C₂₇H₃₃N₉O₄Gd₁]⁺[FA]⁻·4.6H₂O, *M*_{R} = 832.8): C 40.4 (40.8); H 5.2 (4.9); N 15.1 (15.5); Gd 18.9 (18.1). 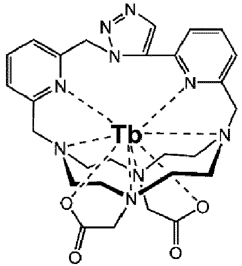 **Synthesis of TD562:** In a glass vial (20 mL), **TD556·**0.1FA·1.6H₂O (40 mg; 69 µmol; 1.0 equiv.) was dissolved in H₂O (15 mL) followed by addition aq. **MOPS/NaOH buffer** (500 mM; pH 7.0; 3.40 mL; 1.70 mmol; 25 equiv.) and aq. **TbCl₃** (100 mM; 745 µL; 75 µmol; 1.1 equiv.) and the mixture was stirred 4 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 27 mg (47%; 1 step; based on **TD556**·0.1FA·1.6H₂O). **ESI-HRMS:** 706.1906 [M]⁺ (theor. [C₂₇H₃₃N₉O₄Tb₁]⁺ = 706.1903). **EA** ([C₂₇H₃₃N₉O₄Tb₁]⁺[FA]⁻ · 4.7H₂O, *M*_{R} = 836.2): C 40.2 (40.5); H 5.2 (4.9); N 15.1 (14.7); Tb 19.0 (18.7). 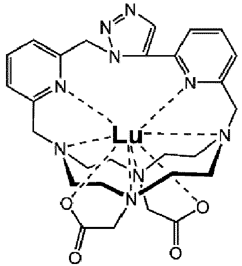 **Synthesis of TD1069:** In a glass vial (20 mL), **TD556·**0.1FA·1.6H₂O (20.8 mg; 35.7 µmol; 1.0 equiv.) was dissolved in H₂O (17 mL) followed by addition aq. **MOPS/NaOH buffer** (500 mM; pH 7.0; 1.80 mL; 900 µmol; 25 equiv.) and aq. **LuCl₃** (100 mM; 400 µL; 40.0 µmol; 1.1 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 26.6 mg (82%; 1 step; based on **TD556·**0.1FA1.6H₂O). **NMR (D₂O, pD ~3): ¹H** δ_{H} 2.64-3.82 (*mc,* C*H*₂-CO, m, 16+4H); 3.95 (*CH*₂-arom., d, 1H, ²*J*_{HH} = 15); 4.15 (C*H*₂-arom., d, 1H, ²*J*_{HH} = 16); 4.29 (*CH*₂-arom., d, 1H, ²*J*_{HH} = 15); 4.85 (*CH*₂-arom., d, 1H, ²*J*_{HH} = 16); 5.60 (C*H*₂-N₃, d, 1H, ²*J*_{HH} = 15); 7.10 (C*H*₂-N₃, d, 1H, ²*J*_{HH} = 15); 7.73 (*arom.,* dd, 1H, ³*J*_{HH} = 8, ⁴*J*_{HH} = 1); 7.80 (*arom.,* dd, 1H, ³*J*_{HH} = 8, ⁴*J*_{HH} = 1); 7.85 (*arom.,* dd, 1H, ³*J*_{HH} = 8, ⁴*J*_{HH} = 1); 7.88 (*C*H-N₃, s, 1H); 8.02 (*arom.,* dd, 1H, ³*J*_{HH} = 8, ⁴*J*_{HH} = 1); 8.25 (*arom.,* t, 1+1H, ³*J*_{HH} = 8). **ESI-HRMS:** 722.2060 [M]⁺ (theor. [C₂₇H₃₃N₉O₄Lu₁]⁺ = 722.2058). **EA** ([C₂₇H₃₃N₉O₄Lu₁]⁺[TFA]⁻·0.3TFA·2.4H₂O, *M*_{R} = 913.0): C 38.9 (38.7); H 4.2 (3.8); N 13.8 (13.7); Lu 19.2 (16.5). 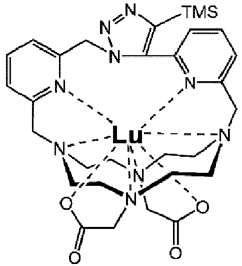 **Synthesis of TD751:** In a glass vial (20 mL), **TD718**·2.6H₂O (14 mg; 21 µmol; 1.0 equiv.) was dissolved in H₂O (18 mL) followed by addition aq. **MES/NaOH buffer** (500 mM; pH 5.2; 1.30 mL; 650 µmol; 31 equiv.) and aq. **LuCl₃** (100 mM; 236 µL; 24 µmol; 1.1 equiv.) and the mixture was stirred 3 h at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 13 mg (65%; 1 step; based on **TD718**·2.6H₂O). **NMR (D₂O, pD ~6): ¹H** δ_{H} 0.10 (C*H*₃, s, 9H); 2.61-3.83 (*mc*, C*H*₂-CO, m, 16+4H); 3.94 (*CH*₂-arom., d, 1H, ²*J*_{HH} = 15); 4.24 (*CH*₂-arom., d, 1H, ²*J*_{HH} = 16); 4.24 (*CH*₂-arom., d, 1H, ²*J*_{HH} = 15); 4.84 (C*H*₂-arom., d, 1H, ²*J*_{HH} = 16); 5.54 (C*H*₂-N₃, d, 1H, ²*J*_{HH} = 15); 6.92 (C*H*₂-N₃, d, 1H, ²*J*_{HH} = 15); 7.70 (*arom.,* dd, 1H, ³*J*_{HH} = 8, ⁴*J*_{HH} = 1); 7.75 (*arom.,* dd, 1H, ³*J*_{HH} = 8, ⁴*J*_{HH} = 1); 7.85 (*arom.,* dd, 1H, ³*J*_{HH} = 8, ⁴*J*_{HH} = 1); 7.98 (*arom.,* dd, 1H, ³*J*_{HH} = 8, ⁴*J*_{HH} = 1); 8.22 (*arom.,* t, 1H, ³*J*_{HH} = 8); 8.26 (*arom.,* t, 1H, ³*J*_{HH} = 8); 8.45 (FA, s, 1H). **¹³C{¹H}** δ_{C} -1.85 (*CH*₃, s); 48.4 (*mc,* s); 48.7 (*mc,* s); 50.7 (*mc,* s); 51.9 (*mc,* s); 52.0 (C*H*₂-N₃, s); 53.2 (*mc,* s); 53.8 (*mc,* s); 54.4 (*mc,* s); 55.5 (*mc,* s); 58.4 (*C*H₂-CO, s); 59.0 (*C*H₂-arom., s); 59.1 (*C*H₂-arom., s); 60.9 (*C*H₂-CO, s); 125.9 (*arom.,* s); 126.7 (*arom.,* s); 128.3 (*arom.,* s); 130.4 (*arom.,* s); 141.4 (*arom.,* s); 142.3 (*arom.,* s); 142.6 (*arom.,* s); 146.3 (*arom.,* s); 147.5 (*arom.,* s); 155.1 (*arom.,* s); 157.7 (*arom.,* s); 158.7 (*arom.,* s); 171.0 (FA, s); 176.8 (CO, s); 178.6 (CO, s). **ESI-HRMS:** 794.2445 [M]⁺ (theor. [C₃₀H₄₁N₉O₄Si₁Lu₁]⁺ = 794.2453). **EA** ([C₃₀H₄₁N₉O₄Si₁Lu₁]⁺[FA]⁻·6.6H₂O, *M*_{R} = 958.7): C 38.8 (39.0); H 5.8 (5.3); N 13.1 (13.0); Si 2.9 (3.0); Lu 18.2 (17.4). 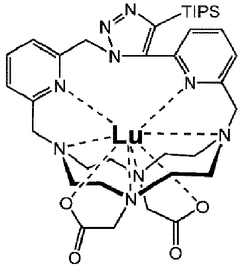 **Synthesis of TD737:** In a glass vial (20 mL), **TD728**·2.6H₂O (15 mg; 20 µmol; 1.0 equiv.) was dissolved in H₂O (17 mL) followed by addition aq. **MOPS/NaOH buffer** (500 mM; pH 7.0; 2.00 mL; 1.00 mmol; 50 equiv.) and aq. **LuCl₃** (100 mM; 220 µL; 22 µmol; 1.1 equiv.) and the mixture was stirred 5 days at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 12 mg (58%; 1 step; based on **TD728**·2.6H₂O). **NMR (D₂O, pD ~5):** δ_{H} 0.96 (*i-Pr,* d, 9H, ³*J*_{HH} = 8); 0.99 (*i-Pr,* d, 9H, ³*J*_{HH} = 8); 1.26 (i-*Pr,* hept, 3H, ³*J*_{HH} = 8); 2.62-3.84 (*mc,* C*H*₂-CO, m, 16+4H); 4.05 (*C*H₂-arom., s, 2H); 4.29 (*C*H₂-arom., d, 1H, ²*J*_{HH} = 17); 4.88 (*C*H₂-arom., d, 1H, ²*J*_{HH} = 17); 5.43 (C*H*₂-N₃, d, 1H, ²*J*_{HH} = 15); 6.74 (C*H*₂-N₃, d, 1H, ²*J*_{HH} = 15); 7.67 (*arom.,* dd, 1H, ³*J*_{HH} = 8, ⁴*J*_{HH} = 1); 7.82 (*arom.,* dd, 1H, ³*J*_{HH} = 8, ⁴*J*_{HH} = 1); 7.83 (*arom.,* dd, 1H, ³*J*_{HH} = 8, ⁴*J*_{HH} = 1); 7.94 (*arom.,* dd, 1H, ³*J*_{HH} = 8, ⁴*J*_{HH} = 1); 8.20 (*arom.,* t, 1H, ³*J*_{HH} = 8); 8.22 *(arom.,* t, 1H, ³*J*_{HH} = 8); 8.43 (FA, s, 1H). **¹³C{¹H}** δ_{C} 12.1 (*i-Pr,* s); 18.8 (*i-Pr,* 2 × s); 47.9 (*mc,* bs); 50.0 (*mc,* bs); 50.4 (*mc,* bs); 52.2 (*mc,* bs); 53.0 (C*H*₂-N₃, s); 54.0 (*mc,* s); 55.2 (*mc,* s); 57.2 (*mc,* s); 57.6 (*mc,* s); 59.3 (*C*H₂-CO, s); 60.0 (*C*H₂-arom., s); 62.3 (*C*H₂-arom., bs); 64.0 (*C*H₂-CO, s); 126.1 (*arom.,* s); 127.4 (*arom.,* s); 128.9 (*arom.,* s); 130.8 (*arom.,* s); 141.6 (*arom.,* s); 143.3 (*arom.,* s); 143.8 (*arom.,* s); 144.6 (*arom.,* s); 147.1 (*arom.,* s); 156.1 (*arom.,* s); 158.4 (*arom.,* s); 159.6 (*arom.,* s); 171.5 (FA, s); 178.0 (CO, s); 179.1 (CO, s). **ESI-HRMS:** 878.3396 [M]⁺ (theor. [C₃₆H₅₃N₉O₄Si₁Lu₁]⁺ = 878.3392). **EA** ([C₃₆H₅₃N₉O₄Si₁Lu₁]⁺[FA]⁻·5.9H₂O, *M*_{R} = 1030.2): C 43.1 (43.4); H 12.2 (11.8); N 12.2 (11.8); Si 2.7 (2.9); Lu 17.0 (16.9).

**Synthesis of TD946:** In a glass vial (20 mL), **TD943**·1.5H₂O (28.1 mg; 45.6 µmol; 1.0 equiv.) was dissolved 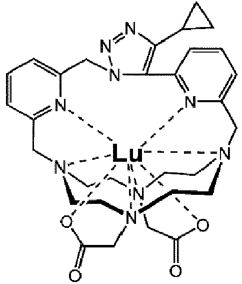 in H₂O (16 mL) followed by addition aq. **MOPS/NaOH buffer** (500 mM; pH 7.0; 3.00 mL; 1.50 mmol; 33 equiv.) and aq. **LuCl₃** (100 mM; 540 µL; 54 µmol; 1.2 equiv.) and the mixture was stirred 3 days at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 32.6 mg (77%; 1 step; based on **TD943**·1.5H₂O). **NMR (D₂O, pD ~5):** δ_{H} 0.69-0.94 (C*H*₂-CH, m, 2H); 0.94-1.07 (*CH*₂-CH, m, 2H); 1.65 (*CH*₂-CH, tt, 1H, ³*J*_{HH} = 8, ³*J*_{HH} = 5); 2.58-3.86 (*mc,* C*H*₂-CO, m, 16+4H); 3.97 (*C*H₂-arom., d, 1H, ²*J*_{HH} = 15); 4.19 (*C*H₂-arom., d, 1H, ²*J*_{HH} = 16); 4.29 (*C*H₂-arom., d, 1H, ²*J*_{HH} = 15); 4.87 (*C*H₂-arom., d, 1H, ²*J*_{HH} = 16); 5.53 (C*H*₂-N₃, d, 1H, ²*J*_{HH} = 15); 6.99 (C*H*₂-N₃, d, 1H, ²*J*_{HH} = 15); 7.73 (*arom.,* dd, 1H, ³*J*_{HH} = 8, ⁴*J*_{HH} = 1); 7.86 (*arom.,* dd, 1H, ³*J*_{HH} = 8, ⁴*J*_{HH} = 1); 7.93 (*arom.,* dd, 1H, ³*J*_{HH} = 8, ⁴*J*_{HH} = 1); 7.99 (*arom.,* dd, 1H, ³*J*_{HH} = 8, ⁴*J*_{HH} = 1); 8.24 (*arom.,* t, 1H, ³*J*_{HH} = 8); 8.29 (*arom.,* t, 1H, ³*J*_{HH} = 8). **ESI-HRMS:** 762.2375 [M]⁺ (theor. [C₃₀H₃₇N₉O₄Lu₁]⁺ = 762.2371). **EA** ([C₃₀H₃₇N₉O₄Lu₁]⁺[TFA]⁻·0.2TFA·1.8H₂O, *M*_{R} = 930.8): C 41.8 (42.2); H 4.4 (4.2); N 13.5 (13.1); F 7.3 (7.0). 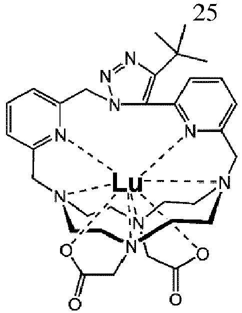 **Synthesis of TD961:** In a glass vial (20 mL), **TD959**·1.5H₂O (14.7 mg; 23.0 µmol; 1.0 equiv.) was dissolved in H₂O (16 mL) followed by addition aq. **MOPS/NaOH buffer** (500 mM; pH 7.0; 1.50 mL; 0.75 mmol; 33 equiv.) and aq. **LuCl₃** (100 mM; 280 µL; 28 µmol; 1.2 equiv.) and the mixture was stirred 3 days at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 20.1 mg (90%; 1 step; based on **TD959**·1.8H₂O). **NMR (D₂O, pD ~4):** δ_{H} 1.18 (*CH*₃, s, 9H); 2.64-3.86 *(mc,* C*H*₂-CO, m, 16+4H); 4.01 (*CH*₂-arom., d, 1H, ²*J*_{HH} = 15); 4.16 (*CH*₂-arom., d, 1H, ²*J*_{HH} = 15); 4.35 (C*H*₂-arom., d, 1H, ²*J*_{HH} = 17); 4.87 (*CH*₂-arom., d, 1H, ²*J*_{HH} = 17); 5.48 (C*H*₂-N₃, d, 1H, ²*J*_{HH} = 15); 6.66 (C*H*₂-N₃, d, 1H, ²*J*_{HH} = 15); 7.70 *(arom.,* dd, 1H, ³*J*_{HH} = 8, ⁴*J*_{HH} = 1); 7.84 (*arom.,* dd, 1H, ³*J*_{HH} = 8, ⁴*J*_{HH} = 1); 7.87 (*arom.,* dd, 1H, ³*J*_{HH} = 8, ⁴*J*_{HH} = 1); 7.93 (*arom.,* dd, 1H, ³*J*_{HH} = 8, ⁴*J*_{HH} = 1); 8.22 (*arom.,* t, 1H, ³*J*_{HH} = 8); 8.26 (*arom.,* t, 1H, ³*J*_{HH} = 8). **ESI-HRMS:** 778.2685 [M]⁺ (theor. [C₃₁H₄₁N₉O₄Lu₁]⁺ = 778.2684). EA ([C₃₁H₄₁N₉O₄Lu₁]⁺[TFA]⁻·0.2TFA·3.0H₂O, *M*_{R}= 968.5): C 41.4 (41.5); H 4.9 (4.6); N 13.0 (12.7); F 7.1 (7.4). 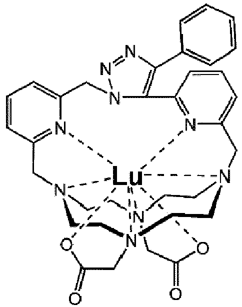 **Synthesis of TD951:** In a glass vial (20 mL), **TD944**· 1.5H₂O (14.4 mg; 22.1 µmol; 1.0 equiv.) was dissolved in H₂O (18 mL) followed by addition aq. **MOPS/NaOH buffer** (500 mM; pH 7.0; 1.20 mL; 600 µmol; 27 equiv.) and aq. **LuCl₃** (100 mM; 265 µL; 26.5 µmol; 1.2 equiv.) and the mixture was stirred 4 days at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 17.4 mg (80%; 1 step; based on **TD944**·1.5H₂O). **NMR (D₂O, pD ~2):** δ_{H} 2.69-3.88 (*mc*, C*H*₂-CO, m, 16+4H); 3.97 (*CH*₂-arom., d, 1H, ²*J*_{HH} = 15); 4.16-4.36 (*CH*₂-arom., m, 2H); 4.88 (*CH*₂-arom., d, 1H, ²*J*_{HH} = 17); 5.51 (C*H*₂-N₃, d, 1H, ²*J*_{HH} = 15); 6.97 (C*H*₂-N₃, d, 1H, ²*J*_{HH} = 15); 7.16-7.25 (*Ph,* m, 2H); 7.28-7.41 (*Ph,* m, 3H); 7.49 (*arom.,* dd, 1H, ³*J*_{HH} = 8, ⁴*J*_{HH} = 1); 7.73 (*arom.,* dd, 1H, ³*J*_{HH} = 8, ⁴*J*_{HH} = 1); 7.83 (*arom.,* dd, 1H, ³*J*_{HH} = 8, ⁴*J*_{HH} = 1); 8.03 (*arom.,* dd, 1H, ³*J*_{HH} = 8, ⁴*J*_{HH} = 1); 8.06 (*arom.,* t, 1H, ³*J*_{HH} = 8); 8.25 (*arom.,* t, 1H, ³*J*_{HH} = 8). **ESI-HRMS:** 798.2379 [M]⁺ (theor. [C₃₃H₃₇N₉O₄Lu₁]⁺ = 798.2371). **EA** ([C₃₃H₃₇N₉O₄Lu₁]⁺[TFA]⁻·0.2TFA·3.0H₂O, *M*_{R} = 988.5): C 43.0 (43.0); H 4.4 (4.3); N 12.8 (12.6); F 6.9 (6.9).

**Synthesis of TD994:** In a glass vial (20 mL), **TD992**·0.3FA·1.9H₂O (41.6 mg; 56.8 µmol; 1.0 equiv.) was 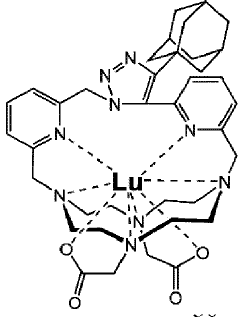 dissolved in H₂O (16 mL) followed by addition aq. **MOPS/NaOH buffer** (500 mM; pH 7.0; 2.85 mL; 1.43 mmol; 25 equiv.) and aq. **LuCl₃** (100 mM; 625 µL; 62.5 µmοl; 1.1 equiv.) and the mixture was stirred 8 days at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 52.3 mg (86%; 1 step; based on **TD992**·0.3FA·1.9H₂O). **NMR (D₂O, pD ~4):** δ_{H} 1.52-1.78 (*Adm.*, m, 9H); 1.80-1.95 (*Adm.*, m, 6H); 2.60-3.83 (*mc*, C*H*₂-CO, m, 16+4H); 3.99 (C*H*₂-arom., d, 1H, ²*J*_{HH} = 15); 4.11 (*CH*₂-arom., d, 1H, ²*J*_{HH} = 15); 4.33 (*CH*₂-arom., d, 1H, ²*J*_{HH} = 17); 4.84 (*CH*₂-arom., d, 1H, ²*J*_{HH} = 17); 5.43 (C*H*₂-N₃,d, 1H, ²*J*_{HH} = 15); 6.58 (C*H*₂-N₃, d, 1H, ²*J*_{HH} = 15); 7.66 (*arom.,* d, 1H, ³*J*_{HH} = 8); 7.80 (*arom.,* d, 1H, ³*J*_{HH} = 8); 7.88-7.92 (*arom.,* m, 2H); 8.18 (*arom.,* t, 1H, ³*J*_{HH} = 8); 8.22 (*arom.,* t, 1H, ³*J*_{HH} = 8). **ESI-HRMS:** 856.3154 [M]⁺ (theor. [C₃₇H₄₇N₉O₄Lu₁]⁺ = 856.3153). **EA** ([C₃₇H₄₇N₉O₄Lu₁]⁺[TFA]⁻·0.4TFA·3.0H₂O, *M*_{R} = 1069.4): C 44.7 (44.3); H 5.0 (4.6); N 11.8 (11.5); F7.5 (7.4); Lu 16.4 (14.0). 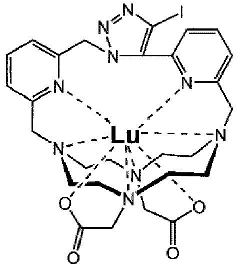 **Synthesis of TD1221:** In a glass vial (20 mL), **TD1204**·0.3FA·2.7H₂O (22.5 mg; 30.5 µmol; 1.0 equiv.) was dissolved in H₂O (18 mL) followed by addition aq. **MOPS/NaOH buffer** (500 mM; pH 7.0; 1.57 mL; 785 µmol; 26 equiv.) and aq. **LuCl₃** (100 mM; 375 µL; 37.5 µmol; 1.2 equiv.) and the mixture was stirred 24 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 24.5 mg (74%; 1 step; based on **TD1204**·0.3FA·2.7H₂O). **NMR (D₂O, pD ~2): ¹H** δ_{H} 2.59-3.83 (*mc*, C*H*₂-CO, m, 16+4H); 3.95 (*CH*₂-arom., d, 1H, ²*J*_{HH} = 15); 4.23 (*CH*₂-arom., d, 1H, ²*J*_{HH} = 17); 4.27 (*CH*₂-arom., d, 1H, ²*J*_{HH} = 15); 4.85 (*CH*₂-arom., d, 1H, ²*J*_{HH} = 17); 5.62 (C*H*₂-N₃, d, 1H, ²*J*_{HH} = 15); 7.01 (C*H*₂-N₃, d, 1H, ²*J*_{HH} = 15); 7.72 (*arom.,* dd, 1H, ³*J*_{HH} = 8, ⁴*J*_{HH} = 1); 7.87 (*arom.,* d, 1H, ⁴*J*_{HH} = 2); 7.90 (*arom.,* dd, 1H, ³*J*_{HH} = 8, ⁴*J*_{HH} = 1); 7.97 (*arom.,* dd, 1H, ³*J*_{HH} = 8, ⁴*J*_{HH} = 1); 8.23 (*arom.,* t, 1H, ³*J*_{HH} = 8); 8.29 (*arom.,* t, 1H, ³*J*_{HH} = 8). ESI-HRMS: 848.1020 [M]⁺ (theor. [C₂₇H₃₂N₉O₄I₁Lu₁]⁺ = 848.1024). EA ([C₂₇H₃₂N₉O₄I₁Lu₁]⁺[TFA]⁻·0.5TFA·4.1H₂O, *M*_{R} = 1092.3): C 33.0 (33.8); H 3.8 (3.6); N 11.5 (11.8); I 11.6 (10.9); F 7.8 (7.0). 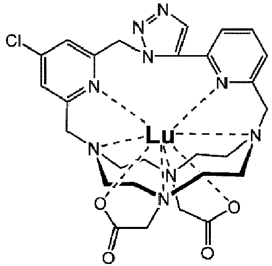 **Synthesis of TD782:** In a glass vial (40 mL), **TD7640**·0.7TFA·1.7H₂O (190 mg; 274 µmol; 1.0 equiv.) was dissolved in H₂O (21 mL) followed by addition aq. **MOPS/NaOH buffer** (500 mM; pH 7.0; 15.4 mL; 7.70 mmol; 28 equiv.) and aq. **LuCl₃** (100 mM; 3.30 mL; 330 µmol; 1.2 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 224 mg (85%; 1 step; based on **TD764**·0.7TFA·1.7H₂O). **NMR (D₂O, pD ~3): ¹H** δ_{H} 2.61-3.84 (*mc*, C*H*₂-CO, m, 16+4H); 3.94 (*CH*₂-arom., d, 1H, ²*J*_{HH} = 15); 4.15 (*CH*₂-arom., d, 1H, ²*J*_{HH} = 16); 4.29 (*CH*₂-arom., d, 1H, ²*J*_{HH} = 15); 4.84 (*CH*₂-arom., d, 1H, ²*J*_{HH} = 16); 5.58 (C*H*₂-N₃, d, 1H, ²*J*_{HH} = 15); 7.10 (C*H*₂-N₃,d, 1H, ²*J*_{HH} = 15); 7.80 (*arom.,* dd, 1H, ³*J*_{HH} = 8, ⁴*J*_{HH} = 1); 7.83 (*arom.,* d, 1H, ⁴*J*_{HH} = 2); 7.85 (*arom.,* dd, 1H, ³*J*_{HH} = 8, ⁴*J*_{HH} = 1); 7.88 (*CH*-N₃, s, 1H); 8.13 (*arom.,* d, 1H, ⁴*J*_{HH} = 2); 8.24 (*arom.,* t, 1H, ³*J*_{HH} = 8). **ESI-HRMS:** 756.1664 [M]⁺ (theor. [C₂₇H₃₂N₉O₄Cl₁Lu₁]⁺ = 756.1668). **EA** ([C₂₇H₃₂N₉O₄Cl₁Lu₁]⁺[TFA]⁻·5.1H₂O, *M*_{R} = 961.9): C 36.2 (36.4); H 4.4 (4.0); N 13.1 (12.9); Cl 3.7 (3.7); F 5.9 (6.4); Lu 18.2 (16.8). 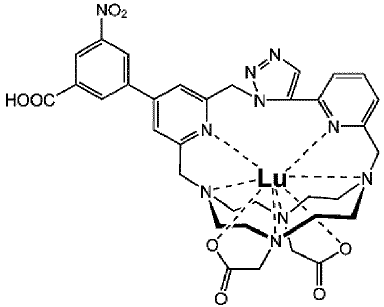 **Synthesis of TD819:** Glass vial (4 mL) was charged with **[TD782**]⁺[TFA]⁻·5.1H₂O (10.0 mg; 10.4 µmol; 1.0 equiv.), **3-borono-5-nitrobenzoic acid** (4.4 mg; 21 µmol; 2.0 equiv.) and **XPhos Pd G2** (0.5 mg; ~0.6 µmol; 6 mol%) and was then three times secured with argon. Under constant flow of argon was then added dry DMF (420 µL) through septum followed by addition of freshly prepared (and briefly washed with argon prior addition) aq.solution of **K₃PO₄**·H₂O (326 mM; 160 µL; 52 µmol; 5.0 equiv.). The mixture was then stirred 16 h under septum (but without external argon) at 80 °C. Resulting heterogenous mixture was diluted with H₂O (2 mL) and the resulting slightly opalescent pale-yellow solution was filtered through syringe microfilter (RC). Filtrate was then directly purified by preparative HPLC (C18; H₂O-MeCN gradient with TFA additive). Fractions with product were joined and directly lyophilized to give product as white fluffy solid. **Yield:** 10 mg (≤96% assuming [**M**]⁺[TFA]·*x*H₂O; *M*_{R} = 1000.7; 1 step; based on [**TD782**]⁺[TFA]⁻·5.1H₂O). **NMR (D₂O, pD ~3): ¹H** δ_{H} 2.66-3.84 (*mc,* C*H*₂-CO, m, 16+4H); 4.07 (*CH*₂-arom., d, 1H, ²*J*_{HH} = 15); 4.17 (*CH*₂-arom., d, 1H, ²*J*_{HH} = 16); 4.39 (*CH*₂-arom., d, 1H, ²*J*_{HH} = 15); 4.87 (*CH*₂-arom., d, 1H, ²*J*_{HH} = 15); 5.72 (C*H*₂-N₃, d, 1H, ²*J*_{HH} = 15); 7.19 (C*H*₂-N₃, d, 1H, ²*J*_{HH} = 15); 7.83 (*arom.,* dd, 1H, ³*J*_{HH} = 8, ⁴*J*_{HH} = 1); 7.87 (*arom.,* dd, 1H, ³*J*_{HH} = 8, ⁴*J*_{HH} = 1); 7.90 (*C*H-N₃, s, 1H); 8.14 (*arom.,* d, 1H, ⁴*J*_{HH} = 2); 8.26 (*arom.,* t, 1H, ³*J*_{HH} = 8); 8.45 (*arom.,* d, 1H, ⁴*J*_{HH} = 2); 8.82 (*arom.,* dm, 1H, ⁴*J*_{HH} = 2); 8.92 (*arom.,* dd, 1H, ⁴*J*_{HH} = 2, ⁴*J*_{HH} = 1); 8.97 (*arom.,* dm, 1H, ⁴*J*_{HH} = 2). **ESI-HRMS:** 887.2121 [M]⁺ (theor. [C₃₄H₃₆N₁₀O₈Lu₁]⁺ = 887.2120). 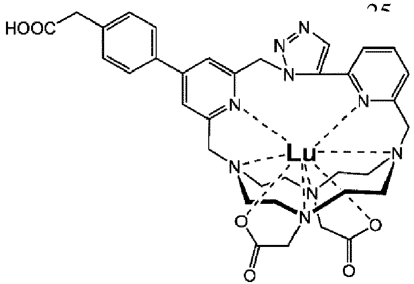 **Synthesis of TD891:** Glass vial (4 mL) was charged with [**TD782**]⁺[TFA]⁻·5.1H₂O (15.0 mg; 15.6 µmol; 1.0 equiv.), 2-(4-**(4,4,5,5-tetramethyl-1,3,2-dioxaborolan-2-yl)phenyl)acetic acid** (8.0 mg; 30.5 µmol; 2.0 equiv.) and **XPhos Pd G2** (0.7 mg; ~0.9 µmol; 6 mol%) and was then three times secured with argon. Under constant flow of argon was then added dry DMF (600 µL) through septum followed by addition of freshly prepared (and briefly washed with argon prior addition) aq. solution of **K₃PO₄**·H₂O (391 mM; 200 µL; 78 µmol; 5.0 equiv.). The mixture was then stirred 16 h under septum (but without external argon) at 80 °C. Resulting heterogenous mixture was diluted with H₂O (2 mL) and the resulting slightly opalescent pale-yellow solution was filtered through syringe microfilter (RC). Filtrate was then directly purified by preparative HPLC (C18; H₂O-MeCN gradient with TFA additive). Fractions with product were joined and directly lyophilized to give product as white fluffy solid. **Yield:** 7.2 mg (41%; 1 step; based on [**TD782**]⁺[TFA]⁻·5.1H₂O). **NMR (D₂O, pD ~3): ¹H** δ_{H} 2.60-3.82 (*mc*, C*H*₂-CO, m, 16+4H); 3.86 (OC-C*H*₂-arom., s, 2H); 3.99 (*CH*₂-arom., d, 1H, ²*J*_{HH} = 15); 4.15 (*CH*₂-arom., d, 1H, ²*J*_{HH} = 16); 4.32 (*CH*₂-arom., d, 1H, ²*J*_{HH} = 15); 4.84 (*CH*₂-arom., d, 1H, ²*J*_{HH} = 16); 5.65 (C*H*₂-N₃, d, 1H, ²*J*_{HH} = 15); 7.13 (C*H*₂-N₃, d, 1H, ²*J*_{HH} = 15); 7.54 (*arom.,* dm, 2H, ³*J*_{HH} = 8); 7.81 (*arom.,* dd, 1H, ³*J*_{HH} = 8, ⁴*J*_{HH} = 1); 7.85 (*arom.,* dd, 1H, ³*J*_{HH} = 8, ⁴*J*_{HH} = 1); 7.90 (*C*H-N₃, s, 1H); 7.92 (*arom.,* dm, 2H, ³*J*_{HH} = 8); 7.99 (*arom.,* d, 1H, ⁴*J*_{HH} = 1); 8.25 (*arom.,* t, 1H, ³*J*_{HH} = 8); 8.30 (*arom.,* d, 1H, ⁴*J*_{HH} = 1). **ESI-HRMS:** 856.2418 [M]⁺ (theor. [C₃₅H₃₉N₉O₆Lu₁]⁺ = 856.2426). EA ([C₃₅H₃₉N₉O₆Cl₁Lu₁]⁺[TFA]⁻·0.7TFA·4.6H₂O, *M*_{R} = 1132.4): C 40.7 (40.6); H 4.4 (4.3); N 11.1 (11.3). 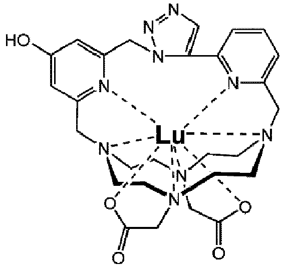 **Synthesis of TD891-OH: TD891-OH** was obtained (in the form of trifluoroacetate salt as white fluffy solid) as by-product during synthesis **TD891**. **Yield:** 5.7 mg. **NMR (D₂O, pD ~3): ¹H** δ_{H} 2.60-3.83 (*mc*, C*H*₂-CO, C*H*₂-arom., m, 16+4+1H); 4.13 (*CH*₂-arom., d, 1H, ²*J*_{HH} = 16); 4.20 (C*H*₂-arom., d, 1H, ²*J*_{HH} = 15); 4.82 (*CH*₂-arom., d, 1H, ²*J*_{HH} = 16); 5.43 (C*H*₂-N₃, d, 1H, ²*J*_{HH} = 15); 6.97 (C*H*₂-N₃,d, 1H, ²*J*_{HH} = 15); 7.09 (*arom.,* d, 1H, ⁴*J*_{HH} = 2); 7.40 (*arom.,* d, 1H, ⁴*J*_{HH} = 2); 7.79 (*arom.,* dd, 1H, ³*J*_{HH} = 8, ⁴*J*_{HH} = 1); 7.84 (*arom.,* dd, 1H, ³*J*_{HH} = 8, ⁴*J*_{HH} = 1); 7.89 (*CH*-N₃, s, 1H); 8.23 (*arom.,* t, 1H, ³*J*_{HH} = 8). ESI-HRMS: 738.2003 [M]⁺ (theor. [C₂₇H₃₃N₉O₅Lu₁]⁺ = 738.2007). 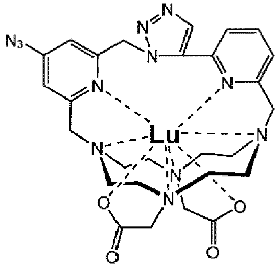 **Synthesis of TD786:** In a glass vial (4 mL), [**TD782**]⁺[TFA]⁻·5.1H₂O (37 mg; 38 µmol; 1.0 equiv.) was dissolved in DMSO (3 mL) followed by addition solid **NaN₃** (54 mg; 831 µmol; 20 equiv.) and the mixture was stirred 2 h at 80 °C. Mixture was then concentrated to remove most of DMSO. Residue was dissolved in H₂O and lyophilized. Crude product was purified by preparative HPLC (C18; H₂O-MeCN gradient with TFA additive). Fractions with product were joined and directly lyophilized to give product in the form of trifluoroacetate salt as white fluffy solid. Yield: 21 mg (55%; 1 step; based on [**TD782**]⁺[TFA]⁻·5.1H₂O). **NMR(D₂O, pD ~4): ¹H** δ_{H} 2.62-3.81 (*mc*, C*H*₂-CO, m, 16+4H); 3.87 (*CH*₂-arom., d, 1H, ²*J*_{HH} = 15); 4.14 (C*H*₂-arom., d, 1H, ²*J*_{HH} = 16); 4.25 (C*H*₂-arom., d, 1H, ²*J*_{HH} = 15); 4.83 (C*H*₂-arom., d, 1H, ²*J*_{HH} = 16); 5.52 (C*H*₂-N₃, d, 1H, ²*J*_{HH} = 15); 7.05 (C*H*₂-N₃, d, 1H, ²*J*_{HH} = 15); 7.38 (*arom.,* d, 1H, ⁴*J*_{HH} = 2); 7.71 (*arom.,* d, 1H, ⁴*J*_{HH} = 2); 7.80 (*arom.,* dd, 1H, ³*J*_{HH} = 8, ⁴*J*_{HH} = 1); 7.84 (*arom.,* dd, 1H, ³*J*_{HH} = 8, ⁴*J*_{HH} = 1); 7.88 (*C*H-N₃, s, 1H); 8.24 (*arom.,* t, 1H, ³*J*_{HH} = 8). **ESI-HRMS:** 763.2075 [M]⁺ (theor. [C₂₇H₃₂N₁₂O₄Lu₁]⁺ = 763.2072). **EA** ([C₂₇H₃₂N₁₂O₄Lu₁]⁺[TFA]⁻·0.2TFA·5.2H₂O, *M*_{R} = 993.0): C 35.6 (35.6); H 4.3 (4.0); N 16.9 (16.6); F 6.9 (6.8). 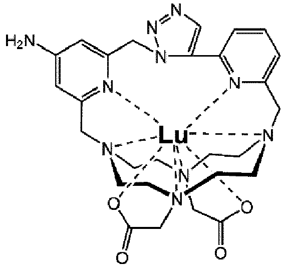 **Synthesis of TD787:** Obtained as side product during synthesis of **TD786** in the form of trifluoroacetate salt as white fluffy solid. **Yield:** 5 mg (~10%; 1 step; based on **TD782·**5.1H₂O). **NMR (D₂O, pD ~2): ¹H** δ_{H} 2.59-3.79 (*mc*, C*H*₂-CO, C*H*₂-arom., m, 16+4+1H); 4.02-4.19 (C*H*₂-arom., m, 2H); 4.25 (C*H*₂-arom., d, 1H, ²*J*_{HH} = 15); 4.79 (C*H*₂-arom., d, 1H, ²*J*_{HH} = 15); 5.27 (C*H*₂-N₃, d, 1H, ²*J*_{HH} = 15); 6.74 (*arom.,* d, 1H, ⁴*J*_{HH} = 2); 6.83 (C*H*₂-N₃, d, 1H, ²*J*_{HH} = 15); 7.03 *(arom.,* d, 1H, ⁴*J*_{HH} = 2); 7.78 (*arom*., dd, 1H, ³*J*_{HH} = 8, ⁴*J*_{HH} = 1); 7.82 (*arom*., dd, 1H, ³*J*_{HH} = 8, ⁴*J*_{HH} = 1); 7.89 (C*H*-N₃, s, 1H); 8.22 (*arom*., t, 1H, ³*J*_{HH} = 8). **ESI-HRMS:** 737.2165 [M]⁺ (theor. [C₂₇H₃₄N₁₀O₄Lu₁]⁺ = 737.2167). 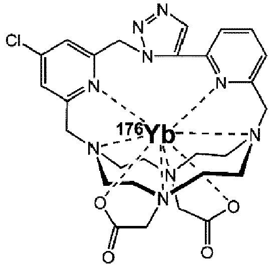 **Synthesis of TD830:** In a glass vial (20 mL), **TD764**·0.7TFA·1.7H₂O (60 mg; 86 µmol; 1.0 equiv.) was dissolved in H₂O (5 mL) followed by addition aq. **MOPS/NaOH buffer** (500 mM; pH 7.0; 5.0 mL; 2.50 mmol; 29 equiv.). and freshly prepared aq. **¹⁷⁶YbCl₃** (~90 µmol; ~1.0 eq.; obtained by dissolving 18.1 mg of **¹⁷⁶Yb₂O** in 0.5 mL of 6 M HCl at 80 °C for several hours, followed by evaporation of the acid and adding 10 mL of H₂O to the residue). Mixture was then stirred 6 h at 80 °C after which was filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 71 mg (≤94% assuming [**M**]⁺[TFA]⁻·*x*H₂O; *M*_{R} = 871.0; 1 step; based on **TD764**·0.7TFA·1.7H₂O). **ESI-HRMS:** 757.1680 [M]⁺ (theor. [C₂₇H₃₂N₉O₄Cl₁¹⁷⁶Yb₁]⁺ = 757.1686). 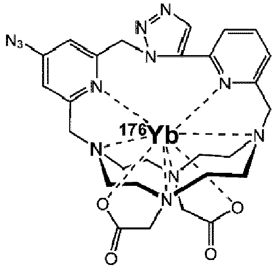 **Synthesis of TD836:** In a glass vial (4 mL), [**TD830**]⁺[TFA]⁻·*x*H₂O (12.1 mg; ~14 µmol; 1.0 equiv.) was dissolved in DMSO (500 µL) followed by addition solid **NaN₃** (9.0 mg; 138 µmol; ~10 equiv.) and the mixture was stirred 30 min at 80 °C. Mixture was then diluted with H₂O (3 mL) and directly purified by preparative HPLC (C18; H₂O-MeCN gradient with TFA additive). Fractions with product were joined and directly lyophilized to give product as white fluffy solid. **Yield:** 9.9 mg (≤81% assuming **[M]**⁺[TFA]⁻·*x*H₂O; *M*_{R} = 877.6; 1 step; based on [**TD830**]⁺[TFA]⁻·*x*H₂O). **ESI-HRMS:** 764.2085 [M]⁺ (theor. [C₂₇H₃₂N₁₂O₄¹⁷⁶Yb₁]⁺ = 764.2090). 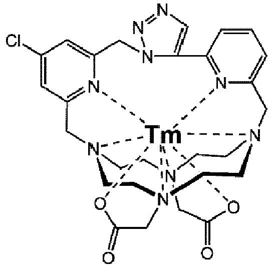 **Synthesis of TD888:** In a glass vial (20 mL), **TD764**·0.6TFA·1.0H₂O (56.5 mg; 84.3 µmol; 1.0 equiv.) was dissolved in H₂O (15 mL) followed by addition aq. **MOPS/NaOH buffer** (500 mM; pH 7.0; 4.0 mL; 2.00 mmol; 24 equiv.). and aq. **TmCl₃** (100 mM; 1.00 mL; 100 µmol; 1.2 equiv.) and the mixture was stirred 6 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 68.0 mg (86%, 1 step; based on **TD764**·0.6TFA·1.0H₂O). **ESI-HRMS:** 750.1608 [M]⁺ (theor. [C₂₇H₃₂N₉O₄Cl₁Tm₁]⁺ = 750.1602). EA ([C₂₇H₃₂N₉O₄Cl₁Tm₁]⁺[TFA]⁻·0.2TFA·2.6H₂O, *M*_{R} = 933.6): C 37.8 (37.7); H 4.0 (3.8); N 13.5 (13.4); Cl 3.8 (3.9); F 7.3 (7.3); Tm 18.1 (17.8). 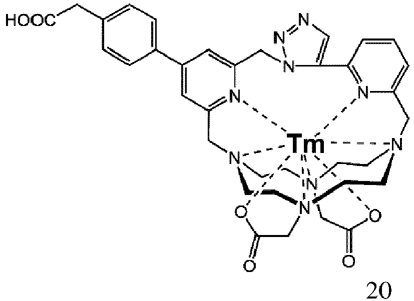 **Synthesis of TD899:** Glass vial (4 mL) was charged with [**TD888**]⁺[TFA]⁻·0.2TFA·2.6H₂O (15.0 mg; 16.1 µmol; 1.0 equiv.), **2-(4-boronophenyl)acetic acid** (4.2 mg; 23.3 µmol; 1.5 equiv.) and **XPhos Pd G2** (0.7 mg; ~0.9 µmol; 6 mol%) and was then three times secured with argon. Under constant flow of argon was then added dry *1,4*-Dioxane (600 µL) through septum followed by addition of freshly prepared (and briefly washed with argon prior addition) aq. solution of **K₃PO₄**·H₂O (391 mM; 200 µL; 78 µmol; 4.9 equiv.). The mixture was then stirred 16 h under septum (but without external argon) at 80 °C. Resulting heterogenous mixture was evaporated to dryness. Residue was re-dissolved in H₂O (3 mL), filtered through syringe microfilter (RC) and purified by preparative HPLC (C18; H₂O-MeCN gradient with TFA additive). Fractions with product were joined and directly lyophilized to give product as white fluffy solid. **Yield:** 10.1 mg (57%; 1 step; based on [**TD888**]⁺[TFA]⁻·0.2TFA·2.6H₂O). **ESI-HRMS:** 850.2362 [M]⁺ (theor. [C₃₅H₃₉N₉O₆Tm₁]⁺ = 850.2360). **EA** ([C₃₅H₃₉N₉O₆Tm₁]⁺[TFA]⁻·0.2TFA·7.0H₂O, *M*_{R} = 1112.6): C 40.4 (40.6); H 4.8 (4.7); N 11.3 (11.1); F 6.1 (6.4). 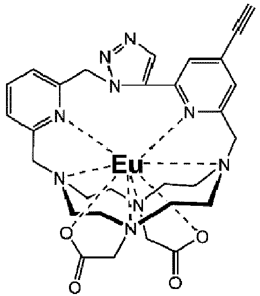 **Synthesis of TD822:** In a glass vial (20 mL), **TD810**·0.5TFA·0.1FA·2.1H₂O (26 mg; 39 µmol; 1.0 equiv.) was dissolved in H₂O (17 mL) followed by addition aq. **AcOH/NaOH buffer** (500 mM; pH 5.8; 2.1 mL; 1.05 mmol; 27 equiv.) and aq. **EuCl₃** (100 mM; 510 µL; 51 µmol; 1.3 equiv.) and the mixture was stirred 3 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 19 mg (≤59% assuming [**M**]⁺[TFA]⁻·*x*H₂O; *M*_{R} = 836.6; 1 step; based on **TD810**·0.5TFA·0.1FA·2.1H₂O). **ESI-HRMS:** 724.1861 [M]⁺ (theor. [C₂₉H₃₃N₉O₄Eu₁]⁺ = 724.1862). 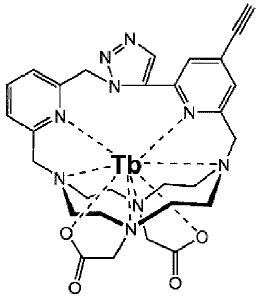 **Synthesis of TD823:** In a glass vial (20 mL), **TD810**·0.5TFA·0.1FA·2.1H₂O (28 mg; 42 µmol; 1.0 equiv.) was dissolved in H₂O (17 mL) followed by addition aq. **AcOH/NaOH buffer** (500 mM; pH 5.8; 2.3 mL; 1.15 mmol; 28 equiv.) and aq. **TbCl₃** (100 mM; 530 µL; 53 µmol; 1.3 equiv.) and the mixture was stirred 2 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 22 mg (≤63%assuming [**M**]⁺[TFA]⁻·*x*H₂O; *M*_{R} = 843.6; 1 step; based on **TD810**·0.5TFA·0.1FA·2.1H₂O). **ESI-HRMS:** 730.1899 [M]⁺ (theor. [C₂₉H₃₃N₉O₄Tb₁]⁺ = 730.1903). 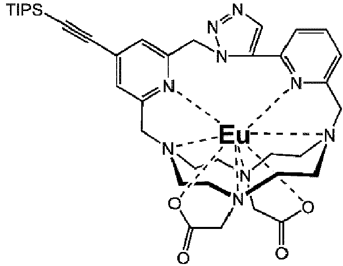 **Synthesis of TD831:** In a glass vial (20 mL), **TD827**·0.8FA·1.6H₂O (50 mg; 63 µmol; 1.0 equiv.) was dissolved in a mixture of abs. EtOH (8 mL) and H₂O (7.5 mL) followed by addition aq. **AcOH/NaOH buffer** (500 mM; pH 5.8; 3.3 mL; 1.65 mmol; 26 equiv.) and aq. **EuCl₃** (100 mM; 740 µL; 74 µmol; 1.2 equiv.) and the mixture was stirred 4 d at 80 °C. Mixture was then diluted with EtOH (100 mL) and filtered through syringe microfilter (RC). The filtrate was concentrated on rotary evaporator. The residue was purified by preparative HPLC (C18; H₂O-MeCN gradient with TFA additive). Fractions with product were joined and directly lyophilized to give product in the form of trifluoroacetate salt as white fluffy solid. **Yield:** 36 mg (≤58% assuming [**M**]⁺[TFA]⁻·*x*H₂O; *M*_{R} = 993.0; 1 step; based on **TD827**·0.8FA·1.6H₂O). **ESI-HRMS:** 880.3189 [M]⁺ (theor. [C₃₈H₅₃N₉O₄Si₁Eu₁]⁺ = 880.3197). 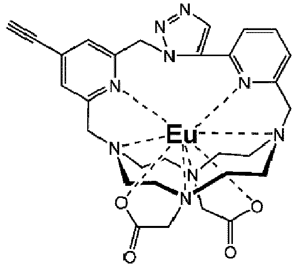 **Synthesis of TD885:** In a glass vial (4 mL), [**TD831**]⁺[TFA]⁻·*x*H₂O (30.5 mg; 30.7 µmol; 1.0 equiv.) was dissolved in a H₂O (3.4 mL) followed by addition aq. **K₂CO₃** (1.0 M; 155 µL; 155 µmol; ~5 equiv.) and the mixture was stirred 40 min at 80 °C. The resulting orange-brown (and not entirely homogenous) mixture was then filtered through syringe microfilter (RC) and the filtrate was purified by preparative HPLC (C18; H₂O-MeCN gradient with TFA additive). Fractions with product were joined and directly lyophilized to give product in the form of trifluoroacetate salt as white fluffy solid. **Yield:** 15.4 mg (≤60% assuming [**M**]⁺[TFA]⁻·*x*H₂O; *M*_{R} = 836.6; 1 step; based on **[TD831]**⁺[TFA]⁻·*x*H₂O). **ESI-HRMS:** 724.1858 [M]⁺ (theor. [C₂₉H₃₃N₉O₄Eu₁]⁺ = 724.1862). 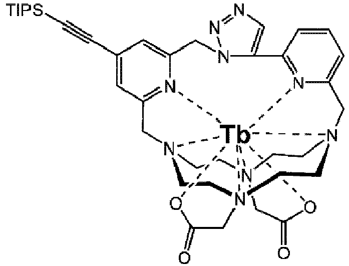 **Synthesis of TD832:** In a glass vial (20 mL), **TD827**·0.8FA·1.6H₂O (47 mg; 59 µmol; 1.0 equiv.) was dissolved in a mixture of abs. EtOH (8 mL) and H₂O (8 mL) followed by addition aq. **AcOH/NaOH buffer** (500 mM; pH 5.8; 3.1 mL; 1.55 mmol; 26 equiv.) and aq. **EuCl₃** (100 mM; 720 µL; 72 µmol; 1.2 equiv.) and the mixture was stirred 4 d at 80 °C. Mixture was then diluted with EtOH (100 mL) and filtered through syringe microfilter (RC). The filtrate was concentrated on rotary evaporator. The residue was purified by preparative HPLC (C18; H₂O-MeCN gradient with TFA additive). Fractions with product were joined and directly lyophilized to give product in the form of trifluoroacetate salt as white fluffy solid. **Yield:** 46 mg (≤78% assuming [**M**]⁺[TFA]⁻·*x*H₂O; *M*_{R} = 999.9; 1 step; based on **TD827**·0.8FA·1.6H₂O). **ESI-HRMS:** 886.3229 [M]⁺ (theor. [C₃₈H₅₃N₉O₄Si₁Tb₁]⁺ = 886.3238). 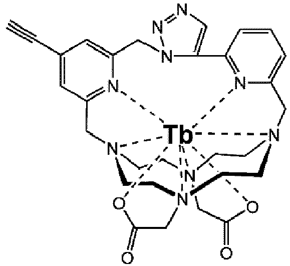 **Synthesis of TD886:** In a glass vial (4 mL), [**TD832**]⁺[TFA]⁻·*x*H₂O (46.2 mg; 46.2 µmol; 1.0 equiv.) was dissolved in a H₂O (3.3 mL) followed by addition aq. **K₂CO₃** (1.0 M; 230 µL; 230 µmol; ~5 equiv.) and the mixture was stirred 40 min at 80 °C. The resulting orange-brown (and not entirely homogenous) mixture was then filtered through syringe microfilter (RC) and the filtrate was purified by preparative HPLC (C18; H₂O-MeCN gradient with TFA additive). Fractions with product were joined and directly lyophilized to give product in the form of trifluoroacetate salt as white fluffy solid. **Yield:** 29.3 mg (≤75% assuming [**M**]⁺[TFA]⁻·*x*H₂O; *M*_{R} = 843.6; 1 step; based on [**TD832**]⁺[TFA]⁻·xH₂O). **ESI-HRMS:** 730.7901 [M]⁺ (theor. [C₂₉H₃₃N₉O₄Tb₁]⁺ = 730.1903). 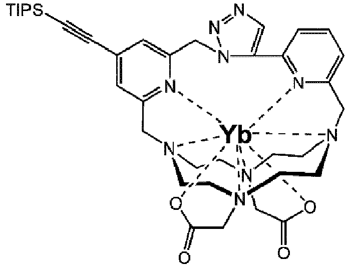 **Synthesis of TD829:** In a glass vial (20 mL), **TD827**·0.8FA·1.6H₂O (31 mg; 39 µmol; 1.0 equiv.) was dissolved in a mixture of abs. EtOH (11 mL) and H₂O (6 mL) followed by addition aq. AcOH/NaOH buffer (500 mM; pH 5.8; 2.0 mL; 1.00 mmol; 26 equiv.) and aq. **YbCl₃** (100 mM; 400 µL; 40 µmol; 1.0 equiv.) and the mixture was stirred 8 h at 80 °C. Mixture was then diluted with MeOH (20 mL) and filtered through syringe microfilter (RC). The filtrate was concentrated on rotary evaporator. The residue was purified by preparative HPLC (C18; H₂O-MeCN gradient with TFA additive). Fractions with product were joined and directly lyophilized to give product in the form of trifluoroacetate salt as white fluffy solid. **Yield:** 38 mg (≤96% assuming [**M**]⁺[TFA]⁻·*x*H₂O; *M*_{R} = 1014.0; 1 step; based on **TD827**·0.8FA·1.6H₂O). **ESI-HRMS:** 901.3372 [M]⁺ (theor. [C₃₈H₅₃N₉O₄Si₁Yb₁]⁺ = 901.3373). 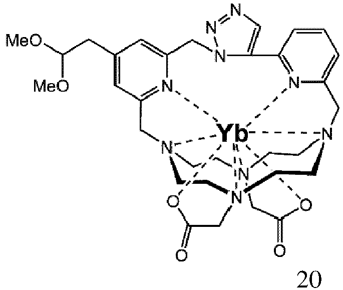 **Synthesis of TD834:** In a glass vial (4 mL), [**TD829**]⁺[TFA]⁻·*x*H₂O (37.9 mg; 37.4 µmol; 1.0 equiv.) was dissolved in a 50% aq. **MeOH** (18 mL) followed by addition aq. **K₂CO₃** (100 mM; 2.0 mL µL; 200 µmol; ~5 equiv.) and the mixture was stirred 1 h at 80 °C. The resulting mixture was then concentrated to ~3 mL and then purified by preparative HPLC (C18; H₂O-MeCN gradient with TFA additive). Fractions with product were joined and directly lyophilized to give product in the form of trifluoroacetate salt as white fluffy solid. **Yield:** 22.5 mg (≤65% assuming [**M**]⁺[TFA]⁻·*x*H₂O; *M*_{R} = 921.8; 1 step; based on [**TD829**]⁺[TFA]⁻·*x*H₂O). **ESI-HRMS:** 809.2570 [M]⁺ (theor. [C₃₁H₄₁N₉O₆Yb₁]⁺ = 809.2563). 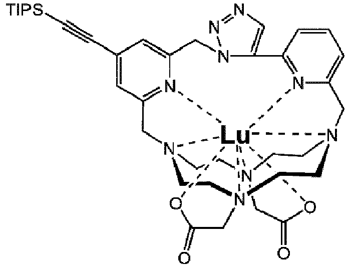 **Synthesis of TD833:** In a glass vial (20 mL), **TD827**·0.8FA·1.6H₂O (64.8 mg; 81 µmol; 1.0 equiv.) was dissolved in a mixture of *i*-PrOH (10 mL) and H₂O (5 mL) followed by addition aq. **MOPS/NaOH buffer** (500 mM; pH 7.0; 4.1 mL; 2.05 mmol; 25 equiv.) and aq. **LuCl₃** (100 mM; 900 µL; 90 µmol; 1.1 equiv.) and the mixture was stirred 20 h at 80 °C. Mixture was then filtered through syringe microfilter (RC). The filtrate was concentrated on rotary evaporator. The residue was purified by preparative HPLC (C18; H₂O-MeCN gradient with TFA additive). Fractions with product were joined and directly lyophilized to give product in the form of trifluoroacetate salt as white fluffy solid. **Yield:** 76.3 mg (78%; 1 step; based on **TD827**·0.8FA·1.6H₂O). **NMR (D₂O, pD ~3): ¹H** δ_{H} 1.06-1.32 (*i-Pr,* m, 21H); 2.63-3.81 (*mc*, C*H*₂-CO, m, 16+4H); 3.90 (C*H*₂-arom., d, 1H, ²*J*_{HH} = 15); 4.15 (C*H*₂-arom., d, 1H, ²*J*_{HH} = 16); 4.28 (C*H*₂-arom., d, 1H, ²*J*_{HH} = 15); 4.84 (C*H*₂-arom., d, 1H, ²*J*_{HH} = 16); 5.55 (C*H*₂-N₃, d, 1H, ²*J*_{HH} = 16); 7.09 (C*H*₂-N₃, d, 1H, ²*J*_{HH} = 16); 7.76 *(arom.,* d, 1H, ⁴*J*_{HH} = 1); 7.80 *(arom.,* dd, 1H, ³*J*_{HH} = 8, ⁴*J*_{HH} = 1); 7.85 *(arom.,* dd, 1H, ³*J*_{HH} = 8, ⁴*J*_{HH} = 1); 7.88 (C*H*+N₃, s, 1H); 8.06 (*arom*., d, 1H, ⁴*J*_{HH} = 1); 8.24 *(arom.,* t, 1H, ³*J*_{HH} = 8). **ESI-HRMS:** 902.3386 [M]⁺ (theor. [C₃₈H₅₃N₉O₄Si₁Lu₁]⁺ = 902.3392). **EA** ([C₃₈H₅₃N₉O₄Si₁Lu₁]⁺[TFA]⁻·1.0TFA·4.0H₂O, *M*_{R} = 1202.0): C 42.0 (41.7); H 5.2 (5.0); N 10.5 (10.4); Si 2.3 (1.9); F 9.5 (9.4). 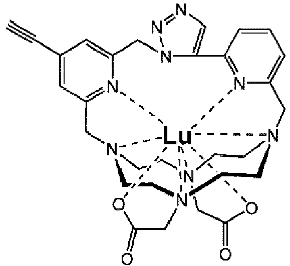 **Synthesis of MIS007:** In a glass vial (40 mL), [**TD833**]⁺[TFA]⁻·1.0TFA·4.0H₂O (19 mg; 16 µmol; 1.0 equiv.) was dissolved in a 50% aq. MeCN (20 mL) followed by addition aq. **K₂CO₃** (100 mM; 2.0 mL; 200 µmol; ~10 equiv.) and the mixture was stirred 1 h at 80 °C. Mixture was then filtered through syringe microfilter (RC). The filtrate was concentrated to ~3 mL and then purified by preparative HPLC (C18; H₂O-MeCN gradient with TFA additive). Fractions with product were joined and directly lyophilized to give product in the form of trifluoroacetate salt as white fluffy solid. Yield: 13 mg (≤81% assuming [**M**]⁺[TFA]⁻·*x*H₂O; *M*_{R} = 1016.0; 1 step; based on [**TD833**]⁺[TFA]⁻·1.0TFA·4.0H₂O). **NMR (D₂O, pD ~5): ¹H** δ_{H} 2.68-3.84 *(mc,* C*H*₂-CO, m, 16+4H); 3.96 (C*H*₂-arom., d, 1H, ²*J*_{HH} = 15); 4.17 (C*H*₂-arom., d, 1H, ²*J*_{HH} = 16); 4.18 (C=CH, s, 1H); 4.30 (C*H*₂-arom., d, 1H, ²*J*_{HH} = 15); 4.86 (C*H*₂-arom., d, 1H, ²*J*_{HH} = 16); 5.61 (C*H*₂-N₃, d, 1H, ²*J*_{HH} = 16); 7.11 (C*H*₂-N₃, d, 1H, ²*J*_{HH} = 16); 7.82 (*arom.,* d, 1H, ⁴*J*_{HH} = 2); 7.82 *(arom.,* dd, 1H, ³*J*_{HH} = 8, ⁴*J*_{HH} = 1); 7.87 *(arom.,* dd, 1H, ³*J*_{HH} = 8, ⁴*J*_{HH} = 1); 7.90 (C*H*-N₃, s, 1H); 8.11 (*arom.,* d, 1H, ⁴*J*_{HH} = 2); 8.27 *(arom.,* t, 1H, ³*J*_{HH} = 8). **ESI-HRMS:** 746.2059 [M]⁺ (theor. [C₂₉H₃₃N₉O₄Lu₁]⁺ = 746.2058). 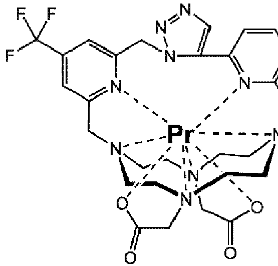 **Synthesis of TD1113:** In a glass vial (20 mL), **TD1063**·0.1FA·1.2H₂O (4.0 mg; 6.2 µmol; 1.0 equiv.) was dissolved in H₂O (10 mL) followed by addition aq. **MOPS/NaOH buffer** (500 mM; pH 7.0; 310 µL; 155 µmol; 25 equiv.) and aq. **PrCl₃** (100 mM; 67 µL; 6.7 µmοl; 1.1 equiv.) and the mixture was stirred 10 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 0.6 mg (≤10% assuming [M]⁺[TFA]⁻·1.0TFA·*x*H₂O; *M*_{R} = 985.9; 1 step; based on **TD1063**·0.1FA·1.2H₂O). **NMR (D₂O, pD ~3): ¹⁹F** δ_{F} -66.45 (bs). **NMR (~0.5 mM TD1113 in -50 mM MOPS/NaOH buffer in 10% D₂O, pH 7.0): ¹⁹F** δ_{F} -66.49 (bs; ΔH_{½} = 135.2 Hz). **ESI-HRMS:** 756.1593 [M]⁺ (theor. [C₂₈H₃₂N₉O₄F₃Pr₁]⁺ = 756.1600). 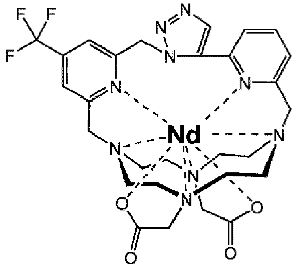 **Synthesis of TD1114:** In a glass vial (20 mL), **TD1063**·0.1FA·1.2H₂O (4.0 mg; 6.2 µmol; 1.0 equiv.) was dissolved in H₂O (10 mL) followed by addition aq. **MOPS/NaOH buffer** (500 mM; pH 7.0; 310 µL; 155 µmol; 25 equiv.) and aq. **NdCl₃** (100 mM; 67 µL; 6.7 µmol; 1.1 equiv.) and the mixture was stirred 10 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 2.0 mg (≤33% assuming [**M**]⁺[TFA]⁻·0.9TFA·*x*H₂O; *M*_{R} = 975.5; 1 step; based on **TD1063**·0.1FA·1.2H₂O). **NMR (D₂O, pD ~3): ¹⁹F** δ_{F} -66.75 (s). **NMR (~0.5 mM TD1114 in ~50 mM MOPS/NaOH buffer in 10% D₂O, pH 7.0): ¹⁹F** δ_{F} -66.76 (s; ΔH_{½} = 47.4 Hz). **ESI-HRMS:** 757.1595 [M]⁺ (theor. [C₂₈H₃₂N₉O₄F₃Nd₁]⁺ = 757.1601). 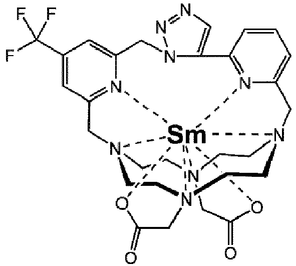 **Synthesis of TD1115:** In a glass vial (20 mL), **TD1063**·0.1FA·1.2H₂O (4.0 mg; 6.2 µmol; 1.0 equiv.) was dissolved in H₂O (10 mL) followed by addition aq. **MOPS/NaOH buffer** (500 mM; pH 7.0; 310 µL; 155 µmol; 25 equiv.) and aq. **SmCl₃** (100 mM; 67 µL; 6.7 µmol; 1.1 equiv.) and the mixture was stirred 10 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 3.6 mg (≤63% assuming [**M**]⁺[TFA]⁻·0.3TFA·*x*H₂O; *M*_{R} = 913.2; 1 step; based on **TD1063**·0.1FA·1.2H₂O). **NMR (D₂O, pD -2): ¹⁹F** δ_{F} -66.03 (s). **NMR (~0.5 mM TD1115 in -50 mM MOPS/NaOH buffer in 10% D₂O, pH 7.0): ¹⁹F** δ_{F} -66.06 (s; ΔH_{½} = 1.9 Hz). **ESI-HRMS:** 767.1713 [M]⁺ (theor. [C₂₈H₃₂N₉O₄F₃Sm₁]⁺ = 767.1721). 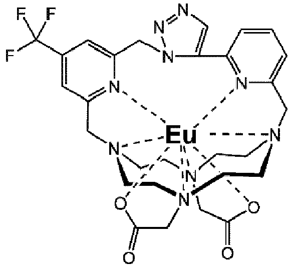 **Synthesis of TD1072:** In a glass vial (20 mL), **TD1063**·0.1FA·1.2H₂O (2.9 mg; 4.6 µmol; 1.0 equiv.) was dissolved in H₂O (10 mL) followed by addition aq. **MOPS/NaOH buffer** (500 mM; pH 7.0; 225 µL; 113 µmol; 25 equiv.) and aq. **EuCl₃** (100 mM; 55 µL; 5.5 µmol; 1.2 equiv.) and the mixture was stirred 8 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 2.7 mg (≤66% assuming [**M**]⁺[TFA]⁻·0.3TFA·xH₂O; *M*_{R} = 914.8; 1 step; based on **TD1063**·0.1FA·1.2H₂O). **NMR (D₂O, pD ~2): ¹⁹F** δ_{F} -62.77 (s). **NMR (~0.5 mM TD1072 in ~50 mM MOPS/NaOH buffer in 10% D₂O, pH 7.0): ¹⁹F** δ_{F} -62.79 (s; ΔH_{½} = 3.3 Hz). ESI-**HRMS:** 768.1738 [M]⁺ (theor. [C₂₈H₃₂N₉O₄F₃Eu₁]⁺ = 768.1736). 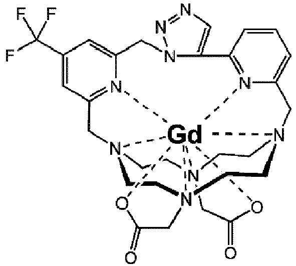 **Synthesis of TD1179:** In a glass vial (20 mL), **TD1063**·0.1FA·1.2H₂O (4.0 mg; 6.5 µmol; 1.0 equiv.) was dissolved in H₂O (13 mL) followed by addition aq. **MOPS/NaOH buffer** (500 mM; pH 7.0; 326 µL; 163 µmol; 25 equiv.) and aq. **GdCl₃** (100 mM; 78 µL; 7.8 µmol; 1.2 equiv.) and the mixture was stirred 4 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 2.2 mg (≤36% assuming [M]⁺[TFA]⁻·0.9TFA·*x*H₂O; *M*_{R} = 988.5; 1 step; based on **TD1063**·0.1FA·1.2H₂O). **NMR (D₂O, pD ~2): ¹⁹F** δ_{F} -62.98 (bs). **NMR (-0.5 mM TD1179 in -50 mM MOPS/NaOH buffer in 10% D₂O, pH 7.0): ¹⁹F** δ_{F} -62.97 (bs; ΔH_{½} = 342.9 Hz). **ESI-HRMS:** 773.1779 [M]⁺ (theor. [C₂₈H₃₂N₉O₄F₃Gd₁]⁺ = 773.1765). 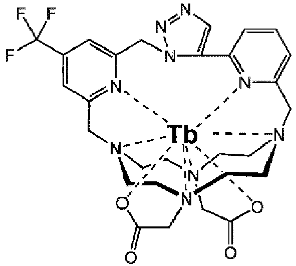 **Synthesis of TD1073:** In a glass vial (20 mL), **TD1063**·0.1FA·1.2H₂O (2.9 mg; 4.6 µmol; 1.0 equiv.) was dissolved in H₂O (10 mL) followed by addition aq. MOPS/NaOH buffer (500 mM; pH 7.0; 225 µL; 113 µmol; 25 equiv.) and aq. **TbCl₃** (100 mM; 55 µL; 5.5 µmol; 1.2 equiv.) and the mixture was stirred 6 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 2.8 mg (≤64% assuming **[M]**⁺[TFA]⁻·0.7TFA·*x*H₂O; *M*_{R} = 967.3; 1 step; based on **TD1063**·0.1FA·1.2H₂O). **NMR (D₂O, pD -2): ¹⁹F** δ_{F} -107.22 (bs). **NMR (~0.5 mM TD1073 in -50 mM MOPS/NaOH buffer in 10% D₂O, pH 7.0): ¹⁹F** δ_{F} -107.50 (bs; ΔH_{½} = 708.6 Hz). **ESI-HRMS:** 774.1779 [M]⁺ (theor. [C₂₈H₃₂N₉O₄F₃Tb₁]⁺ = 774.1777). 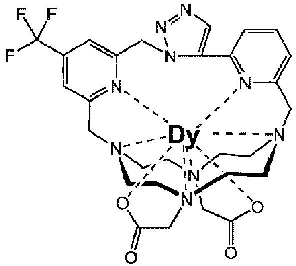 **Synthesis of TD1074:** In a glass vial (20 mL), **TD1063**·0.1FA·1.2H₂O (2.9 mg; 4.6 µmol; 1.0 equiv.) was dissolved in H₂O (10 mL) followed by addition aq. **MOPS/NaOH buffer** (500 mM; pH 7.0; 225 µL; 113 µmol; 25 equiv.) and aq. **DyCl₃** (100 mM; 55 µL; 5.5 µmol; 1.2 equiv.) and the mixture was stirred 5 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. Yield: 3.3 mg (≤75% assuming [**M**]⁺[TFA]⁻·0.7TFA·*x*H₂O; *M*_{R} = 970.9; 1 step; based on **TD1063**·0.1FA·1.2H₂O). **NMR (D₂O, pD ~2): ¹⁹F** δ_{F} -142.86 (s). **NMR (-0.5 mM TD1074 in -50 mM MOPS/NaOH buffer in 10% D₂O, pH 7.0): ¹⁹F** δ_{F} -143.22 (s; ΔH_{½} = 47.2 Hz). **ESI-HRMS:** 779.1816 [M]⁺ (theor. [C₂₈H₃₂N₉O₄F₃Dy₁]⁺ = 779.1816). 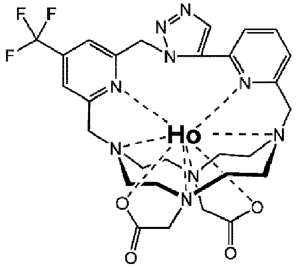 **Synthesis of TD1075:** In a glass vial (20 mL), **TD1063**·0.1FA·1.2H₂O (2.9 mg; 4.6 µmol; 1.0 equiv.) was dissolved in H₂O (10 mL) followed by addition aq. MOPS/NaOH buffer (500 mM; pH 7.0; 225 µL; 113 µmol; 25 equiv.) and aq. **HoCl₃** (100 mM; 55 µL; 5.5 µmol; 1.2 equiv.) and the mixture was stirred 5 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 4.0 mg (≤90% assuming [**M**]⁺[TFA]⁻·0.8TFA·*x*H₂O; *M*_{R} = 984.7; 1 step; based on **TD1063**·0.1FA·1.2H₂O). **NMR (D₂O, pD ~2): ¹⁹F** δ_{F} -85.69 (bs). **NMR (~0.5 mM TD1075 in -50 mM MOPS/NaOH buffer in 10% D₂O, pH 7.0): ¹⁹F** δ_{F} -85.79 (bs; ΔH_{½} = 185.8 Hz). **ESI-HRMS:** 780.1829 [M]⁺ (theor. [C₂₈H₃₂N₉O₄F₃Ho₁]⁺ = 780.1827). 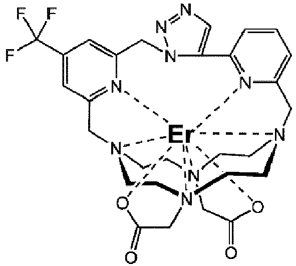 **Synthesis of TD1076:** In a glass vial (20 mL), **TD1063**·0.1FAT·1.2H₂O (2.9 mg; 4.6 µmol; 1.0 equiv.) was dissolved in H₂O (10 mL) followed by addition aq. **MOPS/NaOH buffer** (500 mM; pH 7.0; 225 µL; 113 µmol; 25 equiv.) and aq. **ErCl₃** (100 mM; 55 µL; 5.5 µmol; 1.2 equiv.) and the mixture was stirred 4 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 4.1 mg (≤92% assuming [M]⁺[TFA]⁻·1.0TFA·*x*H₂O; *M*_{R} = 987.1; 1 step; based on **TD1063**·0.1FA·1.2H₂O). **NMR (D₂O, pD -2): ¹⁹F** δ_{F} -47.48 (s). **NMR (~0.5 mM TD1076 in -50 mM MOPS/NaOH buffer in 10% D₂O, pH 7.0): ¹⁹F** δ_{F} -47.42 (s; ΔH_{½} = 19.3 Hz). **ESI-HRMS:** 781.1824 [M]⁺ (theor. [C₂₈H₃₂N₉O₄F₃Er₁]⁺ = 781.1827). 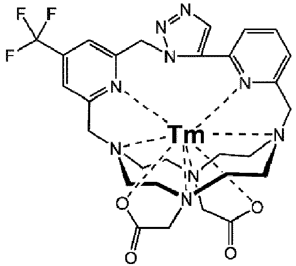 **Synthesis of 1077:** In a glass vial (20 mL), **TD1063**·0.1FA·1.2H₂O (2.9 mg; 4.6 µmol; 1.0 equiv.) was dissolved in H₂O (10 mL) followed by addition aq. **MOPS/NaOH buffer** (500 mM; pH 7.0; 225 µL; 113 µmol; 25 equiv.) and aq. **TmCl₃** (100 mM; 55 µL; 5.5 µmol; 1.2 equiv.) and the mixture was stirred 4 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 4.2 mg (≤92% assuming [**M**]⁺[TFA]⁻·1.0TFA·*x*H₂O; *M*_{R} = 1011.6; 1 step; based on **TD1063**·0.1FA·1.2H₂O). **NMR (D₂O, pD -2): ¹⁹F** δ_{F} -57.57 (bs). **NMR (-0.5 mM TD1077 in ~50 mM MOPS/NaOH buffer in 10% D₂O, pH 7.0): ¹⁹F** δ_{F} -57.51 (bs; ΔH_{½} = 138.72 Hz). **ESI-HRMS:** 784.1863 [M]⁺ (theor. [C₂₈H₃₂N₉O₄F₃Tm₁]⁺ = 784.1866). 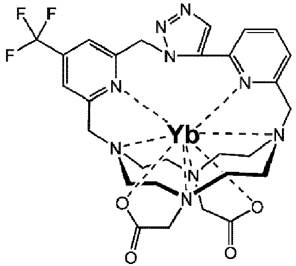 **Synthesis of 1078:** In a glass vial (20 mL), **TD1063**·0.1FA·1.2H₂O (2.9 mg; 4.6 µmol; 1.0 equiv.) was dissolved in H₂O (10 mL) followed by addition aq. **MOPS/NaOH buffer** (500 mM; pH 7.0; 225 µL; 113 µmol; 25 equiv.) and aq. **YbCl₃** (100 mM; 55 µL; 5.5 µmol; 1.2 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 3.7 mg (≤82% assuming [**M**]⁺[TFA]⁻·0.9TFA·*x*H₂O; *M*_{R} = 1004.3; 1 step; based on **TD1063**·0.1FA·1.2H₂O). **NMR (D₂O, pD -2): ¹⁹F** δ_{F} -59.01 (s). **NMR (~0.5 mM TD1078 in ~50 mM MOPS/NaOH buffer in 10% D₂O, pH 7.0): ¹⁹F** δ_{F} -59.00 (s; ΔH_{½} = 18.4 Hz). **ESI-HRMS:** 789.1913 [M]⁺ (theor. [C₂₈H₃₂N₉O₄F₃Yb₁]⁺ = 789.1912). 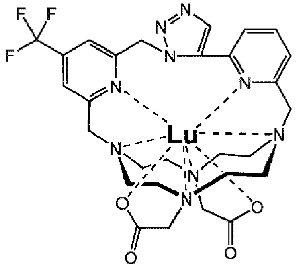 **Synthesis of TD1079:** In a glass vial (20 mL), **TD1063**·0.1FA·1.2H₂O (14.7 mg; 22.8 µmol; 1.0 equiv.) was dissolved in H₂O (18 mL) followed by addition aq. **MOPS/NaOH buffer** (500 mM; pH 7.0; 1150 µL; 575 µmol; 25 equiv.) and aq. **LuCl₃** (100 mM; 250 µL; 25.0 µmol; 1.1 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 16.1 mg (70%; 1 step; based on **TD1063**·0.1FA·1.2H₂O). **NMR (D₂O, pD ~2): ¹H** δ_{H} 2.69-3.85 (*mc,* C*H*₂-CO, m, 16+4H); 4.12 (C*H*₂-arom., d, 1H, ²*J*_{HH} = 15); 4.19 (C*H*₂-arom., d, 1H, ²*J*_{HH} = 16); 4.38 (C*H*₂-arom., d, 1H, ²*J*_{HH} = 15); 4.88 (C*H*₂-arom., d, 1H, ²*J*_{HH} = 16); 5.75 (C*H*₂-N₃, d, 1H, ²*J*_{HH} = 15); 7.23 (C*H*₂-N₃, d, 1H, ²*J*_{HH} = 15); 7.83 *(arom.,* dd, 1H, ³*J*_{HH} = 8, ⁴*J*_{HH} = 1); 7.88 *(arom.,* dd, 1H, ³*J*_{HH} = 8, ⁴*J*_{HH} = 1); 7.90 (C*H*-N₃, s, 1H); 8.12 (*arom*., d, 1H, ⁴*J*_{HH} = 2); 8.28 (*arom*., t, 1H, ³*J*_{HH} = 8), 8.43 (*arom*., d, 1H, ⁴*J*_{HH} = 2). **¹⁹F** δ_{F} -66.10 (s). **NMR (~0.5 mM TD1079 in ~50 mM MOPS/NaOH buffer in 10% D₂O, pH 7.0): ¹⁹F** δ_{F} -66.13 (s; ΔH_{½} = 2.4 Hz). **ESI-HRMS:** 790.1931 [M]⁺ (theor. [C₂₈H₃₂N₉O₄F₃Lu₁]⁺ = 790.1932). **EA** ([C₂₈H₃₂N₉O₄Lu₁F₃]⁺[TFA]⁻·0.2TFA·4.1H₂O, *M*_{R} = 1000.2): C 36.5 (36.2); H 4.1 (3.6); N 12.6 (12.3); F 12.5 (12.0). 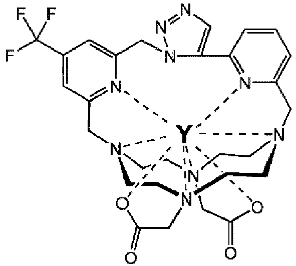 **Synthesis of TD1080:** In a glass vial (20 mL), **TD1063**·0.1FA·1.2H₂O (2.9 mg; 4.6 µmol; 1.0 equiv.) was dissolved in H₂O (10 mL) followed by addition aq. **MOPS/NaOH buffer** (500 mM; pH 7.0; 225 µL; 113 µmol; 25 equiv.) and aq. **YCl₃** (100 mM; 55 µL; 5.5 µmol; 1.2 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 3.6 mg (≤94% assuming **[M]**⁺[TFA]⁻·0.3TFA·*x*H₂O; *M*_{R} = 851.7; 1 step; based on **TD1063**·0.1FA·1.2H₂O). **NMR (D₂O, pD ~2): ¹H** δ_{H} 2.66-3.87 (*mc*, C*H*₂-CO, m, 16+4H); 4.11 (C*H*₂-arom., d, 1H, ²*J*_{HH} = 15); 4.18 (C*H*₂-arom., d, 1H, ²*J*_{HH} = 16); 4.42 (C*H*₂-arom., d, 1H, ²*J*_{HH} = 15); 4.89 (C*H*₂-arom., d, 1H, ²*J*_{HH} = 16); 5.80 (C*H*₂-N₃, d, 1H, ²*J*_{HH} = 16); 7.16 (C*H*₂-N₃, d, 1H, ²*J*_{HH} = 16); 7.84 *(arom.,* dd, 1H, ³*J*_{HH} = 8, ⁴*J*_{HH} = 1); 7.87 (*arom*., dd, 1H, ³*J*_{HH} = 8, ⁴*J*_{HH} = 1); 7.90 (C*H*-N₃, s, 1H); 8.13 *(arom.,* d, 1H, ⁴*J*_{HH} = 2); 8.28 (*arom*., t, 1H, ³*J*_{HH} = 8), 8.43 (*arom*., d, 1H, ⁴*J*_{HH} = 2). **¹⁹F** δ_{F} -66.10 (s). **NMR (∼0.5 mM TD1080 in -50 mM MOPS/NaOH buffer in 10% D₂O, pH 7.0): ¹⁹F** δ_{F} -66.12 (s; ΔH_{½} = 2.1 Hz). **ESI-HRMS:** 704.1583 [M]⁺ (theor. [C₂₈H₃₂N₉O₄F₃Y₁]⁺ = 704.1582). 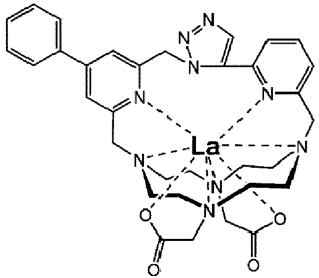 **Synthesis of TD651:** In a glass vial (20 mL), **TD647**·0.2FA·0.9H₂O (30 mg; 46 µmol; 1.0 equiv.) was dissolved in H₂O (17 mL) followed by addition aq. **MOPS/NaOH buffer** (500 mM; pH 7.0; 2.30 mL; 1.15 mmol; 25 equiv.) and aq. **LaCl₃** (100 mM; 500 µL; 50 µmol; 1.1 equiv.) and the mixture was stirred 14 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 15 mg (36%; 1 step; based on **TD647**·0.2FA·0.9H₂O). **NMR (D₂O, pD ~5): ¹H** δ_{H} 2.54-3.63 (*mc,* C*H*₂-CO, m, 16+3H); 3.96 (C*H*₂-arom., d, 1H, ²*J*_{HH} = 15); 4.00 (C*H*₂-arom., d, 1H, ²*J*_{HH} = 14); 4.09 (C*H*₂-CO, 1H, d, ²*J*_{HH} = 17); 4.23 (C*H*₂-arom., d, 1H, ²*J*_{HH} = 15); 4.25 (C*H*₂-arom., d, 1H, ²*J*_{HH} = 14); 5.72 (C*H*₂-N₃, d, 1H, ²*J*_{HH} = 16); 6.62 (C*H*₂-N₃, d, 1H, ²*J*_{HH} = 16); 7.58-7.66 *(Ph,* m, 3H); 7.70 *(arom.,* dd, 1H, ³*J*_{HH} = 8, ⁴*J*_{HH} = 1); 7.81 *(arom.,* dd, 1H, ³*J*_{HH} = 8, ⁴*J*_{HH} = 1); 7.83-7.95 (*Ph, arom.,* m, 2+1H); 8.06 (*arom*., d, 1H, ⁴*J*_{HH} = 2); 8.16 (C*H*-N₃, s, 1H); 8.22 (*arom*., t, 1H, ³*J*_{HH} = 8); 8.42 (FA, s, 1H). **¹³C{¹H}** δ_{C} 53.5 (*C*H₂-N₃, s); 56.3 (*mc*, s); 58.7 (*mc*, s); 58.9 (*mc*, s); 59.2 (*mc*, s); 59.3 (*mc*, s); 60.0 (*mc*, s); 61.9 (*mc*, s); 62.5 (*C*H₂-CO, s); 63.5 (*mc*, s); 65.1 (*C*H₂-CO, s); 65.2 (C*H*₂-arom., s); 66.6 (C*H*₂-arom., s); 124.1 (*arom*., s); 124.6 (*arom*., s); 127.1 (*arom*., s); 127.9 (*arom*., s); 128.1 *(Ph,* s); 130.2 *(Ph,* s); 131.5 *(Ph,* s); 136.1 *(arom,* s); 136.2 *(Ph.,* s); 136.8 (*C*H-N₃, s); 142.0 (*arom*., s); 144.9 (*arom*., s); 153.6 (*arom*., s); 157.0 (*arom*., s); 157.5 (*arom*., s); 159.0 (*arom*., s); 171.0 (FA, s); 179.0 (CO, s); 180.4 (CO, s). **ESI-HRMS:** 762.2024 [M]⁺ (theor. [C₃₃H₃₇N₉O₄La₁]⁺ = 762.2027). **EA** ([C₃₃H₃₇N₉O₄La₁]⁺[FA]⁻·4.8H₂O, *M*_{R} = 894.1): C 45.7 (46.1); H 5.4 (4.7); N 14.1 (13.6); La 15.5 (15.0). 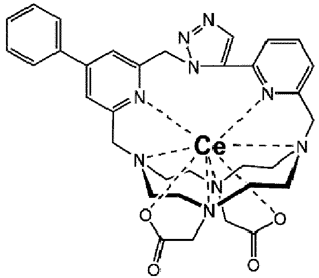 **Synthesis of TD870:** In a glass vial (20 mL), **TD647**·1.3TFA (30.5 mg; 39.4 µmol; 1.0 equiv.) was dissolved in H₂O (17 mL) followed by addition aq. **MOPS/NaOH buffer** (500 mM; pH 7.0; 2.30 mL; 1.15 mmol; 29 equiv.) and aq. **CeCl₃** (100 mM; 545 µL; 54.5 µmol; 1.4 equiv.) and the mixture was stirred 14 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 21.2 mg (58%; 1 step; based on **TD647**·1.3TFA). **ESI-HRMS:** 763.2012 [M]⁺ (theor. [C₃₃H₃₇N₉O₄Ce₁]⁺ = 763.2017). **EA** ([C₃₃H₃₇N₉O₄Ce₁]⁺[FA]⁻·6.5H₂O, *M*_{R} = 925.9): C 44.1 (44.7); H 5.6 (5.1); N 13.6 (13.1); Ce 15.1 (14.5). 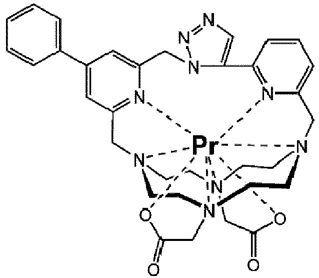 **Synthesis of TD869:** In a glass vial (20 mL), **TD647**·1.3TFA (26.4 mg; 34.1 µmol; 1.0 equiv.) was dissolved in H₂O (17 mL) followed by addition aq. **MOPS/NaOH buffer** (500 mM; pH 7.0; 2.40 mL; 1.20 mmol; 35 equiv.) and aq. **PrCl₃** (100 mM; 500 µL; 50.0 µmol; 1.5 equiv.) and the mixture was stirred 14 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 17.7 mg (56%; 1 step; based on **TD647**·1.3TFA). **ESI-HRMS:** 764.2035 [M]⁺ (theor. [C₃₃H₃₇N₉O₄Pr₁]⁺ = 764.2040). **EA** ([C₃₃H₃₇N₉O₄Pr₁]⁺[FA]⁻·6.4H₂O, *M*_{R} = 924.9): C 44.2 (44.7); H 5.5 (5.0); N 13.6 (13.1); Pr 15.2 (14.8). 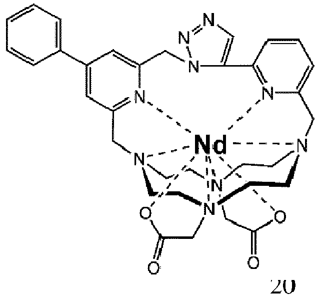 **Synthesis of TD868:** In a glass vial (20 mL), **TD647**·1.3TFA (25.7 mg; 33.2 µmol; 1.0 equiv.) was dissolved in H₂O (17 mL) followed by addition aq. **MOPS/NaOH buffer** (500 mM; pH 7.0; 2.30 mL; 1.15 mmol; 35 equiv.) and aq. **NdCl₃** (100 mM; 460 µL; 46.0 µmol; 1.4 equiv.) and the mixture was stirred 12 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 19.8 mg (65%; 1 step; based on **TD647**·1.3TFA). **ESI-HRMS:** 765.2039 [M]⁺ (theor. [C₃₃H₃₇N₉O₄Nd₁]⁺ = 765.2040). **EA** ([C₃₃H₃₇N₉O₄Nd₁]⁺[FA]⁻·5.6H₂O, *M*_{R} = 913.9): C 44.7 (45.0); H 5.4 (4.6); N 13.8 (13.4); Nd 15.8 (15.2). 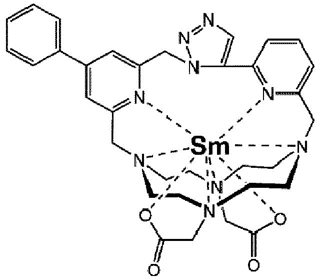 **Synthesis of TD867:** In a glass vial (20 mL), **TD647**·1.3TFA (25.6 mg; 33.1 µmol; 1.0 equiv.) was dissolved in H₂O (17 mL) followed by addition aq. **MOPS/NaOH buffer** (500 mM; pH 7.0; 2.30 mL; 1.15 mmol; 35 equiv.) and aq. **SmCl₃** (100 mM; 460 µL; 46.0 µmol; 1.4 equiv.) and the mixture was stirred 12 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 22.3 mg (73%; 1 step; based on **TD647**·1.3TFA). **ESI-HRMS:** 775.2158 [M]⁺ (theor. [C₃₃H₃₇N₉O₄Sm₁]⁺ = 775.2160). **EA** ([C₃₃H₃₇N₉O₄Sm₁]⁺[FA]⁻5.8H₂O, *M*_{R} = 923.6): C 44.2 (44.7); H 5.4 (4.9); N 13.6 (13.3); Nd 16.3 (15.8). 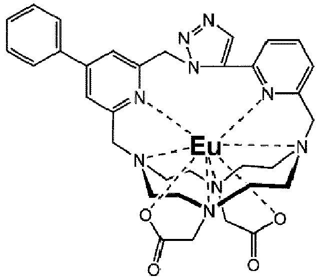 **Synthesis of TD599:** In a glass vial (20 mL), **TD647**·1.3H₂O (50 mg; 77 µmol; 1.0 equiv.) was dissolved in H₂O (15 mL) followed by addition aq. **MOPS/NaOH buffer** (500 mM; pH 7.0; 3.75 mL; 1.88 mmol; 24 equiv.) and aq. **EuCl₃** (100 mM; 825 µL; 83 µmol; 1.1 equiv.) and the mixture was stirred 3 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 44 mg (63%; 1 step; based on **TD647**·1.3H₂O). **ESI-HRMS:** 776.2171 [M]⁺ (theor. [C₃₃H₃₇N₉O₄Eu₁]⁺ = 776.2175). **EA** ([C₃₃H₃₇N₉O₄Eu₁]⁺[FA]⁻·6H₂O, *M*_{R} = 903.6): C 45.2 (45.3); H 5.3 (5.1); N 14.0 (13.7); Eu 16.8 (16.5). 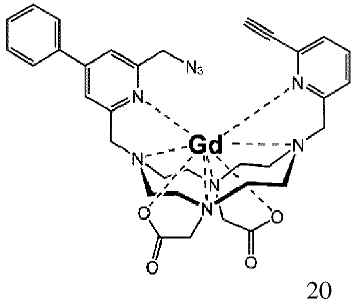 **Synthesis of TD451:** In a glass vial (2 mL), **TD647**·2.2H₂O (2.0 mg; 3.1 µmol; 1.0 equiv.) was dissolved in aq. **MOPS/NaOH buffer** (500 mM; pH 7.0; 155 µL; 77.5 µmol; 25 equiv.) and aq. **GdCl₃** (100 mM; 34 µL; 120 µmol; 1.1 equiv.) and the mixture was stirred 2 d at RT. Mixture was then diluted with H₂O and filtered through syringe microfilter (RC). The filtrate was directly purified by preparative HPLC (C18; H₂O-MeCN gradient with FA additive). Fractions with product were joined and directly lyophilized to give product in the form of formate salt as white fluffy solid. Yield: 0.3 mg. 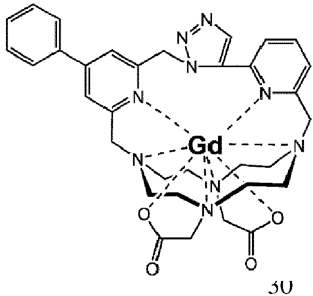 **Synthesis of TD527:** In a glass vial (40 mL), **TD647**·2.2H₂O (75 mg; 113 µmol; 1.0 equiv.) was dissolved in H₂O (32 mL) followed by addition aq. **MOPS/NaOH buffer** (500 mM; pH 7.0; 4.60 mL; 2.30 mmol; 20 equiv.) and aq. **GdCl₃** (100 mM; 1.20 mL; 120 µmol; 1.1 equiv.) and the mixture was stirred 2 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 58 mg (56%; 1 step; based on **TD647**·2.2H₂O). **ESI-HRMS:** 781.2218 [M]⁺ (theor. [C₃₃H₃₇N₉O₄Gd₁]⁺ = 781.2204). **EA** ([C₃₃H₃₇N₉O₄Gd₁]⁺[FA]⁻·5.3H₂O, *M*_{R} = 921.5): C 44.3 (44.7); H 5.3 (4.7); N 13.7 (13.4); Gd 17.1 (16.2). 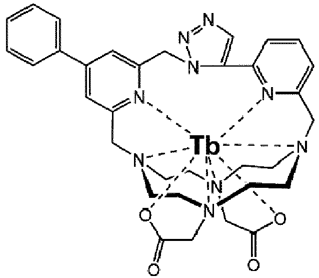 **Synthesis of TD600:** In a glass vial (20 mL), **TD647** · 1.3H₂O (50 mg; 77 µmol; 1.0 equiv.) was dissolved in H₂O (15 mL) followed by addition aq. **MOPS/NaOH buffer** (500 mM; pH 7.0; 3.75 mL; 1.88 mmol; 24 equiv.) and aq. **TbCl₃** (100 mM; 825 µL; 83 µmol; 1.1 equiv.) and the mixture was stirred 3 d at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 49 mg (69%; 1 step; based on **TD647·**1.3H₂O). **ESI-HRMS:** 782.2211 [M]⁺ (theor. [C₃₃H₃₇N₉O₄Tb₁]⁺ = 781.2217). **EA** ([C₃₃H₃₇N₉O₄Tb₁]⁺[FA]⁻4.9H₂O, *M*_{R} = 915.9): C 44.6 (44.7); H 5.3 (5.1); N 13.8 (13.6); Tb 17.4 (17.1). 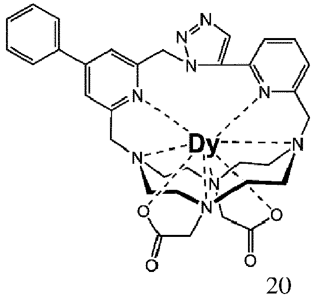 **Synthesis of TD866:** In a glass vial (20 mL), **TD647·**1.3TFA (20.0 mg; 25.8 µmol; 1.0 equiv.) was dissolved in H₂O (17 mL) followed by addition aq. **MOPS/NaOH buffer** (500 mM; pH 7.0; 1.84 mL; 920 µmol; 36 equiv.) and aq. **DyCl₃** (100 mM; 370 µL; 37.0 µmol; 1.4 equiv.) and the mixture was stirred 46 h at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 22.2 mg (89%; 1 step; based on **TD647**·1.3TFA). **ESI-HRMS:** 787.2248 [M]⁺ (theor. [C₃₃H₃₇N₉O₄Dy₁]⁺ = 787.2255). **EA** ([C₃₃H₃₇N₉O₄Dy₁]⁺[FA]⁻·7.7H₂O, *M*_{R} = 970.0): C 42.1 (42.5); H 5.5 (5.0); N 13.0 (12.6); Dy 16.8 (16.1). 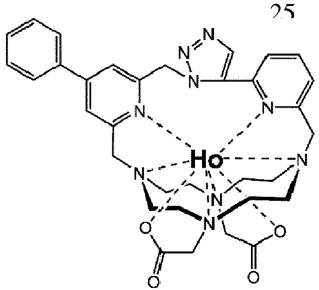 **Synthesis of TD648:** In a glass vial (20 mL), **TD647**·0.2FA·0.9H₂O (22 mg; 34 µmol; 1.0 equiv.) was dissolved in H₂O (17 mL) followed by addition aq. **MOPS/NaOH buffer** (500 mM; pH 7.0; 1.72 mL; 860 µmol; 25 equiv.) and aq. **HoCl₃** (100 mM; 375 µL; 38 µmol; 1.1 equiv.) and the mixture was stirred 20 h at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 24 mg (76%; 1 step; based on **TD647·**0.2FA·0.9H₂O). **ESI-HRMS:** 788.2265 [M]⁺ (theor. [C₃₃H₃₇N₉O₄Ho₁]⁺ = 788.2266). **EA** ([C₃₃H₃₇N₉O₄Ho₁]⁺[FA]⁻·5.0H₂O, *M*_{R} = 923.7): C 44.2 (44.2); H 5.2 (4.8); N 13.6 (13.3); Ho 17.9 (17.3). 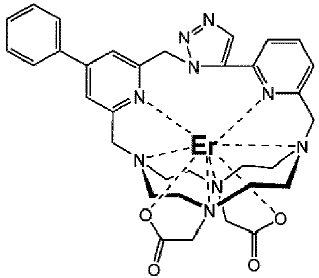 **Synthesis of TD865:** In a glass vial (20 mL), **TD647·**1.3TFA (20.5 mg; 26.5 µmol; 1.0 equiv.) was dissolved in H₂O (17 mL) followed by addition aq. **MOPS/NaOH buffer** (500 mM; pH 7.0; 1.84 mL; 920 µmol; 35 equiv.) and aq. **ErCl₃** (100 mM; 370 µL; 37.0 µmol; 1.4 equiv.) and the mixture was stirred 22 h at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 22.8 mg (90%; 1 step; based on **TD647**·1.3TFA). **ESI-HRMS:** 791.2280 [M]⁺ (theor. [C₃₃H₃₇N₉O₄Er₁]⁺ = 791.2302). **EA** ([C₃₃H₃₇N₉O₄Er₁]⁺[FA]⁻·6.9H₂O, *M*_{R} = 960.3): C 42.5 (42.4); H 5.4 (4.9); N 13.1 (12.7); Er 17.4 (16.8). 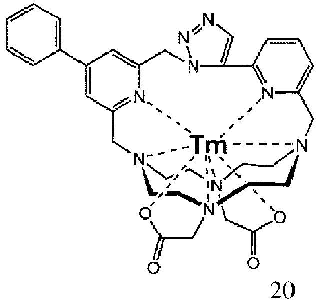 **Synthesis of TD601:** In a glass vial (20 mL), **TD647**·1.3H₂O (49 mg; 75 µmol; 1.0 equiv.) was dissolved in H₂O (15 mL) followed by addition aq. **MOPS/NaOH buffer** (500 mM; pH 7.0; 3.75 mL; 1.88 mmol; 25 equiv.) and aq. **TmCl₃** (100 mM; 825 µL; 83 µmol; 1.1 equiv.) and the mixture was stirred 20 h at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 51 mg (73%; 1 step; based on **TD647·**1.3H₂O). **ESI-HRMS:** 792.2299 [M]⁺ (theor. [C₃₃H₃₇N₉O₄Tm₁]⁺ = 792.2305). **EA** ([C₃₃H₃₇N₉O₄Tm₁]⁺[FA]⁻·4.7H₂O, *M*_{R} = 922.3): C 44.3 (44.4); H 5.2 (5.1); N 13.7 (13.5); Tm 18.3 (18.3). 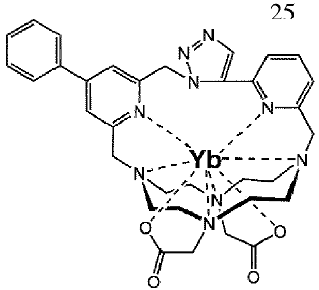 **Synthesis of TD602:** In a glass vial (20 mL), **TD647**·1.3H₂O (49 mg; 75 µmol; 1.0 equiv.) was dissolved in H₂O (15 mL) followed by addition aq. **MOPS/NaOH buffer** (500 mM; pH 7.0; 3.75 mL; 1.88 mmol; 25 equiv.) and aq. **YbCl₃** (100 mM; 825 µL; 83 µmol; 1.1 equiv.) and the mixture was stirred 20 h at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 53 mg (77%; 1 step; based on **TD647·**1.3H₂O). **ESI-HRMS:** 797.2347 [M]⁺ (theor. [C₃₃H₃₇N₉O₄Yb₁]⁺ = 797.2352). **EA** ([C₃₃H₃₇N₉O₄Yb₁]⁺[FA]⁻·4.2H₂O, *M*_{R} = 917.4): C 44.5 (44.1); H 5.1 (5.1); N 13.7 (13.3); Yb 18.4 (18.7). 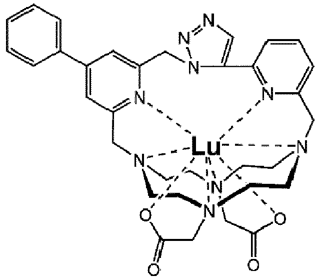 **Synthesis of TD522:** In a glass vial (20 mL), **TD647**·1.3TFA (30.0 mg; 38.8 µmol; 1.0 equiv.) was dissolved in H₂O (17 mL) followed by addition aq. **MOPS/NaOH buffer** (500 mM; pH 7.0; 2.30 mL; 1.15 mmol; 30 equiv.) and aq. **LuCl₃** (100 mM; 500 µL; 50.0 µmol; 1.3 equiv.) and the mixture was stirred 4 h at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 32.6 mg (89%; 1 step; based on **TD647**·1.3TFA). **NMR (D₂O, pD ~5): ¹H** δ_{H} 2.62-3.83 (*mc,* C*H*₂-CO, m, 16+4H); 3.99 (C*H*₂-arom., d, 1H, ²*J*_{HH} = 15); 4.16 (C*H*₂-arom., d, 1H, ²*J*_{HH} = 16); 4.32 (C*H*₂-arom., d, 1H, ²*J*_{HH} = 15); 4.84 (C*H*₂-arom., d, 1H, ²*J*_{HH}= 16); 5.66 (C*H*₂-N₃, d, 1H, ²*J*_{HH}= 15); 7.13 (C*H*₂-N₃, d, 1H, ²*J*_{HH} = 15); 7.60-7.67 *(Ph,* m, 3H); 7.81 *(arom.,* dd, 1H, ³*J*_{HH} = 8, ⁴*J*_{HH} = 1); 7.86 *(arom.,* dd, 1H, ³*J*_{HH} = 8, ⁴*J*_{HH} = 1); 7.90 (C*H*-N₃, s, 1H); 7.91-7.97 *(Ph,* m, 2H); 7.99 *(arom.,* d, 1H, ⁴*J*_{HH} = 2); 8.25 (*arom.,* t, 1H, ³*J*_{HH} = 8); 8.31 (*arom.,* d, 1H, ⁴*J*_{HH} = 2); 8.38 (FA, s, 1H). **¹³C{¹H}** δ_{C} 48.9 (*mc,* bs); 49.1 (*mc,* bs); 51.8 (*mc,* bs); 51.9 (*mc,* bs); 52.9 (C*H*₂-N₃, s); 53.8 (*mc,* s); 53.9 (*mc,* bs); 54.9 (*mc,* s); 56.0 (*mc,* s); 59.2 (*C*H₂-CO, s); 59.6 (C*H*₂-arom., s); 59.7 (*C*H₂-arom., s); 61.0 (*C*H₂-CO, s); 123.7 (*arom.,* s); 126.5 (*arom.,* s); 127.7 (*arom.,* s); 128.3 *(Ph,* s); 130.3 *(Ph,* s); 130.5 (*arom.,* s); 131.8 *(Ph,* s); 136.0 *(Ph,* s); 136.4 (*C*H-N₃, s); 138.4 (*arom.,* s); 142.6 (*arom.,* s); 146.4 (*arom.,* s); 154.7 (*arom.,* s); 156.0 (*arom.,* s); 158.7 (*arom.,* s); 158.8 (*arom.,* s); 170.0 (FA, s); 177.9 (CO, s); 179.4 (CO, s). **ESI-HRMS:** 798.2377 [M]⁺ (theor. [C₃₃H₃₇N₉O₄Lu₁]⁺ = 798.2371). **EA** ([C₃₃H₃₇N₉O₄Lu₁]⁺[FA]⁻·5.5H₂O, *M*_{R} = 942.8): C 43.3 (43.7); H 5.2 (4.6); N 13.4 (13.1); Lu 18.6 (18.0). 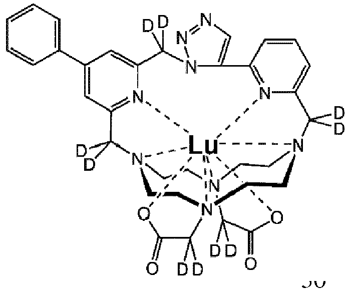 **Synthesis of TD776:** In a glass vial (4 mL), [**TD522**]⁺[FA]⁻·5.5H₂O (9.0 mg; 9.5 µmol; 1.0 equiv.) was dissolved in D₂O (500 µL) and evaporated to dryness. Residue was re-dissolved in D₂O (500 µL) followed by addition of neat **DBU** (29 µL; 194 µmol; 20 equiv.) and the resulting solution was stirred 2 d at 80 °C. Mixture was then evaporated to dryness. Residue was re-dissolved in D₂O (500 µL) followed by addition of another portion of neat **DBU** (15 µL; 100 µmol; ~10 equiv.) and the resulting solution was further stirred 16 h at 80 °C. Mixture was then quenched by addition of **FA** (75 µL; 2.92 mmol; ~300 equiv.) and directly purified by preparative HPLC (C18; H₂O-MeCN gradient with FA additive). Fractions with product were joined and directly lyophilized to give crude product in the form of formate salt as white fluffy solid (¹H NMR analysis found ~1 equiv. of DBU or its byproducts apart from otherwise pure deka-deuterated chelate). Bulk of the material was therefore repurified by preparative HPLC (C18; H₂O-MeCN gradient with TFA additive). Fractions with product were joined and directly lyophilized to give product in the form of trifluoroacetate salt as white fluffy solid. **Yield:** 7.6 mg (≤87% assuming [**M**]⁺[TFA]⁻·*x*H₂O; *M*_{R} = 921.7; 1 step; based on [**TD522**]⁺[FA]⁻·5.5H₂O). **NMR (D₂O, pD ~4): ¹H** δ_{H} 2.64-3.83 *(mc,* m, 16H); 7.59-7.67 *(Ph,* m, 3H); 7.80 *(arom.,* dd, 1H, ³*J*_{HH} = 8, ⁴*J*_{HH} = 1); 7.84 *(arom.,* dd, 1H, ³*J*_{HH} = 8, ⁴*J*_{HH} = 1); 7.89 (C*H*-N₃, s, 1H); 7.90-7.96 *(Ph,* m, 2H); 7.98 (*arom.,* d, 1H, ⁴*J*_{HH} = 2); 8.24 *(arom.,* t, 1H, ³*J*_{HH} = 8); 8.30 (*arom.,* d, 1H, ⁴*J*_{HH} = 2); 8.39 (*FA*, s, 1H). **¹³C{¹H}** δ_{C} 48.9 (*mc,* bs); 49.2 (*mc,* bs); 51.8 (*mc,* bs); 51.9 (*mc,* bs); 52.0-52.9 (*C*D₂-N₃, bm); 53.8 (*mc,* s); 53.9 (*mc,* bs); 54.8 (*mc,* s); 55.9 (*mc,* s); 58.2-59.7 (*C*D₂-CO, 2 × *C*D₂-arom., bm); 59.7-60.9 (*C*D₂-CO, s); 123.8 (*arom.,* s); 126.5 (*arom.,* s); 127.8 *(arom.,* s); 128.3 *(Ph,* s); 130.3 *(Ph,* s); 130.5 (*arom.,* s); 131.8 *(Ph,* s); 136.1 *(Ph,* s); 136.4 (*C*H-N₃, s); 138.5 (*arom.,* s); 142.6 (*arom.,* s); 146.4 (*arom.,* s); 154.8 (*arom.,* s); 156.0 (*arom.,* s); 158.7 (*arom.,* s); 158.8 (*arom.,* s); 170.5 (*FA*, s); 178.0 (CO, s); 179.4 (CO, s). **ESI-HRMS:** 808.3004 [M]⁺ (theor. [C₃₃H₂₇D₁₀N₉O₄Lu₁]⁺ = 808.2998). 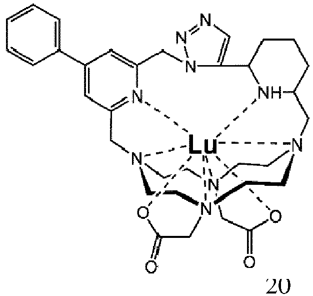 **Synthesis of TD688:** In a glass vial (2 mL), [**TD522**]⁺[FA]⁻·5.5H₂O (1 mg; ~1 µmol; 1.0 equiv.) was dissolved in MeOH (1 mL) followed by addition of solid **NaBH₄** (4 mg; ~100 mmol; ~100 equiv.). The resulting open vial was stirred 1 h at RT after which further portions of **NaBH₄** were added until all starting material was consumed (analyzed by LC-MS). The mixture was then diluted with H₂O (2 mL), neutralized with neat FA (200 µL) and filtered through syringe microfilter (RC). Filtrate was directly purified by preparative HPLC (C18; H₂O-MeCN gradient with FA additive). Fractions with product were joined and directly lyophilized to give product in the form of formate salt as white fluffy solid. **Yield:** <1 mg. **NMR (D₂O, pD ~6): ¹H** δ_{H} 1.42-2.23 (*Pip.,* m, 6H); 2.52-3.70 (*mc,* C*H*₂-CO, C*H*₂-Pip., m, 16+3+2H); 3.76 (*Pip.,* m, 1H); 4.16 (C*H*₂-CO, d, ²*J*_{HH} = 17); 4.22 (C*H*₂-arom., d, 1H, ²*J*_{HH} = 16); 4.32 (C*H*₂-arom., d, 1H, ²*J*_{HH} = 16); 4.78 (*Pip.,* m, 1H); 5.66 (C*H*₂-N₃, d, 1H, ²*J*_{HH} = 16); 6.88 (C*H*₂-N₃, d, 1H, ²*J*_{HH} = 16); 7.68-7.74 *(Ph,* m, 3H); 7.91 (C*H*-N₃, s, 1H); 7.98 *(arom.,* d, 1H, ⁴*J*_{HH} = 2); 7.98-8.02 *(Ph,* m, 2H); 8.25 (*arom.,* d, 1H, ⁴*J*_{HH} = 2); 8.52 (FA, s). **¹³C{¹H}** δ_{C} 22.8 (*Pip*., s); 27.0 (*Pip*., s); 29.5 (*Pip*., s); 50.1 (*Pip*., s); 51.4 (*C*H₂-N₃, s); 52.1 (*mc,* s); 53.4 (*mc,* s); 54.4 (*mc,* s); 54.5 (*Pip*., s); 55.4 (*mc,* s); 55.6 (*mc,* s); 56.0 (*mc,* s); 56.1 (*mc,* s); 56.8 (*mc,* s); 56.0 (*C*H₂-Pip., s); 64.7 (*C*H₂-CO, s); 64.8 (C*H*₂-arom., s); 65.4 (*C*H₂7-Pip., s); 119.8 (*arom.,* s); 123.7 (*arom.,* s); 126.9 *(Ph,* s); 129.0 *(Ph,* s); 130.5 *(Ph,* s); 131.6 (C*H*-N₃, s); 135.7 *(Ph,* s); 138.9 (*arom.,* s); 153.1 (*arom.,* s); 155.9 (*arom.,* s); 159.0 (*arom.,* s); 170.5 (FA, s); 178.8 (CO, s); 179.5 (CO, s). **ESI-HRMS:** 804.2841 [M]⁺ (theor. [C₃₃H₄₃N₉O₄Lu₁]⁺ = 804.2840). 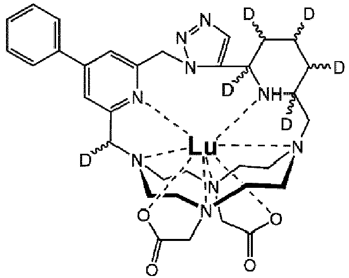 **Synthesis of TD739:** In a glass vial (2 mL), **[TD522**]⁺[FA]⁻·5.5H₂O (10 mg; 11 µmol; 1.0 equiv.) was dissolved in CD₃OD (1 mL) followed by addition of solid **NaBD₄** (44 mg; 1.05 mmol; ~100 equiv.). The resulting open vial was stirred 1 h at RT after which another portion of **NaBD₄** (28 mg; 0.67 mmol; ~60 equiv.) was added. Mixture was further stirred with 1 h at RT. The mixture was then diluted with H₂O (2 mL), neutralized with neat FA (160 µL; ~400 equiv.) and filtered through syringe microfilter (RC). Filtrate was directly purified by preparative HPLC (C18; H₂O-MeCN gradient with FA additive). Fractions with product (major diastereoisomer) were joined and directly lyophilized to give product in the form of formate salt as white fluffy solid. **Yield:** 6 mg (~60%; 1 step; based on [**TD522**]⁺[FA]⁻·5.5H₂O). **NMR (D₂O, pD ~5):** 1.33-1.77 (*Pip*., m, 1H); 1.93-2.09 (*Pip*., m, 2H); 2.42-3.62 (*mc,* C*H*₂-CO, CH₂-Pip. m, 16+3H+1H); 4.07 (C*H*₂-CO, d, 1H, ²*J*_{HH} = 17); 4.19 (C*H*D-arom., s, 1H); 5.56 (C*H*₂-N₃, d, 1H, ²*J*_{HH} = 16); 6.78 (C*H*₂-N₃, d, 1H, ²*J*_{HH} = 16); 7.59-7.65 *(Ph,* m, 3H); 7.82 (C*H*-N₃, s, 1H); 7.87 (*arom.,* d, 1H, ⁴*J*_{HH} = 2); 7.88-7.93 *(Ph,* m, 2H); 8.14 *(arom.,* d, 1H, ⁴*J*_{HH} = 2); 8.43 (FA, s, 1H). **¹³C{¹H}** δ_{C} 23.4 (*Pip.,* bs); 27.7 (*Pip.,* bs); 30.3 (*Pip.,* bs); 50.9 (*Pip.,* bs); 52.7 (C*H*₂-N₃, s); 53.4 (*mc,* s); 54.7 (*mc,* s); 55.3 (*Pip.,* bs); 55.7 (*mc,* s); 56.7 (*mc,* s); 56.8 (*mc,* s); 57.3 (*mc,* s); 57.4 (*mc,* s); 58.1 (*mc,* s); 61.2 (*C*H₂-CO, s); 65.8 (CHD-arom., bs); 66.0 (*C*H₂-Pip., s); 66.8 (*C*H₂-CO, s); 121.1 (*arom.,* s); 125.0 (*arom.,* s); 128.2 *(Ph,* s); 130.3 *(Ph,* s); 131.8 *(Ph,* s); 132.9 (*arom.,* s); 135.9 *(Ph,* s); 140.2 (*arom.,* s); 154.3 (*arom.,* s); 157.2 (*arom.,* s); 160.3 (*arom.,* s); 171.4 (FA, s); 180.1 (CO, s); 180.8 (CO, s). **ESI-HRMS:** 810.3215 [M]⁺ (theor. [C₃₃H₃₇D₆N₉O₄Lu₁]⁺ = 810.3217). 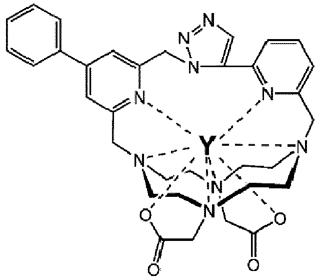 **Synthesis of TD649:** In a glass vial (20 mL), **TD647**·0.2FA·0.9H₂O (38 mg; 59 µmol; 1.0 equiv.) was dissolved in H₂O (16 mL) followed by addition aq. **MOPS/NaOH buffer** (500 mM; pH 7.0; 2.96 mL; 1.48 mmol; 25 equiv.) and aq. **YCl₃** (100 mM; 650 µL; 65 µmol; 1.1 equiv.) and the mixture was stirred 20 h at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 32 mg (65%; 1 step; based on **TD647**·0.2FA·0.9H₂O). **NMR (D₂O, pD ~5): ¹H** δ_{H} 2.58-3.86 (*mc,* C*H*₂-CO, m, 16+4H); 3.99 (C*H*₂-arom., d, 1H, ²*J*_{HH} = 15); 4.15 (C*H*₂-arom., d, 1H, ²*J*_{HH} = 16); 4.37 (C*H*₂-arom., d, 1H, ²*J*_{HH} = 15); 4.85 (C*H*₂-arom., d, 1H, ²*J*_{HH} = 16); 5.71 (C*H*₂-N₃, d, 1H, ²*J*_{HH} = 16); 7.07 (C*H*₂-N₃, d, 1H, ²*J*_{HH} = 16); 7.58-7.68 *(Ph,* m, 3H); 7.78-7.86 (*arom.,* m, 2H); 7.89 (C*H*-N₃, s, 1H); 7.91-7.97 *(Ph,* m, 2H); 7.99 (*arom.,* d, 1H, ⁴*J*_{HH} = 2); 8.26 (*arom.,* t, 1H, ³*J*_{HH} = 8); 8.31 (*arom.,* d, 1H, ⁴*J*_{HH} = 2); 8.42 (FA, s, 1H). **¹³C{¹H}** δ_{C} 49.0 (*mc,* s); 49.2 (*mc,* s); 51.4 *(mc,* s); 51.7 *(mc,* s); 53.0 (*C*H₂-N₃, s); 53.9 *(mc,* s); 54.1 *(mc,* s); 54.7 *(mc,* s); 55.7 *(mc,* s); 59.5 (*C*H₂-CO, s); 59.6 (*C*H₂-arom., s); 59.8 (*C*H₂-arom., s); 61.4 (*C*H₂-CO, s); 123.9 *(arom.,* s); 126.5 *(arom.,* s); 127.6 (*arom.,* s); 128.4 *(Ph,* s); 130.1 (*arom.,* s); 130.3 *(Ph,* s); 131.8 *(Ph,* s); 136.2 *(Ph,* s); 136.8 (*C*H-N₃, s); 138.2 (*arom.,* s); 142.8 (*arom.,* s); 146.0 (*arom.,* s); 155.0 (*arom.,* s); 155.6 (*arom.,* s); 158.6 (*arom.,* s); 158.7 (*arom.,* s); 171.2 (FA, s); 177.7 (CO, s); 179.0 (CO, s). **ESI-HRMS:** 712.2017 [M]⁺ (theor. [C₃₃H₃₇N₉O₄Y₁]⁺ = 712.2022). EA ([C₃₃H₃₇N₉O₄Y₁]⁺[FA]⁻·4.5H₂O, *M*_{R} = 838.7): C 48.7 (48.6); H 5.6 (5.2); N 15.0 (14.7); Y 10.6 (10.3). 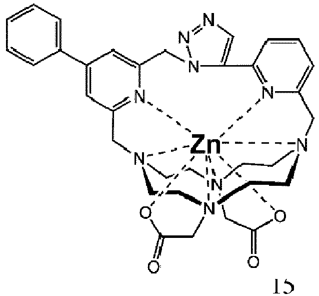 **Synthesis of TD890:** In a glass vial (4 mL), **TD650**·1.1FA·1.6H₂O (15.2 mg; 21.6 µmol; 1.0 equiv.) was dissolved in H₂O (2 mL) followed by addition aq. **MOPS/NaOH buffer** (500 mM; pH 7.0; 1.10 mL; 550 µmol; 25 equiv.) and aq. **Zn(NO₃)₂** (51.7 mM; 460 µL; 23.8 µmol; 1.1 equiv.) and the mixture was stirred 2 h at 80 °C. Mixture was then directly purified by preparative HPLC (C18; H₂O-MeCN gradient with no additive). Fractions with product were joined and directly lyophilized to give product as white fluffy solid. **Yield:** 15.5 mg (91%; 1 step; based on **TD650·**1.1FA·1.6H₂O). **NMR (D₂O, pD ~8): ¹H** δ_{H} 2.43-3.38 *(mc,* C*H*₂-CO, bm, 16+4H); 4.14 (C*H*₂-arom., s, 2H); 4.25 (C*H*₂-arom., bs, 2H); 6.48 (C*H*₂-N₃, bs, 2H); 7.60 (*arom.,* dd, 1H, ³*J*_{HH} = 8, ⁴*J*_{HH} = 1); 7.62-7.68 *(Ph,* m, 3H); 7.85 (*arom.,* dd, 1H, ³*J*_{HH} = 8, ⁴*J*_{HH} = 1); 7.86 (*arom*., d*,* 1H, ⁴*J*_{HH} = 2); 7.91-7.98 *(Ph,* m, 2H); 8.09 *(arom.,* t, 1H,³*J*_{HH} = 8); 8.21 (C*H*-N₃, s, 1H); 8.31 *(arom.,* d, 1H, ⁴*J*_{HH} = 2). **ESI-HRMS:** 688.2339 [M+H]⁺ (theor. [C₃₃H₃₈N₉O₄Zn₁]⁺ = 688.2333). EA ([C₃₃H₃₇N₉O₄Zn₁]·5.5H₂O, *M*_{R} = 788.2): C 50.3 (50.2); H 6.1 (5.8); N 16.0 (15.7); Zn 8.3 (8.5). 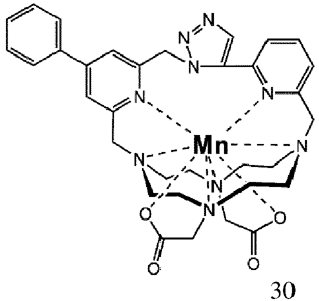 **Synthesis of TD1195:** In a glass vial (4 mL), **TD650**·1.1FA·1.6H₂O (9.0 mg; 12.8 µmol; 1.0 equiv.) was dissolved in H₂O (780 µL) followed by addition aq. **MOPS/NaOH buffer** (500 mM; pH 7.0; 650 µL; 325 µmol; 25 equiv.) and aq. **MnCl₂** (25.0 mM; 570 µL; 14.3 µmol; 1.1 equiv.) and the mixture was stirred 1 h at 80 °C. Mixture was then directly purified by preparative HPLC (C18; H₂O-MeCN gradient with FA additive). Fractions with product were joined and directly lyophilized to give product as white fluffy solid. **Yield:** 7.5 mg (74%; 1 step; based on **TD650·**1.1FA·1.6H₂O). **ESI-HRMS:** 679.2423 [M+H]⁺ (theor. [C₃₃H₃₈N₉O₄Mn₁]⁺ = 679.2422). **EA** ([C₃₃H₃₇N₉O₄Mn₁]·6.4H₂O, *M*_{R} = 794.0): C 49.9 (49.5); H 6.3 (5.9); N 15.9 (15.7); Mn 6.9 (6.2). 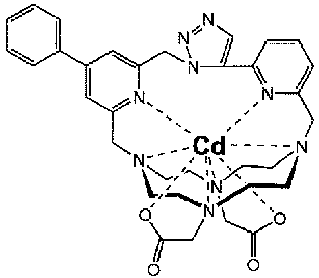 **Synthesis of TD880:** In a glass vial (4 mL), **TD650**·1.1FA·1.6H₂O (35.0 mg; 49.8 µmol; 1.0 equiv.) was dissolved in aq. **MOPS/NaOH buffer** (500 mM; pH 7.0; 2.50 mL; 1.25 mmol; 25 equiv.) and aq. **CdCl₂** (62.6 mM; 875 µL; 54.8 µmol; 1.1 equiv.) and the mixture was stirred 1 h at 80 °C. Mixture was then directly purified by preparative HPLC (C18; H₂O-MeCN gradient with no additive). Fractions with product were joined and directly lyophilized to give product as white fluffy solid. **Yield:** 37.1 mg (90%; 1 step; based on **TD650·**1.1FA·1.6H₂O). **NMR (D₂O, pD ~7): ¹H** δ_{H} 2.24-3.54 *(mc,* C*H*₂-CO, m, 16+4H); 3.60-3.79 (C*H*₂-arom., m, 2H); 4.03 (C*H*₂-arom., d, 1H, ²*J*_{HH} = 13); 4.75 (C*H*₂-arom., d, 1H, ²*J*_{HH} = 14); 5.51 (C*H*₂-N₃, d, 1H, ²*J*_{HH} = 15); 6.95 (C*H*₂-N₃, d, 1H, ²*J*_{HH} = 15); 7.58-7.67 *(Ph,* m, 3H); 7.71 *(arom.,* dd, 1H, ³*J*_{HH} = 8, ⁴*J*_{HH} = 1); 7.80 *(arom.,* dd, 1H, ³*J*_{HH} = 8, ⁴*J*_{HH} = 1); 7.89 *(arom.,* d, 1H, ⁴*J*_{HH} = 2); 7.93-7.98 *(Ph,* m, 2H); 8.04 (C*H*-N₃, s, 1H); 8.15 *(arom.,* t, 1H, ³*J*_{HH} = 8); 8.21 *(arom.,* d, 1H, ⁴*J*_{HH} = 2); **¹³C{¹H}** δ_{C} 47.8 *(mc,* s); 48.3 *(mc,* s); 49.3 *(mc,* s); 50.3 *(mc,* s); 50.9 *(mc,* s); 51.1 *(mc,* s); 51.2 *(mc,* s); 51.5 *(mc,* s); 53.4 (*C*H₂-N₃, s); 56.6 (*C*H₂-CO, s); 57.4 (*C*H₂-arom., s); 57.6 (*C*H₂-CO, s); 58.6 (*C*H₂-arom., s); 124.3 (*arom.,* s); 125.2 (*arom.,* s); 128.2 *(Ph,* s); 128.3 (*arom.,* s); 128.9 (*arom.,* s); 130.2 *(Ph,* s); 131.3 *(Ph,* s); 135.3 (*C*H-N₃, s); 136.6 *(Ph,* s); 138.8 *(arom.,* s); 141.3 *(arom.,* s); 147.4 *(arom.,* s); 153.6 *(arom.,* s); 156.9 (*arom.,* s); 157.9 (*arom.,* s); 158.3 (*arom.,* s); 175.7 (CO, s); 177.4 (CO, s). **ESI-HRMS:** 738.2079 [M+H]⁺ (theor. [C₃₃H₃₈N₉O₄Cd₁]+ = 738.2075). **EA** ([C₃₃H₃₇N₉O₄Cd₁]·5.1H₂O, *M*_{R} = 828.0): C 47.9 (47.6); H 5.7 (5.0); N 15.2 (14.6); Cd 13.6 (13.5). 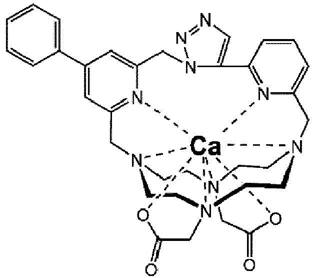 **Synthesis of TD863:** In a glass vial (20 mL), **TD650**·1.1FA·1.6H₂O (8.2 mg; 11.7 µmol; 1.0 equiv.) was dissolved in H₂O (2 mL) followed by addition aq. **MOPS/NaOH buffer** (500 mM; pH 7.0; 1.35 mL; 675 µmol; 58 equiv.) and aq. **Ca(NO₃)₂** (100 mM; 200 µL; 20 µmol; 1.7 equiv.) and the mixture was stirred 24 h at 80 °C. Mixture was then directly purified by preparative HPLC (C18; H₂O-MeCN gradient with no additive). Fractions with product were joined and directly lyophilized to give product as white fluffy solid. **Yield:** 7.4 mg (81%; 1 step; based on **TD650**·1.1FA·1.6H₂O). **NMR (D₂O, pD ~6): ¹H** δ_{H} 2.20-3.58 *(mc,* C*H*₂-CO, m, 16+4H); 3.62 (C*H*₂-arom., d, 1H, ²*J*_{HH} = 14); 3.76 (C*H*₂-arom., d, 1H, ²*J*_{HH} = 15); 4.09 (C*H*₂-arom., d, 1H, ²*J*_{HH} = 14); 4.54 (C*H*₂-arom., d, 1H, ²*J*_{HH} = 15); 5.56 (C*H*₂-N₃, d, 1H, ²*J*_{HH} = 16); 7.02 (C*H*₂-N₃, d, 1H, ²*J*_{HH} = 16); 7.57-7.65 *(Ph,* m, 3H); 7.66 *(arom.,* dd, 1H, ³*J*_{HH} = 8, ⁴*J*_{HH} = 1); 7.72 *(arom.,* dd, 1H, ³*J*_{HH} = 8, ⁴*J*_{HH} = 1); 7.83 *(arom.,* d, 1H, ⁴*J*_{HH} = 2); 7.89 (C*H*-N₃, s, 1H); 7.90-7.95 *(Ph,* m, 2H); 8.09 *(arom.,* t, 1H, ³*J*_{HH} = 8); 8.12 *(arom.,* d, 1H, ⁴*J*_{HH} = 2); **¹³C{¹H}** δ_{C} 48.4 *(mc,* s); 48.5 *(mc,* s); 50.6 *(mc,* s); 50.7 *(mc,* s); 52.6 *(mc,* s); 52.7 *(mc,* s); 53.0 *(mc,* s); 53.3 (*C*H₂-N₃, s); 53.6 *(mc,* s); 57.9 (*C*H₂-CO, s); 59.1 (*C*H₂-arom., s); 59.2 (*C*H₂-arom., s); 59.3 (*C*H₂-CO, s); 123.3 (*arom.,* s); 124.7 (*arom.,* s); 127.0 (*arom.,* s); 128.1 *(Ph,* s); 128.2 (*arom.,* s); 130.1 *(Ph,* s); 130.9 *(Ph,* s); 136.7 (*C*H-N₃, s); 137.3 *(Ph,* s); 139.4 (*arom.,* s); 140.9 (*arom.,* s); 146.7 (*arom.,* s); 153.1 (*arom.,* s); 155.5 (*arom.,* s); 159.3 (*arom.,* s); 159.5 (*arom.,* s); 178.4 (CO, s); 179.7 (CO, s). **ESI-HRMS:** 664.2663 [M+H]⁺ (theor. [C₃₃H₃₈N₉O₄Ca₁]⁺ = 664.2667). **EA** ([C₃₃H₃₇N₉O₄Ca₁]·6.7H₂O, *M*_{R} = 784.5): C 50.5 (50.5); H 6.5 (6.0); N 16.1 (15.6); Ca 5.1 (4.8). 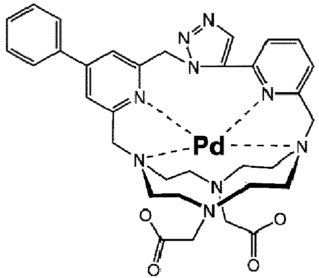 **Synthesis** of **TD1214:** In a glass vial (20 mL), **TD650**·1.1FA·1.6H₂O (9.0 mg; 12.8 µmol; 1.0 equiv.) was dissolved in H₂O (780 µL) followed by addition aq. **MOPS/NaOH buffer** (500 mM; pH 7.0; 650 µL; 325 µmol; 25 equiv.) and aq. **Na₂**[**PdCl₄]** (25 mM; 570 µL; 14.3 µmol; 1.1 equiv.) and the mixture was stirred 4 d at 80 °C. Mixture was then filtered through syringe microfilter (RC) and directly purified by preparative HPLC (C18; H₂O-MeCN gradient with FA additive). Fractions with product were joined and directly lyophilized to give product as white fluffy solid. **Yield:** 6.4 mg (58%; 1 step; based on **TD650**·1.1FA·1.6H₂O). **NMR (D₂O, pD ~5):** 1.54-3.82 *(mc,* C*H*₂-CO, bm, 16+4H); 4.21-5.10 (C*H*₂-arom., bm, 4H overlapped with water signal); 5.69 (C*H*₂-N₃, bs, 1H); 7.42-7.54 *(Ph, arom.,* m, 3+1H); 7.60 (*arom.,* d, 1H, ³*J*_{HH} = 8); 7.65 (C*H*₂-N₃, bs, 1H); 7.69-7.76 *(Ph,* m, 2H); 8.01 *(arom.,* d, 1H, ³*J*_{HH} = 8); 8.08-8.15 (*arom.,* m, 2H); 8.47 (C*H*-N₃, s, 1H). **ESI-HRMS:** 730.2088 [M+H]⁺ (theor. [C₃₃H₃₈N₉O₄Pd₁]⁺ = 730.2076). **EA** ([C₃₃H₃₇N₉O₄Pd₁]·0.2FA·7.0H₂O, *M*_{R} = 865.2): C 46.1 (46.4); H 6.0 (5.7); N 14.6 (14.4); Pd 12.3 (9.6). 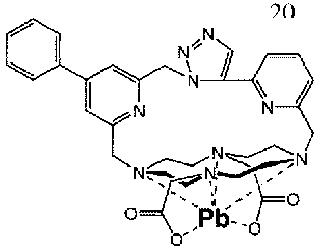 **Synthesis of TD876:** In a glass vial (20 mL), **TD650**·1.1FA·1.6H₂O (8.2 mg; 11.7 µmol; 1.0 equiv.) was dissolved in H₂O (2.5 mL) followed by addition aq. **MOPS/NaOH buffer** (500 mM; pH 7.0; 580 µL; 290 µmol; 25 equiv.) and aq. **Pb(NO₃)₂** (52.4 mM; 300 µL; 15.7 µmol; 1.3 equiv.) and the mixture was stirred 5 min at RT. Mixture was then directly purified by preparative HPLC (C18; H₂O-MeCN gradient with no additive). Fractions with product were joined and directly lyophilized to give product as white fluffy solid. **Yield:** 10.4 mg (94%; 1 step; based on **TD650**·1.1FA·1.6H₂O). **NMR (D₂O, pD ~6):** δ_{H} 2.40-3.62 *(mc,* C*H*₂-CO, bm, 16+4H); 4.20 (C*H*₂-arom., bm, 4H); 5.72 (C*H*₂-N₃, bd, 1H, ²*J*_{HH} = 15); 6.18 (C*H*₂-N₃, bd, 1H, ²*J*_{HH} = 15); 7.56-7.67 *(Ph,* m, 3H); 7.74 *(arom.,* dd, 1H, ³*J*_{HH} = 8, ⁴*J*_{HH} = 1); 7.79 *(arom.,* dd, 1H, ³*J*_{HH} = 8, ⁴*J*_{HH} = 1); 7.86-7.90 *(Ph,* m, 2H); 7.91 *(arom.,* d, 1H, ⁴*J*_{HH} = 2); 7.99 (C*H*-N₃, s, 1H); 8.08 *(arom.,* d, 1H, ⁴*J*_{HH} = 2); 8.16 *(arom.,* t, 1H, ³*J*_{HH} = 8). **ESI-HRMS:** 832.2806 [M+H]⁺ (theor. [C₃₃H₃₈N₉O₄Pb₁]⁺ = 832.2808). **EA** ([C₃₃H₃₇N₉O₄Pb₁]·6.4H₂O, *M*_{R} = 946.2): C 41.9 (42.6); H 5.3 (4.7); N 13.3 (12.6); Pb 21.9 (21.4). 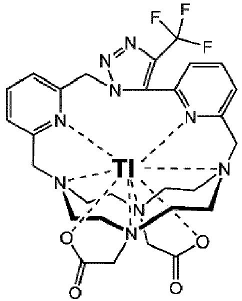 **Synthesis of TD1238: Synthesis:** In a glass vial (20 mL), **TD1188**·0.2TFA·0.4FA·4.3H₂O (6.5 mg; 8.6 µmol; 1.0 equiv.) was dissolved in H₂O (19 mL) followed by addition aq. **MOPS/NaOH buffer** (500 mM; pH 7.0; 950 µL; 475 µmol; 56 equiv.) and solid **Tl(NO₃)₃·**3H₂O (4.2 mg; 9.5 µmol; 1.1 equiv.) and the mixture was stirred 2 h at 80 °C. Then, another portion of **Tl(NO₃)₃**·3H₂O (19.0 mg; 43 µmol; 5.0 equiv.) was added and the mixture was further stirred 20 h at 80 °C. Mixture was then filtered through syringe microfilter (RC) and the filtrate was evaporated to dryness. Residue was re-dissolved in H₂O and directly purified by preparative (HPLC C18; H₂O-MeCN gradient with TFA additive). Fractions with product were joined and directly lyophilized to give product as white solid. **Yield:** 1.4 mg (≤18% assuming [**M**]⁺[TFA]⁻·*x*H₂O; *M*_{R} = 933.0; 1 step; based on **TD1188·**0.2TFA·0.4FA·4.3H₂O). **NMR (D₂O, pD ~4, two set of signals on ¹H, but one on ¹⁹F):** δ_{H} 2.17-5.34 *(mc,* C*H*₂-CO, C*H*₂-arom., m, 16+4H+4H); 5.54-5.70 (C*H*₂-N₃, m, 1H); 6.93-7.07 (C*H*₂-N₃, m, 1H); 7.69-8.44 (*arom.,* m, 6H). **¹⁹F** δ_{F} -58.70 (s). **ESI-HRMS:** 820.2265 [M]⁺ (theor. [C₂₈H₃₂N₉O₄F₃Tl₁]⁺ = 820.2264). 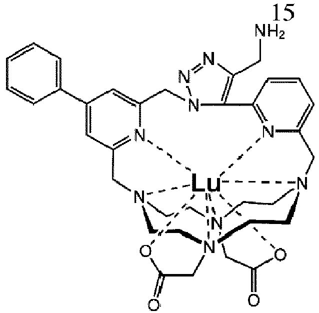 **Synthesis of TD735:** In a glass vial (20 mL), **TD722·**0.7TFA·2.0H₂O (100 mg; 130 µmol; 1.0 equiv.) was dissolved in H₂O (30 mL) followed by addition aq. **MOPS/NaOH buffer** (500 mM; pH 7.0; 7.00 mL; 3.50 mmol; 27 equiv.) and aq. **LuCl₃** (100 mM; 1.50 mL; 150 µmol; 1.2 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 134.2 mg (91%; 1 step; based on **TD722**·0.7TFA·2.0H₂O). **NMR (D₂O, pD ~4): ¹H** δ_{H} 2.65-3.84 *(mc,* C*H*₂-CO, m, 16+4H); 4.01 (C*H*₂-arom., d, 1H, ²*J*_{HH} = 15); 4.04 (C*H*₂-NH₂, d, 1H, ²*J*_{HH} = 15); 4.23 (C*H*₂-arom., d, 1H, ²*J*_{HH} = 16); 4.31 (C*H*₂-NH₂, d, 1H, ²*J*_{HH} = 15); 4.33 (C*H*₂-arom., d, 1H, ²*J*_{HH} = 15); 4.86 (C*H*₂-arom., d, 1H, ²*J*_{HH} = 16); 5.68 (C*H*₂-N₃, d, 1H, ²*J*_{HH} = 15); 7.08 (C*H*₂-N₃, d, 1H, ²*J*_{HH} = 15); 7.60-7.69 *(Ph,* m, 3H); 7.72 *(arom.,* dd, 1H, ³*J*_{HH} = 8, ⁴*J*_{HH} = 1); 7.91 *(arom.,* dd, 1H, ³*J*_{HH} = 8, ⁴*J*_{HH} = 1); 7.92-7.99 *(Ph,* m, 2H); 8.03 *(arom.,* d, 1H, ⁴*J*_{HH} = 2); 8.31 *(arom.,* t, 1H, ³*J*_{HH} = 8); 8.33 *(arom.,* d, 1H, ⁴*J*_{HH} = 2). **¹³C{¹H}** δ_{C} 34.8 (*C*H₂-C≡C, s); 48.9 *(mc,* s); 49.2 *(mc,* s); 51.8 *(mc,* s); 52.0 *(mc,* s); 53.4 (C*H*₂-N₃, s); 53.9 *(mc,* s); 54.1 *(mc,* s); 55.2 *(mc,* s); 56.1 *(mc,* s); 59.2 (*C*H₂-CO, s); 59.7 (C*H*₂-arom., s); 60.0 (C*H*₂-arom., s); 61.1 (*C*H₂-CO, s); 124.0 (*arom.,* s); 126.8 (*arom.,* s); 128.2 (*arom.,* s); 128.4 *(Ph,* s); 130.3 *(Ph,* s); 130.8 *(arom.,* s); 131.9 *(Ph,* s); 136.0 *(Ph,* s); 136.2 (*arom,* s); 140.5 *(arom.,* s); 142.8 (*arom.,* s); 144.4 (*arom.,* s); 155.0 (*arom.,* s); 156.0 (*arom.,* s); 158.9 (*arom.,* s); 160.0 (*arom.,* s); 170.7 (FA, s); 178.8 (CO, s); 179.3 (CO, s). **ESI-HRMS:** 827.2637 [M]⁺ (theor. [C₃₄H₄₀N₁₀O₄Lu₁]⁺ = 827.2636). **EA** ([C₃₄H₄₀N₁₀O₄Lu₁]⁺[TFA]⁻·1.2TFA-3.1H₂O, *M*_{R} = 1133.3): C 40.7 (40.9); H 4.2 (3.9); N 12.4 (12.1); Lu 11.1 (11.0). 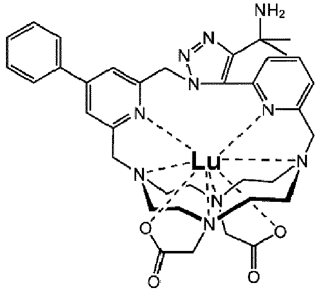 **Synthesis of TD1059:** In a glass vial (20 mL), **TD1054**·0.3TFA·0.8FA·2.3H₂O (10.0 mg; 12.9 µmol; 1.0 equiv.) was dissolved in H₂O (19 mL) followed by addition aq. **MOPS/NaOH buffer** (500 mM; pH 7.0; 800 µL; 400 µmol; 31 equiv.) and aq. **LuCl₃** (100 mM; 150 µL; 15.0 µmol; 1.2 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 10.8 mg (71%; 1 step; based on **TD1054·**0.3TFA·0.8FA·2.3H₂O). **NMR (D₂O, pD ~6): ¹H** δ_{H} 1.18 (C*H*₃, s, 3H); 1.79 (C*H*₃, s, 3H); 2.64-3.85 *(mc,* C*H*₂-CO, m, 16+4H); 4.03 (C*H*₂-arom., d, 1H, ²*J*_{HH} = 15); 4.30 (C*H*₂-arom., d, 1H, ²*J*_{HH} = 16); 4.36 (C*H*₂-arom., d, 1H, ²*J*_{HH} = 18); 4.86 (C*H*₂-arom., d, 1H, ²*J*_{HH} = 18); 5.62 (C*H*₂-N₃, d, 1H, ²*J*_{HH} = 15); 6.83 (C*H*₂-N₃, d, 1H, ²*J*_{HH} = 15); 7.60-7.68 *(Ph,* m, 3H); 7.83 *(arom.,* d, 1H, ³*J*_{HH} = 8); 7.89 *(arom.,* d, 1H, ³*J*_{HH} = 8); 7.91-7.98 *(Ph,* m, 2H); 8.03 (*arom.,* d, 1H, ⁴*J*_{HH} = 2); 8.28 (*arom.,* t, 1H, ³*J*_{HH} = 8); 8.30 *(arom.,* d, 1H, ⁴*J*_{HH} = 2). **ESI-HRMS:** 855.2951 [M]⁺ (theor. [C₃₆H₄₄N₁₀O₄Lu₁]⁺ = 855.2949). **EA** ([C₃₇H₄₅N₁₀O₄Lu₁]⁺[TFA]⁻·1.0TFA·4.6H₂O, *M*_{R} = 1178.6): C 41.8 (41.4); H 4.7 (4.2); N 11.9 (11.7); Lu 14.8 (13.2). 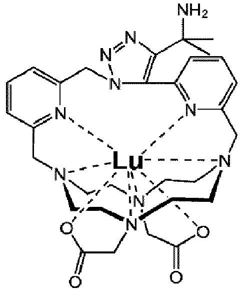 **Synthesis of TD1124:** In a glass vial (20 mL), **TD1105**·0.1FA·2.0H₂O (35.2 mg; 47.1 µmol; 1.0 equiv.) was dissolved in H₂O (17 mL) followed by addition aq. **MOPS/NaOH buffer** (500 mM; pH 7.0; 2.35 mL; 1.18 mmol; 25 equiv.) and aq. **LuCl₃** (100 mM; 520 µL; 52.0 µmol; 1.1 equiv.) and the mixture was stirred 4 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 41.2 mg (77%; 1 step; based on **TD1105**·0.1FA·2.0H₂O). **NMR (D₂O, pD ~3): ¹H** δ_{H} 1.17 (C*H*₃, s, 3H); 1.78 (C*H*₃, s, 3H); 2.64-3.84 (*mc,* C*H*₂-CO, m, 16+4H); 3.97 (C*H*₂-arom., d, 1H, ²*J*_{HH} = 15); 4.25 (C*H*₂-arom., d, 1H, ²*J*_{HH} = 15); 4.35 (C*H*₂-arom., d, 1H, ²*J*_{HH} = 17); 4.86 (C*H*₂-arom., d, 1H, ²*J*_{HH} = 17); 5.56 (C*H*₂-N₃, d, 1H, ²*J*_{HH} = 15); 6.80 (C*H*₂-N₃, d, 1H, ²*J*_{HH} = 15); 7.73 (*arom.,* d, 1H, ³*J*_{HH} = 8); 7.82 (*arom.,* d, 1H, ³*J*_{HH} = 8); 7.88 (*arom.,* d, 1H, ³*J*_{HH} = 8); 7.99 (*arom.,* d, 1H, ³*J*_{HH} = 8); 8.24 (*arom.,* t, 1H, ³*J*_{HH} = 8); 8.27 (*arom.,* t, 1H, ³*J*_{HH} = 8). **ESI-HRMS:** 779.2636 [M]⁺ (theor. [C₃₀H₄₀N₁₀O₄Lu₁]⁺ = 779.2636). **EA** ([C₃₀H₄₀N₁₀O₄Lu₁]⁺[TFA]⁻·1.5TFA·3.9H₂O, *M*_{R} = 1133.9): C 37.1 (37.3); H 4.4 (3.9); N 12.4 (12.0); F 12.6 (12.1). 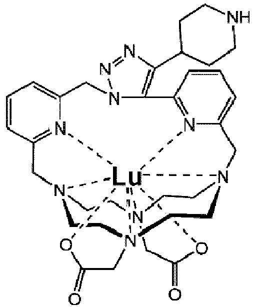 **Synthesis of TD1137:** In a glass vial (20 mL), **TD1127**·1.8TFA·0.1FA (45.9 mg; 52.2 µmol; 1.0 equiv.) was dissolved in H₂O (16 mL) followed by addition aq. **MOPS/NaOH buffer** (500 mM; pH 7.0; 3.15 mL; 1.58 mmol; 30 equiv.) and aq. **LuCl₃** (100 mM; 690 µL; 69.0 µmol; 1.3 equiv.) and the mixture was stirred 2 d at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 58.2 mg (98%; 1 step; based on **TD1127·**1.8TFA·0.1FA). **NMR (D₂O, pD ~4): ¹H** δ_{H} 1.92-2.13 (C*H*₂, m, 3H); 2.17-2.33 (C*H*₂, m, 1H); 2.63-3.83 (*mc*, CH₂-CO, C*H*₂, *CH,* m, 16+4+4+1H); 3.98 (C*H*₂-arom., d, 1H, ²*J*_{HH} = 15); 4.22 (C*H*₂-arom., d, 1H, ²*J*_{HH} = 15); 4.34 (C*H*₂-arom., d, 1H, ²*J*_{HH} = 17); 4.87 (C*H*₂-arom., d, 1H, ²*J*_{HH} = 17); 5.50 (C*H*₂-N₃, d, 1H, ²*J*_{HH} = 15); 6.93 (C*H*₂-N₃, d, 1H, ²*J*_{HH} = 15); 7.67-7.76 (*arom.,* m, 2H); 7.85 *(arom.,* dd, 1H, ³*J*_{HH} = 8, ⁴*J*_{HH} = 1); 7.98 *(arom.,* dd, 1H, ³*J*_{HH} = 8, ⁴*J*_{HH} = 1); 8.23 *(arom.,* t, 1H, ³*J*_{HH} = 8); 8.27 (*arom.*, t, 1H, ³*J*_{HH} = 8). **ESI-HRMS:** 805.2786 [M]⁺ (theor. [C₃₂H₄₂N₁₀O₄Lu₁]⁺ = 805.2793). **EA** ([C₃₂H₄₂N₁₀O₄Lu₁]⁺[TFA]⁻·1.0TFA·5.5H₂O, *M*_{R} = 1131.8): C 38.2 (38.7); H 4.8 (4.5); N 12.4 (11.9); F 10.1 (9.9). 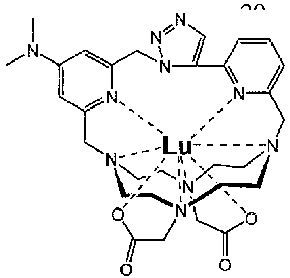 **Synthesis of TD1182:** In a glass vial (20 mL), **TD1176·**0.8FA·4.4H₂O (39.3 mg; 55.4 µmol; 1.0 equiv.) was dissolved in H₂O (16 mL) followed by addition aq. **MOPS/NaOH buffer** (500 mM; pH 7.0; 2.77 mL; 1.39 mmol; 25 equiv.) and aq. **LuCl₃** (100 mM; 610 µL; 61.0 µmol; 1.1 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 44.8 mg (77%; 1 step; based on **TD1176·**0.8FA·4.4H₂O). **NMR (D₂O, pD ~2): ¹H** δ_{H} 2.55-3.78 (*mc*, C*H*₂-CO, CH₃, C*H*₂-arom., m, 16+4+6+2H); 4.11 (C*H*₂-arom., d, 1H, ²*J*_{HH} = 16); 4.13 (C*H*₂-arom., d, 1H, ²*J*_{HH} = 14); 5.31 (C*H*₂-N₃, d, 1H, ²*J*_{HH} = 15); 6.73 *(arom.,* d, 1H, ⁴*J*_{HH} = 2); 6.83 (C*H*₂-N₃, d, 1H, ²*J*_{HH} = 15); 7.07 (*arom.*, d, 1H, ⁴*J*_{HH} = 2); 7.78 (*arom.*, dd, 1H, ³*J*_{HH} = 8, ⁴*J*_{HH} = 1); 7.82 *(arom.,* dd, 1H, ³*J*_{HH} = 8, ⁴*J*_{HH} = 1); 7.89 (C*H*-N₃, s, 1H); 8.22 (*arom.*, t, 1H, ³*J*_{HH} = 8). **ESI-HRMS:** 765.2487 [M]⁺ (theor. [C₂₉H₃₉N₁₀O₄Lu₁]⁺ = 765.2486). **EA** ([C₂₉H₃₈N₁₀O₄Lu₁]⁺[TFA]⁻·1.0TFA·3.4H₂O, *M*_{R} = 1053.9): C 37.6 (37.5); H 4.4 (4.1); N 13.3 (13.1); F 10.8 (10.5). 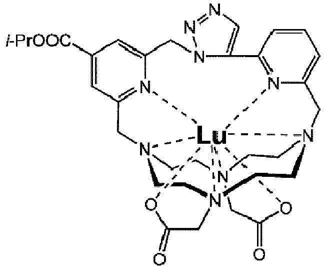 **Synthesis of TD1152:** In a glass vial (20 mL), **TD1148·**0.1FA·1.0H₂O (27.0 mg; 41.0 µmol; 1.0 equiv.) was dissolved in H₂O (17 mL) followed by addition aq. **MOPS/NaOH buffer** (500 mM; pH 7.0; 2.05 mL; 1.03 mmol; 25 equiv.) and aq. **LuCl₃** (100 mM; 450 µL; 45.0 µmol; 1.1 equiv.) and the mixture was stirred 3 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 24.5 mg (60%; 1 step; based on **TD1148**·0.1FA·1.0H₂O). **NMR (D₂O, pD ~3): ¹H** δ_{H} 1.43 (C*H*₃, d, 6H, ³*J*_{HH} = 6); 2.65-3.85 (*mc,* C*H*₂-CO, m, 16+4H); 4.06 (C*H*₂-arom., d, 1H, ²*J*_{HH} = 15); 4.16 (C*H*₂-arom., d, 1H, ²*J*_{HH} = 16); 4.34 (C*H*₂-arom., d, 1H, ²*J*_{HH} = 15); 4.86 (C*H*₂-arom., d, 1H, ²*J*_{HH} = 16); 5.34 (CH, hept, 1H, ³*J*_{HH} = 6); 5.69 (C*H*₂-N₃, d, 1H, ²*J*_{HH} = 15); 7.17 (C*H*₂-N₃, d, 1H, ²*J*_{HH} = 15); 7.81 (*arom.*, dd, 1H, ³*J*_{HH} = 8, ⁴*J*_{HH} = 1); 7.86 (*arom.*, dd, 1H, ³*J*_{HH} = 8, ⁴*J*_{HH} = 1); 7.88 (C*H-*N₃, s, 1H); 8.22 *(arom.,* d, 1H, ⁴*J*_{HH} = 2); 8.25 (*arom.*, t, 1H, ³*J*_{HH} = 8); 8.53 *(arom.,* d, 1H, ⁴*J*_{HH} = 2). **ESI-HRMS:** 808.2425 [M]⁺ (theor. [C₃₁H₃₉N₉O₆Lu₁]⁺ = 808.2426). **EA** ([C₃₁H₃₉N₉O₆Lu₁]⁺[TFA]⁻·0.3TFA·2.5H₂O, *M*_{R} = 1000.9): C 40.3 (40.7); H 4.5 (4.2); N 12.6 (12.2); F 7.4 (7.6). 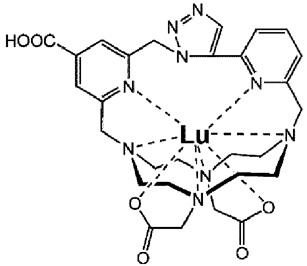 **Synthesis of TD1123**: In a glass vial (20 mL), **TD1092·**0.1FA·1.1H₂O (25.9 mg; 41.0 µmol; 1.0 equiv.) was dissolved in H₂O (17 mL) followed by addition aq. **MOPS/NaOH buffer** (500 mM; pH 7.0; 2.05 mL; 1.03 mmol; 25 equiv.) and aq. **LuCl₃** (100 mM; 450 µL; 45.0 µmol; 1.1 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 24.7 mg (60%; 1 step; based on **TD1092**·0.1FA·1.1H₂O). **NMR (D₂O, pD ~2): ¹H** δ_{H} 2.65-3.86 (*mc*, C*H*₂-CO, m, 16+4H); 4.09 (C*H*₂-arom., d, 1H, ²*J*_{HH} = 15); 4.18 (C*H*₂-arom., d, 1H, ²*J*_{HH} = 16); 4.36 (C*H*₂-arom., d, 1H, ²*J*_{HH} = 15); 4.87 (C*H*₂-arom., d, 1H, ²*J*_{HH} = 16); 5.73 (C*H*₂-N₃, d, 1H, ²*J*_{HH} = 15); 7.19 (C*H*₂-N₃, d, 1H, ²*J*_{HH} = 15); 7.83 (*arom.*, dd, 1H, ³*J*_{HH} = 8, ⁴*J*_{HH} = 1); 7.88 (*arom.*, dd, 1H, ³*J*_{HH} = 8, ⁴*J*_{HH} = 1); 7.90 (C*H*-N₃, s, 1H); 8.21 *(arom.,* d, 1H, ⁴*J*_{HH} = 2); 8.27 *(arom.,* t, 1H, ³*J*_{HH} = 8); 8.50 (*arom.*, d, 1H, ⁴*J*_{HH} = 2). **ESI-HRMS:** 766.1948 [M]⁺ (theor. [C₂₈H₃₃N₉O₆Lu₁]⁺ = 766.1956). **EA** ([C₂₈H₃₃N₉O₆Lu₁]⁺[TFA]⁻·0.6TFA·3.6H₂O, *M*_{R} = 1012.8): C 37.0 (36.7); H 4.1 (3.6); N 12.4 (12.0); F 9.0 (8.6). 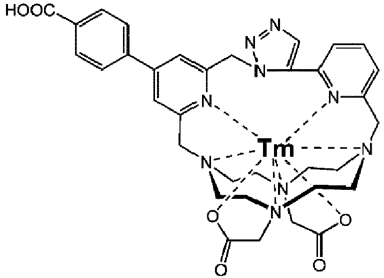 **Synthesis of TD897:** In a glass vial (20 mL), **TD742·**0.1FA.3.2H₂O (48.6 mg; 66.4 µmol; 1.0 equiv.) was dissolved in H₂O (15 mL) followed by addition aq. **MOPS/NaOH buffer** (500 mM; pH 7.0; 3.4 mL; 1.70 mmol; 26 equiv.) and aq. **TmCl₃** (100 mM; 800 µL; 80.0 µmol; 1.2 equiv.) and the mixture was stirred 8 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 65.2 mg (88%; 1step; based on **TD742·**0.1FA.3.2H₂O). **ESI-HRMS:** 836.2206 [M]⁺ (theor. [C₃₄H₃₇N₉O₆Tm₁]⁺ = 836.2203). **EA** ([C₃₄H₄₀N₁₀O₄Tm₁]⁺[TFA]⁻·1.1TFA·2.6H₂O, *M*_{R} = 1121.9): C 40.9 (40.9); H 3.9 (3.8); N 11.2 (11.2); Tm 15.1 (15.2); F 10.7 (10.7).

**Synthesis of TD748:** In a glass vial (20 mL), **TD742**·0.2FA·1.6H₂O (85 mg; 120 µmol; 1.0 equiv.) was 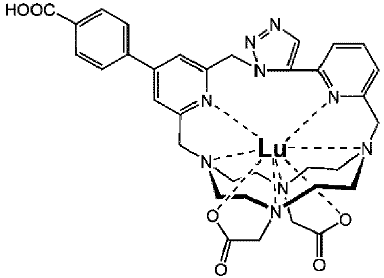 dissolved in H₂O (12 mL) followed by addition aq. **MOPS/NaOH buffer** (500 mM; pH 7.0; 6.00 mL; 3.00 mmol; 25 equiv.) and aq. **LuCl₃** (100 mM; 1.3 mL; 130 µmol; 1.1 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 105 mg (75%; 1step; based on **TD742·**0.1FA·2.9H₂O). **NMR (D₂O, pD ~2): ¹H** δ_{H} 2.64-3.86 *(mc,* C*H*₂-CO, m, 16+4H); 3.98 (C*H*₂-arom., d, 1H, ²*J*_{HH} = 15); 4.18 (C*H*₂-arom., d, 1H, ²*J*_{HH} = 16); 4.34 (C*H*₂-arom., d, 1H, ²*J*_{HH} = 15); 4.87 (C*H*₂-arom., d, 1H, ²*J*_{HH} = 16); 5.71 (C*H*₂-N₃, d, 1H, ²*J*_{HH} = 15); 7.17 (C*H*₂-N₃, d, 1H, ²*J*_{HH} = 15); 7.83 (*arom.*, dd, 1H, ³*J*_{HH} = 8, ⁴*J*_{HH} = 1); 7.88 (*arom.*, dd, 1H, ³*J*_{HH} = 8, ⁴*J*_{HH} = 1); 7.92 (*arom.*, d, 1H, ⁴*J*_{HH} = 2); 7.94 (C*H*-N₃, s, 1H); 7.97 (*arom.*, dm, 2H, ³*J*_{HH} = 9); 8.15 *(arom.,* dm, 2H, ³*J*_{HH} = 9); 8.27 *(arom.,* t, 1H, ³*J*_{HH} = 8); 8.43 (*arom.*, d, 1H, ⁴*J*_{HH} = 2). **ESI-HRMS:** 842.2259 [M]⁺ (theor. [C₃₄H₃₇N₉O₆Lu₁]⁺ = **842.2269).EA** ([C₃₄H₄₀N₁₀O₄Lu₁]⁺[TFA]⁻·1.4TFA·3.2H₂O, *M*_{R} = 1172.9): C 39.7 (40.0); H 3.9 (3.5); N 10.7 (10.3); Lu 14.9 (14.9); F 11.7 (11.8).

**Synthesis of TD785:** In a glass vial (20 mL), **TD742·**0.1FA·2.9H₂O (13 mg; 18 µmol; 1.0 equiv.) was 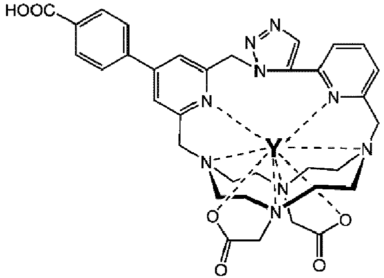 dissolved in H₂O (18 mL) followed by addition aq. **MOPS/NaOH buffer** (500 mM; pH 7.0; 1.20 mL; 600 µmol; 31 equiv.) and aq. **YCl₃** (100 mM; 197 µL; 20 µmol; 1.1 equiv.) and the mixture was stirred 22 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 13 mg (71%; 1step; based on **TD742**·0.1FA·2.9H₂O). **NMR (D₂O, pD ~2): ¹H** δ_{H} 2.60-3.88 *(mc,* C*H*₂-CO, m, 16+4H); 3.98 (C*H*₂-arom., d, 1H, ²*J*_{HH} = 15); 4.16 (C*H*₂-arom., d, 1H, ²*J*_{HH} = 16); 4.38 (C*H*₂-arom., d, 1H, ²*J*_{HH} = 15); 4.86 (C*H*₂-arom., d, 1H, ²*J*_{HH} = 16); 5.94 (C*H*₂-N₃, d, 1H, ²*J*_{HH} = 16); 7.09 (C*H*₂-N₃, d, 1H, ²*J*_{HH} = 16); 7.82 (*arom.,* dd, 1H, ³*J*_{HH} = 8, ⁴*J*_{HH} = 1); 7.85 (*arom.,* dd, 1H, ³*J*_{HH} = 8, ⁴*J*_{HH} = 1); 7.91 (C*H*-N₃, s, 1H); 7.98 (*arom.*, d, 1H, ⁴*J*_{HH} = 2); 8.00 (*arom.*, dm, 2H, ³*J*_{HH} = 9); 8.02 (*arom.*, dm, 2H, ³*J*_{HH} = 9); 8.26 (*arom.*, t, 1H, ³*J*_{HH} = 8); 8.40 (*arom.*, d, 1H, ⁴*J*_{HH} = 2). **ESI-HRMS:** 756.1925 [M]⁺ (theor. [C₃₄H₃₇N₉O₆Y₁]⁺ = 756.1920). **EA** ([C₃₄H₃₇N₉O₆Y₁]⁺[TFA]⁻·0.6TFA·4.8H₂O, *M*_{R} = 1024.5): C 43.6 (43.9); H 4.6 (4.2); N 12.3 (12.0); Y 8.7 (7.9); F 8.9 (8.5). 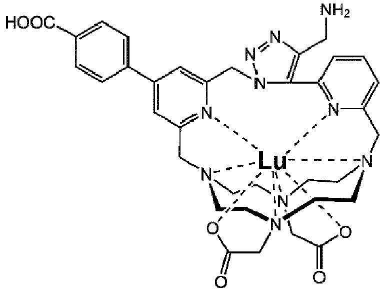 **Synthesis of TD904:** In a glass vial (20 mL), **TD744**·1.0TFA·1.3H₂O (38.6 mg; 46.2 µmol; 1.0 equiv.) was dissolved in H₂O (16 mL) followed by addition aq. **MOPS/NaOH buffer** (500 mM; pH 7.0; 2.80 mL; 1.40 mmol; 30 equiv.) and aq. **LuCl₃** (100 mM; 550 µL; 55.0 µmol; 1.2 equiv.) and the mixture was stirred 16 h at 80 °C. Mixture was then filtered through syringe microfilter (RC). The filtrate was concentrated on rotary evaporator and then directly purified by preparative HPLC (C18; H₂O-MeCN gradient with TFA additive). Fractions with product were joined and directly lyophilized to give product in the form of trifluoroacetate salt as white fluffy solid. **Yield:** 42.2 mg (75%; 1 step; based on **TD744·**1.0TFA·1.3H₂O). **NMR (D₂O, pD ~3): ¹H** δ_{H} 2.62-3.87 *(mc,* C*H*₂-CO, m, 16+4H); 4.03 (C*H*₂-NH₂, d, 1H, ²*J*_{HH} = 15); 4.05 (C*H*₂-arom., d, 1H, ²*J*_{HH} = 15); 4.24 (C*H*₂-arom., d, 1H, ²*J*_{HH} = 16); 4.32 (C*H*₂-NH₂, d, 1H, ²*J*_{HH} = 15); 4.35 (C*H*₂-arom., d, 1H, ²*J*_{HH} = 15); 4.87 (C*H*₂-arom., d, 1H, ²*J*_{HH} = 16); 5.71 (C*H*₂-N₃, d, 1H, ²*J*_{HH} = 15); 7.10 (C*H*₂-N₃, d, 1H, ²*J*_{HH} = 15); 7.73 (*arom.,* dd, 1H, ³*J*_{HH} = 8, ⁴*J*_{HH} = 1); 7.92 *(arom.,* dd, 1H, ³*J*_{HH} = 8, ⁴*J*_{HH} = 1); 8.03 (*arom.,* dm, 2H, ³*J*_{HH} = 9); 8.07 *(arom.,* d, 1H, ⁴*J*_{HH} = 2); 8.21 *(arom.,* dm, 2H, ³*J*_{HH} = 9); 8.31 (*arom.,* t, 1H, ³*J*_{HH} = 8); 8.40 (*arom.*, d, 1H, ⁴*J*_{HH} = 2). **ESI-HRMS:** 871.2536 [M]⁺ (theor. [C₃₅H₄₀N₁₀O₆Lu₁]⁺ = 471.2535). **EA** ([C₃₅H₄₀N₁₀O₆Lu₁]⁺[TFA]⁻·1.1TFA·6.0H₂O, *M*_{R} = 1218.2): C 38.6 (38.6); H 4.4 (4.1); N 11.5 (11.2); F 9.8 (9.5). 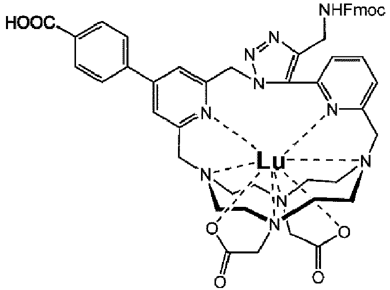 **Synthesis of 905:** In a glass vial (2 mL), [**TD904**]⁺[TFA]⁻·1.1TFA·6.0H₂O (10.0 mg; 8.2 µmol; 1.0 equiv.) was dissolved in aq. **Borate/NaOH buffer** (200 mM; pH 9.0; 2.00 mL; 400 mmol; 50 equiv.). followed by addition of freshly prepared solution of **FmocCl** (3.3 mg; 12.8 µmol; 1.6 equiv.) in MeCN (1.5 mL). The resulting clear solution was stirred 30 min at RT. Mixture was then directly purified by preparative HPLC (C18; H₂O-MeCN gradient with TFA additive). Fractions with product were joined and directly lyophilized to give product in the form of trifluoroacetate salt as white fluffy solid. **Yield:** 9.8 mg (≤99% assuming [**M**]⁺[TFA]⁻·*x*H₂O; *M*_{R} = 1207.0; 1step; based on [**TD904**]⁺[TFA]⁻1.1TFA·6.0H₂O). **NMR** (**CD₃CN; compound is soluble in D₂O but shows broad ¹H signals): ¹H** δ_{H} 2.52-3.64 *(mc,* C*H*₂-CO, m, 16+4H); 3.89 (C*H*₂-arom., d, 1H, ²*J*_{HH} = 15); 4.03 (C*H*₂-NH-Fmoc, dd, 1H, ²*J*_{HH} = 15, ³*J*_{HH} = 3); 4.05 (C*H*₂-arom., d, 1H, ²*J*_{HH} = 16); 4.18 *(Fmoc,* t, 1H, ³*J*_{HH} = 7); 4.26-4.35 *(Fmoc,* m, 2H); 4.36 (C*H*₂-arom., d, 1H, ²*J*_{HH} = 15); 4.52 (C*H*₂-NH-Fmoc, dd, 1H, ²*J*_{HH} = 15, ³*J*_{HH} = 7); 4.78 (C*H*₂-arom., d, 1H, ²*J*_{HH} = 16); 5.50 (C*H*₂-N₃, d, 1H, ²*J*_{HH} = 15); 6.54 (N*H*-Fmoc, dd, 1H, ³*J*_{HH} = 7, ³*J*_{HH} = 3); 7.27-7.33 *(Fmoc,* m, 2H); 7.35-7.43 *(Fmoc,* C*H*₂-N₃, m, 2+1H); 7.57-7.65 (*Fmoc,* C*H*₂-arom., m, 2+1H); 7.70 (*arom.*, dd, 1H, ³*J*_{HH} = 8, ⁴*J*_{HH} = 1); 7.78-7.84 *(Fmoc,* m, 2H); 7.91 (*arom.*, d, 1H, ⁴*J*_{HH} = 2); 7.97 (*arom.*, dm, 2H, ³*J*_{HH} = 9); 8.14 (*arom.*, t, 1H, ³*J*_{HH} = 8); 8.18 *(arom.,* dm, 2H, ³*J*_{HH} = 9); 8.26 *(arom.,* d, 1H, ⁴*J*_{HH} = 2). **ESI-HRMS:** 1093.3219 [M]⁺ (theor. [C₅₀H₅₀N₁₀O₈Lu₁]⁺ = 1093.3215). 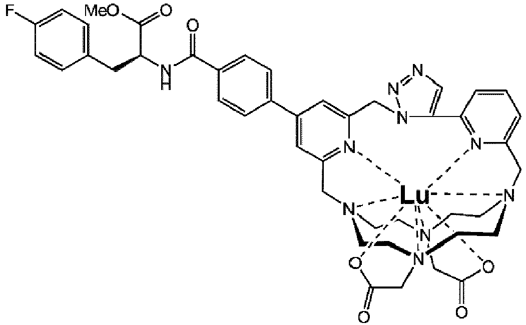 **Synthesis of TD757:** In a glass vial (2 mL), [**TD748**]⁺[FA]⁻·6.4H₂O (0.7 mg; 0.7 µmol; 1.0 equiv.) was dissolved in DMSO (75 µL) followed by addition of **4-Fluoro-L-phenylalanine hydrochloride** (50 mM in DMSO; 22 µL; 1.1 µmol; 1.5 equiv.), **DIPEA** (100 mM in DMSO; 22 µL; 2.2 µmol; 3.1 equiv.) and **HATU** (freshly prepared 50 mM in DMSO; 24 µL; 1.2 µmol; 1.7 equiv.) and the resulting yellow solution was stirred 5 min at RT. Mixture was then diluted with H₂O (750 µL) and directly purified by preparative HPLC (C18; H₂O-MeCN gradient with FA additive). Fractions with product were joined and directly lyophilized to give product as white fluffy solid. **Yield:** 0.7 mg (≤90% assuming [M]⁺[FA]⁻·*x*H₂O; *M*_{R} = 1066.9; 1 step; based on [**TD748]**⁺[FA]⁻·6.4H₂O). **NMR (D₂O, pD ~4): ¹H** δ_{H} 2.56-3.79 *(mc,* C*H*₂-CO, C*H*₂-CH, m, 16+4+2H); 3.82 (C*H*₃, s, 3H); 4.02 (C*H*₂-arom., d, 1H, ²*J*_{HH} = 15); 4.16 (C*H*₂-arom., d, 1H, ²*J*_{HH} = 16); 4.35 (C*H*₂-arom., d, 1H, ²*J*_{HH} = 15); 4.86 (C*H*₂-arom., d, 1H, ²*J*_{HH} = 16); 4.96 (CH₂-C*H*, dd, 1H, ³*J*_{HH} = 10, ³*J*_{HH} = 5); 5.68 (C*H*₂-N₃, d, 1H, ²*J*_{HH} = 15); 7.04-7.20 (C*H*₂-N₃, *arom.*, m, 1+2H); 7.27-7.34 (*arom.*, dd, 2H, ³*J*_{HF} = 9, ³*J*_{HH} = 6); 7.78-7.88 *(arom.,* m, 4H); 7.90 (C*H*-N₃, s, 1H); 7.95-8.06 *(arom.,* m, 3H); 8.26 *(arom.,* t, 1H, ³*J*_{HH} = 8); 8.35 *(arom.,* d, 1H, ⁴*J*_{HH} = 2); 8.46 (*FA*, s, 1H). **¹⁹F** δ_{F} -116.5 (tt, ³*J*_{FH} = 9, ⁴*J*_{FH} = 5). **ESI-HRMS:** 1021.3014 [M]⁺ (theor. [C₄₄H₄₇N₁₀O₇F₁Lu₁]⁺ = 1021.3015). 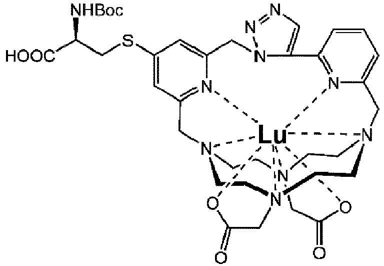 **Synthesis of TD793:** In a glass vial (2 mL), [**TD782**]⁺[TFA]⁻·5.1H₂O (4.4 mg; 4.6 µmol; 1.0 equiv.) was dissolved in DMSO (460 µL) followed by addition of ***N*-Boc-cysteine** (50 mM in DMSO; 185 µL; 9.3 µmol; 2.0 equiv.) and **DIPEA** (8 µL; 46 µmol; 10 equiv.) and the resulting solution was stirred 16 h RT. Mixture was then diluted with H₂O (2 mL) and directly purified by preparative HPLC (C18; H₂O-MeCN gradient with TFA additive). Fractions with product were joined and directly lyophilized to give product as white fluffy solid. **Yield:** 2.0 mg (≤41% assuming [**M**]⁺[TFA]⁻·*x*H₂O; *M*_{R} = 1054.8; 1 step; based on [**TD782**]⁺[HTFA]⁻·5.1H₂O). **NMR (D₂O, pD** ~3): **¹H** δ_{H} 1.28-1.54 (C*H*₃, m, 9H); 2.63-3.95 *(mc,* C*H*₂-CO, C*H*₂-S, C*H*₂-arom., m, 16+4+2+1H); 4.16 (C*H*₂-arom., d, 1H, ²*J*_{HH} = 16); 4.26 (C*H*₂-arom., d, 1H, ²*J*_{HH} = 15); 4.48-4.63 (NH-C*H*-COOH, m, 1H); 4.84 (C*H*₂-arom., d, 1H, ²*J*_{HH} = 16); 5.45-5.59 (C*H*₂-N₃, m, 1H); 7.03 (C*H*₂-N₃, d, 1H, ²*J*_{HH} = 15); 7.59-7.65 *(arom.,* m, 1H); 7.81 (*arom.*, dd, 1H, ³*J*_{HH} = 8,⁴*J*_{HH} = 1); 7.86 (*arom.,* dd, 1H, ³*J*_{HH} = 8, ⁴*J*_{HH} = 1); 7.90 (C*H*-N₃, s, 1H); 7.90-7.95 *(arom.,* m, 1H); 8.26 (*arom.,* t, 1H, ³*J*_{HH} = 8). **ESI-HRMS:** 941.2614 [M]⁺ (theor. [C₃₅H₄₆N₁₀O₈S₁Lu₁]⁺ = 941.2623). 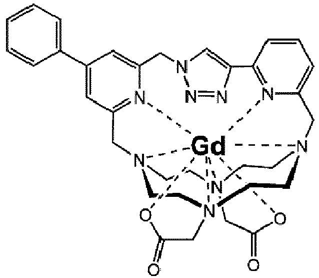 **Synthesis of TD875:** In a glass vial (20 mL), **TD871**·0.6FA·2.3H₂O (10.0 mg; 14.4 µmol; 1.0 equiv.) was dissolved in H₂O (2 mL) followed by addition of aq. **MOPS/NaOH buffer** (500 mM; pH 7.0; 750 µL; 375 µmol; 26 equiv.) and aq. **GdCl₃** (100 mM; 180 µL; 18.0 µmol; 1.3 equiv.). The resulting suspension was stirred 20 d at 80 °C. Then, the mixture was diluted with additional H₂O (16 mL) followed by another portion of aq. **MOPS/NaOH buffer** (500 mM; pH 7.0; 750 µL; 375 µmol; 26 equiv.) and aq. **GdCl₃** (100 mM; 180 µL; 18.0 µmol; 1.3 equiv.). The resulting suspension was further stirred 10 d at 80 °C. Mixture was the filtered through syringe microfilter (RC). The filtrate was evaporated to dryness and then resuspended in H₂O (3.6 mL). Mixture was once more filtered through syringe microfilter (RC) and the filtrate was purified by preparative HPLC (C18; H₂O-MeCN gradient with FA additive). Fractions with product were joined and directly lyophilized to give product as white fluffy solid. **Yield:** 2.1 mg (≤18% assuming [**M**]⁺[FA]⁻·*x*H₂O; *M*_{R} = 826.0; 1 step; based on **TD871**·0.6FA·2.3H₂O). **ESI-HRMS:** 781.2205 [M]⁺ (theor. [C₃₃H₃₇N₉O₄Gd₁]⁺ = 781.2204). 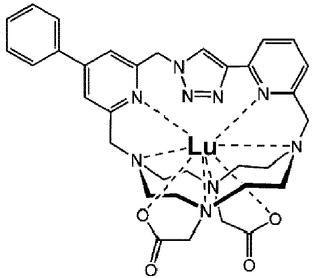 **Synthesis of TD874:** In a glass vial (20 mL), **TD871**·0.6FA·2.3H₂O (10.0 mg; 14.4 µmol; 1.0 equiv.) was dissolved in H₂O (2 mL) followed by addition of aq. **MOPS/NaOH buffer** (500 mM; pH 7.0; 750 µL; 375 µmol; 26 equiv.) and aq. **LuCl₃** (100 mM; 180 µL; 18.0 µmol; 1.3 equiv.). The resulting suspension was stirred 20 d at 80 °C. Then, the mixture was diluted with additional H₂O (16 mL) followed by another portion of aq. **MOPS/NaOH buffer** (500 mM; pH 7.0; 750 µL; 375 µmol; 26 equiv.) and aq. **LuCl₃** (100 mM; 180 µL; 18.0 µmol; 1.3 equiv.). The resulting suspension was further stirred 10 d at 80 °C. Mixture was the filtered through syringe microfilter (RC). The filtrate was evaporated to dryness and then resuspended in H₂O (3.6 mL). Mixture was once more filtered through syringe microfilter (RC) and the filtrate was purified by preparative HPLC (C18; H₂O-MeCN gradient with FA additive). Fractions with product were joined and directly lyophilized to give product as white fluffy solid. **Yield:** 8.6 mg (≤71% assuming [**M**]⁺[FA]⁻·*x*H₂O; *M*_{R} = 843.2; 1 step; based on **TD871**·0.6FA·2.3H₂O). **NMR (D₂O, pD ~6): ¹H** δ_{H} 2.13-3.76 *(mc,* C*H*₂-CO, C*H*₂-arom., m, 16+3+1H); 4.13 (C*H*₂-CO, d, 1H, ²*J*_{HH} = 18); 4.32 (C*H*₂-arom., d, 1H, ²*J*_{HH} = 16); 4.45 (C*H*₂-arom., d, 1H, ²*J*_{HH} = 16); 4.67 (C*H*₂-arom., d, 1H, ²*J*_{HH} = 13); 5.81 (CH₂- N₃, d, 1H, ²*J*_{HH} = 14); 6.08 (C*H*₂- N₃, d, 1H, ²*J*_{HH} = 14); 7.56-7.64 (*Ph, arom.*, m, 3+1H); 7.67 (*arom.*, d, 1H, ⁴*J*_{HH} = 2); 7.79-7.85 *(Ph,* m, 2H); 7.90 *(arom.,* dd, 1H, ³*J*_{HH} = 8, ⁴*J*_{HH} = 1); 8.09 (*arom.*, d, 1H, ⁴*J*_{HH} = 2); 8.16 *(arom.,* t, 1H, ³*J*_{HH} = 8); 8.45 (FA, s, 1H); 8.72 (C*H*-N₃, s, 1H). **ESI-HRMS:** 798.2376 [M]⁺ (theor. [C₃₃H₃₇N₉O₄Lu₁]⁺ = 798.2371). 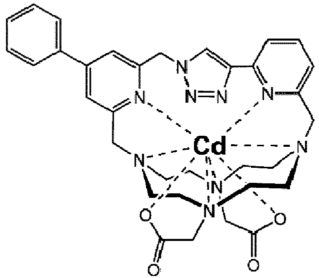 **Synthesis of TD900:** In a glass vial (4 mL), **TD871**·0.6FA·2.3H₂O (4.3 mg; 6.2 µmol; 1.0 equiv.) was dissolved in aq. **MOPS/NaOH buffer** (500 mM; pH 7.0; 640 µL; 320 µmol; 52 equiv.) and aq. **CdCl₂** (62.6 mM; 120 µL; 7.5 µmol; 1.2 equiv.) and the mixture was stirred 1 h at 80 °C. Mixture was then directly purified by preparative HPLC (C18; H₂O-MeCN gradient with no additive). Fractions with product were joined and directly lyophilized to give product as white fluffy solid. **Yield:** 4.2 mg (≤92% assuming **[M]**·*x*H₂O; *M*_{R} = 736.1; 1 step; based on **TD871**·0.6FA·2.3H₂O). **NMR (D₂O, pD ~7): ¹H** δ_{H} 2.00-4.41 (*mc*, C*H*₂-CO, C*H*₂-arom., bm, 16+4+4H); 5.93 (C*H*₂-N₃, bs, 2H); 7.44 (*arom.,* dd, 1H, ³*J*_{HH} = 8, ⁴*J*_{HH} = 1); 7.53-7.63 *(Ph,* m, 3H); 7.73-7.84 (*Ph*, *arom.*, m, 2+1H); 7.88-7.96 (*arom.*, m, 2H); 8.01 (*arom.*, t, 1H, ³*J*_{HH} = 8); 9.74 (C*H*-N₃, s, 1H). **ESI-HRMS:** 738.2081 [M+H]⁺ (theor. [C₃₃H₃₈N₉O₄Cd₁]⁺ = 738.2075). 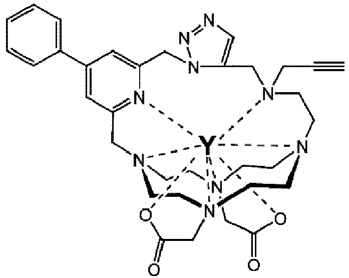 **Synthesis of TD685:** In a glass vial (20 mL), **TD669**·0.1FA·*x*H₂O (17 mg; ~27 µmol; 1.0 equiv.) was dissolved in H₂O (17 mL) followed by addition aq. **MOPS/NaOH buffer** (500 mM; pH 7.0; 2.10 mL; 1.05 mmol; ~40 equiv.) and aq. **YCl₃** (100 mM; 290 µL; 29 µmol; ~1.1 equiv.) and the mixture was stirred 6 weeks at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 4 mg (≤21% assuming [M]⁺[FA]⁻·*x*H₂O; *M*_{R} = 761.7; 1 step; based on **TD669**·0.1FA·*x*H₂O). **NMR (D₂O, pD -5): ¹H** δ_{H} 2.63-3.09 (*mc*, C*H*₂-C*H*₂-N-CH₂-C≡CH, m, 4+2H); 3.11 (C≡C*H*, t, 1H, ⁴*J*_{HH} = 2); 3.13-3.75 (*mc*, C*H₂-*C*H₂*-N-CH₂-C≡CH, C*H*₂-CO, 12+2+2H); 3.78 (C*H*₂-CO, d, 1H, ²*J*_{HH} = 19); 3.79 (C*H*₂-C≡CH, dm, 1H, ²*J*_{HH} = 18); 3.91 (C*H*₂-C≡CH, dd, 1H, ²*J*_{HH} = 18, ⁴*J*_{HH} = 2); 4.01 (C*H*₂-CO, d, 1H, ²*J*_{HH} = 19); 4.09 (C*H*₂-arom., d, 1H, ²*J*_{HH} = 15); 4.25 (C*H*₂-arom., d, 1H, ²*J*_{HH} = 15); 4.39 (C*H*₂-C-N₃, d, 1H, ²*J*_{HH} = 15); 4.75 (C*H*₂-C-N₃, d, 1H, ²*J*_{HH} = 15); 5.67 (C*H*₂-N₃, d, 1H, ²*J*_{HH} = 15); 7.00 (C*H*₂-N₃, d, 1H, ²*J*_{HH} = 15); 7.67-7.73 *(Ph,* m, 3H); 7.94 (*arom.*, d, 1H, ⁴*J*_{HH} = 2); 7.96-8.00 *(Ph,* m, 2H); 8.13 (C*H*-N₃, s, 1H); 8.30 *(arom.,* d, 1H, ⁴*J*_{HH} = 2); 8.52 (FA, s, 1H). **¹³C{¹H}** δ_{C} 41.2 (CH₂-C≡CH, s); 47.8 (*C*H₂-C-N₃, s); 52.6 (CH₂-*C*H₂-N-CH₂-C≡CH, s); 54.1 (*C*H₂-CH₂-N-CH₂-C≡CH, s); 54.4 (C*H*₂-N₃, s); 55.1 *(mc*, s); 57.2 *(mc,* s); 58.0 *(mc,* s); 59.0 (*mc*, s); 59.4 *(mc,* s); 59.5 *(mc,* s); 59.9 *(mc,* s); 60.1 *(mc,* bs); 66.0 (*C*H₂-CO, s); 67.2 (*C*H₂-CO, s); 67.6 (*C*H₂-arom., s); 75.3 (C=CH, s); 81.8 (C=CH, s); 124.8 (*arom.*, s); 127.7 (*arom.*, s); 130.0 *(Ph,* s); 132.1 *(Ph,* s); 133.6 *(Ph,* s); 143.4 *(arom.,* s); 137.5 *(Ph,* s); 138.1 (C*H*-N₃, s); 156.1 *(arom.,* s); 158.6 *(arom.,* s); 161.4 *(arom.,* s); 173.3 (FA, s); 181.2 (CO, s); 181.5 (CO, s). **ESI-HRMS:** 716.2327 [M]⁺ (theor. [C₃₃H₄₁N₉O₄Y1]⁺ = 716.2335). 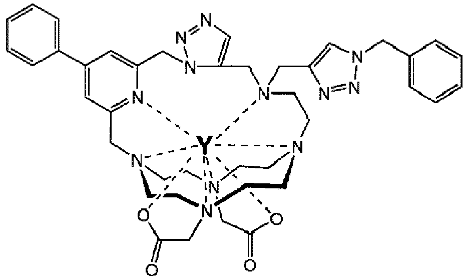 **Synthesis of TD780:** In a glass vial (4 mL), [**TD685**]⁺[FA]⁻·*x*H₂O (3 mg; ~4 µmol; 1.0 equiv.) was dissolved in aq. **MES/NaOH buffer** (500 mM; pH 5.2; 375 µL; 188 µmol; ~50 equiv.) followed by addition of *t*-BuOH ( 240 µL), **Benzyl azide** (500 mM in *t*-BuOH; 12 µL; 6 µmol; ~1.6 equiv.) and aq. solution of **CuSO₄** (500 mm; 3 µL; 1.5 µmol; ~0.4 equiv.). To the resulting faint light blue solution was added freshly prepared aq. solution of **Sodium ascorbate** (100 mM; 15 µL; 1.5 µmol; ~0.4 equiv.) and the color of the solution immediately changed to yellow-green. Mixture was stirred 1 h at RT after which was diluted with H₂O (2.4 mL) and filtered through syringe microfilter (RC). The filtrate was directly purified by preparative HPLC (C18; H₂O-MeCN gradient with TFA additive). Fractions with product were joined and directly lyophilized to give product in the form of trifluoroacetate salt as white fluffy solid. **Yield:** 3 mg (≤74% assuming [**M**]⁺[TFA]⁻·*x*H₂O; *M*_{R} = 962.8; 1 step; based on **TD685**·*x*H₂O). **NMR (D₂O, pD ~3): ¹H** δ_{H} 2.46-4.59 *(mc*, C*H*₂-CO, C*H*₂-arom., C*H*₂-C*H*₂-N-(C*H*₂-C=C)₂, m, 16+4+2+8H); 5.60 (C*H*₂-N₃, d, 1H, ²*J*_{HH} = 16); 5.71 (CH₂-Ph, s, 2H); 6.94 (C*H*₂-N₃, d, 1H, ²*J*_{HH} = 15); 7.37-7.50 *(Ph,* m, 5H); 7.59-7.65 *(Ph,* m, 3H); 7.88 *(arom.,* d, 1H, ⁴*J*_{HH} = 2); 7.89-7.93 *(Ph,* m, 2H); 8.21 (*arom.*, d, 1H, ⁴*J*_{HH} = 2); 8.34 (C*H*-N₃, s, 1H); 8.35 (C*H*-N₃, s, 1H). **ESI-HRMS:** 849.2970 [M]⁺ (theor. [C₄₀H₄₈N₁₂O₄]⁺ = 849.2975).

### Example 7: Synthesis of chains of coordination compounds

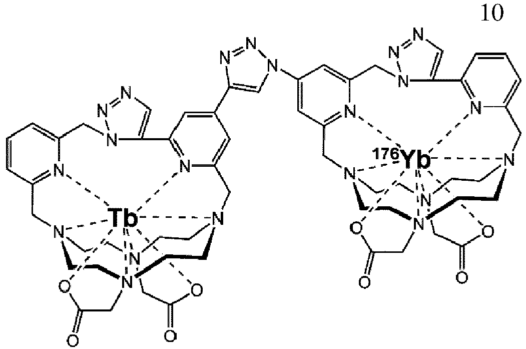

**Synthesis of TD837:** In a glass vial (4 mL), aq. solution of **[TD836**]⁺ [TFA]⁻·*x*H₂O (~20 mM; 274 µL; ~5.5 µmol; 1.0 equiv.) was mixed with aq. solution of **[TD823]**⁺ [TFA]⁻·*x*H₂O (~20 mM; 274 µL; ~5.5 µmol; 1.0 equiv.) followed by addition of aq. **MES/NaOH buffer** (500 mM; pH 5.2; 548 µL; 274 µmol; ~50 equiv.), *t*-BuOH (1.1 mL) and aq. solution of **CuSO₄** (100 mM; 11 µL; 1.1 µmol; ~0.2 equiv.). To the resulting solution was added freshly prepared aq. solution of **sodium ascorbate** (100 mM; 11 µL; 1.1 µmol; ~0.2 equiv.) and the mixture was stirred 2 h at RT. The mixture was then concentrated to ~1 mL and then purified by preparative HPLC (C18; H₂O-MeCN gradient with TFA additive). Fractions with product were joined and directly lyophilized to give product in the form of trifluoroacetate salt as white fluffy solid. **Yield:** 4.7 mg (≤50% assuming [**M**]²⁺[TFA]₂²⁻·*x*H₂O; *M*_{R} = 1721; 1 step; based on [**TD836**]⁺[TFA]⁻·*x*H₂O). **ESI-HRMS:** 747.1998 [M]²⁺ (theor. [C₅₆H₆₅N₂₁O₈Tb₁¹⁷⁶Yb₁]²⁺ = 747.1997). 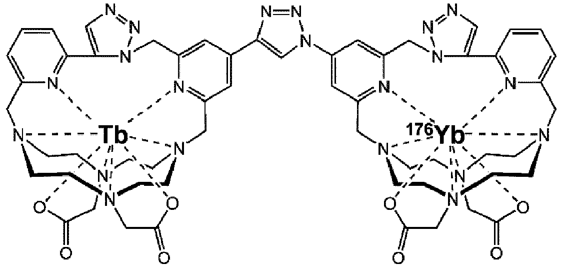 **Synthesis of TD889:** In a glass vial (4 mL), aq. solution of [**TD836**]⁺[TFA]⁻·*x*H₂O (~20 mM; 274 µL; ~5.5 µmol; 1.0 equiv.) was mixed with aq. solution of [**TD886**]⁺[TFA]⁻·*x*H₂O (~20 mM; 274 µL; ~5.5 µmol; 1.0 equiv.) followed by addition of aq. **MES/NaOH buffer** (500 mM; pH 5.2; 548 µL; 274 µmol; ~50 equiv.), *t*-BuOH (1.1 mL) and aq. solution of **CuSO₄** (100 mM; 28 µL; 2.8 µmol; ~0.5 equiv.). To the resulting solution was added freshly prepared aq. solution of **sodium ascorbate** (100 mM; 28 µL; 2.8 µmol; ~0.5 equiv.) and the mixture was stirred 30 min at RT. The mixture was then concentrated to ~1 mL and then purified by preparative HPLC (C18; H₂O-MeCN gradient with TFA additive). Fractions with product were joined and directly lyophilized to give product in the form of trifluoroacetate salt as white fluffy solid. **Yield:** 7.8 mg (≤82% assuming [**M**]²⁺[TFA]₂²⁻·*x*H₂O; *M*_{R} = 1721; 1 step; based on [**TD836**]⁺[TFA]⁻·*x*H₂O). **ESI-HRMS:** 747.2002 [M]²⁺ (theor. [C₅₆H₆₅N₂₁O₈Tb₁¹⁷⁶Yb₁]²⁺ = 747.1997).

### Example 8: Synthesis of dimers of coordination compounds

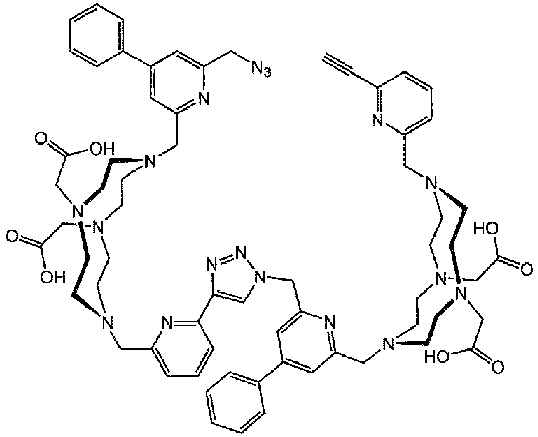

**Synthesis of sc-TD647:** Obtained as side product during synthesis of **TD647**. **Yield:** ~8 mg. **NMR (D₂O): ¹H** δ_{H} 2.69-3.65 *(mc* and C*H*₂-CO, 32+8H); 3.68 (C=CH, s, 1H); 3.70 (C*H*₂-arom., bs, 2H); 3.75-4.12 (C*H*₂-arom., 6H); 4.59 (C*H*₂-N₃., s, 2H); 5.89 (C*H*₂-N₃, s, 2H); 7.36-7.97 *(Ph* and *arom.*, 10+8H); 8.15 (*arom.*, bs, 1H); 8.32 (*arom.*, bs, 1H); 8.64 (C*H*-N₃, s, 1H). **ESI-HRMS:** 1251.6313 [M+H]⁺ (theor. [C₆₆H₇₉N₁₈O₈]⁺ = 1251.6323). 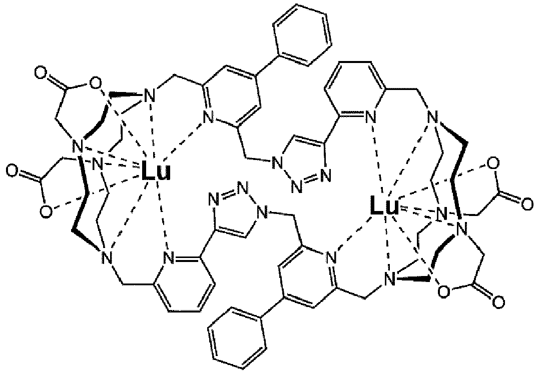 **Synthesis** of **TD613:** In a glass vial (20 mL), **sc-TD647**·*x*H₂O (5.0 mg; ~4 µmol; 1.0 equiv.) was dissolved in H₂O (14 mL) followed by addition aq. **MOPS/NaOH buffer** (500 mM; pH 7.0; 800 µL; 400 mmol; ~ 100 equiv.) and aq. **LuCl₃** (100 mM; 88 µL; 8.8 µmol; ~2.2 equiv.) and the mixture was stirred 1 h at 80 °C. Mixture was then filtered through syringe microfilter (RC). The filtrate was concentrated on rotary evaporator and then directly purified by preparative HPLC (C18; H₂O-MeCN gradient with FA additive). Fractions with product were joined and directly lyophilized to give product in the form of formate salt as white fluffy solid. **Yield:** 4 mg (~60%; 1 step; based on **sc-TD647**·*x*H₂O). **ESI-HRMS:** 798.2368 [M]²⁺ (theor. [C₆₆H₇₄N₁₈O₈Lu₂]²⁺ = 798.2371).

**Synthesis of TD598:** Obtained as side product during synthesis of **TD651** in the form of formate salt as white fluffy solid. **Yield:** 6 mg. **NMR (D₂O, pD ~5): ¹H** δ_{H} 2.47-3.99 *(mc,* C*H*₂-CO, C*H*₂-arom, m, 32+8+4H); 4.42 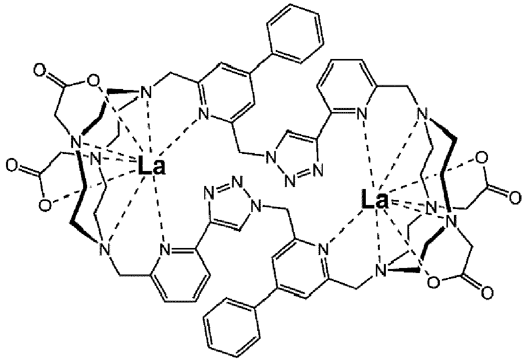 (C*H*₂-arom., d, 2H, ²*J*_{HH} = 17); 4.75 (C*H*₂-arom., d, 2H, ²*J*_{HH} = 17); 5.90 (C*H*₂-N₃, d, 2H, ²*J*_{HH} = 16); 6.20 (C*H*₂-N₃, d, 2H, ²*J*_{HH} = 16); 7.07 *(arom.,* d, 2H, ⁴*J*_{HH} = 2); 7.44 *(arom.,* d, 2H, ⁴*J*_{HH} = 2); 7.47-7.63 *(Ph,* m, 10H); 7.66 (*arom.,* dd, 2H, ³*J*_{HH} = 8, ⁴*J*_{HH} = 1); 7.89 *(arom.,* dd, 2H, ³*J*_{HH} = 8, ⁴*J*_{HH} = 1); 8.15 *(arom.,* t, 2H, ³*J*_{HH} = 8); 8.42 (FA, s); 8.74 (C*H*-N₃, s, 2H). **¹³C{¹H}** δ_{C} 49.3 *(mc,* s); 49.4 *(mc,* s); 51.0 *(mc,* s); 51.6 *(mc,* s); 52.5 *(mc,* s); 53.9 *(mc,* s); 54.0 *(mc,* s); 54.3 *(mc,* s); 55.6 (C*H*₂-N₃, s); 59.2 (C*H*₂-arom., s); 59.8 (*C*H₂-CO, s); 61.2 (C*H*₂-arom., s); 61.9 (*C*H₂-CO, s); 120.7 (*arom.,* s); 121.2 (*arom.,* s); 122.2 (*arom.,* s); 125.4 (*arom.,* s); 126.7 (C*H*-N₃, s); 128.2 (*Ph,* s); 129.6 (*Ph,* s); 131.6 *(Ph,* s); 135.2 (*Ph,* s); 143.0 (*arom.,* s); 145.5 (*arom.,* s); 148.9 (*arom.,* s); 150.2 (*arom.,* s); 157.2 (*arom.,* s); 157.3 (*arom.,* s); 158.9 (*arom.,* s); 170.8 (FA, s); 177.9 (CO, s); 180.5 (CO, s). **ESI-HRMS:** 762.2025 [M]²⁺ (theor. [C₆₆H₇₄N₁₈O₈La₂]²⁺ = 762.2027).

### Example 9: Synthesis of conjugates of coordination compounds with peptides

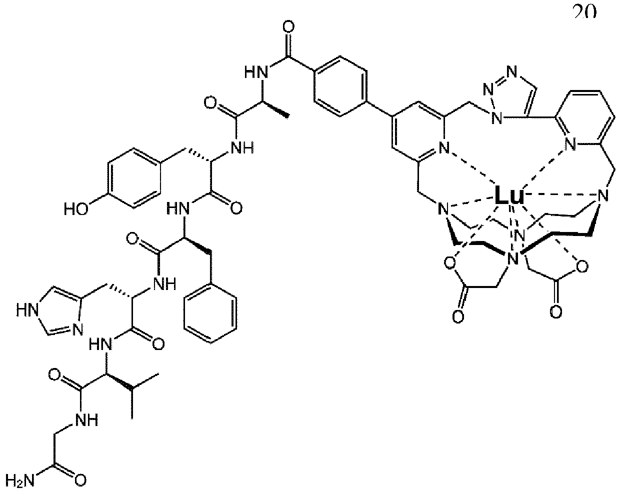

**Synthesis of TD802:** In a glass vial (4 mL), [**TD748**]⁺[TFA]⁻·1.4TFA·3.2H₂O (3.3 mg; 2.8 µmol; 1.0 equiv.) and **H₂N-GVHFYA-H·**1.0TFA·*x*H₂O (3.4 mg; ~4.2 µmol; ~1.5 equiv.) were dissolved in DMSO (1 mL) followed by addition of **DIPEA** (5.0 µL; 29 µmol; ~10 equiv.) and **PyOAP** (freshly prepared 100 mM in DMSO; 52 µL; 5.2 µmol; 1.9 equiv.) and the resulting yellow solution was stirred 20 min at RT. Mixture was then directly purified by preparative HPLC (C18; H₂O-MeCN gradient with TFA additive). Fractions with product were joined and directly lyophilized to give product as white fluffy solid. **Yield:** 4.4 mg (≤96% assuming [**M**]⁺[TFA]⁻·*x*H₂O; *M*_{R} = 1629.5; 1 step; based on [**TD748**]⁺[TFA]⁻·1.4TFA·3.2H₂O). **NMR (D₂O, pD~5): ¹H** δ_{H} 0.93 (^{V}C*H*₃, d, 3H, ³*J*_{HH} = 7); 0.95 (^{V}C*H*₃, d, 3H, ³*J*_{HH} = 7); 1.42 (^{A}C*H*₃, d, 3H, ³*J*_{HH} = 7); 2.03 (^{V}C*H*-(CH₃)₂, dhept, 1H, ³*J*_{HH} = 7, ³*J*_{HH} = 7); 2.62-3.81 *(mc,* C*H*₂-CO, ^{H}C*H*₂, ^{F}C*H*₂, ^{Y}C*H*₂, m, 16+4+2+2+2H); 3.84 (^{G}C*H*₂, d, 1H, ²*J*_{HH} = 17); 3.95 (^{G}C*H*₂, d, 1H, ²*J*_{HH} = 17); 4.01 (C*H*₂-arom., d, 1H, ²*J*_{HH} = 15); 4.02 (^{V}C*H*, d, 1H, ³*J*_{HH} = 7); 4.15 (C*H*₂-arom., d, 1H, ²*J*_{HH} = 16); 4.35 (C*H*₂-arom., d, 1H, ²*J*_{HH} = 15); 4.45 (^{A}C*H*, q, 1H, ³*J*_{HH} = 7); 4.52-4.66 (^{H}C*H*, ^{F}C*H*, ^{Y}C*H*, m, 1+1+1H); 4.85 (C*H*₂-arom., d, 1H, ²*J*_{HH} = 16); 5.67 (C*H*₂-N₃, d, 1H, ²*J*_{HH}= 15); 6.59 (^{Y}*arom.,* dm, 2H, ³*J*_{HH}= 8); 6.96 (^{Y}*arom.,* dm, 2H, ³*J*_{HH}= 8); 7.08-7.18 (C*H*₂-N₃, ^{F}*arom.,* m, 1+2H); 7.19 (^{H}*arom.,* s, 1H); 7.23-7.30 (^{F}*arom.,* m, 3H); 7.81 *(arom.,* dd, 1H, ³*J*_{HH} = 8; ⁴*J*_{HH} = 1); 7.82-7.87 (*arom.,* m, 3H); 7.89 (C*H*-N₃, s, 1H); 8.00 (*arom.,* dm, 2H, ³*J*_{HH} = 8); 8.03 (*arom.,* d, 1H, ⁴*J*_{HH} = 2); 8.24 *(arom.,* t, 1H, ³*J*_{HH} = 8); 8.33 *(arom.,* d, 1H, ⁴*J*_{HH} = 2); 8.48 (^{H}*arom.,* bs, 1H). **ESI-HRMS:** 1515.5593 [M]⁺ (theor. [C₆₈H₈₀N₁₈O₁₂Lu₁]⁺ = 1515.5605). 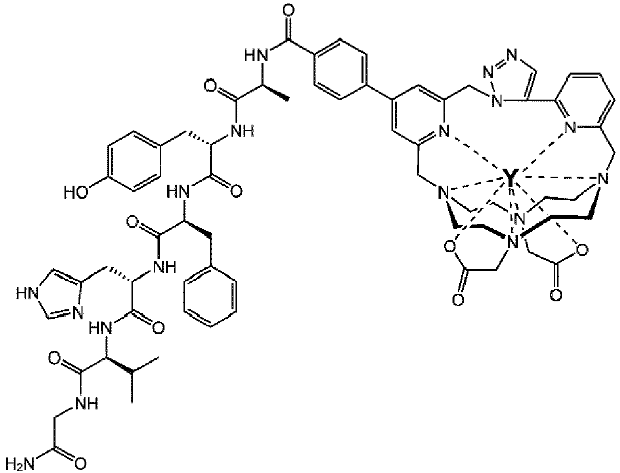 **Synthesis of TD792:** In a glass vial (4 mL), [**TD785**]⁺[TFA]⁻·0.6TFA·4.8H₂O (3.0 mg; 2.9 µmol; 1.0 equiv.) and **H₂N-GVHFYA-H**·1.0TFA·*x*H₂O peptide (3.4 mg; ~4.2 µmol; ~1.4 equiv.) were dissolved in DMSO (1 mL) followed by addition of **DIPEA** (5.0 µL; 29 µmol; ~10 equiv.) and **PyOAP** (freshly prepared 100 mM in DMSO; 52 µL; 5.2 µmol; 1.8 equiv.) and the resulting yellow solution was stirred 20 min at RT. Mixture was then directly purified by preparative HPLC (C18; H₂O-MeCN gradient with TFA additive). Fractions with product were joined and directly lyophilized to give product as white fluffy solid. **Yield:** 4.0 mg (≤89% assuming [**M**]⁺[TFA]⁻·*x*H₂O; *M*_{R} = 1543.3; 1 step; based on [**TD785**]⁺[TFA]⁻· 0.6TFA·4.8H₂O). **NMR (D₂O, pD ~5): ¹H** δ_{H} 0.93 (^{V}C*H*₃, d, 3H, ³*J*_{HH} = 7); 0.95 (^{V}C*H*₃, d, 3H, ³*J*_{HH} = 7); 1.42 (^{A}C*H*₃, d, 3H, ³*J*_{HH} = 7); 2.03 (^{V}C*H*-(CH₃)₂, dhept, 1H, ³*J*_{HH} = 7, ³*J*_{HH} = 7); 2.62-3.81 (*mc,* C*H*₂-CO, ^{H}C*H*₂, ^{F}C*H*₂, ^{Y}C*H*₂, m, 16+4+2+2+2H); 3.84 (^{G}C*H*₂, d, 1H, ²*J*_{HH} = 17); 3.95 (^{G}C*H*₂, d, 1H, ²*J*_{HH} = 17); 4.01 (C*H*₂-arom., d, 1H, ²*J*_{HH} = 15); 4.02 (^{V}C*H*, d, 1H, ³*J*_{HH} = 7); 4.14 (C*H*₂-arom., d, 1H, ²*J*_{HH} = 16); 4.39 (C*H*₂-arom., d, 1H, ²*J*_{HH} = 15); 4.45 (^{A}C*H*, q, 1H, ³*J*_{HH} = 7); 4.52-4.67 (^{H}C*H*, ^{F}C*H*, ^{Y}C*H*, m, 1+1+1H); 4.85 (C*H*₂-arom., d, 1H, ²*J*_{HH} = 16); 5.72 (C*H*₂-N₃, d, 1H, ²*J*_{HH} = 16); 6.59 (^{Y}*arom.,* dm, 2H, ³*J*_{HH} = 8); 6.96 (^{Y}*arom.,* dm, 2H, ³*J*_{HH} = 8); 7.09 (C*H*₂-N₃, d, 1H, ²*J*_{HH}= 16); 7.10-7.16 (^{F}*arom.,* m, 2H); 7.21 (^{H}*arom.,* s, 1H); 7.24-7.30 (^{F}*arom.,* m, 3H); 7.78-7.86 *(arom.,* m, 4H); 7.89 (C*H*-N₃, s, 1H); 8.00 *(arom.,* dm, 2H, ³*J*_{HH} = 8); 8.03 *(arom.,* d, 1H, ⁴*J*_{HH} = 2); 8.25 *(arom.,* t, 1H, ³*J*_{HH} = 8); 8.33 *(arom.,* d, 1H, ⁴*J*_{HH} = 2); 8.53 (^{H}*arom.,* t, 1H, ⁴*J*_{HH} = 2). **ESI-HRMS:** 1429.5264 [M]⁺ (theor. [C₆₈H₈₀N₁₈O₁₂Y₁]⁺ = 1429.5256). 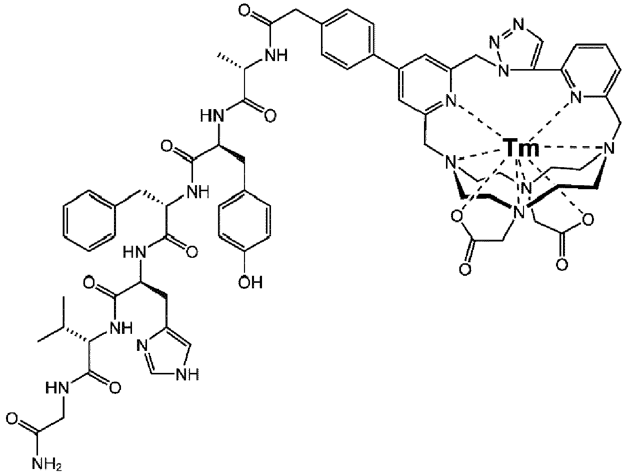 **Synthesis of TD901:** In a glass vial (4 mL), [**TD899**]⁺[TFA]⁻·02TFA·7.0H₂O (1.6 mg; 1.4 µmol; 1.0 equiv.) and **H₂N-GVHFYA-H·**1.0TFA·*x*H₂O (1.6 mg; ~2.0 µmol; ~1.4 equiv.) were dissolved in DMSO (500 µL) followed by addition of **DIPEA** (2.3 µL; 13 µmol; 9 equiv.) and **PyOAP** (freshly prepared 100 mM in DMSO; 25 µL; 2.5 µmol; 1.7 equiv.) and the resulting yellow solution was stirred 40 min at RT. Mixture was then directly purified by preparative HPLC (C18; H₂O--MeCN gradient with TFA additive). Fractions with product were joined and directly lyophilized to give product as white fluffy solid. **Yield:** 2.0 mg (≤85% assuming [**M**]⁺[TFA]⁻·*x*H₂O; *M*_{R} = 1637.5; 1 step; based on [**TD899**]⁺[TFA]⁻·0.2TFA·7.0H₂O). **ESI-HRMS:** 762.2885 [M+H]²⁺ (theor. [C₆₉H₈₃N₁₈O₁₂Tm₁]²⁺ = 762.2887). 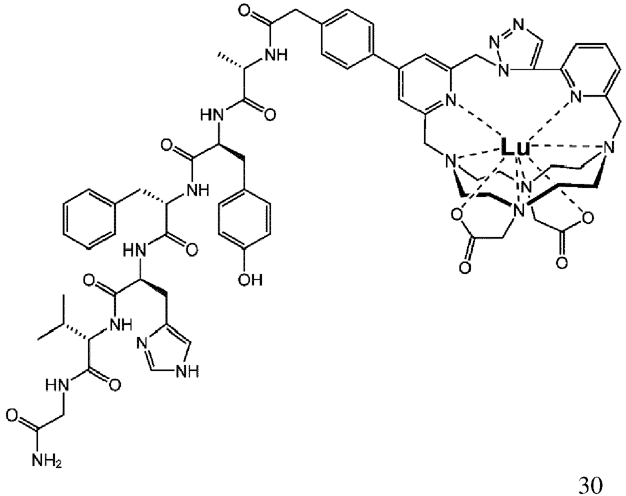 **Synthesis of TD902:** In a glass vial (4 mL), [**TD891**]⁺[TFA]⁻·0.7TFA·4.6H₂O (1.6 mg; 1.4 µmol; 1.0 equiv.) and **H₂N-GVHFYA-H·**1.0TFA·*x*H₂O (1.6 mg; ~2.0 µmol; ~1.4 equiv.) were dissolved in DMSO (500 µL) followed by addition of **DIPEA** (2.3 µL; 13 µmol; 9.4 equiv.) and **PyOAP** (freshly prepared 100 mM in DMSO; 25 µL; 2.5 µmol; 1.8 equiv.) and the resulting yellow solution was stirred 40 min at RT. Mixture was then directly purified by preparative HPLC (C18; H₂O- -MeCN gradient with TFA additive). Fractions with product were joined and directly lyophilized to give product as white fluffy solid. **Yield:** 1.8 mg (≤78% assuming [**M**]⁺[TFA]⁻·*x*H₂O; *M*_{R} = 1643.5; 1 step; based on [**TD891**]⁺[TFA]⁻·0.7TFA·4.6H₂O). **NMR (D₂O, pD ~4, assignment was complicated by two distinguishable diastereomeric set of most of the signals): ¹H** δ_{H} 0.91 (^{V}C*H*₃, d, 3H, ³*J*_{HH} = 7); 0.93 (^{V}C*H*₃, d, 3H, ³*J*_{HH} = 7); 1.36 (^{A}C*H*₃, d, 3H, ³*J*_{HH} = 7); 1.90-4.21 (^{V}C*H*-(CH₃)₂, *mc,* C*H*₂-CO, ^{H}C*H*₂, ^{F}C*H*₂, ^{Y}C*H*₂, ^{G}C*H*₂, C*H*₂-arom., ^{V}C*H*, ^{A}C*H*, m, 1+16+4+2+2+2+2+5+1+1H); 4.21-4.56 (^{H}C*H*, ^{F}C*H*, ^{Y}C*H*, m, 1+1+1H); 4.81-4.90 (C*H*₂-arom., m, 1H); 5.52-5.62 (C*H*₂-N₃, m, 1H); 6.77-7.18 (^{Y}*arom.,* C*H*₂-N₃, ^{F}*arom.,* ^{H}*arom.,* ^{F}*arom.,* m, 4+1+2+1+3H); 7.34-8.10 (*arom.,* C*H*-N₃, 8+1H); 8.21-8.30 (arom., m, 1H); 8.52-8.58 (^{H}*arom.,* m, 1H). **ESI-HRMS:** 765.2919 [M+H]²⁺ (theor. [C₆₉H₈₃N₁₈O₁₂Lu₁]²⁺ = 765.2917). 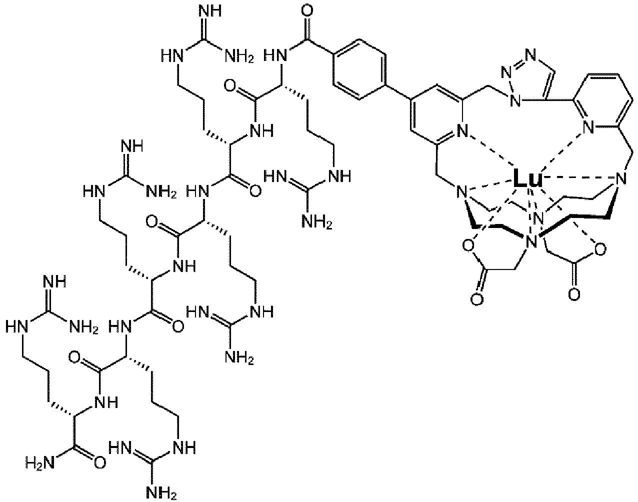 **Synthesis of TD975:** In a glass vial (4 mL), [**TD748**]⁺[TFA]⁻·1.4TFA·3.2H₂O (7.1 mg; 6.1 µmol; 1.0 equiv.) and **H₂N--[^{L}R(Pbf)^{D}R(Pbf)]₃-H·**1.0TFA·*x*H₂O (21 mg; ~8.1 µmol; ~1.3 equiv.) were dissolved in DMSO (1 mL) followed by addition of **NMM** (20 µL; 182 µmol; 30 equiv.) and **PyOAP** (15.5 mg; 29.7 µmol; 5.0 equiv.) and the resulting yellow solution was stirred 20 min at RT. Mixture was then directly purified by preparative HPLC (C18; H₂O-MeCN gradient with TFA additive). Fractions with Pbf protected product were joined, evaporated to dryness and twice co-evaporated with MeOH. Residue was dissolved in TFA (3 mL) and the resulting clear mixture was stirred 16 h at RT. Mixture was evaporated to dryness and the residue was directly purified by preparative HPLC (C18; H₂O-MeCN gradient with TFA additive). Fractions with product were joined and directly lyophilized to give product in the form of trifluoroacetate salt as white fluffy solid (the amount of TFA was determined by qNMR using external capillary solution of 1,3,5-tris(trifluoromethyl)benzene in CCl₄). **Yield:** 13.0 mg (~85% assuming [**M**]⁺[TFA]⁻·5.4TFA·*x*H₂O; *M*_{R}= 2510; 2 steps; based on [**TD748**]⁺[TFA]⁻·1.4TFA·3.2H₂O). **NMR (D₂O, pD ~3): ¹H** δ_{H} 1.41-2.07 (^{R}C*H*₂, m, 24H); 2.63-3.88 (*mc,* C*H*₂-CO, ^{R}C*H*₂, m, 16+4+12H); 4.05 (C*H*₂-arom., d, 1H, ²*J*_{HH} = 15); 4.18 (C*H*₂-arom., d, 1H, ²*J*_{HH} = 16); 4.21-4.57 (^{R}C*H*, C*H*₂-arom., m, 5+1H); 4.53 (^{R}C*H*, t, 1H, ³*J*_{HH} = 7); 4.87 (C*H*₂-arom., d, 1H, ²*J*_{HH} = 16); 5.69 (C*H*₂-N₃, d, 1H, ²*J*_{HH} = 15); 7.19 (C*H*₂-N₃, d, 1H, ²*J*_{HH} = 15); 7.82 *(arom.,* dd, 1H, ³*J*_{HH} = 8; ⁴*J*_{HH} = 1); 7.88 *(arom.,* dd, 1H, ³*J*_{HH} = 8; ⁴*J*_{HH} = 1); 7.91 (C*H*-N₃, s, 1H); 8.03 (*arom.,* dm, 2H, ³*J*_{HH} = 8); 8.06-8.12 (*arom.,* m, 3H); 8.27 (*arom.,* t, 1H, ³*J*_{HH} = 8); 8.39 (*arom.,* d, 1H, ⁴*J*_{HH} = 2). **MALDI-HRMS:** 1777.8487 [M]⁺ (theor. [C₇₀H₁₁₀N₃₄O₁₁,Lu₁]⁺= 1777.8496). 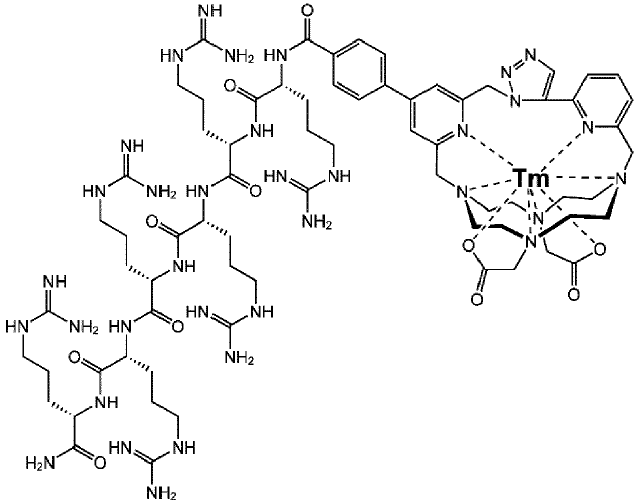 **Synthesis of TD989:** In a glass vial (4 mL), [**TD897**]⁺[TFA]⁻·1.1TFA·2.6H₂O (15.0 mg; 13.4 µmol; 1.0 equiv.) and **TD986·**3.2TFA·5.3H₂O (30.9 mg; 13.4 µmol; 1.0 equiv.) were dissolved in DMSO (1.5 mL) followed by addition of solution of **NMM** (400 mM, 335 µL; 134 µmol; ~10 equiv.) in DMSO and freshly prepared solution of **PyOAP** (110 mM; 210 µL; 23.1 µmol; 1.7 equiv.) in DMSO and the resulting yellow solution was stirred 30 min at RT. Mixture was then directly purified by preparative HPLC (C18; H₂O-MeCN gradient with TFA additive). Fractions with fully protected product were joined, evaporated to dryness and twice co-evaporated with MeOH. Residue was dissolved in TFA (3 mL) and stirred overnight at RT to remove Pbf groups. Resulting blue solution was evaporated to dryness and the residue was purified by preparative HPLC (C18; H₂O-MeCN gradient with TFA additive). Fractions with partly protected product were joined, and evaporated to dryness. Residue was re dissolved in H₂O (10 mL) and transferred via syringe through septum to a glass flask (25 mL) already filled with Pd/C (30 mg) and argon secured. Hydrogen gas was then left bubbling through the suspension for 2 days at RT. Mixture was then filtered through syringe microfilter (RC), and the filtrate was evaporated to dryness. Residue was purified by preparative HPLC (C18; H₂O-MeCN gradient with TFA additive). Fractions with product were joined and directly lyophilized to give product in the form of trifluoroacetate salt as white fluffy solid (the amount of TFA was set arbitrary from analogous **TD975). Yield:** 14.6 mg (~45% assuming [**M**]⁺[TFA]⁻·5.4TFA·*x*H₂O; *M*_{R} = 2505; 3 steps; based on [**TD897**]⁺[TFA]⁻·1.1TFA·2.6H₂O). **MALDI-HRMS:** 1771.8412 [M]⁺ (theor. [C₇₀H₈₃N₃₄O₁₁Tm₁]⁺ = 1771.8435). 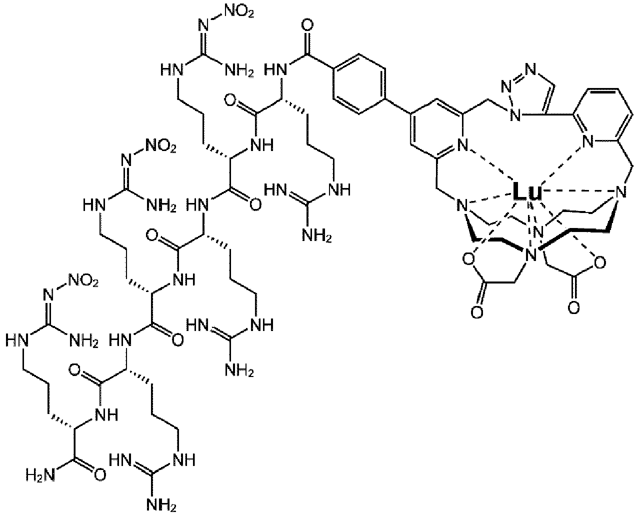 **Synthesis of TD1011:** In a glass vial (4 mL), [**TD748**]⁺[TFA]⁻·1.4TFA·3.2H₂O (15.0 mg; 12.8 µmol; 1.0 equiv.) and **TD9863·**2TFA·5.3H₂O (31 mg; 13.4 µmol; 1.0 equiv.) were dissolved in DMSO (1.5 mL) followed by addition of solution of **NMM** (400 mM, 340 µL; 136 µmol; 11 equiv.) in DMSO and freshly prepared solution of **PyOAP** (110 mM; 210 µL; 23.1 µmol; 1.8 equiv.) in DMSO and the resulting yellow solution was stirred 30 min at RT. Mixture was then directly purified by preparative HPLC (C18; H₂O-MeCN gradient with TFA additive). Fractions with Pbf protected product were joined, evaporated to dryness and twice co-evaporated with MeOH. Residue was dissolved in TFA (3 mL) and the resulting clear mixture was stirred 16 h at RT. Mixture was evaporated to dryness and the residue was suspended in 20 % MeCN (3 mL). Mixture was filtered through syringe microfilter (RC) and the filtrate was directly purified by preparative HPLC (C18; H₂O-MeCN gradient with TFA additive). Fractions with product were joined and directly lyophilized to give product in the form of trifluoroacetate salt as white fluffy solid (the amount of TFA was determined by qNMR using external capillary solution of 1,3,5-tris(trifluoromethyl)benzene in CCl₄). **Yield:** 18.4 mg (~55% assuming [**M**]⁺[TFA]⁻·4.5TFA·*x*H₂O; *M*_{R}= 2540; 2 steps; based on [**TD748**]⁺[TFA]⁻·1.4TFA·3.2H₂O). **NMR (D₂O, pD ~5): ¹H** δ_{H} 1.39-2.07 (^{R}C*H*₂, m, 24H); 2.63-3.85 *(mc,* C*H*₂-CO, ^{R}C*H*₂, m, 16+4+12H); 4.04 (C*H*₂-arom., d, 1H, ²*J*_{HH} = 15); 4.16 (C*H*₂-arom., d, 1H, ²*J*_{HH} = 16); 4.21-4.66 (^{R}C*H*, C*H*₂-arom., m, 6+1H); 4.86 (C*H*₂-arom., d, 1H, ²*J*_{HH} = 16); 5.69 (C*H*₂-N₃, d, 1H, ²*J*_{HH} = 15); 7.18 (C*H*₂-N₃, d, 1H, ²*J*_{HH} = 15); 7.81 *(arom.,* dd, 1H, ³*J*_{HH} = 8; ⁴*J*_{HH} = 1); 7.86j *(arom.,* dd, 1H, ³*J*_{HH} = 8; ⁴*J*_{HH} = 1); 7.90 (C*H*-N₃, s, 1H); 7.95-8.09 *(arom.,* m, 5H); 8.25 *(arom.,* t, 1H, ³*J*_{HH} = 8); 8.37 *(arom.,* d, 1H, ⁴*J*_{HH} = 2). **MALDI-HRMS:** 1912.8054 [M]⁺ (theor. [C₇₀H₁₀₇N₃₇O₁₇Lu₁]⁺ = 1912.8048).

**Synthesis of TD1010:** In a glass vial (4 mL), ), [**TD897**]⁺[TFA]⁻·1.1TFA·2.6H₂O (10.0 mg; 8.9 µmol; 1.0 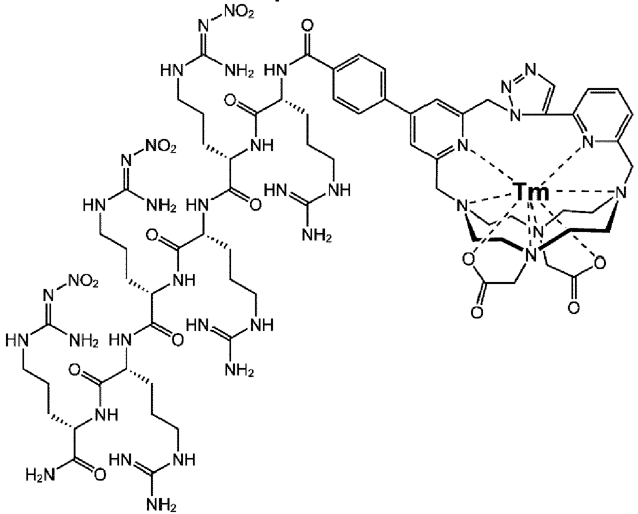 equiv.) and **TD986**·3.2TFA·5.3H₂O (21 mg; 9.1 µmol; 1.0 equiv.) were dissolved in DMSO (1.0 mL) followed by addition of solution of **NMM** (400 mM, 230 µL; 92 µmol; 10 equiv.) in DMSO and freshly prepared solution of **PyOAP** (110 mM; 140 µL; 15.4 µmol; 1.7 equiv.) in DMSO and the resulting yellow solution was stirred 30 min at RT. Mixture was then directly purified by preparative HPLC (C18; H₂O-MeCN gradient with TFA additive). Fractions with Pbf protected product were joined, evaporated to dryness and twice co-evaporated with MeOH. Residue was dissolved in TFA (3 mL) and the resulting clear mixture was stirred 16 h at RT. Mixture was evaporated to dryness and the residue was suspended in 20 % MeCN (3 mL). Mixture was filtered through syringe microfilter (RC) and the filtrate was directly purified by preparative HPLC (C18; H₂O-MeCN gradient with TFA additive). Fractions with product were joined and directly lyophilized to give product in the form of trifluoroacetate salt as white fluffy solid (the amount of TFA was set arbitrary from analogous **TD1011). Yield:** 11.6 mg (~50% assuming [**M**]⁺[TFA]⁻·4.5TFA·*x*H₂O; *M*_{R} = 2530; 2 steps; based on [**TD897**]⁺[TFA]⁻·1.1TFA·2.6H₂O). **MALDI-HRMS:** 1906.7973 [M]⁺ (theor. [C₇₀H₁₀₇N₃₇O₁₇Tm₁]⁺ = 1906.7982).

### Example 10: Charts

**Chart 1.** Formation of *1,5*-cz-[Ln(**Lig**)] from **Lig** and Ln^{III} ions *(metal dependent click-zip*): conversion determined after 1 hour at 80 °C by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. Lig (1.0 equiv.); 1.0 mM aq. Ln^{III} (2.0 equiv.); 50 mM aq. MOPS/NaOH buffer pH 7.0 (100 equiv.). Performed in glass vials with magnetic stirrers. Metal ions are sorted by decreasing ionic radius.

| **Lig** | *λ, nm* | La^{III} | Ce^{III} | Pr^{III} | Nd^{III} | Sm^{III} | Eu^{III} | Gd^{III} | Tb^{III} | Dy^{III} | Ho^{III} | Er^{III} | Tm^{III} | Yb^{III} | Lu^{III} |
|---|---|---|---|---|---|---|---|---|---|---|---|---|---|---|---|
| **TD425** | 275 | 0% | 0% | 0% | 0% | 0% | 0% | 0% | 0% | 0% | 0% | 0% | 0% | 0% | 0% |
| **TD556** | 275 | 1% | 1% | 1% | 2% | 3% | 4% | 5% | 11% | 16% | 24% | 37% | 52% | 64% | 71% |
| **TD576** | *280* | ? | ? | ? | ? | ? | ? | ? | ? | ? | ? | ? | ? | ? | ? |
| **TD582**^{↓} | 275 | 0% | 0% | 5% | 6% | 12% | 14% | 15% | 18% | 18% | 16% | 15% | 14% | 12% | 5% |
| **TD604** | *280* | 0% | 0% | 0% | 0% | 0% | 0% | 0% | 1% | 1% | 1% | 1% | 2% | 2% | 1% |
| **TD647** | *280* | 1% | 1% | 1% | 1% | 3% | 4% | 5% | 9% | 13% | 21% | 32% | 48% | 61% | 64% |
| **TD669** | 275 | 0% | 0% | 0% | 0% | 0% | 0% | 0% | 0% | 0% | 0% | 0% | 0% | 0% | 0% |
| **TD714** ^{∗} | *270* | 0% | 1% | 2% | 3% | 5% | 7% | 9% | 17% | 23% | 26% | 34% | 40% | 40% | 37% |
| **TD722** | *280* | 0% | 0% | 0% | 0% | 1% | 1% | 2% | 3% | 5% | 9% | 17% | 27% | 38% | 49% |
| **TD728** ^{/} | 275 | 0% | 0% | 0% | 0% | 0% | 0% | 0% | 0% | 1% | 1% | 1% | 1% | 2% | 2% |
| **TD742** | *285* | 1% | 1% | 1% | 1% | 3% | 4% | 5% | 9% | 15% | 22% | 34% | 48% | 55% | 66% |
| **TD778 ‡** | *285* | 24% | 20% | 13% | 9% | 4% | 4% | 4% | 9% | 13% | 17% | 25% | 31% | 30% | 26% |
| **TD779 ‡** | *280* | 0% | 0% | 0% | 0% | 1% | 2% | 3% | 6% | 8% | 10% | 12% | 9% | 9% | 5% |
| **TD801** ^{↑} | 275 | 0% | 0% | 0% | 0% | 0% | 0% | 0% | 0% | 0% | 0% | 0% | 0% | 0% | 0% |
| **TD827** ^{/} | 275 | 1% | 2% | 2% | 3% | 7% | 9% | 11% | 21% | 30% | 43% | 56% | 68% | 75% | 77% |
| **TD921^{↓}** | 275 | 0% | 0% | 5% | 4% | 1% | 1% | 0% | 1% | 1% | 1% | 1% | 1% | 1% | 1% |
| **TD943** | *280* | 0% | 0% | 0% | 0% | 0% | 0% | 0% | 1% | 1% | 2% | 2% | 3% | 3% | 5% |
| **TD944** | *270* | 0% | 0% | 0% | 0% | 0% | 0% | 0% | 0% | 0% | 1 % | 1% | 1% | 2% | 2% |
| **TD959** | *275* | 0% | 0% | 0% | 0% | 1% | 1% | 1% | 1% | 1% | 2% | 3% | 4% | 5% | 6% |
| **TD992** | *275* | 0% | 0% | 0% | 0% | 1% | 1% | 1% | 1% | 1% | 2% | 2% | 4% | 5% | 6% |
| **TD1054** | *280* | 0% | 0% | 0% | 1% | 2% | 3% | 3% | 6% | 10% | 16% | 26% | 39% | 53% | 63% |
| **TD1063** | *278* | 0% | 1% | 1% | 2% | 3% | 4% | 5% | 10% | 15% | 22% | 33% | 45% | 53% | 54% |
| **TD1092** 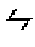 | *280* | 0% | 0% | 0% | 1% | 1% | 2% | 2% | 3% | 5% | 7% | 12% | 19% | 24% | 26% |
| | | 0% | *0* % | 0% | 0% | *2* % | 2% | *3*% | 6% | *8%* | *13%* | *19%* | 26% | *30%* | *30%* |
| **TD1105** 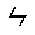 | *275* | 0% | 0% | 0% | 1% | 1% | 1% | 1% | 2% | 2% | 3% | 4% | 5% | 7% | 8% |
| | | 0% | 0% | 0% | 0% | 0% | 0% | 0% | 0% | 0% | 1% | 2% | *3%* | 5% | 9% |
| **TD1127** | 278 | 0% | 0% | 0% | 0% | 0% | 1% | 1% | 1% | 2% | 3% | 4% | 7% | 10% | 13% |
| **TD1148** 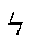 | *280* | 0% | 0% | 0% | 1% | 3% | 4% | 5% | 9% | 14% | 21% | 33% | 45% | 54% | 57% |
| | | 0% | 0% | 0% | 0% | *0%* | 0% | *1%* | 1% | *2%* | *3%* | 4% | 5% | 5% | 6% |
| **TD1160** 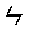 | *280* | - | - | - | - | 0% | - | - | 0% | - | - | - | - | - | 3% |
| | | | | | | *3* % | | | 9% | | | | | | 61% |
| **TD1176** | *285* | 0% | 0% | 1% | 2% | 5% | 6% | 7% | 14% | 20% | 31% | 46% | 63% | 75% | 85% |
| **TD1204** | 278 | 0% | 0% | 0% | 0% | 1% | 1% | 1% | 2% | 2% | 4% | 6% | 11% | 15% | 18% |

| | | | | | | | | | | | | | | | |
|---|---|---|---|---|---|---|---|---|---|---|---|---|---|---|---|
| ? No complex (either Ln(**TD576**) or *1,5*-cz-[Ln(**TD576**)]) was detected, although consumption of the starting ligand was observed (slowest for La, fastest for Lu). Reaction mixtures did not contain visible precipitate. Addition of organic co-solvent (MeOH or MeCN) to reaction mixtures and/or HPLC aliquots did not have significant effect. This behaviour was also observed in the case of a less hydrophobic analogue **TD581.** ^{↓} Obtained data might not accurately reflect abundance of *1,5*-cz-[Ln(**Lig**)] in the whole mixture as non-negligible variation of overall signal intensity was observed throughout the series. ↑ Propionate pendants arms detach during course of the reaction, yielding a mixture of *1,4*-cz-**TD801** and 1,5- **TD801** and their corresponding derivatives with either one or both propionate arms removed. ^{∗} Sum of two diastereoisomers. ^{/} Performed in a mixture of 50% *i*-PrOH in aq. MOPS/NaOH Buffer pH 7.0. ^{‡} Pendant arms of *1,5*-cz-[Ln(**Lig**)] are coordinated in deprotonated iminol form (each bearing -1 charge). As a consequence, these species undergo hydrolysis to acetate arms (i.e. *in situ* yielding *1,5*-cz-[Ln(**TD647**)]). 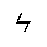 Values in italics: *1,5*-cz-[Ln(**Lig**)] with hydrolysed group in the side chain (Me ester in the case of **TD1092,** *i*-Pr ester in the case of **TD1148,** *t*-Bu ester in the case of **TD1160** and Boc in the case of **TD1105).** - Not performed. | | | | | | | | | | | | | | | |

**Chart 2.** Formation of *1,5*-cz-[Ln(**Lig**)] from **Lig** and Ln^{III} ions (*metal dependent click-zip*): conversion determined after 1 week at 80 °C by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. **Lig** (1.0 equiv.); 1.0 mM aq. Ln^{III} (2.0 equiv.); 50 mM aq. MOPS/NaOH buffer pH 7.0 (100 equiv.). Performed in glass vials with magnetic stirrers. Metal ions are sorted by decreasing ionic radius.

| **Lig** | *λ, nm* | La^{III} | Ce^{III} | Pr^{III} | Nd^{III} | Sm^{III} | Eu^{III} | Gd^{III} | Tb^{III} | Dy^{III} | Ho^{III} | Er^{III} | Tm^{III} | Yb^{III} | Lu^{III} |
|---|---|---|---|---|---|---|---|---|---|---|---|---|---|---|---|
| **TD425** | 275 | 0% | 0% | 0% | 0% | 0% | 0% | 0% | 0% | 0% | 0% | 0% | 0% | 0% | 0% |
| **TD556** | 275 | 50% | 25% | 64% | 81% | 92% | 92% | 91% | 95% | 94% | 95% | 95% | 95% | 95% | 95% |
| **TD582** ^{↓} | 275 | 0% | 0% | 17% | 60% | 86% | 87% | 86% | 82% | 85% | 79% | 74% | 68% | 59% | 26% |
| **TD604** | *280* | 15% | 17% | 16% | 16% | 27% | 51% | 50% | 69% | 67% | 69% | 66% | 71% | 72% | 74% |
| **TD647** | *280* | 49% | 50% | 69% | 79% | 86% | 89% | 92% | 91% | 92% | 95% | 95% | 96% | 96% | 96% |
| **TD669** | 275 | n. d. | n. d. | n. d. | n. d. | 33% | 25% | 24% | err. | 20% | 14% | 12% | 11% | 10% | 7% |
| **TD714** ^{∗} | *270* | 4% | 40% | 80% | 73% | 82% | 85% | 85% | 92% | 91% | 93% | 93% | 94% | 93% | 93% |
| **TD722** | *280* | 2% | 1% | 3% | 4% | 9% | 14% | 19% | 54% | 65% | 82% | 91% | 96% | 96% | 96% |
| **TD728** ^{/} | 275 | 13% | 5% | 23% | 24% | 34% | 35% | 32% | 49% | 54% | 60% | 72% | 84% | 88% | 88% |
| **TD742** | *285* | 40% | 43% | 66% | 76% | 87% | 89% | 91% | 93% | 93% | 94% | 94% | 94% | 93% | 94% |
| **TD764** | 278 | 25% | 20% | 35% | 50% | 73% | 79% | 86% | 91% | 93% | 94% | 95% | 96% | 96% | 96% |
| **TD778** ^{‡} | *285* | 0% | 0% | 0% | 0% | 1% | 3% | 11% | 15% | 18% | 30% | 32% | 31% | 27% | 34% |
| **TD779** ^{‡} | *280* | 0% | 0% | 0% | 0% | 0% | 0% | 0% | 0% | 0% | 0% | 0% | 0% | 0% | 0% |
| **TD801** ^{↑} | 275 | 0% | 0% | 0% | 0% | 0% | 0% | 0% | 0% | 0% | 0% | 0% | 0% | 0% | 0% |
| **TD827** ^{/} | 275 | 46% | 22% | 79% | 84% | 91% | 92% | 93% | 95% | 96% | 96% | 96% | 97% | 97% | 97% |
| **TD921^{↓}** | 275 | 0% | 0% | 55% | 43% | 42% | 31% | 28% | 41% | 37% | 31% | 26% | 18% | 15% | 10% |
| **TD943** | *280* | 5% | 4% | 10% | 16% | 29% | 36% | 40% | 56% | 65% | 77% | 88% | 94% | 97% | 97% |
| **TD944** | *270* | 4% | 3% | 6% | 8% | 16% | 19% | 23% | 37% | 51% | 68% | 84% | 93% | 95% | 94% |
| **TD959** | 275 | 18% | 11% | 27% | 34% | 48% | 51% | 54% | 68% | 76% | 84% | 93% | 97% | 98% | 99% |
| **TD992** | 275 | 22% | 20% | 33% | 40% | 55% | 59% | 61% | 73% | 81% | 89% | 95% | 98% | 99% | 99% |
| **TD1054** | *280* | 4% | 4% | 10% | 11% | 22% | 28% | 33% | 55% | 70% | 79% | 88% | 94% | 97% | 98% |
| **TD1063** | *278* | 5% | 1% | 35% | 52% | 57% | 73% | 74% | 83% | 86% | 90% | 93% | 93% | 95% | 95% |
| **TD1092** 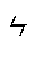 | *280* | 0% | 0% | 0% | 0% | 0% | 0% | 0% | 0% | 0% | 0% | 0% | 0% | 0% | 0% |
| | | *40%* | 37% | 62% | 73% | *88*% | *91%* | 92% | *93%* | 94% | 94% | 94% | 95% | 96% | 97% |
| **TD1105** 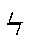 | 275 | 0% | 0% | 0% | 0% | 1% | 1% | 2% | 1% | 0% | 1% | 1% | 1% | 1% | 1% |
| | | 11% | 8% | *21%* | 23% | *44%* | *49%* | 57% | *74%* | 82% | *87%* | *92%* | *94%* | *97%* | *98%* |
| **TD1127** | 278 | 18% | 7% | 15% | 25% | 46% | 53% | 56% | 72% | 80% | 86% | 91% | 93% | 95% | 97% |
| **TD1148** 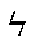 | *280* | 0% | 0% | 0% | 0% | 0% | 0% | 0% | 0% | 0% | 0% | 0% | 0% | 0% | 0% |
| | | *36%* | 27% | *61%* | 71% | *86%* | *90%* | *90%* | *91%* | *93*% | *94*% | 94% | *95*% | *95*% | 96% |
| **TD1160** 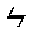 | *280* | - | - | - | - | 0% | - | - | 0% | - | - | - | - | - | 0% |
| | | | | | | *85*% | | | 89% | | | | | | *91%* |
| **TD1176** | *285* | 73% | 60% | 82% | 85% | 92% | 93% | 91% | 94% | 96% | 96% | 96% | 96% | 97% | 96% |
| **TD1204** | 278 | 0% | 0% | 0% | 1 % | 4% | 5% | 5% | 10% | 17% | 23% | 39% | 62% | 76% | 83% |

| | | | | | | | | | | | | | | | |
|---|---|---|---|---|---|---|---|---|---|---|---|---|---|---|---|
| Legend: ^{↓} Obtained data might not accurately reflect abundance of *1,5*-cz-[Ln(**Lig**)] in the whole mixture as non-negligible variation of overall signal intensity was observed throughout the series. ↑ Propionate pendants arms detach during course of the reaction, yielding a mixture of *1,4*-cz-**TD801** and 1,5- **TD801** and their corresponding derivatives with either one or both propionate arms removed. n. d. Could not be precisely determined due to signal overlapping and/or decomplexation during HPLC analysis. ^{∗} Sum of two diastereoisomers. ^{/} Performed in a mixture of 50% *i*-PrOH in aq. MOPS/NaOH Buffer pH 7.0. ^{‡} Pendant arms of *1,5*-cz-[Ln(**Lig**)] are coordinated in deprotonated iminol form (each bearing -1 charge). As a consequence, these species undergo hydrolysis to acetate arms (i.e. *in situ* yielding *1,5*-cz-[Ln(**TD647**)]). err. Accidental evaporation of the whole reaction solvent due to untighten vial cap. 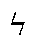 Values in italics: *1,5*-cz-[Ln(**Lig**)] with hydrolysed group in the side chain (Me ester in the case of **TD1092,** *i*-Pr ester in the case of **TD1148,** *t*-Bu ester in the case of **TD1160** and Boc in the case of **TD1105).** - Not performed. | | | | | | | | | | | | | | | |

**Chart 3.** Formation of *1,5*-cz-[Ln(**Lig**)] from **Lig** and Ln^{III} ions *(metal dependent click-zip):* conversion determined after 1 hour at 80 °C (**A**) and 18 hours at 80 °C (**B**) by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. **Lig** (1.0 equiv.); 1.0 mM aq. Ln^{III} (2.0 equiv.); 50 mM aq. MES/NaOH buffer pH 5.2 (100 equiv.). Performed in glass vials with magnetic stirrers. Metal ions are sorted by decreasing ionic radius.

| | | | | | | | | | | | | | | | |
|---|---|---|---|---|---|---|---|---|---|---|---|---|---|---|---|
| **A** | *λ, nm* | La^{III} | Ce^{III} | Pr^{III} | Nd^{III} | Sm^{III} | Eu^{III} | Gd^{III} | Tb^{III} | Dy^{III} | Ho^{III} | Er^{III} | Tm^{III} | Yb^{III} | Lu^{III} |
| **TD750** | *270* | - | - | - | - | - | - | 3% | - | - | - | - | - | - | 19% |
| **B** | *λ, nm* | La^{III} | Ce^{III} | Pr^{III} | Nd^{III} | Sm^{III} | Eu^{III} | Gd^{III} | Tb^{III} | Dy^{III} | Ho^{III} | Er^{III} | Tm^{III} | Yb^{III} | Lu^{III} |
| **TD750** | *270* | - | - | - | - | - | - | 9% | - | - | - | - | - | - | 71% |

| | | | | | | | | | | | | | | | |
|---|---|---|---|---|---|---|---|---|---|---|---|---|---|---|---|
| Legend: - Not performed. | | | | | | | | | | | | | | | |

**Chart 4.** Formation of *1,5*-cz-[M(**Lig**)] from **Lig** and M^{III} ions (*metal dependent click-zip):* conversion determined after 1 hour at 80 °C by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. **Lig** (1.0 equiv.); 1.0 mM aq. M^{III} (2.0 equiv.); 50 mM aq. MOPS/NaOH buffer pH 7.0 (100 equiv.). Performed in glass vials with magnetic stirrers. Metal ions are sorted by decreasing ionic radius.

| **Lig** | *λ, nm* | ^{†}Bi^{III} | Y^{III} | In^{III} | Sc^{III} | Ru^{III} | Ir^{III} | Ga^{III} |
|---|---|---|---|---|---|---|---|---|
| **TD425** | 275 | - | 0% | - | 0% | - | - | - |
| **TD556** | 275 | - | 22% | - | 2% | - | - | - |
| **TD582** | 275 | - | 11% | - | 0% | - | - | - |
| **TD604** | *280* | - | 1% | - | 1% | - | - | - |
| **TD639** | 275 | - | 0% | - | 0% | - | - | - |
| **TD647** | *280* | 29% | 20% | 0% | 7% | - | - | 0% |
| **TD669** | 275 | - | 0% | - | 0% | - | - | - |
| **TD714** ^{∗} | *270* | - | 22% | - | prec. | - | - | - |
| **TD722** | *280* | - | 9% | - | 3% | - | - | - |
| **TD728** ^{/} | 275 | 5% | 1% | - | 0% | 0% | 0% | - |
| **TD742** | 285 | - | 22% | - | 7% | - | - | - |
| **TD764** | *278* | 31% | - | - | - | - | - | - |
| **TD778** ^{‡} | 285 | - | 17% | - | 0% | - | - | - |
| **TD779** ^{‡} | *280* | - | 4% | - | 0% | - | - | - |
| **TD801** | 275 | - | 0% | - | 0% | - | - | - |
| **TD827** ^{/} | 275 | - | 42% | - | 2% | - | - | - |
| **TD921** | 275 | - | 0% | - | 0% | - | - | - |
| **TD943** | *280* | - | 1% | - | 0% | - | - | - |
| **TD944** | *270* | - | 1% | - | 0% | - | - | - |
| **TD959** | 275 | - | 1% | - | 0% | - | - | - |
| **TD992** | 275 | - | 2% | - | 0% | - | - | - |
| **TD1054** | *280* | - | 16% | - | 4% | - | - | - |
| **TD1063** | *278* | - | 20% | - | 1% | - | - | - |
| **TD1063** | *278* | - | 20% | - | 1% | - | - | - |
| **TD1092** 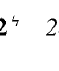 | *280* | - | 6% | - | 1% | - | - | - |
| | | | *12%* | | *0%* | | | |
| **TD1105** 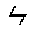 | 275 | - | 2% | - | 0*%* | - | - | - |
| | | | *1*% | | *0%* | | | |
| TD1127 | 278 | - | 2% | - | 0% | - | - | - |
| **TD1148** 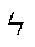 | *280* | - | 18% | - | 0% | - | - | - |
| | | | *3%* | | *0%* | | | |
| **TD1160** 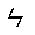 | *280* | - | 1% | - | - | - | - | - |
| | | | *17*% | | | | | |
| **TD1176** | *285* | - | 30% | - | 6% | - | - | - |
| **TD1204** | *278* | - | 3% | - | 0% | - | - | |

| | | | | | | | | |
|---|---|---|---|---|---|---|---|---|
| Legend: prec. Could not be reasonably determined due to formation of precipitate. ^{∗} Sum of two diastereoisomers. ^{/} Performed in a mixture of 50% *i*-PrOH in aq. MOPS/NaOH Buffer pH 7.0. ^{†} Performed in a mixture of 4% DMSO in aq. MOPS/NaOH Buffer pH 7.0. ^{‡} Pendant arms of *1,5*-cz-[Ln(**Lig**)] are coordinated in deprotonated iminol form (each bearing -1 charge). As a consequence, these species undergo hydrolysis to acetate arms (i.e. *in situ* yielding *1,5*-cz-[Ln(**TD647**)]). 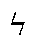 Values in italics: *1,5*-cz-[Ln(**Lig**)] with hydrolysed group in the side chain (Me ester in the case of **TD1092,** *i*-Pr ester in the case of **TD1148,** *t*-Bu ester in the case of **TD1160** and Boc in the case of **TD1105).** - Not performed. | | | | | | | | |

**Chart 5.** Formation of *1,5*-cz-[M(**Lig**)] from **Lig** and M^{III} *(metal dependent click-zip):* conversion determined after 1 week at 80 °C by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. **Lig** (1.0 equiv.); 1.0 mM aq. M^{III} (2.0 equiv.); 50 mM aq. MOPS/NaOH buffer pH 7.0 (100 equiv.). Performed in glass vials with magnetic stirrers. Metal ions are sorted by decreasing ionic radius.

| **Lig** | *λ, nm* | ^{†}Bi^{III} | Y^{III} | In^{III} | Sc^{III} | Ru^{III} | Ir^{III} | Ga^{III} |
|---|---|---|---|---|---|---|---|---|
| **TD425** | *275* | - | 0% | - | 0% | - | - | - |
| **TD556** | *275* | - | 95% | - | 23% | - | - | - |
| **TD582** | *275* | - | 68% | - | 0% | - | - | - |
| **TD604** | *280* | - | 58% | - | 10% | - | - | - |
| **TD639** | *275* | - | 0% | - | 0% | - | - | - |
| **TD647** | *280* | 73% | 93% | 8% | 35% | - | - | 0% |
| **TD669** | *275* | - | 12% | - | 0% | - | - | - |
| **TD714** ^{∗} | *270* | - | 89% | - | prec. | - | - | - |
| **TD722** | *280* | - | 71% | - | 14% | - | - | - |
| **TD728** ^{/} | *275* | 29% | 50% | - | 2% | 0% | 0% | - |
| **TD742** | *285* | - | 94% | - | 52% | - | - | - |
| **TD764** | *278* | 85% | 95% | - | 5% | - | - | - |
| **TD778** ^{‡} | *285* | - | 27% | - | 0% | - | - | - |
| **TD779** ^{‡} | *280* | - | 0% | - | 0% | - | - | - |
| **TD801** | *275* | - | 0% | - | 0% | - | - | - |
| **TD827** ^{/} | *275* | - | 96% | - | 55% | - | - | - |
| **TD921** | *275* | - | 14% | - | 0% | - | - | - |
| **TD943** | *280* | - | 74% | - | 5% | - | - | - |
| **TD944** | *270* | - | 63% | - | 4% | - | - | - |
| **TD959** | *275* | - | 82% | - | 1% | - | - | - |
| **TD992** | *275* | - | 87% | - | 2% | - | - | - |
| **TD1054** | *280* | - | 80% | - | 61% | - | - | - |
| **TD1063** | *278* | - | 91% | - | 8% | - | - | - |
| $TD 1092$ | *280* | - | 0% | - | 0% | - | - | - |
| | | | 95% | | 17% | | | |
| **TD1105** 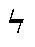 | *275* | - | 0% | - | 0% | - | - | - |
| | | | 87% | | *60* | | | |
| **TD1127** | *278* | - | 83% | - | 29% | - | - | - |
| **TD1148** 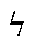 | *280* | - | 0% | - | 0% | - | - | - |
| | | | 94% | | 11% | | | |
| **TD1160** | *280* | - | 0% | - | - | - | - | - |
| | | | *90%* | | | | | |
| **TD1176** | *285* | - | 95% | - | 31% | - | - | - |
| **TD1204** | *278* | - | 22% | - | 16% | - | - | - |

| | | | | | | | | |
|---|---|---|---|---|---|---|---|---|
| Legend: prec. Could not be reasonably determined due to formation of precipitate. ^{∗} Sum of two diastereoisomers. ^{/} Performed in a mixture of 50% *i*-PrOH in aq. MOPS/NaOH Buffer pH 7.0. ^{†} Performed in a mixture of 4% DMSO in aq. MOPS/NaOH Buffer pH 7.0. ^{‡} Pendant arms of *1,5*-cz-[Ln(**Lig**)] are coordinated in deprotonated iminol form (each bearing -1 charge). As a consequence, these species undergo hydrolysis to acetate arms (i.e. *in situ* yielding *1,5*-cz-[Ln(**TD647**)]). 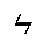 Values in italics: *1,5*-cz-[Ln(**Lig**)] with hydrolysed group in the side chain (Me ester in the case of **TD1092,** *i*-Pr ester in the case of **TD1148,** t-Bu ester in the case of **TD1160** and Boc in the case of **TD1105**). - Not performed. | | | | | | | | |

**Chart 6.** Formation of *1,5*-cz-[M(**Lig**)] from **Lig** and M^{II} ions (*metal dependent click-zip*): conversion determined after 1 hour at 80 °C by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. **Lig** (1.0 equiv.); 1.0 mM aq. M^{II} (2.0 equiv.); 50 mM aq. MOPS/NaOH buffer pH 7.0 (100 equiv.). Performed in glass vials with magnetic stirrers. Metal ions are sorted by decreasing ionic radius.

| **Lig** | *λ, nm* | Ba^{II} | Pb^{II} | Sr^{II} | Ca^{II} | Cd^{II} | Pd^{II} | Mn^{II} | Pt^{II} | Zn^{II} | Cu^{II} | Ni^{II} |
|---|---|---|---|---|---|---|---|---|---|---|---|---|
| **TD639** | *275* | 0% | - | 0% | 0% | - | - | - | - | - | - | - |
| **TD647** | *280* | 0% | 2% | 0% | 16% | 64% | - | - | - | 0% | prec. | 0% |
| **TD714** ^{∗} | *270* | 0% | - | 0% | 0% | 89% | - | - | - | - | - | - |
| **TD728** ^{/} | *275* | - | 0% | - | 0% | 1% | 1% | 0% | 0% | - | - | - |
| **TD764** | *278* | - | 1% | - | 16% | 53% | 10% | 1% | - | - | - | - |
| **TD921** | *275* | 0% | - | 0% | 0% | 92% | - | - | - | - | - | - |
| **TD959** | *275* | - | - | - | 1% | 2% | - | - | - | - | - | - |

| | | | | | | | | | | | | |
|---|---|---|---|---|---|---|---|---|---|---|---|---|
| Legend: prec. Could not be reasonably determined due to formation of precipitate. ^{∗} Sum of two diastereoisomers. ^{/} Performed in a mixture of 50% *i*-PrOH in aq. MOPS/NaOH Buffer pH 7.0. - Not performed. | | | | | | | | | | | | |

**Chart 7.** Formation of *1,5*-cz-[M(**Lig**)] from **Lig** and M^{II} ions (*metal dependent click-zip*): conversion determined after 1 week at 80 °C by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. **Lig** (1.0 equiv.); 1.0 mM aq. M^{II} (2.0 equiv.); 50 mM aq. MOPS/NaOH buffer pH 7.0 (100 equiv.). Performed in glass vials with magnetic stirrers. Metal ions are sorted by decreasing ionic radius.

| **Lig** | *λ, nm* | Ba^{II} | Pb^{II} | Sr^{II} | Ca^{II} | Cd^{II} | Pd^{II} | Mn^{II} | Pt^{II} | Zn^{II} | Cu^{II} | Ni^{II} |
|---|---|---|---|---|---|---|---|---|---|---|---|---|
| **TD639** | *275* | 0% | - | 0% | 0% | - | - | - | - | - | - | - |
| **TD647** | *280* | 0% | 54% | 0% | 87% | 86% | - | - | - | 0% | prec. | 0% |
| **TD714** ^{∗} | *270* | 0% | - | 0% | 6% | 92% | - | - | - | - | - | - |
| **TD728** ^{/} | *275* | - | 14% | - | 37% | 36% | 1% | 1% | 0% | - | - | - |
| **TD764** | *278* | - | 37% | - | 95% | 96% | 9% | 35% | - | - | - | - |
| **TD921** | *275* | 0% | - | 0% | 0% | 96% | - | - | - | - | - | - |
| **TD959** | *275* | - | - | - | 83% | 96% | - | - | - | - | - | - |

| | | | | | | | | | | | | |
|---|---|---|---|---|---|---|---|---|---|---|---|---|
| Legend: prec. Could not be reasonably determined due to formation of precipitate. ^{∗} Sum of two diastereoisomers. ^{/} Performed in a mixture of 50% *i*-PrOH in aq. MOPS/NaOH Buffer pH 7.0. - Not performed. | | | | | | | | | | | | |

**Chart 8.** Formation of *1,5*-cz-[Ln(**Lig**)] from *1,5*-cz-**Lig** *(metal dependent direct complexation):* conversion determined after 1 hour at 80 °C (**A**), 1 week at 80 °C (**B**) and - 6 months at 80 °C (**B**) by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. *1,5*-cz-**Lig** (1.0 equiv.); 1.0 mM aq. Ln^{III} (2.0 equiv.); 50 mM aq. MOPS/NaOH buffer pH 7.0 (100 equiv.). Performed in glass vials with magnetic stirrers. Metal ions are sorted by decreasing ionic radius.

| **A** | *λ, nm* | La^{III} | Ce^{III} | Pr^{III} | Nd^{III} | Sm^{III} | Eu^{III} | Gd^{III} | Tb^{III} | Dy^{III} | Ho^{III} | Er^{III} | Tm^{III} | Yb^{III} | Lu^{III} |
|---|---|---|---|---|---|---|---|---|---|---|---|---|---|---|---|
| **TD650** | *280* | 0% | 0% | 0% | 0% | 0% | 0% | 0% | 0% | 0% | 0% | 0% | 0% | 0% | 0% |
| **B** | *λ, nm* | La^{III} | Ce^{III} | Pr^{III} | Nd^{III} | Sm^{III} | Eu^{III} | Gd^{III} | Tb^{III} | Dy^{III} | Ho^{III} | Er^{III} | Tm^{III} | Yb^{III} | Lu^{III} |
| **TD650** | *280* | 0% | 0% | 0% | 0% | 0% | 0% | 0% | 0% | 0% | 0% | 0% | 0% | 0% | 0% |
| **c** | *λ, nm* | La^{III} | Ce^{III} | Pr^{III} | Nd^{III} | Sm^{III} | Eu^{III} | Gd^{III} | Tb^{III} | Dy^{III} | Ho^{III} | Er^{III} | Tm^{III} | Yb^{III} | Lu^{III} |
| **TD650** | *280* | - | - | - | - | - | - | 0% | - | - | - | - | - | - | 0% |

| | | | | | | | | | | | | | | | |
|---|---|---|---|---|---|---|---|---|---|---|---|---|---|---|---|
| Legend: - Not performed. | | | | | | | | | | | | | | | |

**Chart 9.** Formation of *1,5*-cz-[Ln(**Lig**)] from *1,5*-cz-**Lig** *(metal dependent direct complexation):* conversion determined after 1 min at RT (**A**), 1 hour at RT (**B**), 1 hour at 80 °C (**C**) and 1 week at 80 °C (**D**) by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. *1,5*-cz-**Lig** (1.0 equiv.); 1.0 mM aq. M^{II} *or* M^{III} (2.0 equiv.); 50 mM aq. MOPS/NaOH buffer pH 7.0 (100 equiv.). Performed in glass vials with magnetic stirrers. Metal ions are sorted by decreasing ionic radius.

| **A** | *λ, nm* | Ba^{II} | Pb^{II} | Sr^{II} | ^{†}Bi^{III} | Ca^{II} | Cd^{II} | Y^{III} | In^{III} | Sc^{III} | Zn^{II} | Cu^{II} | Ni^{II} | Ga^{II} |
|---|---|---|---|---|---|---|---|---|---|---|---|---|---|---|
| **TD650** | *280* | - | 98% | - | - | - | - | - | - | - | - | 43% | - | - |
| **B** | *λ, nm* | Ba^{II} | Pb^{II} | Sr^{II} | ^{†}Bi^{III} | Ca^{II} | Cd^{II} | Y^{III} | In^{III} | Sc^{III} | Zn^{II} | Cu^{II} | Ni^{II} | Ga^{II} |
| **TD650** | *280* | - | 99% | - | 2% | 0% | 58% | - | - | - | 59% | 47% | 0% | - |
| **C** | *λ, nm* | Ba^{II} | Pb^{II} | Sr^{II} | ^{†}Bi^{III} | Ca^{II} | Cd^{II} | Y^{III} | In^{III} | Sc^{III} | Zn^{II} | Cu^{II} | Ni^{II} | Ga^{II} |
| **TD650** | *280* | - | 98% | - | 96% | 23% | 98% | - | - | - | 91% | 49% | 9% | - |
| **D** | *λ, nm* | Ba^{II} | Pb^{II} | Sr^{II} | ^{†}Bi^{III} | Ca^{II} | Cd^{II} | Y^{III} | In^{III} | Sc^{III} | Zn^{II} | Cu^{II} | Ni^{II} | Ga^{II} |
| **TD650** | *280* | 0% | 98% | 0% | 90% | 96% | 99% | 0% | 0% | 0% | 99% | 78% | 11% | 0% |

| | | | | | | | | | | | | | | |
|---|---|---|---|---|---|---|---|---|---|---|---|---|---|---|
| Legend: ^{†} Performed in a mixture of ~4% DMSO in aq. MOPS/NaOH Buffer pH 7.0. - Not performed. | | | | | | | | | | | | | | |

**Chart 10**. Formation of *1,4*-cz-[Ln(**Lig**)] from *1,4*-cz-**Lig** *(metal dependent direct complexation):* conversion determined after 1 week at 80 °C (**A**) by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. *1,4*-cz-**Lig** (1.0 equiv.); 1.0 mM aq. Ln^{III} (2.0 equiv.); 50 mM aq. MOPS/NaOH buffer pH 7.0 (100 equiv.). Performed in glass vials with magnetic stirrers. Metal ions are sorted by decreasing ionic radius.

| **A** | *λ, nm* | La^{III} | Ce^{III} | Pr^{III} | Nd^{III} | Sm^{III} | Eu^{III} | Gd^{III} | Tb^{III} | Dy^{III} | Ho^{III} | Er^{III} | Tm^{III} | Yb^{III} | Lu^{III} |
|---|---|---|---|---|---|---|---|---|---|---|---|---|---|---|---|
| **TD622** | *275* | 0% | 0% | 3% | 6% | 34% | 45% | 46% | 61% | 66% | 68% | 70% | 71% | 51% | 53% |

**Chart 11.** Formation of *1,4*-cz-[Ln(**Lig**)] from *1,4*-cz-**Lig** *(metal dependent direct complexation):* conversion determined after 1 hour at 80 °C (**A**) and 1 week at 80 °C (**B**) by analytical HPLC (H₂O-MeCN system with 0.1% FA additive; detection at *λ*; rounded to integers; aliquots from reaction mixtures were diluted with H₂O, vortexed, centrifuged and then injected to HPLC). Initial component concentrations: 0.5 mM aq. *1,4*-cz-**Lig** (1.0 equiv.); 1.0 mM aq. M^{II} *or* M^{III} (2.0 equiv.); 50 mM aq. MOPS/NaOH buffer pH 7.0 (100 equiv.). Performed in glass vials with magnetic stirrers. Metal ions are sorted by decreasing ionic radius.

| **A** | *λ, nm* | Ba^{II} | Pb^{II} | Sr^{II} | ^{†}Bi^{III} | Ca^{II} | Cd^{II} | Y^{III} | In^{III} | Sc^{III} | Zn^{II} | Cu^{II} | Ni^{II} | Ga^{II} |
|---|---|---|---|---|---|---|---|---|---|---|---|---|---|---|
| **TD622** | *275* | - | 98% | - | 98% | 20% | 98% | - | - | - | 88% | 42% | 41% | - |
| **B** | *λ, nm* | Ba^{II} | Pb^{II} | Sr^{II} | ^{†}Bi^{III} | Ca^{II} | Cd^{II} | Y^{III} | In^{III} | Sc^{III} | Zn^{II} | Cu^{II} | Ni^{II} | Ga^{II} |
| **TD622** | 275 | 0% | 99% | 0% | 91% | 21% | 99% | 51% | 13% | 0% | 99% | 48% | 44% | 8% |

| | | | | | | | | | | | | | | |
|---|---|---|---|---|---|---|---|---|---|---|---|---|---|---|
| Legend: ^{†} Performed in a mixture of ~4% DMSO in aq. MOPS/NaOH Buffer pH 7.0. - Not performed. | | | | | | | | | | | | | | |

**Chart 12.** Formation of *1,5*-cz-[Lu(**Lig**)] from **Lig** (*metal dependent click-zip*) with various absolute concentrations: conversion determined after 1 hour at 80 °C (**A**), 1 day at 80 °C (**B**) and 1 week at 80 °C (**C**) by analytical HPLC (H₂O-MeCN system with 0.1% FA additive; detection at 280 nm; rounded to integers; aliquots from reaction mixtures were diluted with H₂O, vortexed, centrifuged and then injected to HPLC). Performed with constant ratio of reagents (**Lig**: Lu^{III} : aq. MOPS/NaOH buffer pH 7.0 = 1.0: 1.5 : 50). Performed in glass vials with magnetic stirrers.

| | | | | | |
|---|---|---|---|---|---|
| **Lig,** mM | 5.0 × 10¹ | 5.0 × 10⁰ | 5.0 × 10⁻¹ | 5.0 × 10⁻² | 5.0 × 10⁻³ |
| Lu^{III}, mM | 7.5 × 10¹ | 7.5 × 10⁰ | 7.5 × 10⁻¹ | 7.5 × 10⁻² | 7.5 × 10⁻³ |
| aq. MOPS/NaOH buffer pH 7.0, mM | 2.5 × 10³ | 2.5 × 10² | 2.5 × 10¹ | 2.5 × 10⁰ | 2.5 × 10⁻¹ |
| **TD647** (**A**) | 71% | 74% | 70% | 60% | 15% |
| **TD647** (**B**) | 84% | 96% | 97% | 96% | 59% |
| **TD647** (**C**) | 86% | 96% | 97% | 96% | 57% |

**Chart 13.** pH dependent formation of *1,5-* and *1,4*-cz-**Lig** isomers from **Lig** (*pH dependent click-zip*): conversion determined after 1 hour at 80 °C (**A**) and 1 day at 80 °C (**B**) by analytical HPLC (H₂O-MeCN system with 0.1% FA additive; detection at *λ*; rounded to integers; aliquots from reaction mixtures were diluted with H₂O, vortexed and then injected to HPLC). Initial component concentrations: 0.5 mM aq. **Lig** (1.0 equiv.); 1.0 M HClO₄ (pH ~0)/0.1 M HClO₄ (pH ~1)/50 mM aq. buffer (100 equiv.). Performed in glass vials with magnetic stirrers.

| **A** | *λ, nm* | Syste m | aq. HClO₄ | | aq. Citrate/NaOH buffer | | | | | | | aq. Borate/NaOH buffer | | |
|---|---|---|---|---|---|---|---|---|---|---|---|---|---|---|
| | | pH | ~0 | ~1 | 2.2 | 3.0 | 3.8 | 4.6 | 5.4 | 6.2 | 7.0 | 8.6 | 9.4 | 10.2 |
| | *275* | *1,5* | 13% | 13% | 13% | 10% | 3% | 1% | 1% | 0% | 0% | 0% | 1% | 1% |
| **TD42 5** | | *1,4* | 0% | 0% | 0% | 0% | 0% | 0% | 0% | 0% | 0% | 0% | 0% | 0% |
| **TD64 7** | *280* | *1,5* | 9% | 44% | 17% | 6% | 1% | 1% | 1% | 1% | 1% | 2% | 8% | 17% |
| | | *1,4* | 2% | 3% | 2% | 1% | 1% | 1% | 1% | 1% | 2% | 3% | 3% | 2% |

| **B** | *λ, nm* | Syste m | aq. HClO₄ | | aq. Citrate/NaOH buffer | | | | | | | aq. Borate/NaOH buffer | | |
|---|---|---|---|---|---|---|---|---|---|---|---|---|---|---|
| | | pH | ~0 | ~1 | 2.2 | 3.0 | 3.8 | 4.6 | 5.4 | 6.2 | 7.0 | 8.6 | 9.4 | 10.2 |
| **TD42 5** | *275* | *1,5* | 88% | 90% | 93% | 89% | 57% | 15% | 6% | 6% | 8% | 10% | 11% | 12% |
| | | *1,4* | 0% | 0% | 0% | 0% | 0% | 0% | 0% | 1% | 4% | 5% | 4% | 2% |
| **TD64 7** | *280* | *1,5* | 72% | 91% | 88% | 69% | 22% | 13% | 12% | 11% | 19% | 26% | 60% | 80% |
| | | *1,4* | 8% | 5% | 6% | 6% | 6% | 5% | 6% | 12% | 23% | 32% | 17% | 5% |

**Chart 14.** Alkali metal dependent formation of *1,5*-cz-**Lig** and *1,4*-cz-**Lig** isomers from **Lig** (*alkali metal dependent click-zip*) at pH 10.2: conversion determined after 1 hour at 80 °C (**A**) and 1day at 80 °C (**B**) by analytical HPLC (H₂O-MeCN system with 0.1% FA additive; detection at *λ*; rounded to integers; aliquots from reaction mixtures were diluted with H₂O, vortexed and then injected to HPLC). Initial component concentrations: 0.5 mM aq. **Lig** (1.0 equiv.); 50 mM aq. Borate/(M^{I})₂CO₃ buffer (M^{I}= Li^{I}- Cs^{I}; 100 equiv.). Performed in plastic Eppendorf test tubes with magnetic stirrers.

| **A** | *λ, nm* | System | aq. Borate/(M^{I})₂CO₃ buffer (pH 10.2) | | | | |
|---|---|---|---|---|---|---|---|
| | | M^{I} | Li^{I} | Na^{I} | K^{I} | Rb^{I} | Cs^{I} |
| **TD425** | *275* | *1,5* | 0% | 1% | 0% | 1% | 1% |
| | | *1,4* | 0% | 0% | 1% | 1% | 1% |
| **TD647** | *280* | *1,5* | 0% | 15% | 1% | 0% | 0% |
| | | *1,4* | 0% | 1% | 2% | 2% | 2% |
| **TD921** | *275* | *1,5* | 12% | 30% | 2% | 2% | 2% |
| | | *1,4* | 1% | 2% | 2% | 2% | 2% |

| **B** | *λ, nm* | System | aq. Borate/(M^{I})₂CO₃ buffer (pH 10.2) | | | | |
|---|---|---|---|---|---|---|---|
| | | M^{I} | ^{Ψ}Li^{I} | Na^{I} | K^{I} | Rb^{I} | Cs^{I} |
| **TD425** | *275* | *1,5* | 2% | 9% | 10% | 10% | 11% |
| | | *1,4* | 0% | 1% | 10% | 10% | 10% |
| **TD647** | *280* | *1,5* | 9% | 85% | 10% | 6% | 7% |
| | | *1,4* | 2% | 1% | 40% | 41% | 39% |
| **TD582** | *275* | *1,5* | 53% | 69% | 11% | 10% | 10% |
| | | *1,4* | 3% | 2% | 25% | 25% | 26% |
| **TD581** | *280* | *1,5* | 10% | 25% | 7% | 7% | 6% |
| | | *1,4* | 19% | 26% | 32% | 32% | 31% |
| **TD703** | *270* | *1,5* | 24% | 75% | 14% | 14% | 13% |
| | | *1,4* | 14% | 10% | 20% | 20% | 20% |
| **TD714** | *270* | *1,5* | 15% | 81% | 10% | 9% | 9% |
| | | *1,4* | 12% | 7% | 46% | 45% | 44% |
| **TD921** | *275* | *1,5* | 86% | 89% | 23% | 21% | 21% |
| | | *1,4* | 6% | 4% | 31% | 31% | 31% |

| | | | | | | | |
|---|---|---|---|---|---|---|---|
| Legend: ^{Ψ} Reaction mixture contained a visible precipitate, which was shown to be analyte free (all forms). | | | | | | | |

**Chart 15.** Relaxitivities of Gd^{III} chelates **TD451** and **TD527** determined by standard inversion recovery pulse sequence on relaxometer (the minispec mq20 NMR analyzer; 0.5T; *Bruker*) at 40.0 °C (concentration of chelates: 1.0 mM in aq. MOPS/NaOH buffer, pH 7.0).

| | | |
|---|---|---|
| species | formally | relaxivity, mM⁻¹s⁻¹ |
| **TD451** | [Gd(**TD647**)]) | 2.39 |
| **TD527** | *1,5-cz-*[Gd(**TD647**)]) | 1.48 |

**Chart 17.** Dissociation of chelates in aq. 6.0 M HCl at 80 °C (*acid decomplexation kinetic inertness*): fraction of intact chelate determined after 1 hour, 1 day, 1 week and 1 month by analytical HPLC (H₂O-MeCN system with 0.1% FA additive; detection at 280 nm; rounded to integers; aliquots from reaction mixtures were appropriately diluted to prevent re-complexation, vortexed, centrifuged and then injected to HPLC). Performed in glass vials with magnetic stirrers.

| chelate | formally | *λ, nm* | 1 hour | 1 day | 1 week | 1 month | *t*_{1/2}, hours *^{f}* |
|---|---|---|---|---|---|---|---|
| **TD875** ^{A} | *1,4*-cz-[Gd^{III}(**TD647**)] | 278 | 0% | 0% | 0% | 0% | too unstable |
| **TD874** ^{A} | *1,4*-cz-[Lu^{III}(**TD647**)] | 278 | 0% | 0% | 0% | 0% | too unstable |
| **TD900** ^{A} | *1,4-*cz-[Cd^{II}(**TD647**)] | 278 | 0% | 0% | 0% | 0% | too unstable |
| **TD651** ^{B} | *1,5*-cz-[La^{III}(**TD647**)] | 278 | 0% | 0% | 0% | 0% | too unstable |
| **TD870** ^{B} | *1,5*-cz-[Ce^{III}(**TD647**)] | 278 | 0% | 0% | 0% | 0% | too unstable |
| **TD869** ^{B} | *1,5*-cz-[Pr^{III}(**TD647**)] | 278 | 0% | 0% | 0% | 0% | too unstable |
| **TD868** ^{B} | *1,5*-cz-[Nd^{III}(**TD647**)] | 278 | 0% | 0% | 0% | 0% | too unstable |
| **TD867** ^{B} | *1,5-*cz-[Sm^{III}(**TD647**)] | 278 | 0% | 0% | 0% | 0% | too unstable |
| **TD599** ^{B} | *1,5*-cz-[Eu^{III}(**TD647**)] | 278 | 0% | 0% | 0% | 0% | too unstable |
| **TD527** ^{B} | *1,5*-cz-[Gd^{III}(**TD647**)] | 278 | 2% | 0% | 0% | 0% | **1.7E⁻¹** |
| **TD600** ^{B} | *1,5*-cz-[Tb^{III}(**TD647**)] | 278 | 72% | 0% | 0% | 0% | **2.7E⁰** |
| **TD866** ^{B} | *1,5*-cz-[Dy^{III}(**TD647**)] | 278 | 95% | 25% | 0% | 0% | **1.2E¹**±1.3E⁻¹ |
| **TD648** ^{B} | *1,5*-cz-[Ho^{III}(**TD647**)] | 278 | 99% | 81% | 17% | 0% | **6.8E¹**±2.7E⁰ |
| **TD865** ^{B} | *1,5*-cz-[Er^{III}(**TD647**)] | 278 | 99% | 95% | 71% | 24% | **3.4E²**±2.5E⁰ |
| **TD601** ^{B} | *1,5*-cz-[Tm^{III}(**TD647**)] | 278 | 100% | 99% | 92% | 69% | **1.4E³**±1.2E¹ |
| **TD602** ^{B} | *1,5-*cz-[Yb^{III} (**TD647**)] | 278 | 100% | 100% | 98% | 90% | **4.7E³**±2.1E¹ |
| **TD522** ^{B} | *1,5*-cz-[Lu^{III}(**TD647**)]^{∞} | 278 | 100% | 100% | 99% | 98% | **3.3E⁴**±1.4E³ |
| **TD649** ^{B} | *1,5*-cz-[Y^{III}(**TD647**)] | 278 | 98% | 69% | 7% | 0% | **4.5E¹**±3.1E⁻¹ |
| **TD876** ^{A} | *1.5*-cz-[Pb^{II}(**TD647**)] | 278 | 0% | 0% | 0% | 0% | too unstable |
| **TD863** ^{A} | *1.5*-cz-[Ca^{II}**TD647**)] | 278 | 0% | 0% | 0% | 0% | too unstable |
| **TD880** ^{A} | *1.5*-cz-[Cd^{II}**TD647**)] | 278 | 43% | 0% | 0% | 0% | **1.2E⁰** |
| **TD890** ^{A} | *1.5*-cz-[Zn^{II}**D647**)] | 278 | 0% | 0% | 0% | 0% | too unstable |
| **TD624-La** ^{A,∗} | ^{↓} [La^{III}(*p*-**NO₂BnDOTA**)] | *285* | 0% | 0% | 0% | 0% | too unstable |
| **TD624-Gd** ^{A,∗} | ^{↓} [Gd^{III}(*p*-**NO₂BnDOTA**)] | *285* | 0% | 0% | 0% | 0% | too unstable |
| **TD624-Lu** ^{A,∗} | ^{↓} [Lu^{III}(*p*-**NO₂BnDOTA**)] | *285* | 0% | 0% | 0% | 0% | too unstable |
| **TD925** ^{B} | *1,5*-cz-[Gd^{III}(**TD921**)] | 275 | 77% | 0% | 0% | 0% | **2.6**E⁰ |
| **TD748** ^{B} | *1.5*-cz-[Lu^{III}(**TD742**)] ^{∞} | *285* | 100% | 100% | 100% | 97% | **1.5E⁴**±2.9E² |
| **TD735** ^{B} | *1,5*-cz-[Lu^{III}(**TD722**)] ^{∞} | *280* | 100% | 100% | 99% | 98% | **2.3E⁴**±8.3E² |
| **TD739** ^{B,∗} | - | 275 | 100% | 90% | 43% | 15% | **1.5E²**±2.6E¹ |
| **TD734** ^{B,∗} | *1,5*-cz-[Lu^{III}(**TD714**)] ^{∞} | *280* | 100% | 100% | 98% | 95% | **1.6E⁴**±1.5E³ |
| **TD737** ^{B} | *1,5*-cz-[Lu^{III}(**TD728**)] | *280* | 100% (87%/13%) 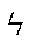 | 100% (3%/97%) 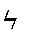 | 99% (0%/99%) 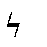 | 98% (0%/98%) 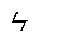 | - |

| | | | | | | | |
|---|---|---|---|---|---|---|---|
| Legend: ^{A} Aliquots (2 µL) were neutralized with 500 mM FA/NaOH buffer (*pH* 3.6; 200 µL) prior measurement. ^{B} Aliquots (2 µL) were neutralized with 500 mM MOPS/NaOH buffer (pH 7.0; 200 µL) prior measurement. *^{f}* Dissociation data were fitted by exponential decay (equation y = exp(-*x*/*t*); *y* = fraction of intact chelate; *x* = time; *t* = decay constant). Chelate stability is expressed as half-life *t*_{½} (in hours; highlighted bold), derived from decay constant (*t*½ = ln(2) × *t*). The error of the fitting is not displayed for single data point fitting. ^{∗} Sum of two diastereoisomers. ^{↓} Stock solutions preparation: In Eppendorf tube (0.5 mL), **2,2′,2ʺ,2‴-(2-(4-nitrobenzyl)-1,4,7,10-tetraazacyclododecane-1,4,7,10-tetrayl)tetraacetic acid tetrahydrochloride** (10.0 mM in H₂O; 100 µL; 1 µmol; 1.0 equiv.) was mixed with corresponding aq. **LnCl₃** (100 mM; 23.5 µL; 1.1 µmοl; 1.1 equiv.), H₂O (59 µL) and aq. **NaOH** (400 mM; 17.5 µL; 7.0 µmοl; 7.0 equiv.). The resulting mixtures were then stirred 16 h at RT (full conversion was confirmed by LC-MS) followed by centrifugation. Supernatants were then directly used as stock solutions for the dissociation assay. -- Isolated only as a click-zipped complex (no parent ligand exists). ^{∞} Additional data points were collected over the period of 6 months (included in the fitting). 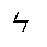 Values in brackets: chelate with intact TIPS group/chelate with hydrolysed TIPS group. - The half-life cannot be rigorously determined due to a parallel hydrolysis of TIPS group. | | | | | | | |

**Chart 18.** Dissociation of chelates in aq. 1.0 M HCl at 80 °C (*acid decomplexation kinetic inertness*): fraction of intact chelate determined after 1 hour, 1 day, 1 week and 1 month by analytical HPLC (H₂O-MeCN system with 0.1% FA additive; detection at 280 nm; rounded to integers; aliquots from reaction mixtures were neutralized with aq. MOPS/NaOH Buffer pH 7.0, vortexed, centrifuged and then injected to HPLC). Performed in glass vials with magnetic stirrers.

| chelate | | formally | *λ, nm* | 1 hour | 1 day | 1 week | 1 month | *t*_{½}, hours *^{f}* |
|---|---|---|---|---|---|---|---|---|
| | **TD875** ^{A} | *1,4*-cz-[Gd^{III}(**TD647**)] | 278 | 0% | 0% | 0% | 0% | too unstable |
| | **TD874** ^{A} | *1,4*-cz-[Lu^{III}(**TD647**)] | 278 | 0% | 0% | 0% | 0% | too unstable |
| | **TD900** ^{A} | *1,4*-cz-[Cd^{II}(**TD647**)] | 278 | 0% | 0% | 0% | 0% | too unstable |
| | **TD651** ^{B} | *1,5*-cz-[La^{III}(**TD647**)] | 278 | 0% | 0% | 0% | 0% | too unstable |
| | **TD870** ^{B} | *1,5*-cz-[Ce^{III}(**TD647**)] | 278 | 0% | 0% | 0% | 0% | too unstable |
| | **TD869** ^{B} | *1,5*-cz-[Pr^{III}(**TD647**)] | 278 | 0% | 0% | 0% | 0% | too unstable |
| | **TD868** ^{B} | *1,5*-cz-[Nd^{III}(**TD647**)] | 278 | 0% | 0% | 0% | 0% | too unstable |
| | **TD867** ^{B} | *1,5*-cz-[Sm^{III}(**TD647**)] | 278 | 62% | 0% | 0% | 0% | **1.5E⁰** |
| | **TD599** ^{B} | *1,5*-cz-[Eu^{III}(**TD647**)] | 278 | 92% | 10% | 0% | 0% | **7.3E⁰**±2.6E⁻¹ |
| | **TD527** ^{B} | *1,5*-cz-[Gd^{III}(**TD647**)] | 278 | 98% | 58% | 1% | 0% | **3.0E¹**±4.9E⁻¹ |
| | **TD600** ^{B} | *1,5*-cz-[Tb^{III}(**TD647**)] | 278 | 99% | 96% | 78% | 23% | **3.7E²**±3.2E¹ |
| | **TD866** ^{B} | *1,5*-cz-[Dy^{III}(**TD647**)] | 278 | 100% | 99% | 96% | 87% | **3.6E³**±5.0E¹ |
| | **TD648** ^{B} | *1,5*-cz-[Ho^{III}(**TD647**)] | 278 | 100% | 100% | 99% | 96% | **1.3E⁴**±2.6E² |
| | **TD865** ^{B} | *1,5*-cz-[Er^{III}(**TD647**)] | 278 | 100% | 100% | 100% | 99% | **5.9E⁴**±1.1E³ |
| | **TD601** ^{B} | *1,5*-cz-[Tm^{III}(**TD647**)] | 278 | 100% | 100% | 100% | 100% | too stable |
| | **TD602** ^{B} | *1,5*-cz-[Yb^{III}(**TD647**)] | 278 | 100% | 100% | 100% | 100% | too stable |
| | **TD522** ^{B} | *1,5*-cz-[Lu^{III}(**TD647**)] | 278 | 100% | 100% | 100% | 100% | too stable |
| | **TD649** ^{B} | *1,5*-cz-[Y^{III}(**TD647**)] | 278 | 100% | 100% | 98% | 92% | **5.9E³**±1.4E² |
| | **TD876** ^{A} | *1.5*-cz-[Pb^{II}(**TD647**)] | 278 | 0% | 0% | 0% | 0% | too unstable |
| | **TD863** ^{A} | *1.5*-cz-[Ca^{II}(**TD647**)] | 278 | 0% | 0% | 0% | 0% | too unstable |
| | **TD880** ^{A} | *1.5*-cz-[Cd^{II}(**TD647**)] | 278 | 100% | 99% | 90% | 63% | **1.1E³**±7.7E⁰ |
| | **TD890** ^{A} | *1.5*-cz-[Zn^{II}(**TD647**)] | 278 | 0% | 0% | 0% | 0% | too unstable |
| | **TD624-La** ^{A,∗} | [La^{III}(*p*-**NO₂BnDOTA**)] | *285* | 0% | 0% | 0% | 0% | too unstable |
| | **TD624-Gd** ^{A,∗} | [Gd^{III}(*p*-**NO₂BnDOTA**)] | *285* | 15% | 0% | 0% | 0% | **3.7E⁻¹** |
| | **TD624-Lu** ^{A,∗} | [Lu^{III}(*p*-**NO₂BnDOTA**)] | *285* | 12% | 0% | 0% | 0% | **3.3E⁻¹** |
| | **TD925** ^{B} | *1,5*-cz-[Gd^{III}(**TD921**)] | 275 | 100% | 82% | 21% | 0% | **7.7E¹**±2.3E⁰ |

| | | | | | | | | |
|---|---|---|---|---|---|---|---|---|
| Legend: ^{A} Aliquots (2 µL) were neutralized with 125 mM FA/NaOH buffer (*pH* 3.6; 200 µL) prior measurement. ^{B} Aliquots (2 µL) were neutralized with 500 mM MOPS/NaOH buffer (pH 7.0; 200 µL) prior measurement. *^{f}* Dissociation data were fitted by exponential decay (equation *y* = exp(-*x*/*t*); *y* = fraction of intact chelate; *x* = time; *t* = decay constant). Chelate stability is expressed as half-life *t*_{½} (in hours; highlighted bold), derived from decay constant (*t*½ = ln(2) × *t*). The error of the fitting is not displayed for single data point fitting. ^{∗} Sum of two diastereoisomers. ^{↓} Stock solutions preparation: In Eppendorf tube (0.5 mL), **2,2′,2ʺ,2‴-(2-(4-nitrobenzyl)-1,4,7,10-tetraazacyclododecane-1,4,7,10-tetrayl)tetraacetic acid tetrahydrochloride** (10.0 mM in H₂O; 100 µL; 1 µmol; 1.0 equiv.) was mixed with corresponding aq. **LnCl₃** (100 mM; 23.5 µL; 1.1 µmol; 1.1 equiv.), H₂O (59 µL) and aq. **NaOH** (400 mM; 17.5 µL; 7.0 µmοl; 7.0 equiv.). The resulting mixtures were then stirred 16 h at RT (full conversion was confirmed by LC-MS) followed by centrifugation. Supernatants were then directly used as stock solutions for the dissociation assay. | | | | | | | | |

**Chart 19.** Dissociation of chelates in aq. 0.1 M^{III} HCl at 80 °C (*acid decomplexation kinetic inertness*): fraction of intact chelate determined after 1 hour, 1 day, 1 week and 1 month by analytical HPLC (H₂O-MeCN system with 0.1% FA additive; detection at 280 nm; rounded to integers; aliquots from reaction mixtures were diluted with H₂O, vortexed and then injected to HPLC). Performed in glass vials with magnetic stirrers.

| chelate | | formally | *λ, nm* | 1 hour | 1 day | 1 week | 1 month | *t*_{½}*,* hours *^{f}* |
|---|---|---|---|---|---|---|---|---|
| | **TD875** ^{A} | *1,4*-cz-[Gd^{III}(**TD647**)] | 278 | 0% | 0% | 0% | 0% | too unstable |
| | **TD874** ^{A} | *1,4*-cz-[Lu^{III}(**TD647**)] | 278 | 0% | 0% | 0% | 0% | too unstable |
| | **TD900** ^{A} | *1,4*-cz-[Cd^{II}(**TD647**)] | 278 | 56% | 0% | 0% | 0% | **1.2E⁰** |
| | **TD651** ^{B} | *1,5-*cz-[La^{III}(**TD647**)] | 278 | 0% | 0% | 0% | 0% | too unstable |
| | **TD870** ^{B} | *1,*5-cz-[Ce^{III}(**TD647**)] | 278 | 0% | 0% | 0% | 0% | too unstable |
| | **TD869** ^{B} | *1,5*-cz-[Pr^{III}(**TD647**)] | 278 | 23% | 0% | 0% | 0% | **4.7E⁻¹** |
| | **TD868** ^{B} | *1,5*-cz-[Nd^{III}(**TD647**)] | 278 | 76% | 0% | 0% | 0% | **1.2E⁰** |
| | **TD867** ^{B} | *1,5*-cz-[Sml^{lIl}(**TD647**)] | 278 | 99% | 86% | 35% | 1% | **1.1E²**±7.5E^{~1} |
| | **TD599** ^{B} | *1,5*-cz-[Eu^{III}(**TD647**)] | 278 | 100% | 97% | 83% | 21% | **3.8E²**±5.8E¹ |
| | **TD527** ^{B} | *1,5*-cz-[Gd^{III}(**TD647**)] | 278 | 100% | 99% | 95% | 82% | **2.5E³**±3.7E¹ |
| | **TD600** ^{B} | *1,5*-cz-[Tb^{III}(**TD647**)] | 278 | 100% | 100% | 99% | 99% | **3.2E⁴**±3.3E³ |
| | **TD866** ^{B} | *1,5*-cz-[Dy^{III}(**TD647**)] | 278 | 100% | 100% | 100% | 100% | **9.5E⁴**±3.0E³ |
| | **TD648** ^{B} | *1,5*-cz-[Ho^{III}(**TD647**)] | 278 | 100% | 100% | 100% | 100% | too stable |
| | **TD865** ^{B} | *1,5*-cz-[Er^{III}(**TD647**)] | 278 | 100% | 100% | 100% | 100% | too stable |
| | **TD601** ^{B} | *1,5*-cz-[Tm^{III}(**TD647**)] | 278 | 100% | 100% | 100% | 100% | too stable |
| | **TD602** ^{B} | *1,5*-cz-[Yb^{III}(**TD647**)] | 278 | 100% | 100% | 100% | 100% | too stable |
| | **TD522** ^{B} | *1,5*-cz-[Lu^{III}(**TD647**)] | 278 | 100% | 100% | 100% | 100% | too stable |
| | **TD649** ^{B} | *1,5*-cz-[Y^{III}(**TD647**)] | 278 | 100% | 100% | 100% | 100% | too stable |
| | **TD876** ^{A} | *1.5*-cz-[Pb^{II}(**TD647**)] | 278 | 0% | 0% | 0% | 0% | too unstable |
| | **TD863** ^{A} | *1.5*-cz-[Ca^{II}(**TD647**)] | 278 | 0% | 0% | 0% | 0% | too unstable |
| | **TD880** ^{A} | *1.5*-cz-[Cd^{II}(**TD647**)] | 278 | 100% | 100% | 100% | 100% | too stable |
| | **TD890** ^{A} | *1.5*-cz-[Zn^{II}(**TD647**)] | 278 | 46% | 0% | 0% | 0% | **4.7E⁻¹** |
| | **TD624-La** ^{A,∗} | [La^{III}(*p*-**NO₂BnDOTA**)] | *285* | 9% | 0% | 0% | 0% | **2.9E⁻¹** |
| | **TD624-Gd** ^{A,∗} | [Gd^{III}(*p*-**NO₂BnDOTA**)] | *285* | 88% | 2% | 0% | 0% | **4.9E⁰**±2.9E⁻¹ |
| | **TD624-Lu** ^{A,∗} | [Lu^{III}(*p*-**NO₂BnDOTA**)] | 285 | 76% | 2% | 0% | 0% | **2.5E⁰**±9.6E⁻² |
| | **TD925** ^{B} | *1,5*-cz-[GdI^{III}(**TD921**)] | 275 | 100% | 98% | 90% | 60% | **9.9E²**±2.2E¹ |

| | | | | | | | | |
|---|---|---|---|---|---|---|---|---|
| Legend: ^{A} Aliquots (2 µL) were diluted with H₂O (200 µL) prior measurement. ^{B} Aliquots (2 µL) were neutralized with 500 mM MOPS/NaOH buffer (pH 7.0; 200 µL) prior measurement. *^{f}* Dissociation data were fitted by exponential decay (equation y = exp(-*x*/*t*); *y* = fraction of intact chelate; *x* = time; *t* = decay constant). Chelate stability is expressed as half-life *t*½ (in hours; highlighted bold), derived from decay constant (*t*½ = ln(2) × *t*). The error of the fitting is not displayed for single data point fitting. ^{∗} Sum of two diastereoisomers. ^{↓} Stock solutions preparation: In Eppendorf tube (0.5 mL), **2,2′,2ʺ,2‴-(2-(4-nitrobenzyl)-1,4,7,10-tetraazacyclododecane-1,4,7,10-tetrayl)tetraacetic acid tetrahydrochloride** (10.0 mM in H₂O; 100 µL; 1 µmol; 1.0 equiv.) was mixed with corresponding aq. **LnCl₃** (100 mM; 23.5 µL; 1.1 µmol; 1.1 equiv.), H₂O (59 µL) and aq. **NaOH** (400 mM; 17.5 µL; 7.0 µmol; 7.0 equiv.). The resulting mixtures were then stirred 16 h at RT (full conversion was confirmed by LC-MS) followed by centrifugation. Supernatants were then directly used as stock solutions for the dissociation assay. | | | | | | | | |

**Chart 20.** Dissociation of chelates in aq. 0.1 M^{III} HCl at RT (*acid decomplexation kinetic inertness*): fraction of intact chelate determined after 1 hour, 1 day, 1 week and 1 month by analytical HPLC (H₂O-MeCN system with 0.1% FA additive; detection at 280 nm; rounded to integers; aliquots from reaction mixtures were diluted with H₂O, vortexed and then injected to HPLC). Performed in glass vials with magnetic stirrers.

| chelate | | formally | *λ*, *nm* | 1 hour | 1 day | 1 week | 1 month | *t*_{½}, hours *^{f}* |
|---|---|---|---|---|---|---|---|---|
| | **TD875** ^{A} | *1*,*4*-cz-[Gd^{III}(**TD647**)] | 278 | 98% | 58% | 6% | 0% | **3.2E¹**±2.1E⁰ |
| | **TD874** ^{A} | *1*,*4*-cz-[Lu^{III} (**TD647**)] | 278 | 99% | 68% | 14% | 0% | **5.1E¹**±4.8E⁰ |
| | **TD900^{A}** | *1,4*-cz-[Cd^{II}(**TD647**)] | 278 | 100% | 99% | 91% | 63% | **1.3E³**±5.8E⁰ |
| | **TD651**^{B} | *1,5*-cz-[La^{III}(**TD647**)] | 278 | 76% | 4% | 0% | 0% | **2.5E⁰±**2.5E⁻¹ |
| | **TD870** ^{B} | *1,5-*cz-[Ce^{III}(**TD647**)] | 278 | 95% | 37% | 0% | 0% | **1.7E¹±** 2.9E⁻¹ |
| | **TD869** ^{B} | *1,5*-cz-[Pr^{III}(**TD647**)] | 278 | 98% | 71% | 12% | 0% | **5.2E¹**±2.2E⁰ |
| | **TD868** ^{B} | *1*,*5*-cz-[Nd^{III}(**TD647**)] | 278 | 99% | 90% | 54% | 2% | **1.8E²** ± 1.2E¹ |
| | **TD867** ^{B} | *1*,*5*-cz-[Sm^{III}(**TD647**)] | 278 | 100% | 100% | 99% | 96% | **1.3E⁴**±4.4E² |
| | **TD599** ^{B} | *1,5*-cz-[Eu^{III}(**TD647**)] | 278 | 100% | 100% | 100% | 100% | too stable |
| | **TD527** ^{B} | *1*,*5*-cz-[Gd^{III}(**TD647**)] | 278 | 100% | 100% | 100% | 100% | too stable |
| | **TD600**^{B} | *1,5*-cz-[Tb^{III}(**TD647)**] | 278 | 100% | 100% | 100% | 100% | too stable |
| | **TD866** ^{B} | *1*,*5*-cz-[Dy^{III}(**TD647**)] | 278 | 100% | 100% | 100% | 100% | too stable |
| | **TD648** ^{B} | *1*,*5*-cz-[Ho^{III}(**TD647**)] | 278 | 100% | 100% | 100% | 100% | too stable |
| | **TD865** ^{B} | *1*,*5*-cz-[Er^{III}(**TD647**)] | 278 | 100% | 100% | 100% | 100% | too stable |
| | **TD601** ^{B} | *1*,*5*-cz-[Tm^{III}(**TD647**)] | 278 | 100% | 100% | 100% | 100% | too stable |
| | **TD602**^{B} | *1*,*5*-cz-[Yb^{III}(**TD647**)] | 278 | 100% | 100% | 100% | 100% | too stable |
| | **TD522** ^{B} | *1*,*5*-cz-[Lu^{III}(**TD647**)] | 278 | 100% | 100% | 100% | 100% | too stable |
| | **TD649** ^{B} | *1*,*5*-cz-[Y^{III}(**TD647**)] | 278 | 100% | 100% | 100% | 100% | too stable |
| | **TD876** ^{A} | *1*,*5*-cz-[Pb^{II}(**TD647**)] | 278 | 6% | 0% | 0% | 0% | **2.4E⁻¹** |
| | **TD863** ^{A} | *1*,*5*-cz-[Ca^{II}(**TD647**)] | 278 | 39% | 0% | 0% | 0% | **7.3E**^{**-**1} |
| | **TD880** ^{A} | *1*,*5*-cz-[Cd^{II}(**TD647**)] | 278 | 100% | 100% | 100% | 100% | too stable |
| | **TD890** ^{A} | *1*,*5*-cz-[Zn^{II}(**TD647)**] | 278 | 100% | 93% | 54% | 5% | **1.9E²** ± 8.5E⁰ |
| **TD624-La** ^{A,∗} | | [La^{III}(***p*-NO₂BnDOTA)**] | *285* | 95% | 36% | 0% | 0% | **1.6E¹** ± 1.8E⁻¹ |
| **TD624-Gd** ^{A,∗} | | [Gd^{III}(***p*-NO₂BnDOTA**)] | *285* | 100% | 99% | 92% | 79% | **2.1E³** ± 1.2E² |
| **TD624-Lu** ^{A,∗} | | [Lu^{III}(***p*-NO₂BnDOTA**)] | *285* | 99% | 98% | 87% | 67% | **1.2E³** ± 8.7E¹ |
| | **TD925** ^{B} | *1*,*5*-cz-[Gd^{III}(**TD921**)] | 275 | 100% | 100% | 100% | 100% | too stable |

| | | | | | | | | |
|---|---|---|---|---|---|---|---|---|
| Legend: ^{A} Aliquots (2 µL) were diluted with H₂O (200 µL) prior measurement. ^{B} Aliquots (2 µL) were neutralized with 500 mM MOPS/NaOH buffer (pH 7.0; 200 µL) prior measurement. *^{f}* Dissociation data were fitted by exponential decay (equation y = exp(-*x*/*t*); y = fraction of intact chelate; *x* = time; *t* = decay constant). Chelate stability is expressed as half-life *t_{½}* (in hours; highlighted bold), derived from decay constant (*t*_{½} = ln(2) × *t*). The error of the fitting is not displayed for single data point fitting. ^{∗} Sum of two diastereoisomers. ↓ Stock solutions preparation: In Eppendorf tube (0.5 mL), **2,2,2",2'"-(2-(4-nitrobenzyl)-1,4,7,10-tetraazacyclododecane-1,4,7,10-tetrayl)tetraacetic acid tetrahydrochloride** (10.0 mM in H₂O; 100 µL; 1 µmol; 1.0 equiv.) was mixed with corresponding aq. **LnCl₃** (100 mM; 23.5 µL; 1.1 µmol; 1.1 equiv.), H₂O (59 µL) and aq. **NaOH** (400 mM; 17.5 µL; 7.0 µmol: 7.0 equiv.). The resulting mixtures were then stirred 16 h at RT (full conversion was confirmed by LC-MS) followed by centrifugation. Supernatants were then directly used as stock solutions for the dissociation assay. | | | | | | | | |

**Chart 21.** Cleavage of model hexapeptide-chelate conjugates into parent chelate and individual amino acids in aq. 3.0 M HCl at 80 °C over the course of 1 week (*acid peptide hydrolysis kinetics*): fractions of starting chelate-peptide conjugate (*S*), chelate-containing intermediates (*I*), parent chelate **(*P*)** and free ligand (*L*) determined by analytical HPLC (H₂O-MeCN system with 0.1% FA additive; detection at 285 nm; rounded to integers; aliquots from reaction mixtures were neutralized with MOPS buffer, pH 7.0, vortexed and then injected to HPLC). Performed in glass vials with magnetic stirrers.

| chelate | species | | 1 h | 1 d | 2d | 3d | 4 d | 5 d | 6 d | 7 d |
|---|---|---|---|---|---|---|---|---|---|---|
| **TD802'** | *1*,*5*-cz-[Lu^{III}(**Lig-AYFHVG-NH₂**)] | *S* | 0% | 0% | 0% | 0% | 0% | 0% | 0% | 0% |
| | *1,5*-cz-[Lu^{III}(**Lig-AYFHVG-OH**)] | *I* | 48% | 0% | 0% | 0% | 0% | 0% | 0% | 0% |
| | *1*,*5*-cz-[Lu^{III}(**Lig-AYFHV-OH)**] | *I* | 2% | 0% | 0% | 0% | 0% | 0% | 0% | 0% |
| | *1*,*5*-cz-[Lu^{III}(**Lig-AYFH-OH)**] | *I* | 5% | 0% | 0% | 0% | 0% | 0% | 0% | 0% |
| | *1*,*5*-cz-[Lu^{III}(**Lig-AYF-OH)**] | *I* | 9% | 0% | 0% | 0% | 0% | 0% | 0% | 0% |
| | *1*,*5*-cz-[Lu^{III}(**Lig-AY-OH**)] | *I* | 8% | 0% | 0% | 0% | 0% | 0% | 0% | 0% |
| | *1*,*5*-cz-[Lu^{III}(**Lig-A-OH)**] | *I* | 21% | 12% | 2% | 0% | 0% | 0% | 0% | 0% |
| | *1,5*-cz-[Lu^{III}(**Lig**)] | ***P*** | **7 %** | **88%** | **98%** | **100%** | **100%** | **100%** | **100%** | **100%** |
| | *1*,*5*-cz-**Lig** | *L* | 0% | 0% | 0% | 0% | 0% | 0% | 0% | 0% |

| chelate | species | | 1 h | 1 d | 2d | 3d | 4 d | 5 d | 6 d | 7 d |
|---|---|---|---|---|---|---|---|---|---|---|
| **TD792** ' | *1*,*5*-cz-[Y^{III}(**Lig-AYFHVG-NH₂**)] | *S* | 0% | 0% | 0% | 0% | 0% | 0% | 0% | 0% |
| | *1*,*5*-cz-[Y^{III}(**Lig-AYFHVG-OH**)] | *I* | 46% | 0% | 0% | 0% | 0% | 0% | 0% | 0% |
| | *1*,*5*-cz-[Y^{III}(**Lig-AYFHV-OH**)] | *I* | 2% | 0% | 0% | 0% | 0% | 0% | 0% | 0% |
| | *1*,*5*-cz-[Y^{III}(**Lig-AYFH-OH**)] | *I* | 5% | 0% | 0% | 0% | 0% | 0% | 0% | 0% |
| | *1*,*5*-cz-[Y^{III}(**Lig-AYF-OH**)] | *I* | 9% | 0% | 0% | 0% | 0% | 0% | 0% | 0% |
| | *1*,*5*-cz-[Y^{III}(**Lig-AY-OH**)] | *I* | 9% | 0% | 0% | 0% | 0% | 0% | 0% | 0% |
| | *1*,*5*-cz-[Y^{III}(**Lig-A-OH**)] | *I* | 22% | 13% | 2% | 0% | 0% | 0% | 0% | 0% |
| | *1*,*5*-cz-[Y^{III}(**Lig-COOH**)] | ***P*** | **7%** | **84%** | **91%** | **90%** | **87%** | **84%** | **81%** | **77%** |
| | *1,5*-cz-**Lig** ^{#} | *L* | 0% | 3% | 7% | 10% | 13% | 16% | 19% | 23% |

| chelate | species | | 1 h | 1 d | 2d | 3d | 4 d | 5 d | 6 d | 7 d |
|---|---|---|---|---|---|---|---|---|---|---|
| **TD902** " | *1*,*5*-cz-[Lu^{III}(**Lig-AYFHVG-NH₂**)] | *S* | 0% | 0% | 0% | 0% | 0% | 0% | 0% | 0% |
| | *1*,*5*-cz-[Lu^{III}(**Lig-AYFHVG-OH)**] | *I* | 32% | 0% | 0% | 0% | 0% | 0% | 0% | 0% |
| | *1*,*5*-cz-[Lu^{III}(**Lig-AYFHV-OH**)] | *I* | 1% | 0% | 0% | 0% | 0% | 0% | 0% | 0% |
| | *1*,*5*-cz-[Lu^{III}(**Lig-AYFH-OH**)] | *I* | 4% | 0% | 0% | 0% | 0% | 0% | 0% | 0% |
| | *1,5*-cz-[Lu^{III}(**Lig-AYF-OH)]** | *I* | 6% | 0% | 0% | 0% | 0% | 0% | 0% | 0% |
| | *1,5*-cz-[Lu^{III}(**Lig-AY-OH**)] | *I* | 5% | 0% | 0% | 0% | 0% | 0% | 0% | 0% |
| | *1*,*5*-cz-[Lu^{III}(**Lig-A-OH)**] | *I* | 13% | 0% | 0% | 0% | 0% | 0% | 0% | 0% |
| | *1*,*5*-cz-[Lu^{III}(**Lig**)] | ***P*** | **39%** | **100%** | **100%** | **100%** | **100%** | **100%** | **100%** | **100%** |
| | *1*,*5*-cz-**Lig** | *L* | 0% | 0% | 0% | 0% | 0% | 0% | 0% | 0% |
| **TD901** " | *1,5*-cz-[Tm^{III}(**Lig-AYFHVG-NH₂)**] | *S* | 0% | 0% | 0% | 0% | 0% | 0% | 0% | 0% |
| | *1*,*5*-cz-[Tm^{III}(**Lig-AYFHVG-OH**)] | *I* | 34% | 0% | 0% | 0% | 0% | 0% | 0% | 0% |
| | *1,5*-cz-[Tm^{III}(**Lig-AYFHV-OH**)] | *I* | 1% | 0% | 0% | 0% | 0% | 0% | 0% | 0% |
| | *1*,*5*-cz-[Tm^{III}(**Lig-AYFH-OH**)] | *I* | 4% | 0% | 0% | 0% | 0% | 0% | 0% | 0% |
| | *1*,*5*-cz-[Tm^{III}(**Lig-AYF-OH)**] | *I* | 6% | 0% | 0% | 0% | 0% | 0% | 0% | 0% |
| | *1,5*-cz-[Tm^{III}(**Lig-AY-OH**)] | *I* | 4% | 0% | 0% | 0% | 0% | 0% | 0% | 0% |
| | *1,5*-cz-[Tm^{III}(**Lig-A-OH**)] | *I* | 12% | 0% | 0% | 0% | 0% | 0% | 0% | 0% |
| | *1,5*-cz-[Tm^{III}(**Lig**)] | ***P*** | **37%** | **100**% | **100**% | **100**% | **100**% | **100**% | **100**% | **99%** |
| | *1*,*5*-cz-**Lig** | *L* | 0% | 0% | 0% | 0% | 0% | 0% | 0% | 1% |

| | | | | | | | | | | |
|---|---|---|---|---|---|---|---|---|---|---|
| Legend: ' Hexapeptide is connected to the chelate through aromatic amide moiety. " Hexapeptide is connected to the chelate through aliphatic amide moiety. ^{#} Sum of *1*,*5*-cz-**Lig** and *1,5*-cz-**Lig-A-OH**. | | | | | | | | | | |

**Chart 22.** Cleavage of model hexapeptide-chelate conjugates into parent chelate and individual amino acids in aq. 2.0 M HCl at 80 °C over the course of 1 week (*acid peptide hydrolysis kinetics*): fractions of starting chelate-peptide conjugate (*S*), chelate-containing intermediates (*I*), parent chelate (***P***) and free ligand (*L*) determined by analytical HPLC (H₂O-MeCN system with 0.1% FA additive; detection at 285 nm; rounded to integers; aliquots from reaction mixtures were neutralized with MOPS buffer, pH 7.0, vortexed and then injected to HPLC). Performed in glass vials with magnetic stirrers.

| chelate | species | | 1 h | 1 d | 2d | 3d | 4 d | 5 d | 6 d | 7 d |
|---|---|---|---|---|---|---|---|---|---|---|
| **TD802'** | *1*,*5*-cz-[Lu^{III}(**Lig-AYFHVG-NH₂**)] | *S* | 0% | 0% | 0% | 0% | 0% | 0% | 0% | 0% |
| | *1,5*-cz-[Lu^{III}(**Lig-AYFHVG-OH**)] | *I* | 65% | 0% | 0% | 0% | 0% | 0% | 0% | 0% |
| | *1*,*5*-cz-[Lu^{III}(**Lig-AYFHV-OH)**] | *I* | 1 % | 0% | 0% | 0% | 0% | 0% | 0% | 0% |
| | *1,5*-cz-[Lu^{III}(**Lig-AYFH-OH)]** | *I* | 4% | 0% | 0% | 0% | 0% | 0% | 0% | 0% |
| | *1,5*-cz-[Lu^{III}(**Lig-AYF-OH)]** | *I* | 7% | 0% | 0% | 0% | 0% | 0% | 0% | 0% |
| | *1,5*-cz-[Lu^{III}(**Lig-AY-OH**)] | *I* | 6% | 1% | 0% | 0% | 0% | 0% | 0% | 0% |
| | *1*,*5*-cz-[Lu^{III}(**Lig-A-OH)**] | *I* | 13% | 30% | 8% | 3% | 1% | 0% | 0% | 0% |
| | *1,5*-cz-[Lu^{III}(**Lig**)] | ***P*** | **5 %** | **69%** | **92%** | **97%** | **99%** | **100%** | **100%** | **100%** |
| | *1*,*5*-cz-**Lig** | *L* | 0% | 0% | 0% | 0% | 0% | 0% | 0% | 0% |

| chelate | species | | 1 h | 1 d | 2d | 3d | 4 d | 5 d | 6 d | 7 d |
|---|---|---|---|---|---|---|---|---|---|---|
| **TD792** ' | *1*,*5*-cz-[Y^{III}(**Lig-AYFHVG-NH₂**)] | *S* | 1 % | 0% | 0% | 0% | 0% | 0% | 0% | 0% |
| | *1*,*5*-cz-[Y^{III}(**Lig-AYFHVG-OH**)] | *I* | 63% | 0% | 0% | 0% | 0% | 0% | 0% | 0% |
| | *1,5*-cz-[Y^{III}(**Lig-AYFHV-OH**)] | *I* | 1 % | 0% | 0% | 0% | 0% | 0% | 0% | 0% |
| | *1*,*5*-cz-[Y^{III}(**Lig-AYFH-OH**)] | *I* | 4% | 0% | 0% | 0% | 0% | 0% | 0% | 0% |
| | *1*,*5*-cz-[Y^{III}(**Lig-AYF-OH**)] | *I* | 7% | 0% | 0% | 0% | 0% | 0% | 0% | 0% |
| | *1*,*5*-cz-[Y^{III}(**Lig-AY-OH**)] | *I* | 6% | 0% | 0% | 0% | 0% | 0% | 0% | 0% |
| | *1*,*5*-cz-[Y^{III}(**Lig-A-OH**)] | *I* | 14% | 28% | 10% | 4% | 1% | 0% | 0% | 0% |
| | *1*,*5*-cz-[Y^{III}(**Lig-COOH**)] | ***P*** | **5 %** | **70%** | **88%** | **93%** | **94%** | **94%** | **93%** | **92%** |
| | *1,5*-cz-**Lig** ^{#} | *L* | 0% | 1% | 2% | 4% | 5% | 6% | 7% | 8% |

| chelate | species | | 1 h | 1 d | 2d | 3d | 4 d | 5 d | 6 d | 7 d |
|---|---|---|---|---|---|---|---|---|---|---|
| **TD902** " | *1*,*5*-cz-[Lu^{III}(**Lig-AYFHVG-NH₂**)] | *S* | 0% | 0% | 0% | 0% | 0% | 0% | 0% | 0% |
| | *1,5*-cz-[Lu^{III}(**Lig-AYFHVG-OH**)] | *I* | 48% | 0% | 0% | 0% | 0% | 0% | 0% | 0% |
| | *1*,*5*-cz-[Lu^{III}(**Lig-AYFHV-OH**)] | *I* | 1% | 0% | 0% | 0% | 0% | 0% | 0% | 0% |
| | *1*,*5*-cz-[Lu^{III}(**Lig-AYFH-OH**)] | *I* | 3% | 0% | 0% | 0% | 0% | 0% | 0% | 0% |
| | *1,5*-cz-[Lu^{III}(**Lig-AYF-OH)]** | *I* | 5% | 0% | 0% | 0% | 0% | 0% | 0% | 0% |
| | *1,5*-cz-[Lu^{III}(**Lig-AY-OH**)] | *I* | 3% | 0% | 0% | 0% | 0% | 0% | 0% | 0% |
| | *1*,*5*-cz-[Lu^{III}(**Lig-A-OH)**] | *I* | 9% | 0% | 0% | 0% | 0% | 0% | 0% | 0% |
| | *1*,*5*-cz-[Lu^{III}(**Lig**)] | ***P*** | ***3*1%** | **100%** | **100%** | **100%** | **100%** | **100%** | **100%** | **100%** |
| | *1*,*5*-cz-**Lig** | *L* | 0% | 0% | 0% | 0% | 0% | 0% | 0% | 0% |
| **TD901** " | *1,5*-cz-[Tm^{III}(**Lig-AYFHVG-NH₂)**] | *S* | 0% | 0% | 0% | 0% | 0% | 0% | 0% | 0% |
| | *1*,*5*-cz-[Tm^{III}(**Lig-AYFHVG-OH**)] | *I* | 50% | 0% | 0% | 0% | 0% | 0% | 0% | 0% |
| | *1,5*-cz-[Tm^{III}(**Lig-AYFHV-OH**)] | *I* | 1% | 0% | 0% | 0% | 0% | 0% | 0% | 0% |
| | *1,5*-cz-[Tm^{III}(**Lig-AYFH-OH)**] | *I* | 3% | 0% | 0% | 0% | 0% | 0% | 0% | 0% |
| | *1,5*-cz-[Tm^{III}(**Lig-AYF-OH**)] | *I* | 5% | 0% | 0% | 0% | 0% | 0% | 0% | 0% |
| | *1,5*-cz-[Tm^{III}(**Lig-AY-OH**)] | *I* | 3% | 0% | 0% | 0% | 0% | 0% | 0% | 0% |
| | *1,5*-cz-[Tm^{III}(**Lig-A-OH**)] | *I* | 8% | 0% | 0% | 0% | 0% | 0% | 0% | 0% |
| | *1,5*-cz-[Tm^{III}(**Lig**)] | ***P*** | **30%** | **100%** | **100%** | **100%** | **100%** | **100%** | **100%** | **100%** |
| | *1*,*5*-cz-**Lig** | *L* | 0% | 0% | 0% | 0% | 0% | 0% | 0% | 0% |

| | | | | | | | | | | |
|---|---|---|---|---|---|---|---|---|---|---|
| Legend: ' Hexapeptide is connected to the chelate through aromatic amide moiety. " Hexapeptide is connected to the chelate through aliphatic amide moiety. ^{#} Sum of *1*,*5*-cz-**Lig** and *1,5*-cz-**Lig-A-OH**. | | | | | | | | | | |

**Chart 23.** Cleavage of model hexapeptide-chelate conjugates into parent chelate and individual amino acids in aq. 1.0 M HCl at 80 °C over the course of week (*acid peptide hydrolysis kinetics*): fractions of starting chelate-peptide conjugate (S), chelate-containing intermediates (*I*), parent chelate (P) and free ligand (*L*) determined by analytical HPLC (H₂O-MeCN system with 0.1% FA additive; detection at 285 nm; rounded to integers; aliquots from reaction mixtures were neutralized with MOPS buffer, pH 7.0, vortexed and then injected to HPLC). Performed in glass vials with magnetic stirrers.

| chelate | species | | 1 h | 1 d | 2d | 3d | 4 d | 5 d | 6 d | 7 d |
|---|---|---|---|---|---|---|---|---|---|---|
| **TD802'** | *1*,*5*-cz-[Lu^{III}(**Lig-AYFHVG-NH₂**)] | *S* | 4% | 0% | 0% | 0% | 0% | 0% | 0% | 0% |
| | *1,5*-cz-[Lu^{III}(**Lig-AYFHVG-OH**)] | *I* | 80% | 1% | 0% | 0% | 0% | 0% | 0% | 0% |
| | *1*,*5*-cz-[Lu^{III}(**Lig-AYFHV-OH)**] | *I* | 1 % | 0% | 0% | 0% | 0% | 0% | 0% | 0% |
| | *1,5*-cz-[Lu^{III}(**Lig-AYFH-OH**)] | *I* | 2% | 1% | 0% | 0% | 0% | 0% | 0% | 0% |
| | *1,5*-cz-[Lu^{III}(**Lig-AYF-OH)]** | *I* | 3% | 2% | 0% | 0% | 0% | 0% | 0% | 0% |
| | *1,5*-cz-[Lu^{III}(**Lig-AY-OH**)] | *I* | 3% | 5% | 1% | 0% | 0% | 0% | 0% | 0% |
| | *1*,*5*-cz-[Lu^{III}(**Lig-A-OH)**] | *I* | 6% | 55% | 40% | 28% | 17% | 10% | 6% | 3% |
| | *1,5*-cz-[Lu^{III}(**Lig**)] | ***P*** | **2 %** | **34%** | **59%** | **72%** | **83%** | **90%** | **94%** | **97%** |
| | *1*,*5*-cz-**Lig** | *L* | 0% | 0% | 0% | 0% | 0% | 0% | 0% | 0% |

| chelate | species | | 1 h | 1 d | 2d | 3d | 4 d | 5 d | 6 d | 7 d |
|---|---|---|---|---|---|---|---|---|---|---|
| **TD792** ' | *1*,*5*-cz-[Y^{III}(**Lig-AYFHVG-NH₂**)] | *S* | 5% | 0% | 0% | 0% | 0% | 0% | 0% | 0% |
| | *1*,*5*-cz-[Y^{III}(**Lig-AYFHVG-OH**)] | *I* | 77% | 1% | 0% | 0% | 0% | 0% | 0% | 0% |
| | *1*,*5*-cz-[Y^{III}(**Lig-AYFHV-OH**)] | *I* | 1 % | 0% | 0% | 0% | 0% | 0% | 0% | 0% |
| | *1*,*5*-cz-[Y^{III}(**Lig-AYFH-OH**)] | *I* | 2% | 1% | 0% | 0% | 0% | 0% | 0% | 0% |
| | *1*,*5*-cz-[Y^{III}(**Lig-AYF-OH**)] | *I* | 4% | 2% | 0% | 0% | 0% | 0% | 0% | 0% |
| | *1*,*5*-cz-[Y^{III}(**Lig-AY-OH**)] | *I* | 3% | 5% | 1% | 0% | 0% | 0% | 0% | 0% |
| | *1*,*5*-cz-[Y^{III}(**Lig-A-OH**)] | *I* | 6% | 56% | 35% | 26% | 16% | 8% | 5% | 4% |
| | *1*,*5*-cz-[Y^{III}(**Lig-COOH**)] | ***P*** | **2 %** | ***35%*** | **64%** | **73%** | **83%** | **91%** | **93%** | **95%** |
| | *1,5*-cz-**Lig**^{#} | *L* | 0% | 0% | 0% | 1% | 1% | 1% | 2% | 2% |

| chelate | species | | 1 h | 1 d | 2d | 3d | 4 d | 5 d | 6 d | 7 d |
|---|---|---|---|---|---|---|---|---|---|---|
| **TD902** " | *1*,*5*-cz-[Lu^{III}(**Lig-AYFHVG-NH₂**)] | *S* | 3% | 0% | 0% | 0% | 0% | 0% | 0% | 0% |
| | *1,5*-cz-[Lu^{III}(**Lig-AYFHVG-OH**)] | *I* | 67% | 0% | 0% | 0% | 0% | 0% | 0% | 0% |
| | *1*,*5*-cz-[Lu^{III}(**Lig-AYFHV-OH**)] | *I* | 1% | 0% | 0% | 0% | 0% | 0% | 0% | 0% |
| | *1*,*5*-cz-[Lu^{III}(**Lig-AYFH-OH**)] | *I* | 2% | 0% | 0% | 0% | 0% | 0% | 0% | 0% |
| | 1*,5*-cz-[Lu^{III}(**Lig-AYF-OH)]** | *I* | 3% | 0% | 0% | 0% | 0% | 0% | 0% | 0% |
| | *1,5*-cz-[Lu^{III}(**Lig-AY-OH**)] | *I* | 2% | 0% | 0% | 0% | 0% | 0% | 0% | 0% |
| | *1*,*5*-cz-[Lu^{III}(**Lig-A-OH)**] | *I* | 4% | 3% | 0% | 0% | 0% | 0% | 0% | 0% |
| | *1*,*5*-cz-[Lu^{III}(**Lig**)] | ***P*** | **19%** | **97%** | **100%** | **100%** | **100%** | **100%** | **100%** | **100%** |
| | *1*,*5*-cz-**Lig** | *L* | 0% | 0% | 0% | 0% | 0% | 0% | 0% | 0% |

| Chelate | species | | 1 h | 1 d | 2d | 3d | 4 d | 5 d | 6 d | 7 d |
|---|---|---|---|---|---|---|---|---|---|---|
| **TD901** " | *1,5*-cz-[Tm^{III}(**Lig-AYFHVG-NH₂)**] | *S* | 2% | 0% | 0% | 0% | 0% | 0% | 0% | 0% |
| | *1*,*5*-cz-[Tm^{III}(**Lig-AYFHVG-OH**)] | *I* | 68% | 1% | 0% | 0% | 0% | 0% | 0% | 0% |
| | *1*,*5*-cz-[Tm^{III}(**Lig-AYFHV-OH**)] | *I* | 1% | 0% | 0% | 0% | 0% | 0% | 0% | 0% |
| | *1*,*5*-cz-[Tm^{III}(**Lig-AYFH-OH**)] | *I* | 2% | 0% | 0% | 0% | 0% | 0% | 0% | 0% |
| | *1,5*-cz-[Tm^{III}(**Lig-AYF-OH**)] | *I* | 3% | 0% | 0% | 0% | 0% | 0% | 0% | 0% |
| | *1,5*-cz-[Tm^{III}(**Lig-AY-OH**)] | *I* | 2% | 0% | 0% | 0% | 0% | 0% | 0% | 0% |
| | *1,5*-cz-[Tm^{III}(**Lig-A-OH**)] | *I* | 4% | 4% | 0% | 0% | 0% | 0% | 0% | 0% |
| | *1,5*-cz-[Tm^{III}(**Lig**)] | ***P*** | **19%** | **95%** | **100%** | **100%** | **100%** | **100%** | **100%** | **100%** |
| | *1*,*5*-cz-**Lig** | *L* | 0% | 0% | 0% | 0% | 0% | 0% | 0% | 0% |

| | | | | | | | | | | |
|---|---|---|---|---|---|---|---|---|---|---|
| Legend: ' Hexapeptide is connected to the chelate through aromatic amide moiety. " Hexapeptide is connected to the chelate through aliphatic amide moiety. ^{#} Sum of *1*,*5*-cz-**Lig** and *1,5*-cz-**Lig-A-OH**. | | | | | | | | | | |

**Chart 24.** Cell penetrating properties of **TD975, TD989, TD1010** and **TD1011** (CPP-conjugates). Protocol: Grown cells (CCRF-CEM; T-lymphoblastic leukemia) were centrifuged (250 × g, RT), washed with PBS and resuspended in RPMI medium Dutch modification (with HEPES buffer, without antibiotics) to a concentration 2.5 × 10⁷ mL⁻¹. Suspension was transferred into 12 (4 CPP-conjugate combinations, each in triplicate) Eppendorf tubes (400 µL of suspension to each). Stock solution of CPP conjugates (5.0 mM, 50 µL) were added to reach 0.5 mM final concentration of given CPP-conjugate. Cells were then incubated 2 h at 37 °C. After incubation, samples were centrifuged (900 × g, RT). Medium was carefully pipetted out and the pellets twice washed with 1 mL PBS. Resulting pellets were treated with HCl (3.0 M, 500 µL) and stirred 7 days at 80 °C. Resulting solutions were then analyzed for cell uptake.

| CPP conjugates incubated with cells | cell uptake, % | | | | | |
|---|---|---|---|---|---|---|
| 0.5 mM **TD975** + 0.5 mM **TD989** | **TD975***^{a}* | 0.54±0.012% *^{b}* | 0.57±0.039 *^{c}* | **TD989** *^{a}* | 0.47±0.049 *^{b}* | 0.47±0.052 *^{c}* |
| 0.5 mM **TD1011** + 0.5 mM **TD1010** | **TD1011***^{a}* | 0.13±0.013% *^{b}* | 0.15±0.017 *^{c}* | **TD1010** *^{a}* | 0.13±0.003 ^{b} | 0.14±0.019 ^{c} |
| 0.5 mM **TD975** + 0.5 mM **TD1010** | **TD975** *^{a}* | 0.72±0.006% *^{b}* | 0.73±0.020 *^{c}* | **TD1010** *^{a}* | 0.06±0.036 *^{b}* | 0.06±0.027 *^{c}* |
| 0.5 mM **TD1011** + 0.5 mM **TD989** | **TD1011** *^{a}* | 0.07±0.014% *^{b}* | 0.07±0.049 *^{c}* | **TD989** *^{a}* | 0.69±0.003 *^{b}* | 0.66±0.018 *^{c}* |

| | | | | | | |
|---|---|---|---|---|---|---|
| Legend: *^{a}* analysed as **TD748** and **TD897** after acid hydrolysis of parent CPP conjugates. *^{b}* quantified by LC-MS: UV integration at 285 nm; ESI-MS integration of EIC⁺ 841-843 m/z ([**TD748**]⁺) and 835-837 m/z ([**TD897]**⁺). The quantification was based on calibration (of both UV and ESI-MS modes) using stock solutions of **TD748** and **TD897** with known concentrations. *^{c}* quantified by ICP. | | | | | | |

### Abbreviations and definitions

- cyclene: 1,4,7,10-tetraazacyclododecane
- Cbz: benzylchloroformate
- ^{t}Bu: terc-butyl
- Boc: benzyloxycarbonyl
- DCM: dichloromethane
- TFA: trifluoroacetic acid
- TIPS: triisopropylsilyl
- TMS: trimethylsilyl
- Ph: phenyl
- Fmoc: fluorenylmethyloxykarbonyl
- iPr: isopropyl
- MOPS: 3-(N-morpholino)propanesulfonic acid
- DIPEA: N,N-Diisopropylethylamine
- LC-MS: liquid chromatography - mass spectrometry
- MRI: magnetic resonance imaging
- aryl: substituent derived from at least one aromatic hydrocarbon ring, e.g. phenyl or naphtyl
- alkyl: substituent derived from an alkane (linear or branched) by removing one hydrogen, e.g. methyl or ethyl
- arylalkyl: substituent derived from an aryl-substituted alkane (linear or branched) by removing one hydrogen, e.g. benzyl
- arylalkynyl: substituent derived from an aryl-substituted alkyne by removing one hydrogen (thus containing a triple bond), which may be linear or branched
- click-zip: closed cage ligand or complex, wherein the click reaction between the azide and the triple bond took place and the triazole bridge was formed, thereby "zipping" the metal coordination place.
- *1,5*-cz-[**M**(**Lig**)] from **Lig**: and **M** ions is a general notation of the coordination compounds according to the present invention. "1,5-cz-" stands for a 1,5-triazole bridged cage, [**M**(**Lig**)] stands for a coordination compound of a metal cation and a ligand (the compound according to general formula (**I**)).

## Claims

**1.** Compound of general formula (I) 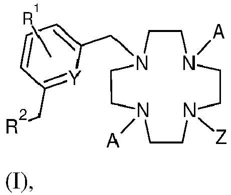 wherein
Y is selected from a group consisting of nitrogen; N-oxide;
R¹ is selected from the group consisting of H; halogen; -OH; -N₃; -CH₂N₃; -NR₂, wherein R is independently selected from H or C₁ to C₆ alkyl, which may be branched or linear; C₆ to C₁₀ aryl, which can optionally be substituted with -NH₂, -NO₂, -COOH, -CH₂Cl and/or -CH₂COOH; C₇ to C₁₀ arylalkyl, which can optionally be substituted with -NH₂, -NO₂, -COOH, -CH₂Cl and/or -CH₂COOH;
-CF₃; -COOR, wherein R is as defined above; 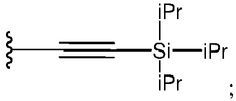 -CH₂CH(OMe)₂; 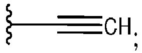
R² is 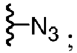
A are independently selected from the group consisting of H; -(CH₂)ₙCOOH, wherein n is an integer from 1 to 3; -CH(CH₃)COOH; -CH((CH₂)ₙCH₃)COOH, wherein n is as defined above; -CH₂P(=O)(OR)₂, wherein R is as defined above;
-CH₂C(=O)(NH₂); -CH₂C(=O)(NH)-CH₂COOH; -CH₂P(=O)(OH)(Ar), wherein Ar is phenyl, which can optionally be substituted with C₁ to C₆ alkyl;
Z is selected from a group consisting of 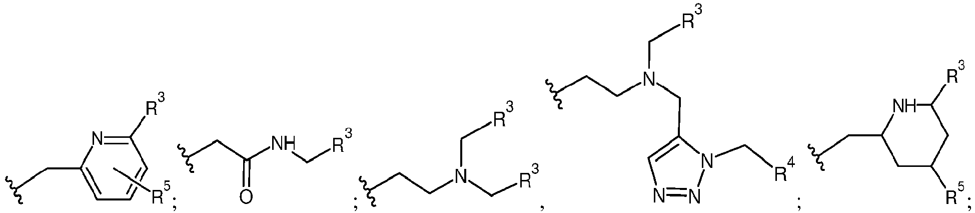 wherein
R³ is 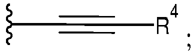
R⁴ is selected from the group consisting of H; halogen; piperidinyl; trimethylsilyl; triisopropylsilyl; phenyl; C₁ to C₆ alkyl, which may be branched or linear; C₃ to C₆ cycloalkyl; -CF₃; -CH₂NHR⁶, wherein R⁶ is selected from H, fluorenylmethyloxykarbonyl and benzyloxycarbonyl; -C(CH₃)₂(NHR⁶), wherein R⁶ is as defined above; adamantyl; 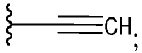
R⁵ is H; -CF₃; halogen; -OH; C₇ to C₁₀ arylalkyl, which can optionally be substituted with -NH₂, -NO₂, - COOH, -CH₂Cl and/or -CH₂COOH; or 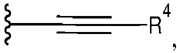 wherein R⁴ is as defined above;
and/or R² and R³ together form a 1,2,3-triazole group of formula 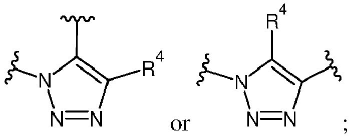 wherein R⁴ is defined above;
with the proviso that at most one A is H.

**2.** Compound of general formula (I) according to claim 1, wherein:
Y is nitrogen;
R¹ is selected from the group consisting of H; Cl; -N(CH₃)₂; phenyl, which can optionally be substituted with -COOH or -CH₂COOH; benzyl, which can optionally be substituted with -COOH or -CH₂COOH; -CF₃; - COOCH₃; -COOCH(CH₃)₂; -COOtBu; 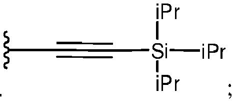
A are independently selected from the group consisting of H; -(CH₂)ₙCOOH, wherein *n* is 1 or 2; - CH₂P(=O)(OH)₂; -CH₂P(=O)(OH)(OEt); -CH₂P(=O)(OEt)₂; -CH₂C(=O)(NH₂); -CH₂C(=O)(NH)-CH₂COOH; -CH₂P(=O)(OH)(Ph);
Z is selected from a group consisting of 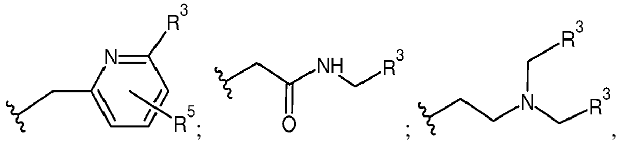 wherein
R³ is 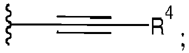
R⁴ is selected from the group consisting of H; halogen; piperidinyl; trimethylsilyl; triisopropylsilyl; phenyl; cyclopropyl, terc-butyl; -CF₃; -CH₂NH₂; -CH₂N(H)(Fmoc); -C(CH₃)₂(NH₂); -C(CH₃)₂(NHBoc); adamantyl;
R⁵ is H or 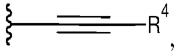 wherein R⁴ is as defined above;
with the proviso that at most one A is H.

**3.** Compound according to claim 1 or 2,
wherein Z is 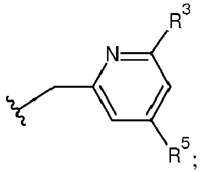
R³ is 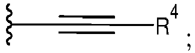
R⁴ and R⁵ are as defined above, preferably R⁴ and R⁵ are H.

**4.** Compound of general formula (I) according to claim 1, wherein R² and R³ together form a 1,2,3-triazole group of formula 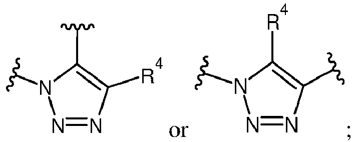 such that the bridge between two opposite nitrogen atoms of the cyclen moiety is an entity of general formula (IIa) or (IIb) 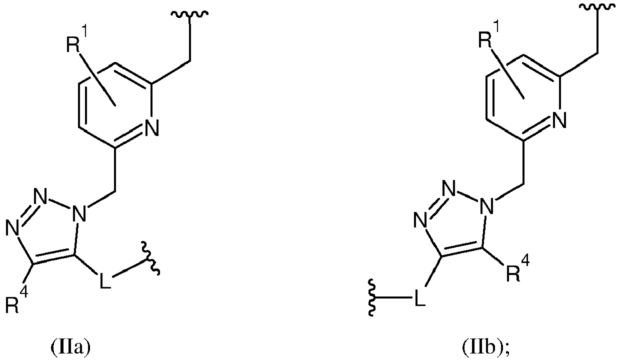 wherein
L is selected from the group consisting of 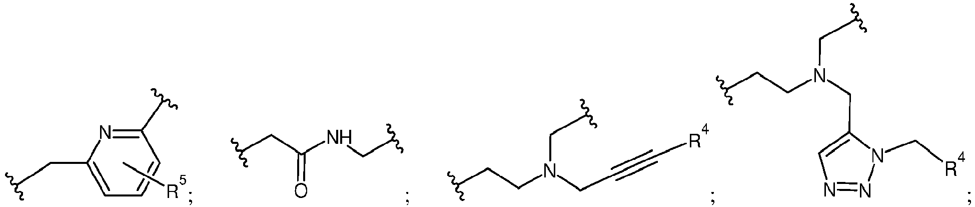 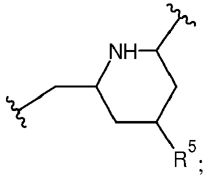
and R¹, R⁴ and R⁵ are as defined in claim 1;
preferably L is 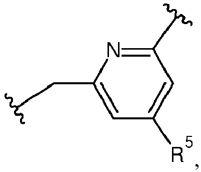 more preferably L is

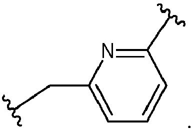

**5.** Compound of general formula (I) according to any one of the preceding claims, selected from the group comprising compounds, wherein Y is nitrogen, Z is 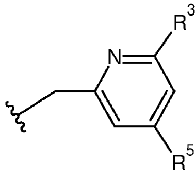 and the remaining substituents are
| | | | | | | |
|---|---|---|---|---|---|---|
| present in the following combinations: | | | | | | |
| Compou nd | R¹ | A | R² | R³ | R⁴ | R⁵ |
|---|---|---|---|---|---|---|
| TD556 | H | -(CH₂)COOH | | | H | H |
| TD810 | H | -(CH₂)COOH | | | H | 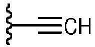 |
| TD827 | 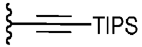 | -(CH₂)COOH | | 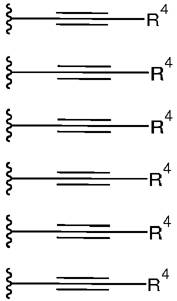 | H | H |
| TD718 | H | -(CH₂)COOH | 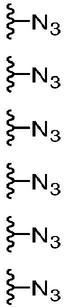 | | TMS | H |
| TD728 | H | -(CH₂)COOH | | | TIPS | H |
| TD1204 | H | -(CH₂)COOH | | | I | H |
| TD944 | H | -(CH₂)COOH | 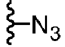 | 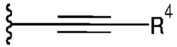 | Ph | H |
| TD943 | H | -(CH₂)COOH | 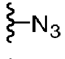 | 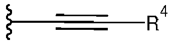 | cyclopropyl | H |
| TD959 | H | -(CH₂)COOH | 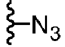 | 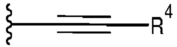 | tBu | H |
| TD992 | H | -(CH₂)COOH | 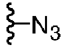 | 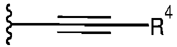 | adamantyl | H |
| TD705 | H | -CH₂P(=O)(OEt)₂ and -(CH₂)COO^{t}Bu | 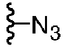 | 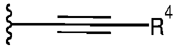 | H | H |
| TD714 | H | -CH₂P(=O)(OH)₂ and -(CH₂)COOH | 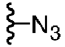 | 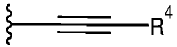 | H | H |
| TD921 | H | -CH₂P(=O)(Ph)(OH) | 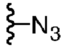 | 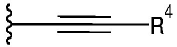 | H | H |
| TD580 | H | -CH₂P(=O)(OEt)₂ | 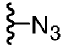 | 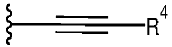 | H | H |
| TD582 | H | -CH₂P(=O)(OH)(OEt) | 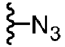 | 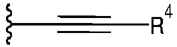 | H | H |
| TD581 | H | -CH₂P(=O)(OH)₂ | 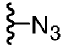 | 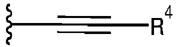 | H | H |
| TD575 | Ph | -CH₂P(=O)(OEt)₂ | 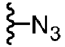 | 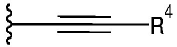 | H | H |
| TD576 | Ph | -CH₂P(=O)(OH)₂ | 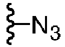 | 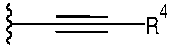 | H | H |
| TD764 | Cl | -(CH₂)COOH | 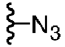 | 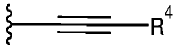 | H | H |
| TD1063 | -CF₃ | -(CH₂)COOH | 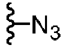 | 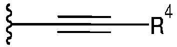 | H | H |
| TD1092 | -COOCH₃ | -(CH₂)COOH | 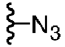 | 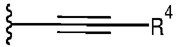 | H | H |
| TD1148 | -COOⁱPr | -(CH₂)COOH | 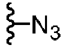 | 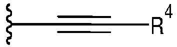 | H | H |
| TD1160 | -COO^{t}Bu | -(CH₂)COOH | 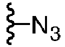 | 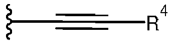 | H | H |
| TD1176 | -N(CH₃)₂ | -(CH₂)COOH | 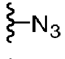 | 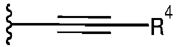 | H | H |
| TD647 | Ph | -(CH₂)COOH | 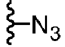 | 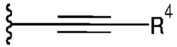 | H | H |
| TD722 | Ph | -(CH₂)COOH | 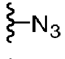 | 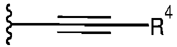 | -CH₂NH₂ | H |
| TD1054 | Ph | -(CH₂)COOH | 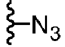 | 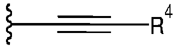 | -C(CH₃)₂NH₂ | H |
| TD1105 | H | -(CH₂)COOH | 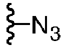 | 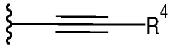 | -C(CH₃)₂NHBoc | H |
| TD1127 | H | -(CH₂)COOH | 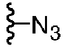 | 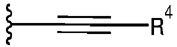 | 4-piperidinyl | H |
| TD742 | 4-carboxyphen yl | -(CH₂)COOH | | 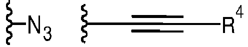 | H | H |
| TD744 | 4-carboxyphen yl | -(CH₂)COOH | | 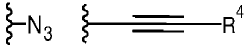 | -CH₂-NH₂ | H |
| TD779 | Ph | -(CH₂)COONH₂ | | 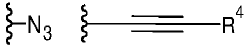 | H | H |
| TD778 | Ph | -(CH₂)CONH-CH₂COOH | | 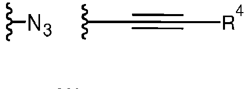 | H | H |
| cz-TD556 | H | -(CH₂)COOH | 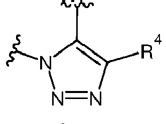 | | H | H |
| cz-TD764 | Cl | -(CH₂)COOH | 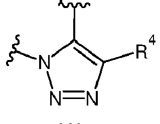 | | H | H |
| cz-TD1063 | -CF₃ | -(CH₂)COOH | 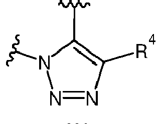 | | H | H |
| TD1188 | H | -(CH₂)COOH | 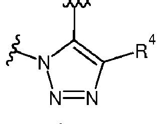 | | -CF₃ | H |
| TD650 | Ph | -(CH₂)COOH | 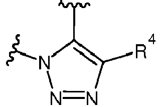 | | H | H |
| TD871 | Ph | -(CH₂)COOH | 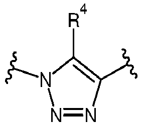 | | H | H |

**6.** A method of preparation of the compound of the general formula (I) according to any one of the claims 1 to 5, **characterized in that** it comprises the following steps:
i) providing an alkyne intermediate of general formula Z-Cl, wherein Z is as defined in claim 1;
ii) providing an azide intermediate of general formula (III) 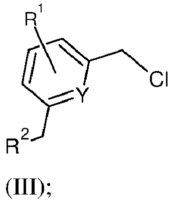 wherein Y, R¹ and R² are as defined in claim 1;
iii) providing a cyclen derivative of general formula (IV) 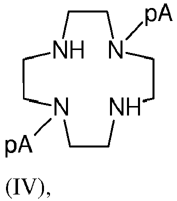 wherein pA is selected from the group comprising -(CH₂)ₙCOO^{t}Bu, wherein n is an integer from 1 to 3; benzyloxycarbonyl; -CH(CH₃)COO^{t}Bu;
- CH₂P(=O)(OR)₂, wherein R is C₁ to C₆ alkyl, which may be branched or linear;
- CH₂C(=O)(NH₂); -CH₂C(=O)(NH)-CH₂COOH; -CH₂P(=O)(OH)(Ar), wherein Ar is phenyl, which can optionally be substituted with C₁ to C₆ alkyl;
iv-A) reacting the cyclen derivative of general formula (IV) with alkyne intermediate Z-Cl to obtain an intermediate of general formula (V) 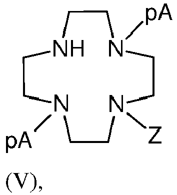 wherein Z is as defined in claim 1, and pA is defined above;
or
iv-B) reacting the cyclen derivative of general formula (IV) with the azide intermediate of general formula (III) to obtain an intermediate of general formula (VI) 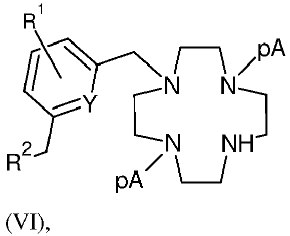 wherein Y, R¹, R² and pA are as defined above;
v-A) reacting the intermediate of general formula (V) with the azide intermediate of general formula (III) to obtain an intermediate of general formula (VII) 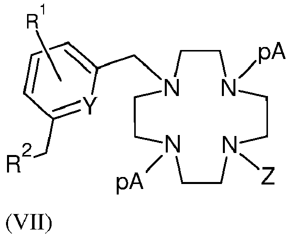 wherein Y, R¹, R², pA and Z are as defined above;
or
v-B) reacting the intermediate of general formula (VI) with the alkyne intermediate Z-Cl to obtain the intermediate of general formula (VII);
vi) optionally, hydrolysis of protecting groups, resulting in the compound of general formula (I).

**7.** A coordination compound of the compound of the general formula (I) according to any one of the claims 1 to 5 with a metal cation, selected from the group consisting of lanthanide(III) cations, Na⁺, Ba²⁺, Pb²⁺, Sr²⁺, Ca²⁺, Cd²⁺, Zn²⁺, Mn²⁺, Pt²⁺, Cu²⁺, Ni²⁺, Sc³⁺, Y³⁺, Bi³⁺, In³⁺, Ru³⁺, Ir³⁺, Ga³⁺, Tl³⁺, Pd²⁺; preferably the metal cation is selected from the group consisting of La³⁺, Ce³⁺, Pr³⁺, Nd³⁺, Sm³⁺, Eu³⁺, Gd³⁺, Tb³⁺, Dy³⁺, Ho³⁺, Er³⁺, Tm³⁺, Yb³⁺, Lu³⁺, Y³⁺, Sc³⁺, Bi³⁺, In³⁺, Tl³⁺, Pb²⁺, Ca²⁺, Cd²⁺, Zn²⁺, Cu²⁺, Ni²⁺, Mn²⁺, Pd²⁺, Na⁺.

**8.** A method of preparation of the coordination compound according to claim 7, **characterized in that** it comprises the following steps:
i) synthesis of the compound of the general formula (I) according to claim 6;
ii) providing a salt of an inorganic acid and a metal cation, selected from the group consisting of lanthanide(III) cations, Na⁺, Ba²⁺, Pb²⁺, Sr²⁺, Ca²⁺, Cd²⁺, Zn²⁺, Mn²⁺, Pt²⁺, Cu²⁺, Ni²⁺, Sc³⁺, Y³⁺, Bi³⁺, In³⁺, Ru³⁺, Ir³⁺, Ga³⁺, Tl³⁺, Pd²⁺; preferably, the salt is selected from a group comprising chloride, nitrate, acetate, formate, trifluoroacetate, trifluoromethylsulfonate;
iii) mixing the compound of the general formula (I) from step i) with the metal salt from step ii) in aqueous solution, preferably for at least 1 hour at temperature in the range of from 25 to 100 °C, resulting in chelation of the metal cation by the compound of general formula (I) and formation of the coordination compound according to claim 7;
iv) optionally, transformation of the coordination compound from step iii), wherein the transformation is selected from at least one of the following reactions:
- substitution reaction of halogen group present in R¹ or R⁴, with a phenylboronic acid, thus transforming the halogen substituent into -COOH;
- substitution reaction of halogen group present in R¹ or R⁴, with -N₃ group by reaction with NaN₃;
- hydrolysis of TIPS protecting group present in R¹ or R⁴ substituent, wherein R¹ or R⁴ is 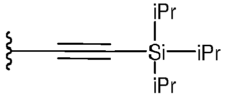 , resulting in R¹ or R⁴ being 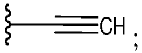
- addition reaction of methanol to R¹, wherein R¹ is 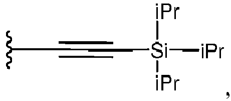 thus transforming the triple bond to dimethyl acetal;
- hydrolysis of triisopropylsilyl group from R¹, wherein R¹ is 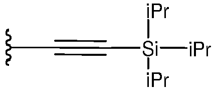 , thus transforming R¹ into 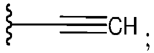
- deuteration of CH₂ groups of the pendant arms in D₂O in presence of DBU, transforming them into CD₂ groups;
- selective reduction of pyridyl cycle of Z substituent, wherein Z is 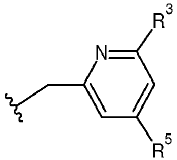 and R³ and R⁵ are as defined in claim 1, using NaBH₄ or NaBD₄ as the reducing agent, thus transforming the Z group into 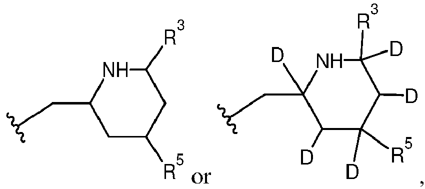 respectively;
- reaction of NH₂ group of R¹ or R⁴, wherein R¹ or R⁴ is -CH₂NH₂, with FmocCl, resulting in transformation of -CH₂NH₂ group into -CH₂NHFmoc group;
- reaction of -COOH group present in R¹ or R⁴, with an amino group of an aminoacid or peptide, thus forming a peptide bond;
- S-alkylation reaction of a halogen, preferably Cl, of R¹ or R⁴ substituent with a thiol, resulting in trasforming the halogen into sulfide;
- reaction of 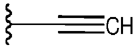 group of R¹ or R³, wherein R¹ or R³ contains 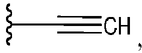 with an azide, preferably with alkyl- or arylazide, thereby forming a triazole bridge connecting the alkyl- or aryl- to R¹ or R³ group.
- reaction of -N₃ group of R¹, wherein R¹ contains -N₃ group, with a substituent comprising a triple bond, thereby forming a triazole bridge connecting the substituent with the coordination compound.

**9.** A coordination compound chain containing at least two coordination compounds according to claim 7, bound via a triazole bridge formed between R¹ or R⁵ group of the first coordination compound and R¹ or R⁵ group of the following coordination compound.

**10.** A coordination compound dimer of general formula (X) 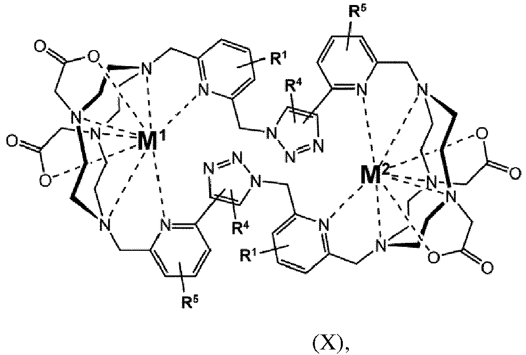 wherein M¹ and M² are metal cations as defined in claim 7, and R¹, R⁴ and R⁵ are as defined in claim 1.

**11.** A conjugate for drug tracing, which contains the coordination compound according to claim 7 or the coordination compound chain according to claim 9, conjugated to a peptide or to a protein;
wherein the coordination compound according to claim 7 or the coordination compound chain according to claim 9 contain R¹ group selected from -NH₂; -COOH; C₆ to C₁₀ aryl, substituted with -NH₂, -COOH, or -CH₂COOH;
which is conjugated to the peptide or the protein via an amide bond formed between -COOH group present in R¹ and amino group of the peptide or the protein or via an amide bond formed between -NH₂ group present in R¹ and carboxyl group of the peptide or the protein;
and wherein the peptide is selected from the group comprising oligopeptides of 3 to 20 aminoacids;
and wherein the protein is selected from the group comprising antibodies, preferably monoclonal antibodies.

**12.** A method of drug tracing, **characterized in that** it comprises the following steps:
i) providing a cell culture or a tissue to be analyzed, containing at least one conjugate according to claim 11, wherein the peptide or the protein is of the peptide-based or protein-based drug to be traced;
ii) hydrolyzing the cell culture or tissue from step i) using a strong acid, preferably HCl, obtaining a hydrolyzate;
iii) qualitative and/or quantitative analysis of the hydrolyzate of step ii) for the presence of the coordination compound of claim 7 or of the coordination compound chain of claim 9, preferably using LC-MS.

**12.** Use of the coordination compound according to claim 7 or of the coordination compound chain according to claim 9 or of the coordination compound dimer according to claim 10 or of the conjugate according to claim 11 in pharmacy, preferably for development and testing of new drugs, more preferably for drug marking and tracing.

**13.** The coordination compound according to claim 7 or the coordination compound chain according to claim 9 or the coordination compound dimer according to claim 10 for use in medical diagnostics, preferably as MRI contrast agents.

**14.** The coordination compound according to claim 7 or the coordination compound chain according to claim 9 or the coordination compound dimer according to claim 10, wherein M is selected from the group comprising ⁴⁴Sc, ⁴⁷Sc, ⁶⁴Cu, ⁶⁷Cu, ⁸⁶Y, ⁹⁰Y, ¹⁴⁰Nd, ¹⁴⁹Pm, ¹⁵¹Pm, ¹⁵³Sm, ¹⁵⁹Gd, ¹⁴⁹Tb, ¹⁶¹Tb, ¹⁶⁵Dy, ¹⁶¹Ho, ¹⁶⁶Ho, ¹⁶⁹Er, ¹⁶⁷Tm, ¹⁷⁵Yb, ¹⁷⁷Lu, for use in medicine as radiodiagnostic and/or radiopharmaceutic agents.
